# EUROPEAN PATENT APPLICATION

(11) **EP 4 477 759 A1**
(43) Date of publication of application: **18.12.2024**
(21) Application number: 23753159.5
(22) Date of filing: 08.02.2023
(51) Int. Cl.: C12Q 1/6853, C12Q 1/6869, C12N 15/10, C12N 9/22, C12N 15/113, C12Q 1/6811, C12Q 1/6876, C12N 15/62

(54) **METHOD FOR PREDICTING OFF-TARGET WHICH CAN OCCUR IN PROCESS OF EDITING GENOME BY USING PRIME EDITING SYSTEM**

(30) Priority: 08.02.2022 KR 20220016521; 28.11.2022 KR 20220161819
(71) Applicant: Toolgen Incorporated, Seoul 07789 (KR)
(72) Inventor: LEE, Jeongjoon, Seongnam-si, Gyeonggi-do 13532 (KR); KWON, Jeonghun, Seoul 08279 (KR); KIM, Minyoung, Seoul 04129 (KR); JO, Anna, Anyang-si, Gyeonggi-do 14075 (KR); KIM, Young Ho, Yongin-si, Gyeonggi-do 16839 (KR)
(74) Representative: dompatent von Kreisler Selting Werner - Partnerschaft von Patent- und Rechtsanwälten mbB
(86) International application number: PCT/KR2023/001867
(87) International publication number: WO 2023/153811

(57) **Abstract**

The present application relates to a method for predicting an off-target which can occur in the process of editing a genome by using a prime editing system.

## Description

### Technical Field

The present application relates to a method for predicting an off-target in a prime editing system, one type of gene editing system.

### Background Art

Genome editing by CRISPR/Cas systems is a field of active research. Although various studies have been conducted on modified guide RNAs and the like and Cas proteins have been developed for genetic manipulation, gene editing methods by CRISPR/Cas systems still have problems. Many problems resulting from genetic manipulation methods by CRISPR/Cas systems have motivated the development of more sophisticated genome editing technologies. Such motivation led to the development of base editing, a more sophisticated genome editing technology. However, base editing still has a limited range of applications.

David R. Liu et al. developed prime editing technology, a "search-and-replace" genome editing technology usable in inducing insertions, deletions, all 12 base-to-base conversions, and combinations thereof to the genome.

Although a new platform for genome editing referred to as "prime editing" was developed by David R. Liu et al., methods for predicting off-targets that may occur in genome editing by prime editing have not yet been developed. Due to the development of prime editing, a new platform for genome editing, there is a need to develop new methods for predicting off-targets that are more suitable for prime editing systems.

### Disclosure

### Technical Problem

Off-targets occurring in gene editing processes cause strong side effects. Accordingly, various methods for predicting off-targets have been developed. However, methods known to date are developed to target traditional CRISPR/Cas systems and thus are challenging to apply to new gene editing systems, such as prime editing systems. Hence, the present application discloses a method or system for predicting an off-target in a prime editing system, the method or system developed to target the prime editing system.

### Technical Solution

Some embodiments of the present application provide a method for predicting off-target occurring in process of genome editing by prime editing system, comprising:
(a) obtaining a manipulated cell,
   wherein the manipulated cell comprises a manipulated genome DNA, wherein the manipulated genome DNA comprises a tag sequence, and the manipulated genome DNA is generated through a following process which a prime editor protein and tpegRNA are involved, comprising:
   (i) contacting the genome DNA with a prime editor protein and a tpegRNA (tagmentation pegRNA), wherein the prime editor comprises a Cas protein and a reverse transcriptase, and wherein the tpegRNA comprises a spacer and an extension region comprising a tag template,
   (ii) the tag sequence is inserted into the genome DNA by reverse transcription process performed by the reverse transcriptase using the tag template of the tpegRNA as a template of reverse transcription;
(b) analyzing the manipulated genome DNA to obtain information on a tagmentation,
   wherein the information on the tagmentation comprises information on a region of genome DNA of which the tag sequence is inserted.

In certain embodiments, the method for predicting off-target may further comprises:
obtaining information of the off-target based on the information on the tagmentation, wherein the information of the off-target comprises information on whether an off-target candidate exists, and information on the region of the off-target candidate if the off-target candidate exists.

In certain embodiments, the method for predicting off-target may further comprises:
verifying information on a on-target, and comparing the information on the on-target with the the information on the tagmentation.

In certain embodiments, the method for predicting off-target may further comprises:
confirming information on whether a off-target candidate exists by confirming an on-target information and comparing the on-target information with the information on the tagmentation.

In certain embodiments, the tag sequence may be inserted into a region in the genome DNA which is specified by the spacer of tpegRNA.

In certain embodiments, the region where the tag sequence is inserted may be associated with (corresponds to) off-target candidate regions or on-target regions.

In certain embodiments, information on the region where the tag sequence is inserted may comprise information on a chromosome where the tag sequence is located, and information on a region in the chromosome where the tag sequence is present.

In certain embodiments, the information on the region of the candidate of the off-target may comprise information on a chromosome where the off-target candidate is located, and the region in the chromosome where the off-target candidate is present.

In certain embodiments, the information on the tagmentation may further comprise: insertion ratio of the tag sequence by a region where the tag sequence is inserted.

In certain embodiments, the information on the off-target may further comprise: off-target predicting score of the off-target candidate.

In certain embodiments, the information on the off-target may further comprise: a number of the off-target candidates predicted.

In certain embodiments, the manipulated cell may be obtained by a method comprises: contacting the prime editor protein or a nucleic acid encoding the prime editor protein and the tpegRNA or a nucleic acid encoding the tpegRNA with a cell.

In certain embodiments, the manipulated cell may be obtained by a method comprises: introducing the prime editor protein or a nucleic acid encoding the prime editor protein and the tpegRNA or a nucleic acid encoding the tpegRNA into a cell.

In certain embodiments, the method may further comprise: obtaining a DNA from the manipulated cell, wherein it is performed prior to (b).

In certain embodiments, the tpegRNA may comprise:
spacer; gRNA core; and an extension region comprising a primer binding site, a tag template, and a reverse transcription template.

In certain embodiments, the reverse transcription template of the tpegRNA may comprise an editing template and homology region.

In certain embodiments, the manipulated genome DNA may comprise an editing.

In certain embodiments, the spacer, the gRNA core, and the extension region may be located in the order of the spacer, the gRNA core, and the extension region in the 5' to 3' direction.

In certain embodiments, the tag template may be located in between primer binding site and the reverse transcription template in the extension region.

In certain embodiments, the tpegRNA may further comprise 3' engineering region comprising RNA protection motif.

In certain embodiments, the method for predicting off-target may further comprise:
verifying a predetermined prime editing system, comprising one or more of follows:
information on a predetermined cell, information on a predetermined pegRNA, information on a predetermined prime editor protein.

In certain embodiments, the predetermined cell may be different from the cell used in the method for predicting off-target.

In certain embodiments, a sequence of the spacer of the tpegRNA may be the same as a sequence of the spacer of the predetermined pegRNA, and a sequence of the primer binding site of the tpegRNA may be the same as a sequence of the primer binding site of the predetermined pegRNA.

In certain embodiments, a sequence of the spacer of the tpegRNA may be the same as a sequence of the spacer of the predetermined pegRNA, a sequence of the primer binding site of the tpegRNA may be the same as a sequence of the primer binding site of the predetermined pegRNA,and a sequence of the reverse transcription template of the tpegRNA may be the same as a sequence of the reverse transcription template of the predetermined pegRNA.

In certain embodiments, the prime editor protein used in the method for predicting off-target may be the same or different to the predetermined prime editor protein.

In certain embodiments, the length of the tag template may be 5nt to 60nt.

In certain embodiments, the length of the tag template may be 10nt to 50nt.

In certain embodiments, the prime editor protein may be a PE-nuclease comprising a Cas protein which have double-strand break activity.

In certain embodiments, the prime editor protein may be a PEmax-nuclease.

In certain embodiments, the Cas protein which included in the prime editor protein may be nickase.

In certain embodiments, the prime editor protein may be a PE2 prime editor protein.

In certain embodiments, the manipulation of DNA genome may further involve any one or more of a dnMLH1, a gRNA, and an additional Cas protein, and an additional prime editor protein.

In certain embodiments, wherein the (b) may comprise: analyzing the manipulated genome DNA tag-specifically.

In certain embodiments, the (b) may comprise: sequencing the manipulated genome DNA.

In certain embodiments, the (b) comprises:
generating a tag-specific library from the manipulated genome DNA; generating an amplified tag-specific library by amplifying tag-specific library; and sequencing the amplified tag-specific library.

Some embodiments of the present application provide a method for predicting off-target occurring in process of genome editing by prime editing system, comprising:
(a) preparing a population of cells comprising one or more manipulated cells,
   wherein the manipulated cell comprises a manipulated genome DNA, wherein the manipulated genome DNA comprises a tag sequence, and the manipulated genome DNA is generated through a following process which a prime editor protein and tpegRNA are involved, comprising:
   (i) contacting the genome DNA with a prime editor protein and a tpegRNA (tagmentation pegRNA), wherein the prime editor protein comprises a Cas protein and a reverse transcriptase, and , wherein the tpegRNA comprises a spacer and an extension region comprising a tag template,
   (ii) the tag sequence is inserted into the genome DNA, wherein the insertion of the tag sequence is achieved through a reverse transcription process performed by the reverse transcriptase using the tag template of the tpegRNA as a template of reverse transcription;
(b) obtaining a tagmentation information by analyzing results obtained through a process comprising a sequencing the manipulated genome DNA of one or more manipulated cells,
   wherein the tagmentation information comprises information on one or more sites where each tag sequence is inserted; and
(c) obtaining information on the off-target based on the tagmentation information,
   wherein the information on the off-target comprises information on whether a off-target candidate exists, and information on the region of one or more off-target candidates.

Some embodiments of the present application provide a tagmentation pegRNA (tpegRNA) comprising:
a spacer; a gRNA core; and an extension region compring a tag template.

In certain embodiments, the spacer, the gRNA core, and the extension region comprising the tag template may be located in the order of the spacer, the gRNA core, and the extension region comprising the tag template in the 5' to 3' direction.

In certain embodiments, the extension region may comprise the tag template, a primer binding site, and a reverse transcription template.

In certain embodiments, the tag template may be located in between primer binding site and the reverse transcription template in the extension region.

In certain embodiments, the reverse transcription template may be located in between the tag template and the primer binding site.

In certain embodiments, the primer binding site, the tag template and the reverse transcription template may be located in the order of the reverse transcription template, the tag template, and the primer binding site in the 5' to 3' direction in the extension region.

In certain embodiments, the reverse transcription template may comprise an editing template and a homology region.

In certain embodiments, the tag template may have a length of 5nt to 60nt.

In certain embodiments, the tag template may have a length of 10nt to 50nt.

In certain embodiments, the tpegRNA may further comprise 3' engineering region comprising an RNA protection motif.

In certain embodiments, the RNA protection motif may have a length of 10nt to 60nt.

In certain embodiments, the tpegRNA may have a length of 100nt to 350nt.

Some embodiments of the present application provide a composition for predicting off-target which are capable of occuring in process of genome editing by prime editing system, comprising:
a tpegRNA; and
a prime editor comprising a Cas protein and a reverse transcriptase.

### Advantageous Effects

A method for predicting an off-target in a prime editing system, according to some embodiments of the present application, uses a molecular mechanism of the prime editing system and thus has numerous advantages in predicting the off-target in the prime editing system compared to other known off-target prediction methods.

### Description of Drawings

FIG. 1 illustrates structural examples of traditional guide RNA (gRNA), prime editing guide RNA (pegRNA), and tagmentation pegRNA (tpegRNA);
FIG. 2 relates to an exemplary embodiment of a tpegRNA, wherein the tpegRNA illustrated in FIG. 2 includes an extension region including a DNA synthesis template, a tag template, and a primer binding site (PBS);
FIG. 3 relates to an exemplary embodiment of a tpegRNA, wherein the tpegRNA illustrated in FIG. 3 includes an extension region including a primer binding site, a tag template, an editing template, and a homology region;
FIG. 4 relates to a tag insertion mechanism using a tpegRNA of an off-target prediction system of the present application, specifically illustrating an example of a DNA molecule where a nick is made at an on-target or off-target candidate site and a prime editor protein/tpegRNA complex that induces a nick;
FIG. 5 relates to a tag insertion mechanism using a tpegRNA of an off-target prediction system of the present application, specifically illustrating a region where a primer binding site of the tpegRNA functions as a primer for genome DNA and an annealing site, followed by performing reverse transcription by a reverse transcriptase (RT) using a tag template and the like as a template;
FIG. 6 relates to a tag insertion mechanism using a tpegRNA of an off-target prediction system of the present application, illustrating that a tag sequence and the like were added to an endogenous DNA strand (3' DNA flap) by performing reverse transcription, followed by installing the tag sequence and complementary sequences thereto at an on-target site or off-target candidate site of genome DNA through a process including removing the 5' DNA flap and DNA repair;
FIG. 7 illustrates an exemplary process of TAgmentation of Prime Editor sequencing (TAPE-seq) that is an off-target prediction system of the present application;
FIG. 8 shows results for the insertion rate of a tag sequence by incubation periods;
FIG. 9 shows a map of a green fluorescent protein (GFP)-piggyBac vector;
FIGS. 10 to 15 show enrichment results of GFP-positive cells, specifically wherein FIGS. 10 to 11 show results for HEK293T, FIGS. 12 to 13 show results for HeLa, and FIGS. 14 to 15 show results for K562;
FIG. 16 shows the number of candidate off-target regions found by TAPE-seq, by incubation periods after transfecting HEK294T cells with HEK4 (+2 G to T) pegRNA;

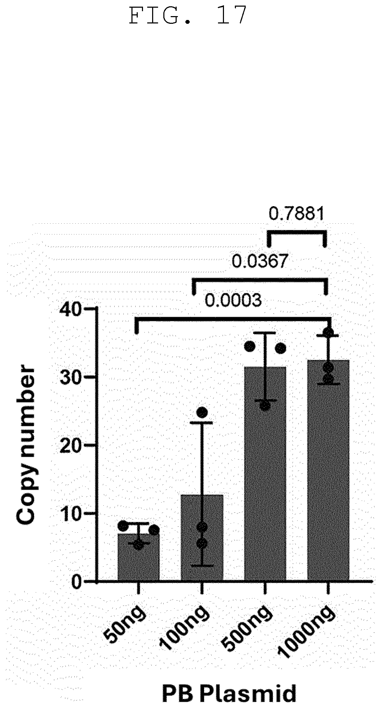

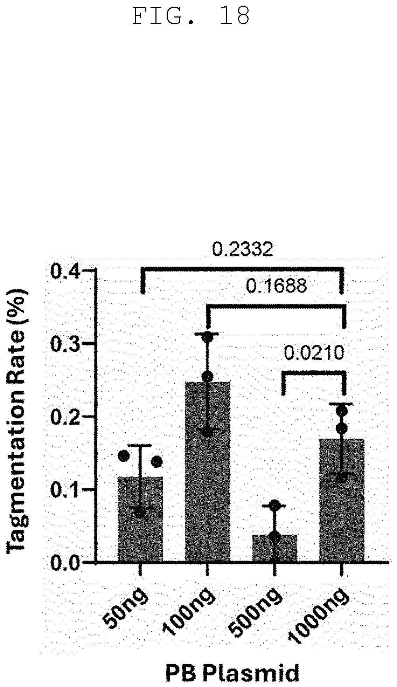

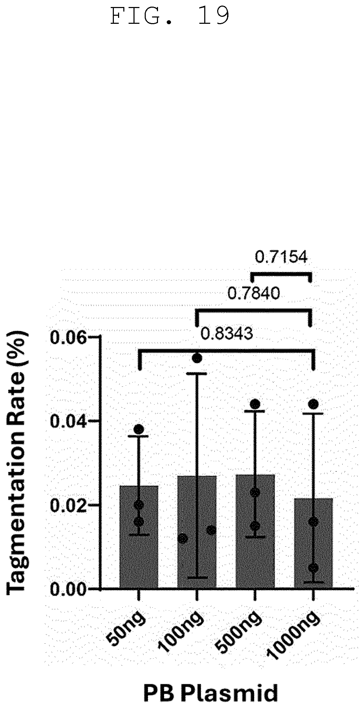

FIGS. 17 to 19 show experimental results to find an amount of a piggyBac vector optimal for co-transfection with a transposase plasmid, specifically wherein FIG. 17 is a graph showing the copy number of the piggyBac constructs found in cells by quantitative polymerase chain reaction (PCR), by the amount of a piggyBac plasmid (PB plasmid), FIG. 18 is a graph showing tagmentation rates at an on-target site by the amount (ng) of the piggyBac plasmid used for HEK293T transfection, and FIG. 19 is a graph showing tagmentation rates at Off-target site 1 by the amount (ng) of the piggyBac plasmid used for HEK293T transfection;
FIG. 20 shows analysis results for tagmentation rates by probe sequence length, wherein the tagmentation rates at on-target sites were analyzed;
FIG. 21 shows analysis results for tagmentation rates by probe sequence length, wherein the tagmentation rates at off-target sites were analyzed;
FIG. 22 shows analysis results for prime editing rates and tagmentation rates at on-target sites for nine different pegRNAs;
FIG. 23 shows analysis results for tagmentation rates at six target sites of *HEK4* (+2 G to T) pegRNA and *HBB (+4* A to T) pegRNA;
FIG. 24 shows editing ratios in Case 1 to Case 2 determined by targeted deep sequencing using a PE-analyzer, wherein nine different pegRNAs were analyzed;
FIG. 25 shows examination results for tagmentation with and without prime editing at ten different on-target and off-target sites;

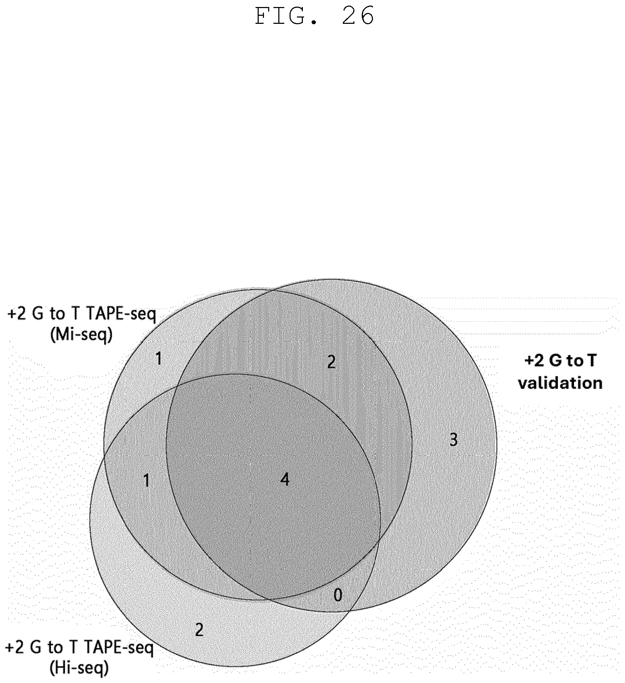

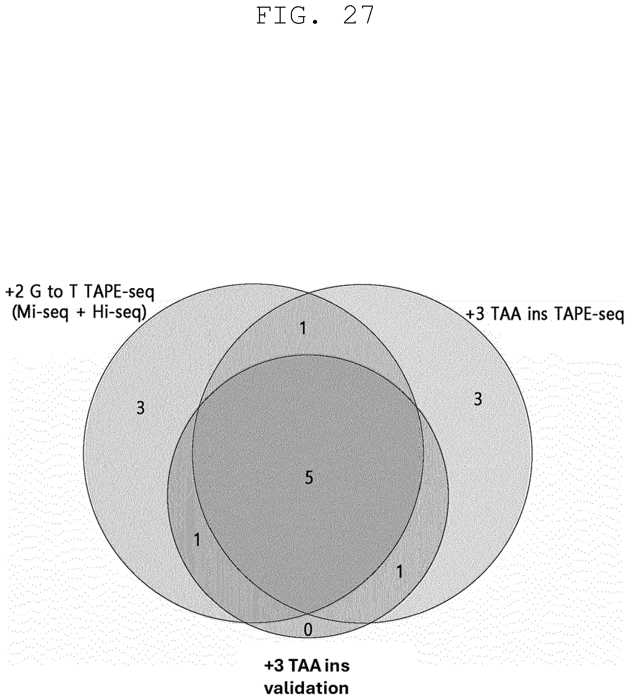

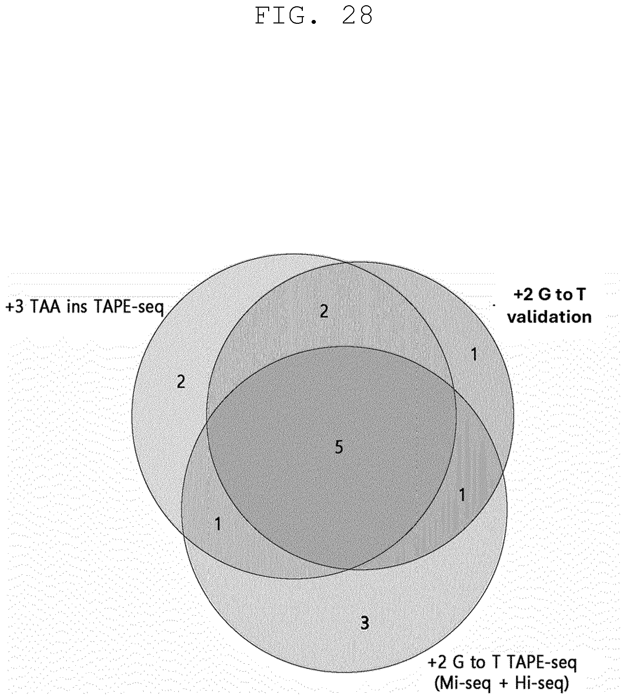

FIGS. 26 to 28 show comparison results for validated regions with off-target sites of HEK4 pegRNA predicted by TAPE-seq, wherein FIG. 26 shows comparison results for validated regions with off-target sites of *HEK4* (+2 G to T) pegRNA predicted by TAPE-seq, FIG. 27 shows comparison results for validated sites of *HEK4* (+3 TAA ins), combination of off-target sites of *HEK4* (+2 G to T) predicted by TAPE-seq using Mi-seq and off-target sites of *HEK4* (+2 G to T) predicted by TAPE-seq using Hi-seq, and off-target sites of *HEK4* (+3 TAA ins) predicted by TAPE-seq (Mi-seq), and FIG. 28 shows comparison results for validated sites of *HEK4* (+2 G to T), combination of off-target sites of *HEK4* (+2 G to T) predicted by TAPE-seq using Mi-seq and off-target sites of *HEK4* (+2 G to T) predicted by TAPE-seq using Hi-seq, and off-target sites of *HEK4* (+3 TAA ins) predicted by TAPE-seq (Mi-seq);

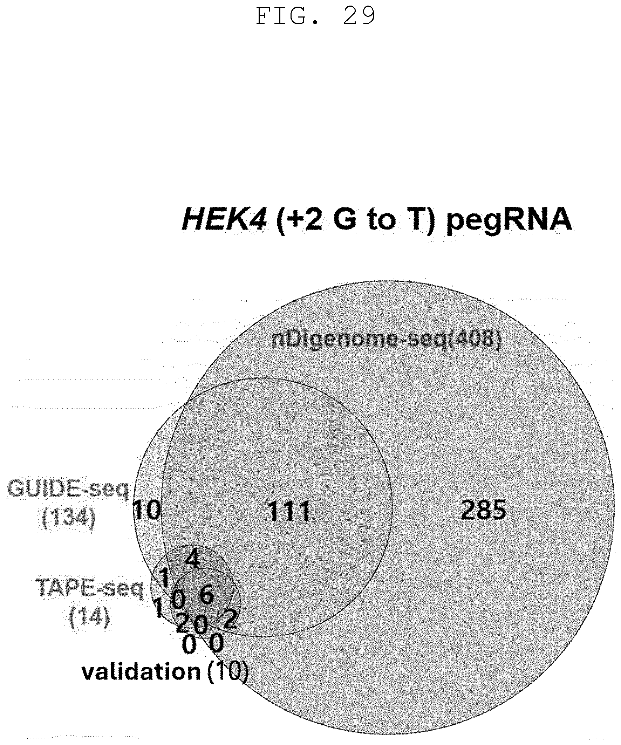

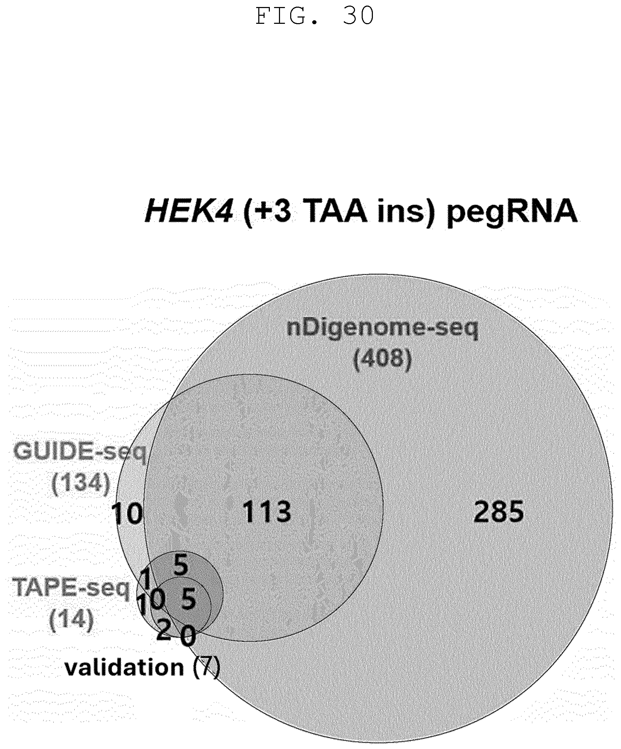

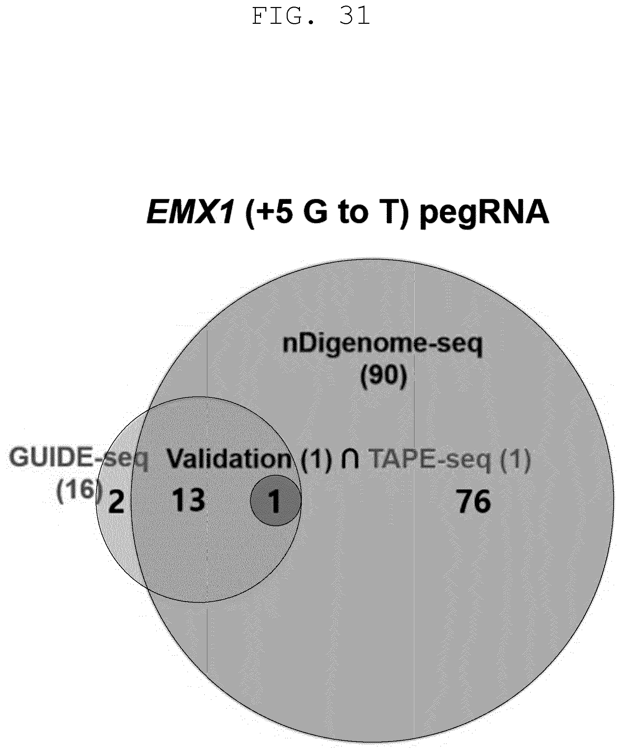

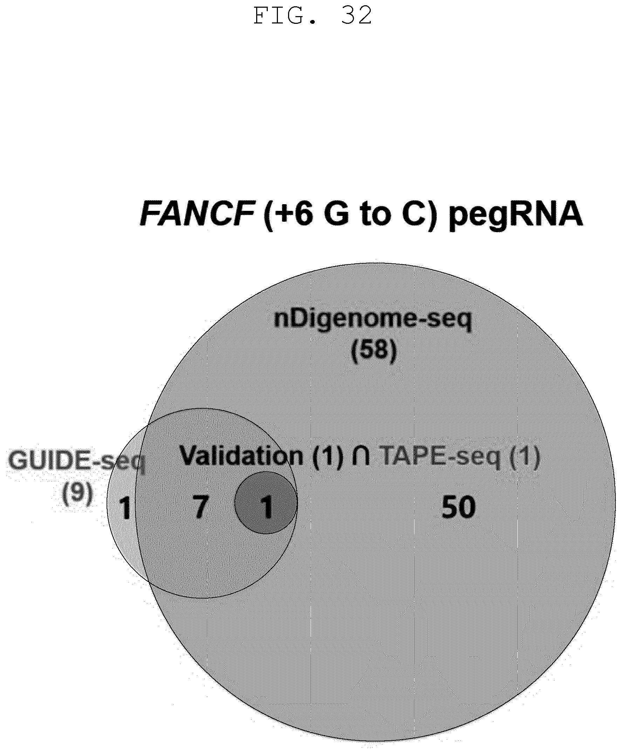

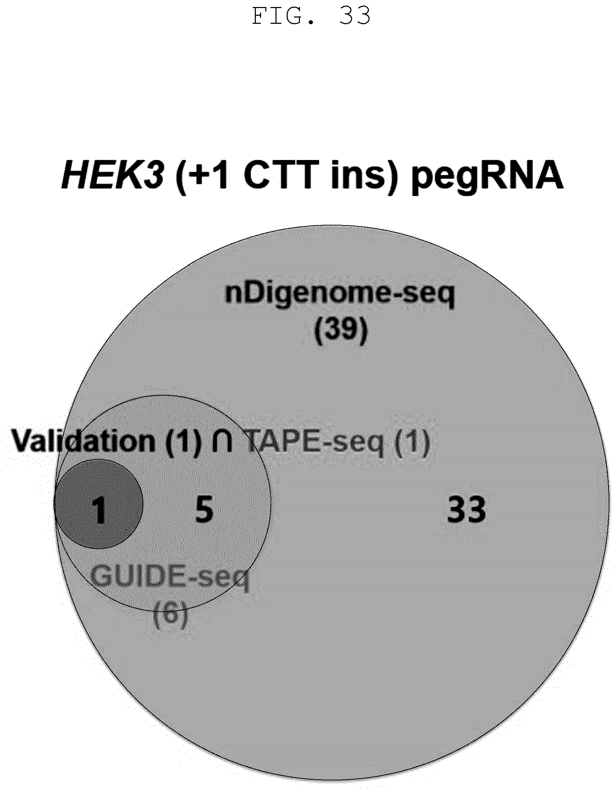

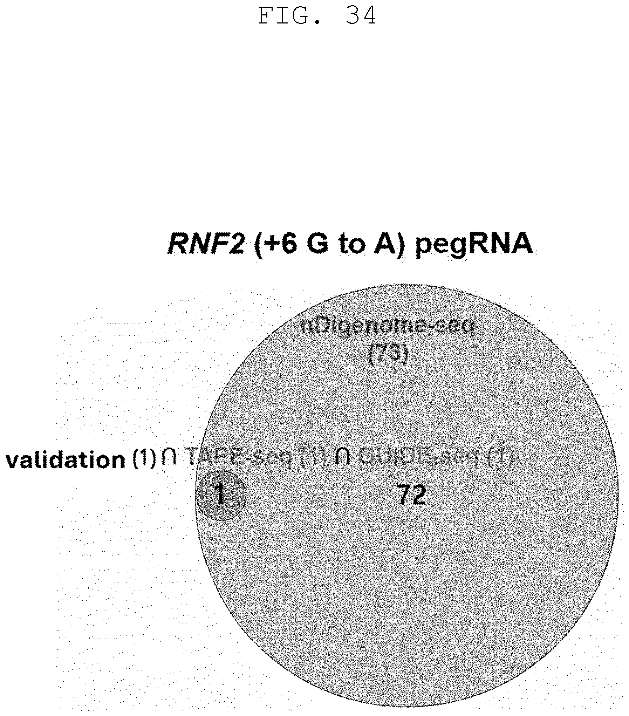

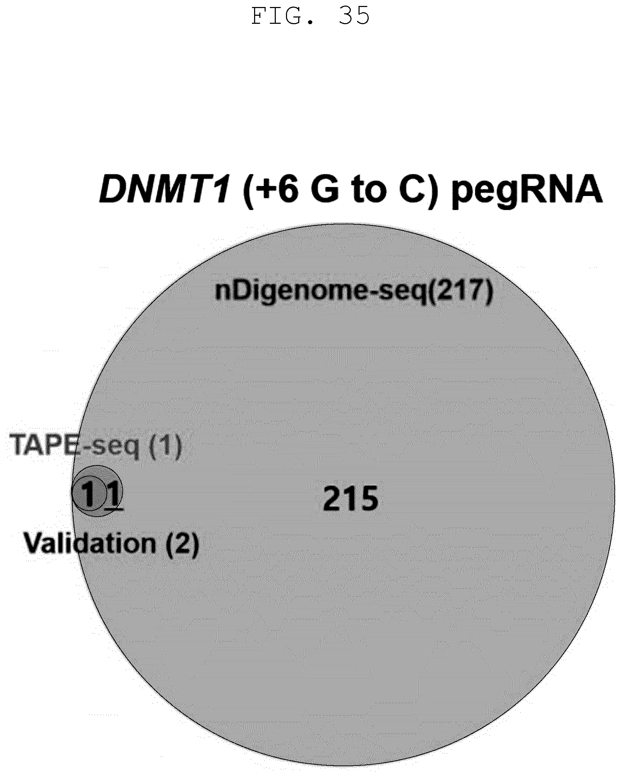

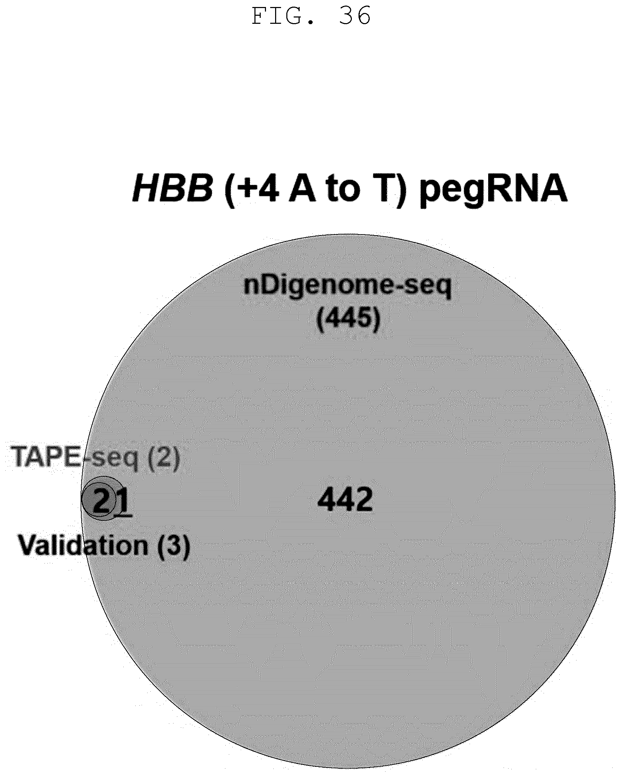

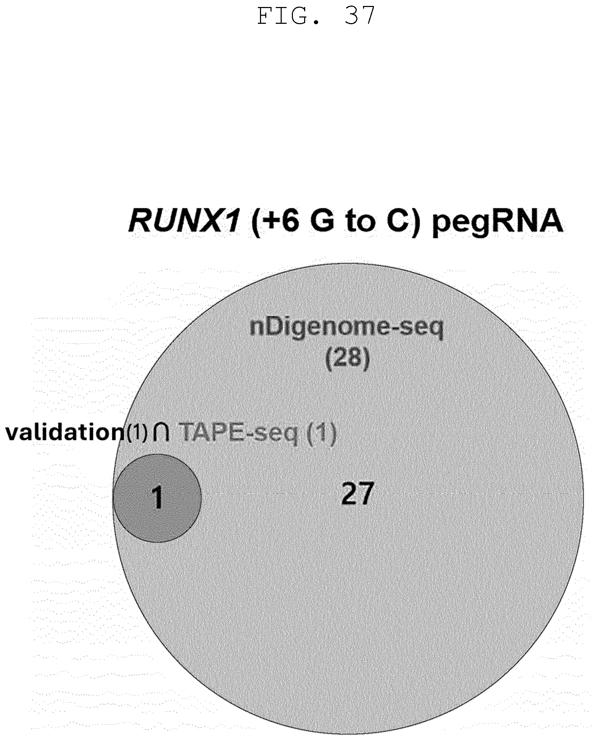

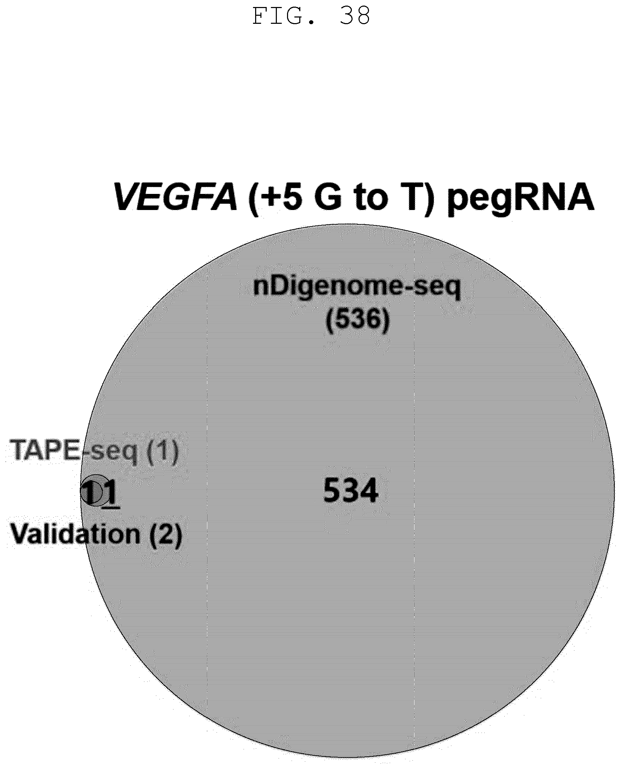

FIGS. 29 to 38 relate to a comparison of results predicted by TAPE-seq with results predicted by other off-target prediction methods, wherein FIG. 29 shows results for *HEK4* (+2 G to T) pegRNA, FIG. 30 shows results for *HEK4* (+3 TAA ins) pegRNA, FIG. 31 shows results for *EMX1* (+5 G to T) pegRNA, FIG. 32 shows results for *FANCF* (+6 G to C) pegRNA, FIG. 33 shows results for *HEK3* (+1 CTT ins) pegRNA, FIG. 34 shows results for *RNF2* (+6 G to A) pegRNA, FIG. 35 shows results for *DNMT1* (+6 G to C) pegRNA, FIG. 36 shows results for *HBB* (+4 A to T) pegRNA, FIG. 37 shows results for *RUNX1* (+6 G to C) pegRNA, and FIG. 38 shows results for *VEGFA* (+5 G to T) pegRNA;
FIG. 39 shows analysis results for validated off-targets, related to the results in FIGS. 29 to 38, missed by each prediction method;
FIG. 40 shows analysis results for tagmentation rates of PE2 TAPE-seq and PE4 TAPE-seq;
FIGS. 41 to 43 show comparison results for off-targets predicted by PE2 TAPE-seq, off-targets predicted by PE4 TAPE-seq, and bona-fide off-targets, the bona-fide off-targets validated by targeted deep sequencing, wherein FIG. 41 shows results related to HEK293T, FIG. 42 shows results related to HeLa, and FIG. 43 shows results related to K562;
FIG. 44 shows analysis results for a summary of the number of target sites missed regarding FIGS. 41 to 43, wherein FIG. 44A shows the analysis results by each prediction method, and FIG. 44B shows the analysis results by each cell;
FIGS. 45 to 47 compare TAPE-seq off-target prediction results with validation results by cells, wherein FIG. 45 compares validation results in HEK293T with TAPE-seq prediction results in each cell, FIG. 46 compares validation results in HeLa cells with the TAPE-seq prediction results in each cell, and FIG. 47 compares validation results in K562 cells with the TAPE-seq prediction results in each cell;
FIG. 48 shows analysis results for the number of validated off-targets missed by TAPE-seq prediction results in each cell;
FIG. 49 shows analysis results for tagmentation rates of TAPE-seq using PE2, PE2-nuclease, and PEmax-nuclease used with an epegRNA;

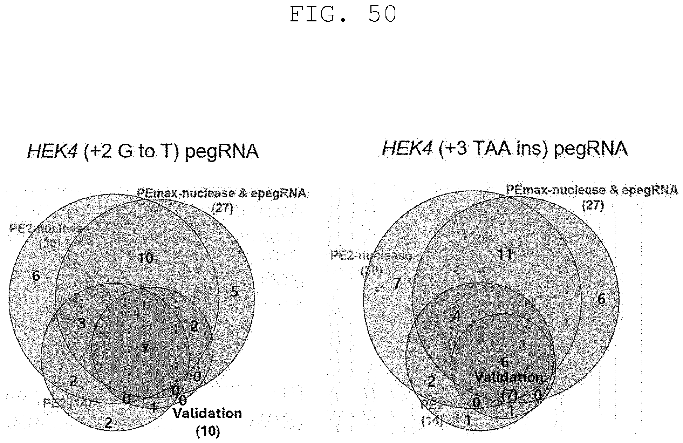

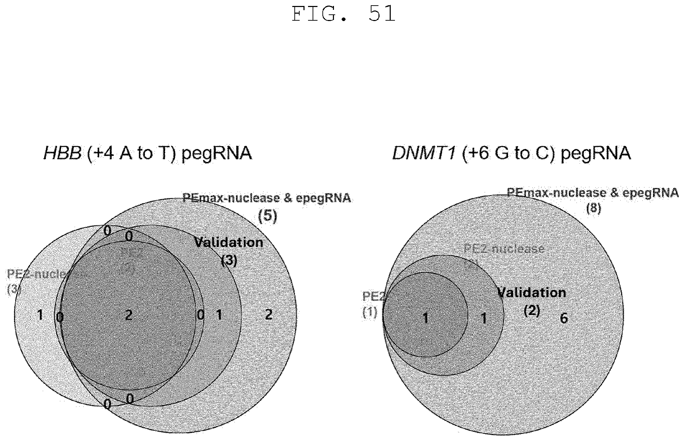

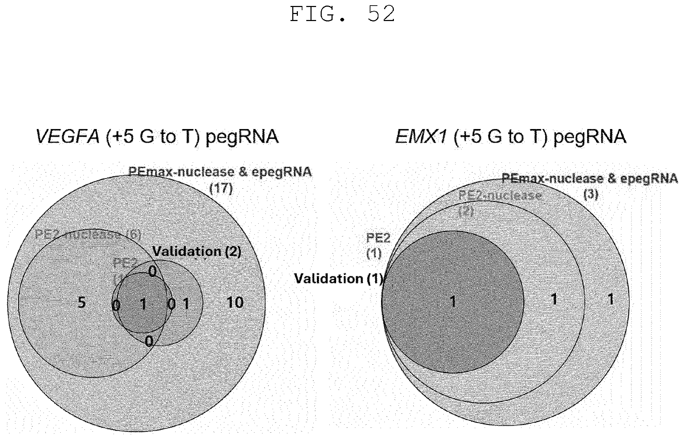

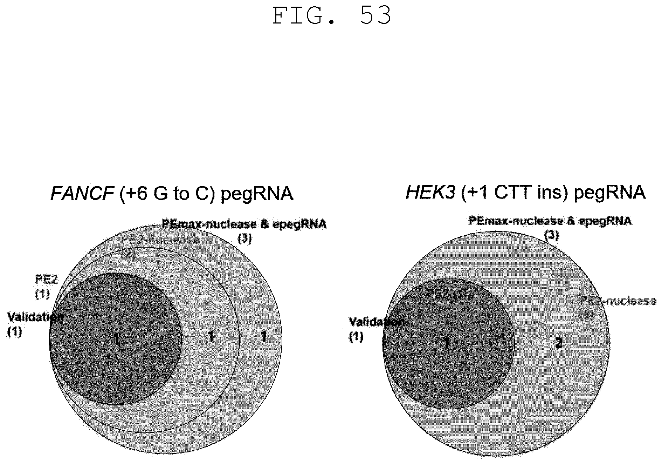

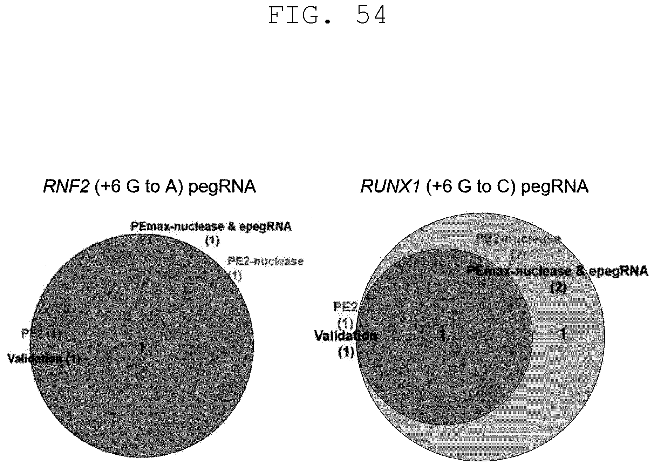

FIGS. 50 to 54 show comparison results for off-targets predicted by each TAPE-seq method (PE2 TAPE-seq, PE2-nuclease TAPE-seq, and TAPE-seq using PEmax-nuclease with epegRNAs) with validated off-target regions, wherein FIG. 50 shows results for *HEK4* (+2 G to T) pegRNA (or epegRNA) and *HEK4* (+3 TAA ins) pegRNA, FIG. 51 shows results for *HBB* (+4 A to T) pegRNA and *DNMT1* (+6 G to C) pegRNA, FIG. 52 shows results for *VEGFA* (+5 G to T) pegRNA and *EMX1* (+5 G to T) pegRNA, FIG. 53 shows results for *FANCF* (+6 G to C) pegRNA and *HEK3* (+1 CTT ins) pegRNA, and FIG. 54 shows results for *RNF2* (+6 G to A) pegRNA and *RUNX1* (+6 G to C) pegRNA;

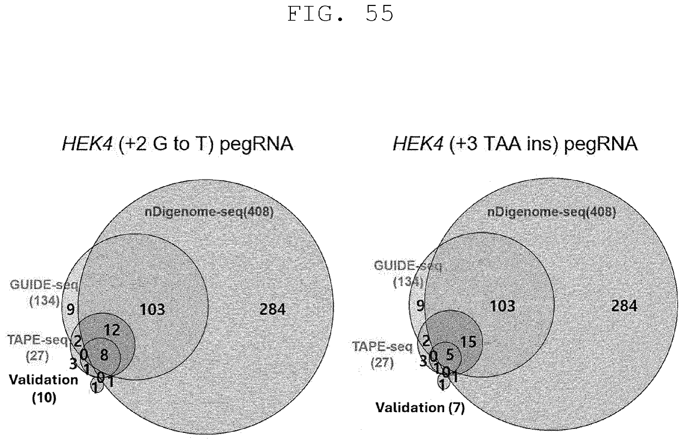

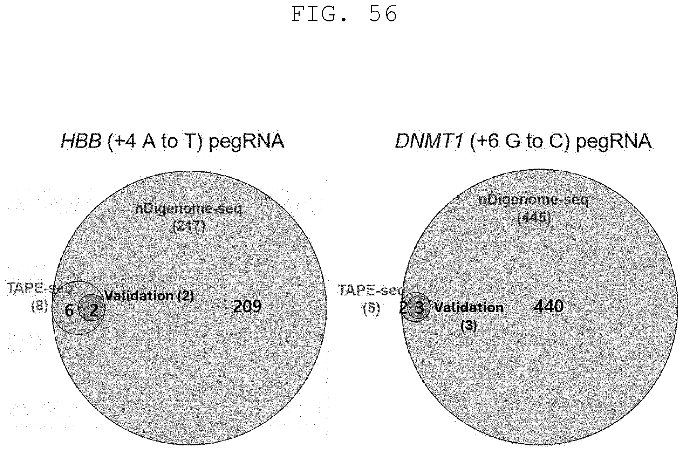

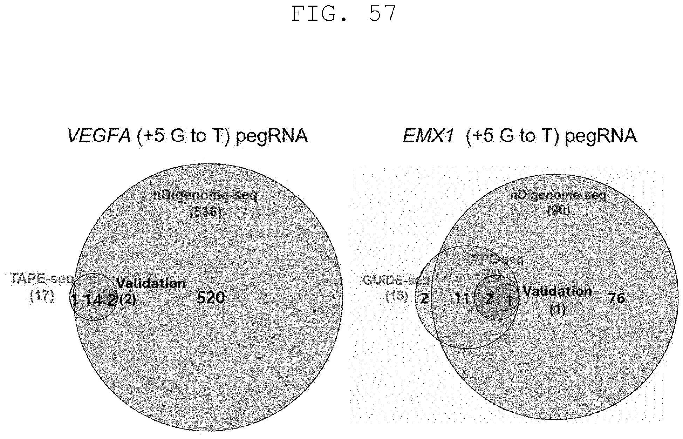

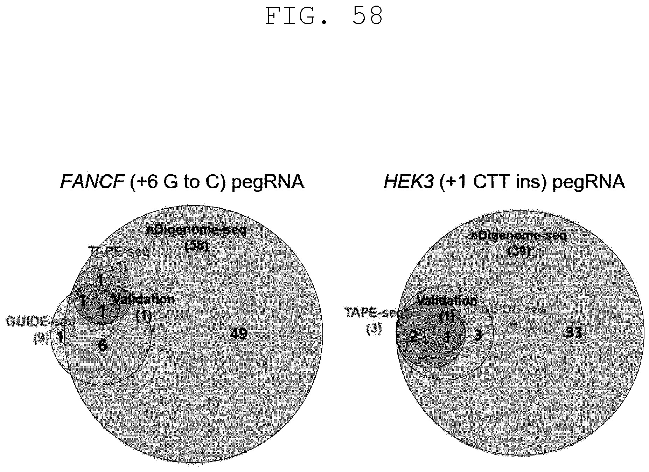

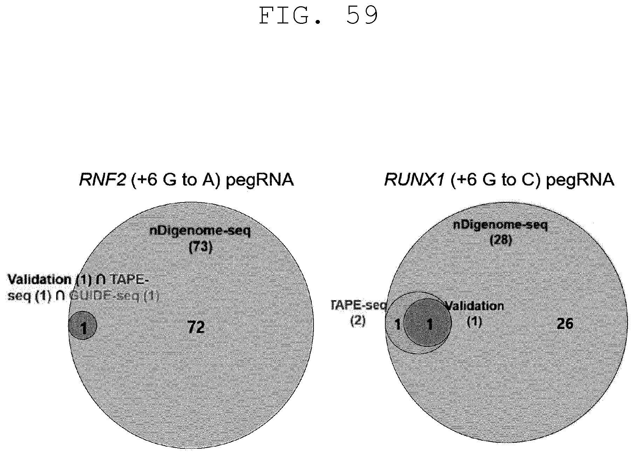

FIGS. 55 to 59 show comparison results for off-target prediction results of nDigenome-seq, GUIDE-seq, and TAPE-seq (TAPE-seq using PEmax-nuclease with epegRNAs) with validated off-targets, wherein FIG. 55 shows results for *HEK4* (+2 G to T) pegRNA and *HEK4* (+3 TAA ins) pegRNA, FIG. 56 shows results for *HBB* (+4 A to T) pegRNA and *DNMT1* (+6 G to C) pegRNA, FIG. 57 shows results for *VEGFA* (+5 G to T) pegRNA and *EMX1* (+5 G to T) pegRNA, FIG. 58 shows results for *FANCF* (+6 G to C) pegRNA and *HEK3* (+1 CTT ins) pegRNA, and FIG. 59 shows results for *RNF2* (+6 G to A) pegRNA and *RUNX1* (+6 G to C) pegRNA;
FIG. 60 shows analysis results for miss rates of GUIDE-seq, nDigenome-seq, TAPE-seq (PE2), TAPE-seq (PE2-nuclease), and TAPE-seq (using PEmax-nuclease with an epegRNA) ;

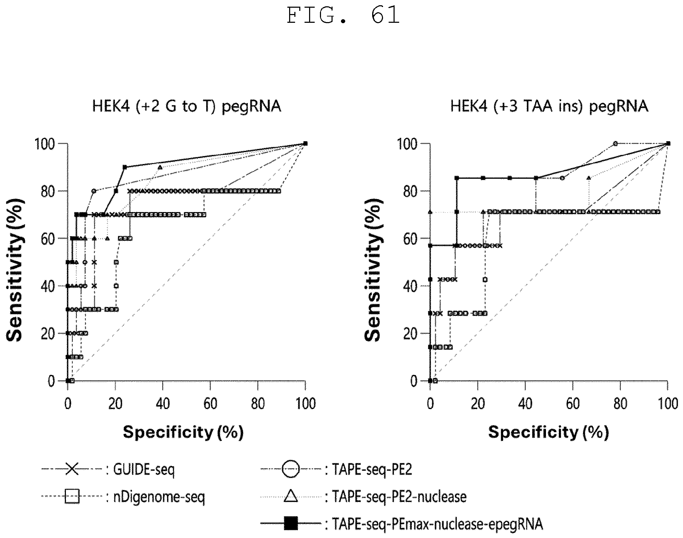

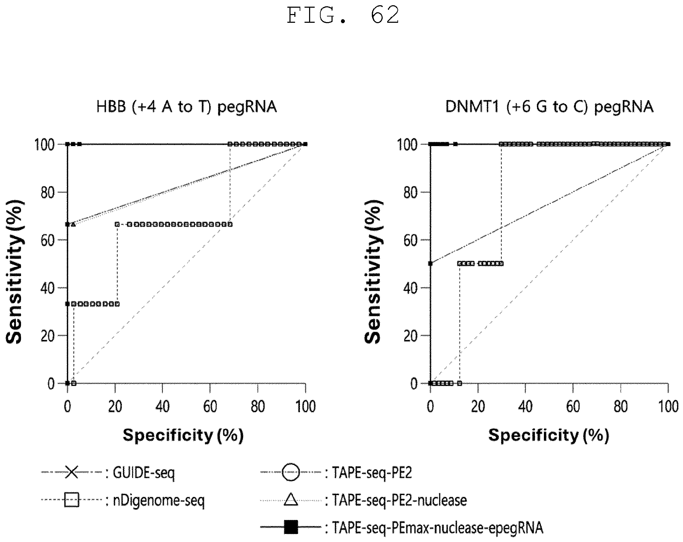

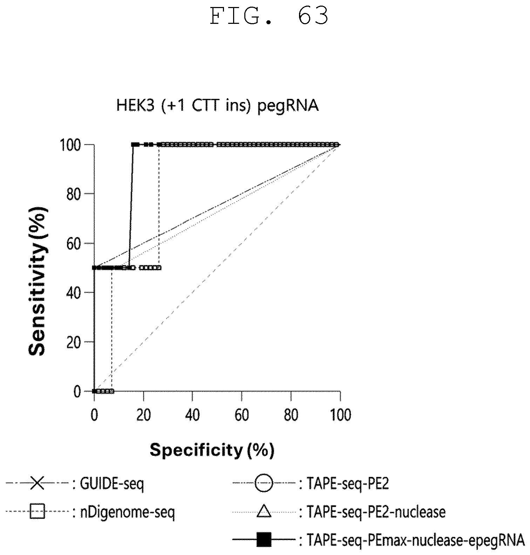

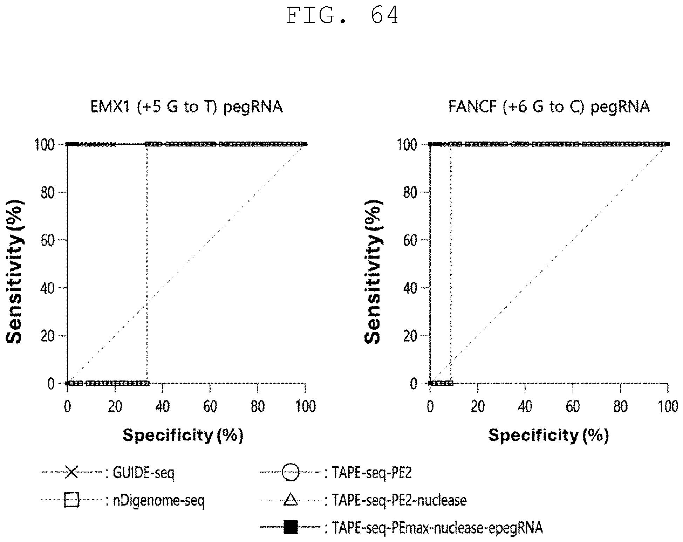

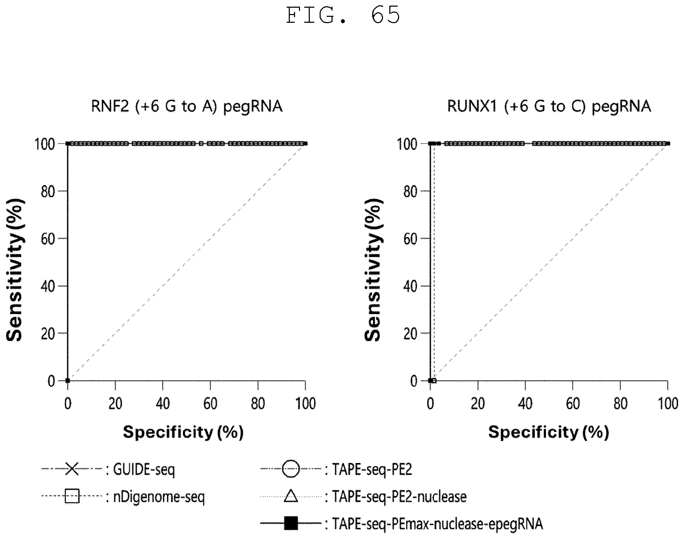

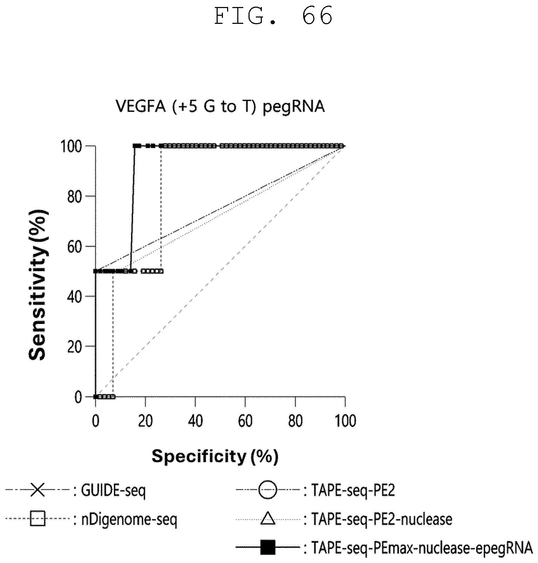

FIGS. 61 to 66 show comparison results for GUIDE-seq, nDigenome-seq, TAPE-seq (PE2), TAPE-seq (PE2-nuclease), and TAPE-seq (PEmax-nuclease with epegRNAs) using receiver operating characteristic (ROC) curves, wherein FIG. 61 shows results for *HEK4* (+2 G to T) pegRNA and *HEK4* (+3 TAA ins) pegRNA, FIG. 62 shows results for *HBB* (+4 A to T) pegRNA and *DNMT1* (+6 G to C) pegRNA, FIG. 63 shows results for *HEK3* (+1 CTT ins) pegRNA, FIG. 64 shows results for *EMX1* (+5 G to T) pegRNA and *FANCF* (+6 G to C) pegRNA, FIG. 65 shows results for *RNF2* (+6 G to A) pegRNA and *RUNX1* (+6 G to C) pegRNA, and FIG. 66 shows results for *VEGFA* (+5 G to T) pegRNA;
FIG. 67 shows analysis results for areas under ROC curves calculated on the basis of the analysis results in FIGS. 61 to 66;

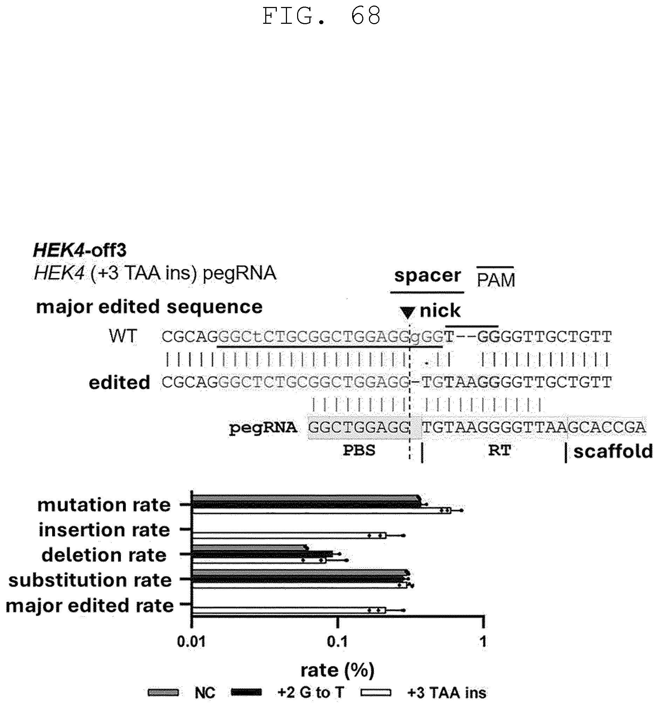

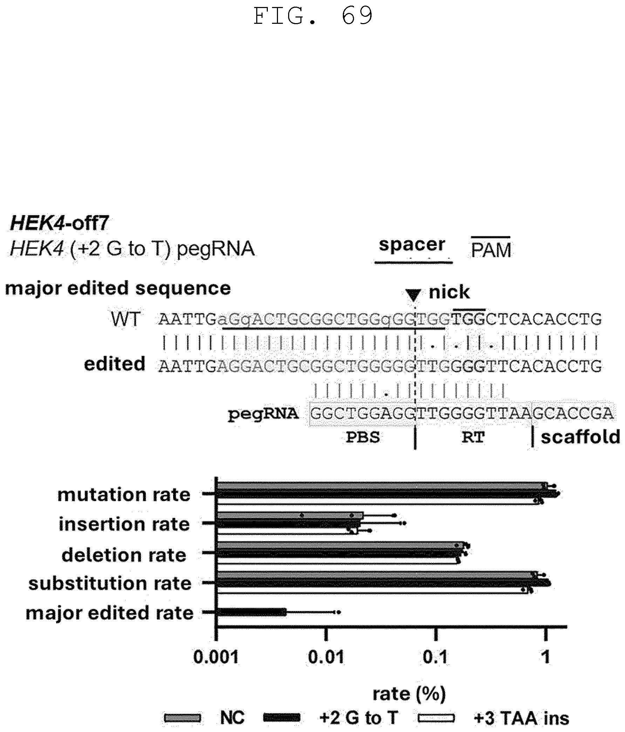

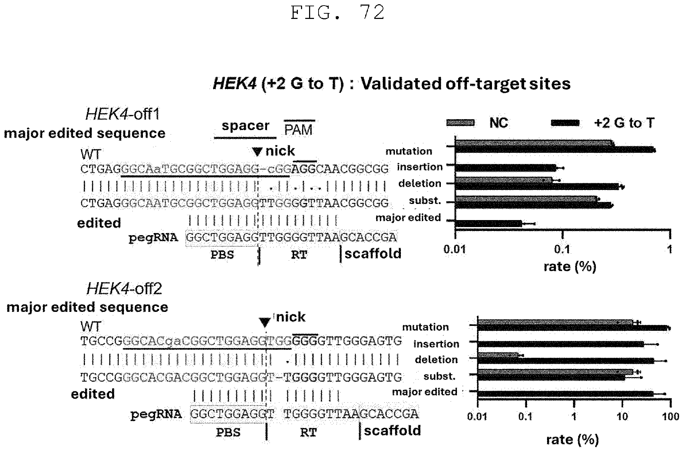

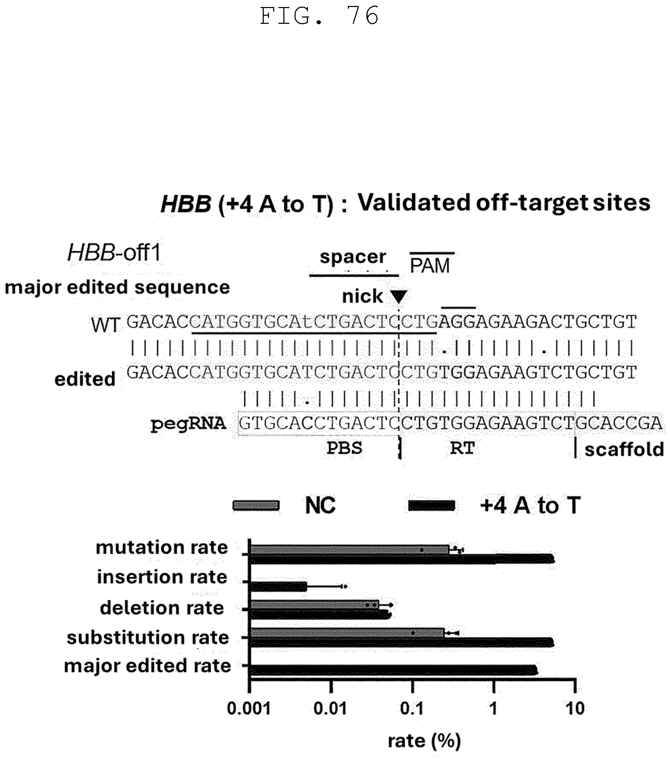

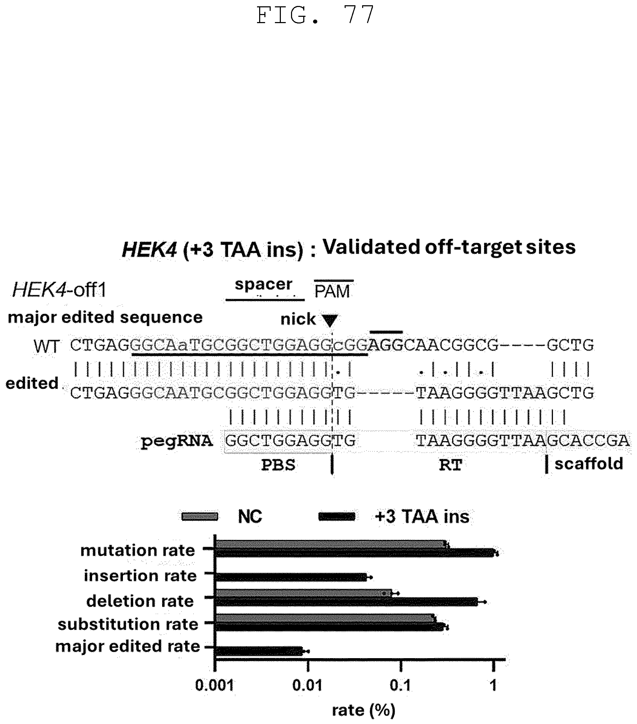

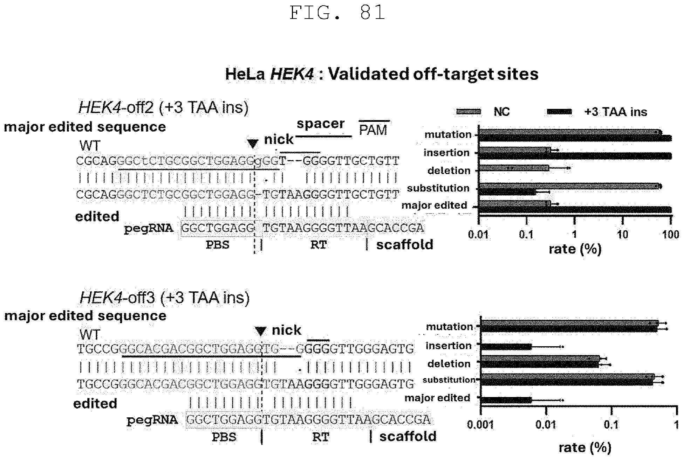

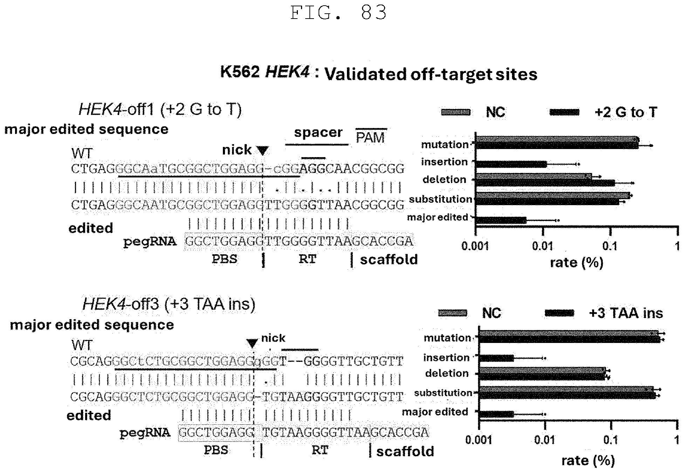

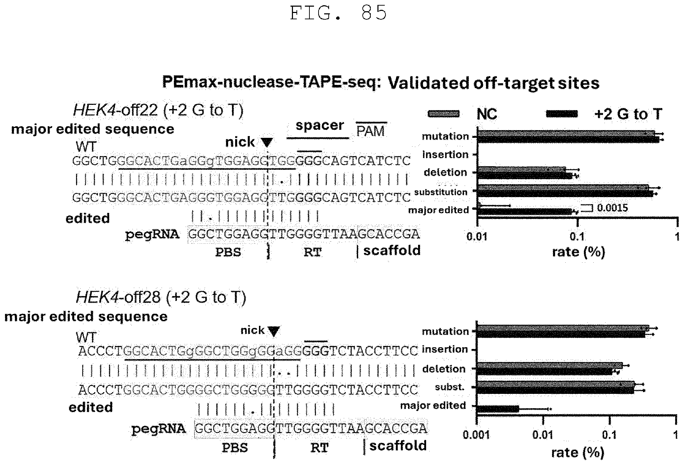

FIGS. 68 to 87 show analysis results for editing patterns of off-target sites analyzed by targeted deep sequencing, wherein FIG. 68 shows results related to an editing pattern induced by *HEK4* (+3 TAA ins) pegRNA, FIGS. 69 to 71 show results related to editing patterns induced by *HEK4* (+2 G to T) pegRNA, FIGS. 72 to 75 show results for editing patterns at validated off-target sites related to *HEK4* (+2 G to T) pegRNA, FIG. 76 shows results for an editing pattern at a validated off-target site related to *HBB* (+4 A to T) pegRNA, FIGS. 77 to 80 show results for editing patterns at validated off-target sites related to *HEK4 (+3* TAA ins) pegRNA, FIGS. 81 to 82 show results in HeLa cells, specifically showing results for *HEK4* (+3 TAA ins) pegRNA and *HEK4* (+2 G to T) pegRNA, FIGS. 83 to 84 show results in K562 cells, specifically showing results for *HEK4* (+3 TAA ins) pegRNA and *HEK4* (+2 G to T) pegRNA, and FIGS. 85 to 87 show results for editing patterns at off-target sites validated by TAPE-seq performed using PEmax-nuclease, specifically showing results for *HEK4* (+2 G to T) pegRNA, *DNMT1* (+6 G to C) pegRNA, *HBB* (+4 A to T) pegRNA, and *VEGFA* (+5 to T) pegRNA;
FIGS. 88 to 90 show analysis results for ROC curves constructed using the number of mismatches in each region (target region, PBS, and RT template) of tpegRNAs, wherein FIG. 88 shows results for *HEK4* (+2 G to T) pegRNA, *HEK4 (+3* TAA ins) pegRNA, and *HBB* (+4 A to T) pegRNA, FIG. 89 shows results for *HEK3* (+1 CTT ins) pegRNA, *FANCF* (+6 G to C) pegRNA, and *EMX1* (+5 G to T) pegRNA, and FIG. 90 shows results for *DNMT1* (+6 G to C) pegRNA, *RUNX1* (+6 G to C) pegRNA, and *VEGFA* (+5 G to T) pegRNA;
FIG. 91 shows analysis results for areas under the ROC curves calculated on the basis of the analysis results in FIGS. 88 to 90;
FIG. 92 shows analysis results of mismatch rates for false positive sites predicted by TAPE-seq and validated sites; and
FIG. 93 shows a vector map of piggyBac PE2 all-in-one

### plasmid (pAllin1-PE2).

### Best Mode

### Definition of terms

Unless otherwise defined, all technical and scientific terms used herein have the same meaning as commonly understood by those skilled in the art to which the present disclosure pertains. The following references provide those skilled in the art with general definitions of many terms used herein: [Singleton et al., Dictionary of Microbiology and Molecular Biology (2nd ed. 1994); The Cambridge Dictionary of Science and Technology (Walker ed., 1988); The Glossary of Genetics, 5th Ed., R. Rieger et al. (eds.), Springer Verlag (1991); and Hale & Marham, The Harper Collins Dictionary of Biology (1991)]. The following terms used herein have the meanings assigned thereto unless otherwise specified.

### "Linked" or "linkage"

As used herein, the term "linked" or "linkage" means that two or more elements existing in one conceptualizable structure are linked, either directly or indirectly (for example, via other elements such as linkers), and is not intended to imply that other additional elements cannot exist between the two or more elements. For example, a description such as "element B linked to element A" is intended to include both cases where one or more other elements exist between elements A and B (that is, element A is linked to element B via one or more other elements) and where one or more other elements do not exist between elements A and B (that is, elements A and B are linked directly), but is not to be interpreted as being limited.

### Sequence identity

As used herein, the term "sequence identity" is used in relation to the degree of similarity between two or more sequences. For example, the term "sequence identity" is used with a term referring to a reference sequence and a term expressing a ratio (such as percentages) . For example, the term "sequence identity" may be used to describe a sequence that is similar to or practically the same as a reference nucleotide sequence. When describing a sequence as "having at least 90% sequence identity to sequence A", the reference sequence herein is sequence A. For example, a reference sequence and a sequence subject to measuring a percentage of sequence identity may be aligned to calculate the percentage of sequence identity. Additionally, the percentage of sequence identity may be calculated by including all mismatches, deletions, and insertions for one or more nucleotides. A method for calculating and/or determining percentages of sequence identity is not particularly limited and may be calculated and/or determined by any reasonable methods or algorithms available to those skilled in the art.

### Notation of amino acid sequence

Unless otherwise described, amino acid sequences are described herein in the N-terminal to C-terminal direction, using either a one-letter or three-letter notation of amino acid. For example, when written in RNVP, this means a peptide in which arginine, asparagine, valine, and proline are linked in such an order in the N-terminal to C-terminal direction. For another example, when written in Thr-Leu-Lys, this means a peptide in which threonine, leucine, and lysine are linked in such an order in the N-terminal to C-terminal direction. Amino acids unable to be represented by the one-letter notation may be notated using other letters and further elaborated.

Each amino acid is notated in the following manner: alanine (Ala, A); arginine (Arg, R); asparagine (Asn, N); aspartic acid (Asp, D); cysteine (Cys, C); glutamic acid (Glu, E); glutamine (Gln, Q); glycine (Gly, G); histidine (His, H); isoleucine (Ile, I); leucine (Leu, L); lysine (Lys K); methionine (Met, M); phenylalanine (Phe, F); proline (Pro, P); serine (Ser, S); threonine (Thr, T); tryptophan (Trp, W); tyrosine (Tyr, Y); and valine (Val, V).

### Notation of nucleic acid sequence

As used herein, the symbols A, T, C, G, and U are interpreted as having meanings understood by those skilled in the art, which may be interpreted appropriately as a base, nucleoside, or nucleotide in DNA or RNA, depending on context and technology. For example, the respective symbols may be interpreted as adenine (A), thymine (T), cytosine (C), guanine (G), or uracil (U) when referring to a base, or as adenosine (A), thymidine (T), cytidine (C), guanosine (G), or uridine (U) when referring to a nucleoside. When referring to a nucleotide in a sequence, this may be interpreted as meaning nucleotides containing the respective nucleosides above.

### Orientation of sequence disclosed

Unless otherwise specified or described, nucleotide sequences (such as DNA sequences, RNA sequences, or DNA/RNA hybrid sequences) disclosed herein must be understood as starting from the 5' to 3' direction. Unless otherwise specified or described, amino acid sequences disclosed herein must be understood as starting from the N- to C-terminal direction.

### Target sequence

As used herein, the term "target sequence" means a specific sequence recognized by a guide RNA or gene editing tools (such as Cas/traditional gRNA complexes, prime editor enzyme/pegRNA complexes, and the like) to cleave a target gene or target nucleic acid. The target sequence may be appropriately selected depending on the purpose. For example, the "target sequence" may refer to a sequence contained in a sequence of a target gene or target nucleic acid while being complementary to a sequence of a spacer contained in a guide RNA (such as pegRNAs) (in which case the target sequence may complementarily bind to the sequence of the spacer of the guide RNA). For another example, the "target sequence", a sequence contained in the sequence of the target gene or target nucleic acid, may refer to a complementary sequence to the sequence complementary to the sequence of the spacer contained in the guide RNA (in which case the target sequence may have a sequence that is practically the same as the sequence of the spacer of the guide RNA). As described above, the target sequence may be used to refer to the sequence complementary to the sequence of the spacer contained in the guide RNA and/or the sequence practically the same as the sequence of the spacer of the guide RNA, but is not to be interpreted as being limited. In some embodiments, the target sequence may be disclosed as a PAM sequence-containing sequence. In some embodiments, the target sequence may be disclosed as a PAM sequence-free sequence. The target sequence will be interpreted appropriately depending on the context in which the content thereof is described. Typically, the sequence of the spacer is determined in consideration of the sequence of the target gene or target nucleic acid and the PAM sequence that an editing protein of a CRISPR/Cas system recognizes. The target sequence may refer only to a sequence of a specific strand that complementarily binds to a guide RNA of a CRISPR/Cas complex, may refer only to a sequence of a specific strand that does not complementarily bind to the guide RNA, or may refer to the target double strand as a whole containing a portion of the specific strand, which is interpreted appropriately depending on the context. The definition of the term target sequence herein is disclosed to describe a strand where the target sequence may be present, so the use of the term target sequence is not intended to distinguish sequences of on-targets and off-targets. The term "target sequence" may be used regarding sequences of on-targets. Additionally, the term "target sequence" may be used regarding sequences of off-targets. In other words, in some embodiments, an intended target sequence may be called a sequence of an on-target, and an unintended target sequence may be called a sequence of an off-target. For example, in some embodiments, the sequence of the on-target may be called the target sequence (in which case the target sequence and the sequence of the spacer of the guide RNA may, for example, be practically the same). For another example, in some embodiments, the sequence of the off-target may be called the target sequence (in which case none or one or more mismatches may, for example, exist between the target sequence and the sequence of the spacer of the guide RNA). The term target sequence may be interpreted in relation to the on-target and the off-target appropriately depending on the content of the relevant paragraph.

### Spacer binding strand

As used herein, the term "spacer binding strand" refers to a strand containing a sequence that forms complementary binding to a part or all of a sequence in the spacer region of a guide nucleic acid in a gene editing system (such as CRISPR/Cas gene editing systems, prime editing systems, and the like) involving guide nucleic acids (such as guide RNAs). Typically, DNA molecules, such as genomes, have a double-stranded structure. In the double strand, a strand having a sequence complementary to a part or all of the sequence in the spacer region of the guide nucleic acid, thereby forming complementary binding thereto, may be called the spacer binding strand.

### Spacer non-binding strand

As used herein, the term "spacer non-binding strand" refers to a strand other than the "spacer binding strand" referring to a strand containing a sequence that forms complementary binding to a part or all of a sequence in the spacer region of a guide nucleic acid, in a gene editing system (such as CRISPR/Cas gene editing systems, prime editing systems, and the like) involving guide nucleic acids (such as guide RNAs). Typically, DNA molecules, such as genomes, have a double-stranded structure, and the term "spacer non-binding strand" may be used to refer to a strand other than the spacer binding strand in the double strand. For example, in the editing of a DNA molecule by a prime editing system, a strand containing a sequence that forms complementary binding to a part or all of a sequence in the spacer region of a pegRNA may be called the "spacer binding strand", and a strand containing a sequence that forms complementary binding to the primer binding site (PBS) of the pegRNA may be called the "spacer non-binding strand". For example, in Prime Editing Version 2, a nick is induced in the spacer non-binding strand by Cas9 (H840A), and a 3' DNA flap is formed on the spacer non-binding strand.

### First and second strands of DNA molecule

Typically, DNA molecules, such as genomes, have a double helix structure composed of two strands. Such a DNA molecule composed of two strands may be called double-stranded DNA. For the description of CRISPR/Cas-based gene editing systems, both strands of a DNA molecule are sometimes required to be called differentially. A single strand of a DNA molecule may be called a first strand. In this case, a strand, other than the first strand, of double-stranded DNA may be called a second strand. In each embodiment, the first and second strands may be set randomly. For example, in some embodiments, when a single strand of a DNA molecule is called the first strand, the other strand of the DNA molecule may be called the second strand. In some embodiments, a spacer binding strand may, for example, be called the first strand. In some embodiments, a spacer non-binding strand may, for another example, be called the first strand. As described above, a single strand of a DNA molecule may be called the first strand, and the other strand may be called the second strand, as needed.

### Upstream and downstream

As used herein, the terms "upstream" and "downstream" are relative terms to define the linear position of at least two elements located in a nucleic acid molecule (whether single-stranded or double-stranded) oriented in the 5' to 3' direction. For example, when describing a first element as being upstream of a second element in a nucleic acid molecule, the first element herein may be located somewhere toward 5' relative to the second element. For example, when a single-nucleotide polymorphism (SNP) is on the 5' side of a nick site, the SNP may be described as being located upstream of the Cas9-induced nick site. For another example, when describing the first element as being downstream of the second element in a nucleic acid molecule, the first element herein may be located somewhere toward the 3' direction relative to the second element. For example, when an SNP is on the 3' side of a nick site, the SNP may be described as being located downstream of a Cas9-induced nick site. The nucleic acid molecule may be DNA (double-stranded or single-stranded), RNA (double-stranded or single-stranded), or a hybrid of DNA and RNA.

### Nuclear localization signal or sequence (NLS)

The term "NLS" refers to an amino acid sequence that facilitates the import of protein into the nucleus of a cell. For example, the import of protein may be facilitated by nuclear transport. The NLS is well-known and will be apparent to those skilled in the art. For example, exemplary sequences of the NLS are described in PCT Application No. PCT/EP2000/011690 (Publication No. WO2021/038547), the content of which is incorporated herein by reference for an exemplary NLS. In some embodiments, the NLS may contain an amino acid sequence of PKKKRKV (SEQ ID NO: 1), KRPAATKKAGQAKKKK (SEQ ID NO: 2), PAAKRVKLD (SEQ ID NO: 3), RQRRNELKRSP (SEQ ID NO: 4), NQSSNFGPMKGGNFGGRSSGPYGGGGQYFAKPRNQGGY (SEQ ID NO: 5), RMRIZFKNKGKDTAELRRRRVEVSVELRKAKKDEQILKRRNV (SEQ ID NO: 6), VSRKRPRP (SEQ ID NO: 7), PPKKARED (SEQ ID NO: 8), PQPKKKPL (SEQ ID NO: 9), SALIKKKKKMAP (SEQ ID NO: 10), DRLRR (SEQ ID NO: 11), PKQKKRK (SEQ ID NO: 12), RKLKKKIKKL (SEQ ID NO: 13), REKKKFLKRR (SEQ ID NO: 14), KRKGDEVDGVDEVAKKKSKK (SEQ ID NO: 15), RKCLQAGMNLEARKTKK (SEQ ID NO: 16), or MDSLLMNRRKFLYQFKNVRWAKGRRETYLC (SEQ ID NO: 17), but are not limited thereto. One or more NLSs may be fused selectively to a protein for gene editing, such as Cas proteins or prime editor proteins. The protein-fused NLSs may be used for facilitating such proteins linked thereto to move into the desired site, the nucleus.

### Protein, peptide, and polypeptide

As used herein, the terms "protein", "peptide", and "polypeptide" are used interchangeably and refer to polymers of amino acid residues linked by peptide (amide) bonds. These terms refer to proteins, peptides, or polypeptides with any size, structure, or function. Typically, these proteins, peptides, or polypeptides may be at least 3 or more amino acids in length. In some embodiments, the protein, peptide, or polypeptide may refer to an individual protein or a combination of proteins. For example, the protein, peptide, or polypeptide may be used as a term including all meanings of an individual protein, a fusion protein in which two or more elements (in which case at least one of the two elements is protein) are fused, and a protein complex in which two or more elements (in which case at least one of the two elements is protein) are complexed. In some embodiments, one or more amino acids in the protein, peptide, or polypeptide may be modified. In this case, the modifications included in the protein, peptide, or polypeptide may, for example, be modifications caused by chemical substances, such as a carbohydrate group, a hydroxyl group, a phosphate group, a farnesyl group, an isofarnesyl group, and a fatty acid group, conjugation, functionalization, or addition of linkers and the like for other modification. In some embodiments, the protein, peptide, or polypeptide may be a single molecule or a multimolecular complex. In some embodiments, the protein, peptide, or polypeptide may be a naturally occurring protein. In some embodiments, the protein, peptide, or polypeptide may be a protein fragment. In some embodiments, the protein, peptide, or polypeptide may be naturally occurring, recombinant, synthetic, or prepared by any combination thereof. Any proteins provided herein may be prepared by any methods known in the art. For example, any proteins provided herein may be prepared by recombinant protein expression and purification, which is particularly appropriate for a fusion protein containing a peptide linker. Inventions for recombinant protein expression and purification are widely known. See [Green, Michael R., and Joseph Sambrook. "Molecular cloning." A Laboratory Manual 4th (2012) .], the entire contents of which are incorporated herein by reference.

### Functional equivalent

The term "functional equivalent" or "equivalent" refers to a secondary molecule or conceptualizable element that is functionally equivalent but not necessarily equivalent to a primary molecule or conceptualizable element in structure. For example, a "Cas9 equivalent" refers to a protein that has the same or substantially the same or similar function as Cas9 but does not necessarily have the same amino acid sequence. Throughout the present application, when a specific protein is mentioned, the specific protein mentioned is intended to include all functional equivalents thereof. For example, when written as "protein X", the term protein X may be interpreted as meanings including functional equivalents thereof. In this aspect, the "functional equivalents" or "equivalents" of protein X include any paralogs, orthologs, fragments, homologs, naturally occurring, manipulated, mutated, and synthesized versions of protein X having equivalent functions. For example, when the term Cas protein is used, this term may be interpreted as including equivalents of the Cas protein (such as Cas nickases). For another example, when the term reverse transcriptase is used, this term may be interpreted as including equivalents of the reverse transcriptase.

### Circular permutant

As used herein, the term "circular permutant" refers to a polypeptide or protein containing a circular permutation, which is a structural rearrangement of the protein whereby the order of amino acids found in the amino acid sequence of the protein is changed. The circular permutant, compared to wild-type counterparts thereof, is a protein with a modified N-terminal end and/or C-terminal end. For example, the wild-type C-terminal half of a protein becomes the new N-terminal half. For example, the circular permutation (or CP) refers to a topological rearrangement in the primary sequence of a protein in which the sequence is split at different sites to prepare new adjacent N- and C-termini while linking the N- and C-termini thereof simultaneously with peptide linkers. As a result, a protein that differs in connectivity but overall is the same or similar in three-dimensional (3D) shape may be prepared. For example, a protein structure with improved or modified features, including reduced proteolysis susceptibility, improved catalytic activities, modified matrix or ligand binding, and/or improved thermal stability, may be prepared. Circular permutant proteins may naturally occur (for example, concanavalin A and lectins). Additionally, circular permutations may occur as a result of post-translational modifications or may be manipulated using recombinant techniques. Circular permutants of a specific protein may be included in equivalents of the specific protein.

As an example of the circular permutant, "circularly permuted Cas9" refers to any Cas9 proteins or variants thereof resulting from circular permutants thereof with locally rearranged N- and C-termini. Such circularly permuted Cas9 proteins ("CP-Cas9") or variants thereof have the ability to bind to DNA when complexed with a guide RNA. See [Oakes, Benjamin L., Dana C. Nadler, and David F. Savage. "Protein engineering of Cas9 for enhanced function." Methods in enzymology. Vol. 546. Academic Press, 2014. 491-511.; and Oakes, Benjamin L., et al. "CRISPR-Cas9 circular permutants as programmable scaffolds for genome modification." Cell 176.1-2 (2019): 254-267.], each of which is incorporated herein by reference. The disclosure herein includes new CP-Cas9 as long as the new CP-Cas9 has the ability to bind to DNA when the resulting circularly permuted protein is complexed with a gRNA, or in consideration of any previously known CP-Cas9. Exemplary sequences of the CP-Cas9 protein are disclosed in WO2020191233A1 (Application No. PCT/US2020/023712), the entire contents of which are incorporated herein by reference.

### Fusion protein

As used herein, the term "fusion protein" refers to a hybrid polypeptide containing a domain or protein derived from at least two different types of elements (in which case at least one of the elements is protein). For example, the fusion protein may be a hybrid polypeptide including proteins derived from two different types of proteins. One type of protein may be located at the amino-terminal (N-terminal) portion of the fusion protein or the carboxy-terminal (C-terminal) portion of the fusion protein, thus forming an "amino-terminal fusion protein" or "carboxy-terminal fusion protein", respectively. In some embodiments, the fusion protein may be used to refer to an element having a single molecule form in which two or more elements are linked by covalent bonds. In other embodiments, the fusion protein may be used to refer to an element having a multimolecular complex form in which two or more elements are linked by non-covalent bonds.

### Linker

As used herein, the term "linker" may refer to a molecule linking two other molecules or moieties. The linker linking two types of proteins in a fusion protein may be an amino acid sequence. For example, Cas9 may be linked to a reverse transcriptase by an amino acid linker sequence to form a fusion protein. Additionally, the linker linking two nucleotide sequences in conjunction may be a nucleotide sequence. For example, in traditional guide RNAs, a crRNA is linked to a tracrRNA via a linker, thus forming a single-stranded guide RNA. In other embodiments, the linker is an organic molecule, group, polymer, or chemical moiety. In some embodiments, the linker may have a length of 1 to 200 amino acids, but is not limited thereto. In some embodiments, the linker may have a length of 1 to 500 nucleotides, but is not limited thereto. Furthermore, longer linkers may also be considered.

### Bispecific ligand

As used herein, the term "bispecific ligand" or "bispecific moiety" refers to a ligand binding to two different types of ligand-binding domains. In certain embodiments, the ligand is a small molecular compound, peptide, or polypeptide. In other embodiments, the ligand-binding domain is a dimerization domain that may be installed as a peptide tag in a protein. In various embodiments, two types of proteins, each independently containing dimerization domains that are the same or different, may be induced to dimerize through the binding of each dimerization domain to the bispecific ligand. As used herein, the "bispecific ligand" may equivalently refer to "chemical inducer of dimerization" or "CID".

### Dimerization domain

The term "dimerization domain" refers to a ligand-binding domain binding to the binding moiety of a bispecific ligand. A first dimerization domain binds to a first binding moiety of the bispecific ligand, and a second dimerization domain binds to a second binding moiety of the same bispecific ligand. When the first dimerization domain is fused to a first protein, and the second dimerization domain is fused to a second protein, the first and second proteins may dimerize in the presence of the bispecific ligand. In this case, the bispecific ligand has at least one moiety binding to the first dimerization domain and at least another moiety binding to the second dimerization domain. In some embodiments, the dimerization domain (such as the first dimerization domain) may be linked to a Cas protein. In some embodiments, the dimerization domain (such as the second dimerization domain) may be linked to a reverse transcriptase.

### Nickase

The term "nickase" refers to a Cas protein in which one of two nuclease domains is inactivated. The nickase may cleave only one strand of a target DNA molecule.

### Flap endonuclease

As used herein, the term "flap endonuclease" refers to an enzyme that catalyzes the removal of a 5' single-stranded DNA flap. Such enzymes process the removal of a 5' flap formed during a cellular process, including DNA replication. In some embodiments, a prime editing method may use a flap endonuclease that is endogenous or exogenously provided to remove 5' flap of endogenous DNA formed in a target region during prime editing. The flap endonuclease is known in the art and disclosed in [Patel, Nikesh, et al. "Flap endonucleases pass 5'-flaps through a flexible arch using a disorder-thread-order mechanism to confer specificity for free 5'-ends." Nucleic acids research 40.10 (2012): 4507-4519.; and Tsutakawa, Susan E., et al. "Human flap endonuclease structures, DNA double-base flipping, and a unified understanding of the FEN1 superfamily." Cell 145.2 (2011): 198-211.] in detail, each of which is incorporated herein by reference. An exemplary flap endonuclease may be flap structure-specific endonuclease 1 (FEN1). The sequence of FEN1 is disclosed in WO2020191233A1 (Application No. PCT/US2020/023712).

### Effective amount

As used herein, the term "effective amount" refers to an amount of biologically active agent sufficient to derive the desired biological response. For example, in some embodiments, the effective amount of a prime editor protein may refer to an amount of protein sufficient to edit a nucleotide sequence in a target region, such as genomes. In some embodiments, the effective amount of the prime editor protein provided in the present application, such as fusion proteins including a nickase Cas9 domain and a reverse transcriptase, may refer to an amount of a fusion protein sufficient to induce the editing of an intended target region to be specifically bound and edited by the fusion protein. As will be appreciated by those skilled in the art, the effective amount of an agent, such as fusion proteins, nucleases, hybrid proteins, protein dimers, complexes of proteins (or protein dimers) and polynucleotides, or polynucleotides, may vary with various factors, such as the desired biological responses, specific genes to be edited, genomes to be edited, target regions to be edited, cells or tissues to be targeted, and the agent to be used.

### About

As used herein, the term "about" means a degree of approximation to any quantity and refers to an amount, level, value, number, frequency, percent, dimension, size, amount, weight, or length that varies by 30%, 25%, 20%, 25%, 10%, 9%, 8%, 7%, 6%, 5%, 4%, 3%, 2%, or 1% with respect to a reference amount, level, value, number, frequency, percent, dimension, size, amount, weight, or length.

### CRISPR/Cas system

### Overview of CRISPR/Cas system

### CRISPR

This "CRISPR" section is intended for the benefit of technologists, and the terms used in this section are not intended to limit the terms disclosed herein.

CRISPR is a family of DNA sequences (that is, CRISPR clusters) found in bacteria and archaea, indicating the snippets of prior infections by viruses that have invaded prokaryotes. The snippets of DNA are used by prokaryotic cells to detect and destroy DNA from subsequent attacks by similar viruses and compose a prokaryotic immune defense system along with an array of CRISPR-associated RNAs and CRISPR-associated proteins (Cas proteins). CRISPR clusters are transcribed and processed into a CRISPR RNA (crRNA). Subsequently, Cas9/crRNA/tracrRNA endonucleolytically cleaves a linear or circular dsDNA target complementary to the RNA. Specifically, the target strand not complementary to the crRNA is first cleaved endonucleolytically and then trimmed 3'-5' exonucleolytically. DNA binding and cleavage typically require protein and both RNAs. However, single guide RNAs (sgRNA, or simply gRNA) have been developed, and such single-stranded RNAs are manipulated so that aspects of both the crRNA and the tracrRNA are combined into a single RNA species. For example, see, [Jinek, Martin, et al. "A programmable dual-RNA-guided DNA endonuclease in adaptive bacterial immunity." science 337.6096 (2012): 816-821.], the entire contents of which are incorporated herein by reference. Cas9 recognizes a short motif (protospacer adjacent motif or PAM) in the CRISPR repeat sequences to help distinguish self versus non-self. Not only CRISPR biology but also sequences and structures of Cas9 nucleases are well known to those skilled in the art (for example, see [Ferretti, Joseph J., et al. "Complete genome sequence of an M1 strain of Streptococcus pyogenes." Proceedings of the National Academy of Sciences 98.8 (2001): 4658-4663.; Deltcheva, Elitza, et al. "CRISPR RNA maturation by trans-encoded small RNA and host factor RNase III." Nature 471.7340 (2011): 602-607.; and Jinek, Martin, et al. "A programmable dual-RNA-guided DNA endonuclease in adaptive bacterial immunity." science 337.6096 (2012): 816-821.], the entire contents of each of which are incorporated herein by reference). Cas9 orthologs have been described in various species including, but not limited to, *Streptococcus pyogenes (S. pyogenes)* and *Streptococcus thermophilus (S. thermophilus)* . Additional suitable Cas9 nucleases and sequences will be apparent to those skilled in the art, based on the disclosure herein, and such Cas9 nucleases and sequences contain Cas9 sequences from organisms and loci disclosed in [Chylinski, Krzysztof, Anais Le Rhun, and Emmanuelle Charpentier. "The tracrRNA and Cas9 families of type II CRISPR-Cas immunity systems." RNA biology 10.5 (2013): 726-737.], the entire contents of which are incorporated herein by reference.

### CRISPR/Cas system and editing of DNA molecules by same

A CRISPR/Cas system developed from the above-described CRISPR is a technology for editing a desired DNA molecule (such as genomes of cells) at a desired site using a Cas protein derived from the CRISPR system of a cell and a guide nucleic acid directing the Cas protein to a target region. For example, the Cas protein forms a Cas/gRNA complex with a guide RNA (gRNA). The Cas/gRNA complex is directed to a desired site by the guide RNA included therein. The Cas protein included in the Cas/gRNA complex induces a double-strand break (DSB) or nick (in the case of nickases) at the desired site. When using the CRISPR/Cas system, not only the genomes of cells but also DNA molecules not located in the genome may be editable. Since the discovery of CRISPR, single-stranded guide RNAs (single guide RNAs; sgRNAs) in which a tracrRNA and a crRNA are linked, as described above, have been developed (see [Jinek, Martin, et al. "A programmable dual-RNA-guided DNA endonuclease in adaptive bacterial immunity." science 337.6096 (2012): 816-821.], the entire contents of which are incorporated herein by reference) regarding the CRISPR/Cas system. Additionally, various classes and/or types of Cas proteins, such as Cas9, Cas12a (cpf1), Cas12b (c2c1), Cas12e (CasX), Cas12k (c2c5), Cas14, Cas14a, Cas13a (c2c2), Cas13b (c2c6) Cas nicakse (such as Cas9 nickases), dead Cas, and the like, have been developed. In some embodiments, the Cas protein may be called a CRISPR enzyme. For an understanding of the CRISPR/Cas system, see WO2018/231018 (International Publication No.), the entire contents of which are incorporated herein by reference. The Cas protein (or CRISPR enzyme) that may be used in the CRISPR/Cas system is to be further described below for the benefit of technologists.

### Cas protein

### Overview of Cas protein

Regarding the CRISPR/Cas system, the Cas protein may be used to refer to a protein that makes a nick or DSB in a desired region to complete editing or that helps induce editing. The term Cas protein may be used to include equivalents thereof. Typically, Cas proteins have nuclease activity to cleave nucleic acids. For example, some Cas proteins may induce a double-strand break (DSB), which may be called Cas nucleases. For another example, some Cas proteins may induce a nick, which may be called Cas nickases. Several Cas proteins are modified not to have nuclease activity, which may be called dead Cas. In the CRISPR/Cas system, the Cas protein may be used interchangeably with the CRISPR enzyme. A representative example of the Cas protein is Cas9.

As used herein, the term Cas protein is used to collectively refer to an editing protein capable of making a DSB or nick in a target region or to inactive the Cas protein, as used in CRISPR/Cas systems. Examples of the Cas protein may include Cas9, Cas9 variants, Cas9 nickases (nCas9), dead Cas9, Cpf1 (Cas12a) (type V CRISPR-Cas system), C2c1 (Cas12b) (type V CRISPR-Cas system), C2c2 (Cas13a) (type VI CRISPR-Cas system), and C2c3 (type V CRISPR-Cas system), but are not limited thereto. Examples of additional Cas proteins are described in [Abudayyeh, Omar O., et al. "C2c2 is a single-component programmable RNA-guided RNA-targeting CRISPR effector." Science 353.6299 (2016): aaf5573.], the entire contents of which are incorporated herein by reference.

In one embodiment, the Cas protein may be a Cas protein (for example, Cas9 or Cpf1) derived from various microorganisms, such as *Streptococcus pyogenes, Streptococcus thermophilus, Streptococcus sp., Staphylococcus aureus, Campylobacter jejuni, Nocardiopsis dassonvillei, Streptomyces pristinaespiralis, Streptomyces viridochromogenes, Streptomyces viridochromogenes, Streptosporangium roseum, Streptosporangium roseum, Alicyclobacillus acidocaldarius, Bacillus pseudomycoides, Bacillus selenitireducens, Exiguobacterium sibiricum, Lactobacillus delbrueckii, Lactobacillus salivarius, Microscilla marina, Burkholderiales bacterium, Polaromonas naphthalenivorans, Polaromonas sp., Crocosphaera watsonii, Cyanothece sp., Microcystis aeruginosa, Synechococcus sp., Acetohalobium arabaticum, Ammonifex degensii, Caldicellulosiruptor bescii, Candidatus Desulforudis, Clostridium botulinum, Clostridium difficile, Finegoldia magna, Natranaerobius thermophilus, Pelotomaculum thermopropionicum, Acidithiobacillus caldus, Acidithiobacillus ferrooxidans, Allochromatium vinosum, Marinobacter sp., Nitrosococcus halophilus, Nitrosococcus watsonii, Pseudoalteromonas haloplanktis, Ktedonobacter racemifer, Methanohalobium evestigatum, Anabaena variabilis, Nodularia spumigena, Nostoc sp., Arthrospira maxima, Arthrospira platensis, Arthrospira sp., Lyngbya sp., Microcoleus chthonoplastes, Oscillatoria sp., Petrotoga mobilis, Thermosipho africanus,* or *Acaryochloris marina.*

Hereinbelow, a Cas9 protein, the representative example of the Cas protein, is to be illustrated.

### Cas9 protein

In CRISPR/Cas9 systems, proteins having nuclease activity to cleave nucleic acids or proteins having inactivated nuclease activity are called Cas9 proteins. The term Cas9 protein is used to include equivalents thereof. Additionally, the Cas9 protein is sometimes called a Cas9 nuclease, casn1 nuclease, or clustered regularly interspaced short palindromic repeat (CRISPR)-associated nuclease. The Cas9 protein correspond to Class 2 Type II in the CRISPR/Cas system classification, and examples thereof include proteins derived from *Streptococcus pyogenes, Streptococcus thermophilus, Streptococcus sp., Streptomyces pristinaespiralis, Streptomyces viridochromogenes, Streptomyces viridochromogenes, Streptosporangium roseum,* or *Streptosporangium roseum.* The sequences and structures of the Cas9 protein are well known to those skilled in the art (for example, see [Ferretti, Joseph J., et al. "Complete genome sequence of an M1 strain of Streptococcus pyogenes." Proceedings of the National Academy of Sciences 98.8 (2001): 4658-4663.; Deltcheva, Elitza, et al. "CRISPR RNA maturation by trans-encoded small RNA and host factor RNase III." Nature 471.7340 (2011) : 602-607.; and Jinek, Martin, et al. "A programmable dual-RNA-guided DNA endonuclease in adaptive bacterial immunity." science 337.6096 (2012): 816-821.], the entire contents of each of which are incorporated herein by reference). Additional Cas9 proteins and sequences are disclosed in [Chylinski, Krzysztof, Anais Le Rhun, and Emmanuelle Charpentier. "The tracrRNA and Cas9 families of type II CRISPR-Cas immunity systems." RNA biology 10.5 (2013): 726-737.], the entire contents of which are incorporated herein by reference).

For example, a DNA cleavage domain of Cas9 is known to include two subdomains: an NHN nuclease subdomain and an RucC1 subdomain. The NHN subdomain cleaves a strand complementary to the gRNA, whereas the RuvC1 subdomain cleaves a non-complementary strand. Inactivation of any one of these subdomains may silence the nuclease activity of the inactivated subdomain, and inactivation of both of these subdomains may silence the entire nuclease activity of Cas9. For example, a H840A mutation provides a Cas9 nickase. For example, both D10A and H840A mutations completely inactivate the nuclease activity of *S. pyogenes* Cas9 (see [Jinek, Martin, et al. "A programmable dual-RNA-guided DNA endonuclease in adaptive bacterial immunity." science 337.6096 (2012): 816-821.]). In some embodiments, a protein containing a Cas9 fragment may be provided. For example, the protein may include any one or more selected from the following two Cas9 domains: the gRNA binding domain of Cas9 and the DNA cleavage domain of Cas9. In some embodiments, the Cas9 variant may be provided. The Cas9 variant is homologous to Cas9 or the fragment thereof. For example, the Cas9 variant may be at least about 70% identical, at least about 80% identical, at least about 90% identical, at least about 95% identical, at least about 96% identical, at least about 97% identical, at least about 98% identical, at least about 99% identical, at least about 99.5% identical, at least about 99.6% identical, at least about 99.7% identical, at least about 99.8% identical, or at least about 99.9% identical to wild-type Cas9 (such as SpCas9). In some embodiments, the Cas9 variant may have 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more amino acid changes compared to wild-type Cas9 (such as SpCas9). In some embodiments, the Cas9 variant may include the Cas9 fragment (such as the gRNA binding domain and/or the DNA cleavage domain). In some embodiments, a fragment of the Cas9 variant may be at least about 70% identical, at least about 80% identical, at least about 90% identical, at least about 95% identical, at least about 96% identical, at least about 97% identical, at least about 98% identical, at least about 99% identical, at least about 99.5% identical, at least about 99.6% identical, at least about 99.7% identical, at least about 99.8% identical, or at least about 99.9% identical to a fragment of corresponding wild-type Cas9. In some embodiments, a wild-type Cas9 fragment or the fragment of the Cas9 variant may be at least 10%, at least 15%, at least 20%, at least 25%, at least 30%, at least 35%, at least 40%, at least 45%, at least 50%, at least 55%, at least 60%, at least 65%, at least 70%, at least 75%, at least 80%, at least 85%, at least 90%, at least 95%, at least 96%, at least 97%, at least 98%, at least 99%, at least 99.5%, or at least 99.9% or more of the amino acid length of corresponding wild-type Cas9.

### Guide RNA

### Overview of guide RNA

In the CRISPR/Cas system, the Cas protein associates with the guide nucleic acid to form a Cas/guide nucleic acid complex. Typically, in CRISPR/Cas systems, a guide RNA (gRNAs) is used as the guide nucleic acid, and the Cas protein associates with the guide RNA to form a Cas/gRNA complex. The Cas/gRNA complex may be called a ribonucleoprotein (RNP). The Cas/gRNA complex makes a nick or double-strand break (DSB) in a target region containing a sequence corresponding to (for example, complementary to) a sequence of a spacer of the guide RNA (gRNA), and the Cas protein induces the DSB or nick. A site where the DSB or nick is made may be near the PAM sequence in the genome.

Cas/gRNA targeting involves a protospacer adjacent motif (PAM) in the genome and the sequence of the spacer of the guide RNA. The Cas protein (such as Cas9), directed to the target region by the PAM and the sequence of the spacer of the guide RNA, makes the DSB in the target region.

In the CRISPR/Cas gene editing system, an RNA having the function of directing the Cas protein to the target region to recognize a specific sequence contained in a target DNA molecule is called the guide RNA.

The functional configuration of the guide RNA is primarily divided as follows: 1) a scaffold sequence portion and 2) a guide domain containing a guide sequence. The scaffold sequence portion, with which the Cas protein (such as Cas9 proteins) interacts, is a portion where the resulting complex is formed by binding to the Cas protein. Typically, the scaffold sequence portion includes a tracrRNA and a crRNA repeat sequence portion, and the scaffold sequence is determined by the type of Cas protein used. The guide sequence is a portion that may complementarily bind to a nucleotide sequence portion of a certain length in a target nucleic acid (such as genomes of cells or target DNA molecules). The guide sequence may be artificially modified and is determined by the target nucleotide sequence of interest related to the desired gene editing.

In some embodiments, the guide RNA may be described as including the crRNA and the tracrRNA. The crRNA may include a spacer and a repeat sequence. A portion of the repeat sequence of the crRNA may interact with (for example, complementarily bind to) the tracrRNA portion. As described above, the single-stranded guide RNA (single guide RNA; sgRNA) in which the tracrRNA and the crRNA are linked (see [Jinek, Martin, et al. "A programmable dual-RNA-guided DNA endonuclease in adaptive bacterial immunity." science 337.6096 (2012): 816-821.], the entire contents of which are incorporated herein by reference) may be provided. In other words, the guide RNA may be provided as being doublestranded or single-stranded.

In some embodiments, the sgRNA may be described as including a guide domain, a first complementary domain, a linker domain, and a second complementary domain. In this case, the sgRNA may contain an additional domain including any one or more from the proximal and tail domains, but is not particularly limited thereto. In this case, the linker domain links the first and second complementary domains, and a part or all of the first complementary domain forms a complementary binding to a part or all of the second complementary domain. As a result, the first complementary domain, the linker domain (for example, including polynucleotide linkers), and the secondary complementary domain form a secondary structure, such as a loop structure (see [PCT Application No. PCT/KR2018/006803, Publication No. WO2018/231018]).

The term guide RNA also includes equivalent guide nucleic acid molecules that associate with Cas9 equivalents, homologs, orthologs, or paralogs, whether naturally occurring or unnaturally occurring (for example, being manipulated, recombinant, or the like), and enable localization of the Cas9 equivalents and the like to a specific target nucleotide sequence. As described above, the Cas9 equivalent may include other Cas proteins derived from any type of CRISPR system (such as types II, V, and VI), including Cpf1 (type V CRISPR-Cas system), C2c1 (type V CRISPR-Cas system), C2c2 (type VI CRISPR-Cas system), and C2c3 (type V CRISPR-Cas system). Additional Cas equivalents are described in [Abudayyeh, Omar O., et al. "C2c2 is a single-component programmable RNA-guided RNA-targeting CRISPR effector." Science 353.6299 (2016): aaf5573.], the entire contents of which are incorporated herein by reference. A guide RNA used in traditional CRISPR/Cas systems may be called a "traditional" guide RNA to be compared with a modified guide RNA called a prime editing guide RNA (pegRNA) invented for a prime editing method and a composition to be described herein. The prime editing guide RNA (pegRNA) may have a form in which an extension arm is linked to the 3' or 5' end of the traditional guide RNA.

The guide RNA or pegRNA may include any one or more from the spacer, a gRNA core, the extension arm (particularly in pegRNAs), and a transcription terminator. Furthermore, various structural elements may be additionally included without being limited thereto. The spacer contains the sequence of the spacer, and the sequence of the spacer refers to a sequence in the guide RNA or pegRNA binding to a sequence in a region containing a protospacer sequence in the target region. The gRNA core may be called a gRNA scaffold or backbone sequence and refers to a sequence in the gRNA or pegRNA responsible for binding to the Cas9 or an equivalent thereof. The gRNA core is free of a spacer or targeting sequence used to guide the Cas9 to the target region (target DNA). The extension arm (particularly in pegRNAs), an element included in the pegRNA, includes a primer binding site (PBS) and a DNA synthesis template sequence for installing a single-stranded DNA flap containing the desired genetic modification by a polymerase (such as reverse transcriptases). The extension arm may be located at the 3' or 5' end of the pegRNA and is designed to install the desired genetic modification. The extension arm in the pegRNA may be called an extension region. In some embodiments, the guide RNA or pegRNA may further include a transcription termination sequence at the 3' of a molecule.

### Guide sequence of guide RNA

The guide RNA may include the guide domain containing the guide sequence. The guide sequence may be used interchangeably with the sequence of the spacer. The guide domain may be used interchangeably with the spacer. The guide sequence, a portion that may be artificially designed, is determined by the target nucleotide sequence of interest. In some embodiments, the guide sequence may be designed to target a sequence adjacent to the PAM sequence located in the desired DNA molecule to be edited. As described above, localization of the Cas/gRNA complex to the target site (such as on-target sites) may be directed. The structure of the guide nucleic acid may vary with the CRISPR type. For example, the guide RNA used in the CRISPR/Cas9 gene editing system may have a structure of 5'-[guide domain]-[scaffold] -3' .

In one embodiment, the guide sequence may have a length of 5 nt to 40 nt. In one embodiment, the guide sequence contained in the guide domain of the guide RNA may have a length of 10 nt to 30 nt. In one embodiment, the guide sequence may have a length of 15 nt to 25 nt. In one embodiment, the guide sequence may have a length of 18 nt to 22 nt. In one embodiment, the guide sequence may have a length of 20 nt. In one embodiment, a target sequence (including all target sequences present in a spacer binding strand and a spacer non-binding strand) serving as a sequence in the genome that forms complementary binding to the guide sequence may have a length of 5 nt to 40 nt or 5 bp to 40 bp. In one embodiment, the target sequence, serving as the sequence in the genome that forms complementary binding to the guide sequence, may have a length of 10 nt to 30 nt or 10 bp to 30 bp. In one embodiment, the target sequence may have a length of 15 nt to 25 nt or 15 bp to 25 bp. In one embodiment, the target sequence may have a length of 18 nt to 22 nt or 18 bp to 22 bp. In one embodiment, the target sequence may have a length of 20 nt or 20 bp.

### PAM

There are two requirements for traditional CRISPR/Cas systems to cleave target DNA molecules. First, a base sequence (nucleotide sequence) of a certain length recognizable by a Cas protein (such as Cas9 proteins) is required to be present in a target gene or a target nucleic acid. In this case, the base sequence (nucleotide sequence) of a certain length recognizable by the Cas9 protein is called a protospacer adjacent motif (PAM) sequence. The PAM sequence is a unique sequence determined by the Cas9 protein. Second, a sequence that may complementarily bind to a sequence of a spacer contained in a guide RNA is required to be present near the PAM sequence of a certain length. In this case, the PAM sequence may be used to contain all sequences present in a spacer non-binding strand and a spacer binding strand.

As described above, the Cas/gRNA complex in the CRISPR/Cas system is directed to the target region by the guide sequence of the gRNA and the PAM sequence of the target DNA molecule (for example, genomes of cells). In the target DNA molecule, the PAM sequence may be located in a strand to which the guide sequence does not bind, rather than a strand to which the guide sequence of the guide RNA binds. The PAM sequence may be determined independently depending on the type of Cas protein used. In one embodiment, the PAM sequence may be any one selected from (disclosed in the 5' to 3' direction): NGG (SEQ ID NO: 19); NNNNRYAC (SEQ ID NO: 20); NNAGAAW (SEQ ID NO: 21); NNNNGATT (SEQ ID NO: 22); NNGRR(T) (SEQ ID NO: 23); TTN (SEQ ID NO: 24); and NNNVRYAC (SEQ ID NO: 25). Each N may independently be A, T, C, or G. Each R may independently be A or G. Each Y may independently be C or T. Each W may independently be A or T. For example, when using SpCas9 as the Cas protein, the PAM sequence may be NGG (SEQ ID NO: 19). For example, when using *Streptococcus thermophilus* Cas9 (StCas9) as the Cas protein, the PAM sequence may be NNAGAAW (SEQ ID NO: 21). For example, when using *Neisseria meningitides* Cas9 (NmCas9), the PAM sequence could be NNNNGATT (SEQ ID NO: 22). For example, when using *Campylobacter jejuni* Cas9 (CjCas9), the PAM may be NNNVRYAC (SEQ ID NO: 25). In one embodiment, the PAM sequence may be linked to the 3' end of the target sequence present in the spacer non-binding strand (in which case the target sequence present in the spacer non-binding strand refers to the sequence not binding to the guide RNA). In one embodiment, the PAM sequence may be located at the 3' end of the target sequence present in the spacer non-binding strand. The target sequence present in the spacer non-binding strand refers to the sequence not binding to the guide sequence of the guide RNA. The target sequence present in the spacer non-binding strand is complementary to the target sequence present in the spacer binding strand.

The site where the DSB or nick is made may be near the PAM sequence of the genome. In one embodiment, the site where the DSB or nick is made may range from -0 to -20 or +0 to +20 relative to the 5' or 3' end of the PAM sequence present in the spacer non-binding strand. In one embodiment, the site where the DSB or nick is made may range from -1 to -5 or +1 to +5 of the PAM sequence in the spacer non-binding strand. For example, in the CRISPR/Cas system using SpCas9, SpCas9 is well known to cleave between the third and fourth nucleotides located upstream of the PAM sequence.

### Genome editing process by traditional CRISPR/Cas system

For the benefit of technologists, a genome editing process by a traditional CRISPR/Cas system is briefly disclosed using the following examples. In this case, the traditional CRISPR/Cas system refers to a system capable of editing a DNA molecule using the Cas protein and a traditional gRNA.

For example, an environment capable of contacting a desired DNA molecule to be edited with a Cas/gRNA complex may be provided. When aiming for genome editing in a cell, the Cas protein or a nucleic acid encoding the same and the guide RNA or a nucleic acid encoding the same may be introduced into the cell, thereby achieving the environment capable of contacting the Cas protein and the guide RNA with the genome DNA of the cell. Under the environment capable of contacting the Cas protein and the guide RNA with the genome DNA of the cell, the Cas protein and the guide RNA may form a Cas/gRNA complex. Even in the absence of the genome DNA of the cell, the Cas/gRNA complex may be formed when both the Cas protein and gRNA are present in an appropriate environment. The Cas/gRNA complex is directed to a target region where a pre-designed target sequence is present by involving the PAM sequence of the genome and a guide sequence of the gRNA contained in the Cas/gRNA complex. The Cas/gRNA complex directed to the target region makes a DSB (for example, in the case of Cas9) in the target region. Then, the DNA where the DSB is made (cleaved) is repaired through a DNA repair process, thereby completing gene editing in the target region or at the target site. The two main pathways for repairing the DSB made in DNA are homology-directed repair (HDR) and nonhomologous end joining (NHEJ). HDR, a naturally occurring DNA repair system between the two pathways, may be used to modify genomes in various organisms, including humans. HDR-mediated repair may be primarily used to insert a desired sequence into a target region or target site or to induce specific point mutations, but is not limited thereto. HDR-mediated repair may be performed using HDR, the DNA repair system, and HDR templates (such as donor templates that may be supplied from outside the cell). NHEJ refers to a DSB repair process in DNA and, contrary to HDR, joins the cleaved ends without HDR templates, meaning that this repair process does not require HDR templates. NHEJ may be a DNA repair mechanism that may be selected primarily to induce an indel. The indel (insertion/deletion) may refer to a variation in which some nucleotides are deleted from the middle of a nucleotide array of a nucleic acid before gene editing, any nucleotides are inserted, and/or such insertions and deletions are combined. The occurrence of some indels in a target gene may lead to inactivation of the corresponding gene. HDR and NHEJ, the DNA repair mechanisms, are disclosed in [Sander, Jeffry D., and J. Keith Joung. "CRISPR-Cas systems for editing, regulating and targeting genomes." Nature biotechnology 32.4 (2014): 347-355.] in detail, the entire contents of which are incorporated herein by reference.

The traditional CRISPR/Cas system, which is the basis of the prime editing system, has been described hereinabove in detail for the benefit of technologists. The present application relates to a new system for predicting off-targets that may occur in a DNA editing process by a prime editing system. Hereinbelow, in advance to describing the off-target prediction system in the prime editing system provided by the present application, a prime editing system, which is the basis of the off-target prediction system, and a process of editing a DNA molecule by the prime editing system will be described in detail.

### Prime editing system

### Overview of prime editing system

Prime editing, developed by David R. Liu et al., is a technology for editing a DNA molecule (such as genomes) using a Cas protein, a polymerase (such as reverse transcriptases), and a specialized guide RNA including a DNA synthesis template to integrate or insert desired editing into a target region of a DNA molecule. A description and various embodiments of prime editing are disclosed in [Anzalone, Andrew V., et al. "Search-and-replace genome editing without double-strand breaks or donor DNA." Nature 576.7785 (2019): 149-157.; Chen, Peter J., et al. "Enhanced prime editing systems by manipulating cellular determinants of editing outcomes." Cell 184.22 (2021): 5635-5652.; and PCT Application No. PCT/US2020/023712, Publication No. WO2020191233A1], the entire contents of each of which are incorporated herein by reference.

In prime editing, to introduce desired editing into a target region in a target DNA molecule, genomes are edited by using (1) a prime editor protein including a Cas protein a polymerase (such as reverse transcriptases), and (2) a prime editing guide RNA (pegRNA). Various embodiments of prime editing are disclosed in PCT Application No. PCT/US2020/023712 (Publication No. WO2020191233A1), the entire contents of which are incorporated herein by reference.

Prime editing, a versatile and precise genome editing method to directly write new genetic information into a target region in a DNA molecule (such as genomes) using the prime editor protein including the Cas protein, is a new-platform genome editing method developed by David R. Liu et al. Prime editing primarily uses the Cas protein, the polymerase, and the pegRNA, wherein the pegRNA has a form in which an extension arm is linked to a traditional guide RNA. In this case, the extension arm includes an extension region. The extension region includes an editing template serving as a template of the desired editing so that the desired editing is inserted into the target region. In this case, the insertion of the desired editing into the target region is performed through numerous processes including polymerization by the polymerase (such as reverse transcriptases) linked to the Cas protein. Polymerization by the polymerase is performed on a spacer non-binding strand using the DNA synthesis template included in the extension region of the pegRNA as a polymerization template.

For example, in Prime Editing Version 2, PE2, a nick is made in a spacer non-binding strand (induced by and/or made by a Cas protein included in a PE2 prime editor protein), followed by performing polymerization (reverse transcription) by a reverse transcriptase on a DNA synthesis template strand in the 5' to 3' direction from the site where the nick is made relative to the spacer non-binding. The reverse transcription is performed using the DNA synthesis template, included in an extension region, as a template of the reverse transcription. In such a polymerization process, a sequence complementary to a part or all of the DNA synthesis template is encoded at the site of the spacer non-binding strand where the nick is made. The sequence encoded in such a manner forms a 3' DNA flap. The 3' DNA flap includes an editing, and the editing has a DNA sequence complementary to an editing template included in the DNA synthesis template. Subsequently, a 5' DNA flap is removed through a 5' DNA flap cleavage process (which may, for example, involve FEN1, a 5' DNA flap endonuclease). Additionally, the desired editing is integrated into the desired site through the ligation of the 3' DNA flap and a process of cellular DNA repair and/or replication. The process of editing a DNA molecule by Prime Editing Version 2 (PE2) is described in [Anzalone, Andrew V., et al. "Search-and-replace genome editing without double-strand breaks or donor DNA." Nature 576.7785 (2019): 149-157.] in detail, the entire contents of which are incorporated herein by reference.

The term editing used in relation to prime editing may refer to an editing integrated into a DNA molecule as a result of the prime editing system. For example, the editing may be used to refer to an editing integrated into the spacer non-binding strand, an editing to be integrated into the spacer binding strand, and/or an editing to be integrated into the double strand. This is because the editing installed in the 3' flap is installed ultimately in the spacer non-binding strand and the spacer binding strand through the ligation of the 3' DNA flap and a process including cellular DNA repair and/or replication, as described above. The editing may include any one or a combination of insertions of one or more nucleotides, deletions of one or more nucleotides, and substitutions of one or more nucleotides with other nucleotides.

For example, the editing may include insertions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be inserted may or may not be located in succession in a nucleic acid. For example, the editing may include deletions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be deleted may or may not be located in succession in a nucleic acid. For example, the editing may include substitutions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be substituted may or may not be located in succession in a nucleic acid. For another example, the editing may include the above-described insertions and substitutions. For another example, the editing may include the above-described deletions and substitutions. For another example, the editing may include the above-described insertions, deletions, and substitutions. As [Anzalone, Andrew V., et al. "Search-and-replace genome editing without double-strand breaks or donor DNA." Nature 576.7785 (2019): 149-157.], the publication first disclosed by David R. Liu et al. on prime editing, described the prime editing scope as "All 4 transition point mutations; All 8 transversion point mutations; Insertions (1 bp to ≥ 44 bp); Deletions (1 bp to ≥ 80 bp); combinations of the above", embodiments of the editing enabled to be installed in a DNA molecule by prime editing are diverse. Furthermore, prime editing technology is still being developed and improved, so the prime editing scope is not limited to the scope disclosed in the above document. [Anzalone, Andrew V., et al. "Search-and-replace genome editing without double-strand breaks or donor DNA." Nature 576.7785 (2019): 149-157.], the entire contents of which are incorporated herein by reference, describes prime editing as a versatile and precise genome editing method that "writes" new genetic information directly at specific DNA regions. In this light, the description herein for genetic information enabled to be inserted or installed into DNA through prime editing should not be interpreted as being limited.

In some cases, prime editing may be considered a "search-and-replace" genome editing technology. This is because the prime editor (or prime editor complex) used to perform prime editing may search and locate the desired target region to be edited while installing an alternative strand containing the desired editing instead of a corresponding endogenous DNA strand of the target region. PCT Application No. PCT/US2020/023712 (Publication No. WO2020191233A1), the entire contents of which are incorporated herein by reference, discloses that the prime editor described in the above document is not limited to reverse transcriptases, and such reverse transcriptases are merely one type of DNA polymerase that may be used in prime editing. Thus, whenever a reverse transcriptase is mentioned, those skilled in the art will appreciate that any suitable DNA polymerase may be used instead of the reverse transcriptase. Similarly, it will also be fully appreciated that not only Cas9, nCas9, or the like but also proteins or domains that are functionally equivalent to Cas9 may be used in prime editing.

The guide RNA specialized for prime editing (that is, pegRNAs) is complexed with the Cas protein (for example, complexed with a fusion protein including the Cas protein) and ultimately installs the desired editing at the target site in the target region of the DNA molecule (such as genomes) through the prime editing process. The pegRNA includes the editing template to deliver desired information to the target DNA. The alternative strand containing the sequence corresponding to the editing template is prepared from the editing template, and this alternative strand is used to replace the corresponding endogenous DNA strand. The prime editing mechanism may involve nicking the target region in a single strand of DNA and exposing a 3'-hydroxyl group to deliver the information from the pegRNA to the target DNA. Subsequently, the prime editing mechanism includes delivering the desired information into the target region, using the exposed 3'-hydroxyl group, through a DNA polymerization process based on the sequence that may deliver the desired information into the pegRNA. In various embodiments, the extension region that provides the polymerization template of the editing-containing alternative strand may be formed from RNA or DNA. In the case of the RNA extension region, the polymerase used in prime editing may be an RNA-dependent DNA polymerase (such as reverse transcriptases). In the case of the DNA extension region, the polymerase used in prime editing may be a DNA-dependent DNA polymerase. A strand newly synthesized by prime editing (that is, the alternative DNA strand containing the desired editing) may be homologous to the genome target sequence, except for including the desired nucleotide modification. The newly synthesized strand of DNA may also be called a single-stranded DNA flap (such as 3' single-stranded DNA flaps), which will replace the corresponding endogenous strand.

In various embodiments, prime editing operates by contacting the target DNA molecule with the Cas protein complexed with the prime editing guide RNA (pegRNA) (in which case the Cas protein is included in the prime editor protein). One example of editing a DNA molecule (such as genomes) by prime editing may be described as follows. After contacting a nCas9 (which may, for example, be included in the prime editor protein)/pegRNA complex with a DNA molecule, the pegRNA guides the nCas9 to bind to a target region. A nick is introduced (nCas9 introduces the nick) into one strand of the DNA strand in the target region, thus preparing the available 3' end of one strand of the DNA strand. The available 3' end is located in the target region. In certain embodiments, the nick may be made in a strand not hybridized to a portion of the pegRNA sequence, which means the spacer non-binding strand. In other certain embodiments, the nick may be made in a strand to be hybridized to a portion of the pegRNA sequence, which means the spacer binding strand. The region located at the 3' end of the DNA strand formed by nicking of the Cas9 nickase (the region located upstream of the nick site) interacts with a portion of the extension region of the pegRNA for reverse transcription priming. In certain embodiments, the DNA strand at the 3' end is hybridized to a primer binding site (PBS) or a reverse transcriptase-priming sequence contained in the extension region of the pegRNA. A single strand of DNA is synthesized by the reverse transcriptase (which may, for example, be included in a prime editing fusion protein) in the direction from the 3' end of the primed region towards the 5' end of the pegRNA. In other words, the single strand of DNA is synthesized in the 5' to 3' direction relative to the spacer non-binding strand (PAM-containing sequence) hybridized to the primer binding site. The single strand of DNA synthesized in such a manner includes the desired nucleotide modification (such as one or more base modifications, one or more insertions, one or more deletions, or a combination thereof). The synthesized single strand of DNA may be called a 3' single-stranded DNA flap. When the 3' single strand invades into endogenous DNA, a 5' endogenous DNA flap formed (unedited) is removed. The removal of the 5' endogenous DNA flap may be performed through a 5' flap cleavage process. The 3' single-stranded DNA flap invaded into the endogenous DNA is ligated. DNA repair then operates. As a result, the desired editing is integrated fully into the target region.

The purposes of the prime editing system are achievable, for example, by factors including the prime editor protein, the pegRNA, and the like. Hereinbelow, the prime editor protein and the pegRNA used in prime editing are to be described.

### Prime editor protein

### Overview of prime editor protein

In some embodiments, the prime editor protein (or prime editing construct) means a construct in the form of a complex or a fusion protein including the Cas protein and the polymerase. The prime editor protein may be called terms such as a prime editing protein, a prime editing construct, a prime editing enzyme, a prime editor enzyme, and a prime editing fusion protein. The prime editor protein may include a structure represented by [Cas]-[P] or [P]-[Cas], wherein "P" refers to any polymerase (such as reverse transcriptases) or an element derived therefrom, and "Cas" refers to the Cas protein (such as SpCas9 variants including Cas9 nickases or wild-type SpCas9) or an element derived therefrom. The "]-[" or "-", indicating that the Cas protein and the polymerase are linked, may refer to an element such as any linker having the function of linking the Cas protein and the polymerase in a covalent or noncovalent manner or to a bond.

As described above, the prime editor protein includes the Cas protein (such as Cas9 nickases) and the reverse transcriptase (or DNA polymerase). The prime editor protein may be in the form of a fusion protein composed of one molecule or in the form of a complex composed of two or more molecules, but is not particularly limited. Prime editing may be performed on the target region by the prime editor protein in the presence of the pegRNA. The prime editor protein forms a complex with the pegRNA, in which case the complex may be called a prime editor protein/pegRNA complex. In some embodiments, the prime editor protein may be called a prime editing protein.

In some embodiments, the term "prime editing system" may refer to the editing of a DNA molecule performed using the prime editor protein and the pegRNA or the prime editor protein and the pegRNA. As described above, the term "prime editing system" may be used as a comprehensive concept to describe content related to prime editing. In some embodiments, the prime editing system may further include other elements or use thereof in addition to the prime editor protein and the pegRNA. For example, the prime editing system may further include a traditional guide RNA capable of directing second site nicking of an unedited strand or use thereof.

In some embodiments, the prime editor protein includes:
(i) the Cas protein; and
(ii) the polymerase.

Hereinbelow, the Cas protein and the polymerase, included in the prime editor protein, are to be described.

### Prime editor protein element 1 - Cas protein

The prime editor protein includes the Cas protein and the polymerase. The prime editor protein may include the Cas protein described in detail in the "CRISPR/Cas system" section. The term Cas9 protein is used to include equivalents thereof. The Cas protein may be called a CRISPR enzyme, a nucleic acid programmable DNA binding protein (napDNAbp), a CRISPR protein, or the like.

In some embodiments, the Cas protein may be Cas12a, Cas12b1 (C2c1), Cas12c (C2c3), Cas12e (CasX), Cas12d (CasY), Cas12g, Cas12h, Cas12i, Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cas13a (C2c2), Cas13b, Cas13c, Cas13d, Cas14, xCas9, circularly permuted Cas9, an Argonaute (Ago) domain, or fragments, homologs, or variants thereof, but is not particularly limited thereto. In some embodiments, the Cas protein may be a Cas protein having nickase activity. The Cas protein having nickase activity may be a Cas9 nickase or Cas12 nickase (such as Cas12a nickases, Cas12b1 nickases, or the like), but is not limited thereto. In some embodiments, the Cas protein may be a Cas protein having nuclease activity. In some embodiments, the Cas protein may include one or more amino acid substitutions or amino acid variations in an NHN domain and/or a RuvC domain. For example, the variant may contain an amino acid sequence having about 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% sequence identity compared to the amino acid sequence of a wild-type Cas protein or parent Cas protein. For example, the variant may include one or more insertions, one or more deletions, one or more substitutions, or a combination thereof compared to the amino acid sequence of a wild-type Cas protein or parent Cas protein.

For example, the Cas protein may be Cas9 derived from *Streptococcus pyogenes* (SpCas9), Cas9 derived from *Campylobacter jejuni* (CjCas9), Cas9 derived from *Staphylococcus aureus* (SaCas9), or variants thereof. For example, the Cas protein may be SpyMac, iSpymac, GeoCas9, xCas9, circularly permuted Cas9, or variants thereof. For example, the SpCas9 variant may include variations in amino acid residues of one or more insertions, one or more deletions, one or more substitutions, or a combination thereof, compared to the amino acid sequence of wild-type SpCas9. For example, the SpCas9 variant including a H840A substitution provides a Cas protein having nickase activity. For example, the SpCas9 variant including a D10A substitution provides a Cas protein having nickase activity. For example, the SpCas9 variant may include R221K and N394K substitutions. For example, the SpCas9 variant may have a form in which one or more amino acid residues selected from D10, R221, L244, N394, H840, K1211, and L1245 of wild-type SpCas9 are substituted with another amino acid residue. For example, the SpCas9 variant may include any one or more from D10A, R221K, L244Q, N394K, H840A, K1211Q, and L1245V. In some embodiments, the Cas protein may be: an SpCas9 variant having nickase activity including H840A; an SpCas9 variant having nickase activity including R221K, N394K, and H840A (see [Chen, Peter J., et al. "Enhanced prime editing systems by manipulating cellular determinants of editing outcomes." Cell 184.22 (2021): 5635-5652.]; a wild-type SpCas9 variant having nuclease activity (that is, inducing a DSB) (see Adikusuma, Fatwa, et al. "Optimized nickase-and nuclease-based prime editing in human and mouse cells." Nucleic acids research 49.18 (2021): 10785-10795.]); or an SpCas9 variant having nuclease activity including R221K and N394K, but is not limited thereto. In some embodiments, the Cas protein may be codon optimized. In some embodiments, the prime editor protein may include a PAMless Cas protein.

Various examples of the Cas protein that may be included in the prime editor protein are described in [U.S. Patent Application No. 17/219,672] in detail.

In some embodiments, wild-type SpCas9 may contain the following amino acid sequence of SEQ ID NO: 28:

























 (SEQ ID NO: 28).

In some embodiments, a wild-type SpCas9 variant including a H840A variation may contain the following amino acid sequence of SEQ ID NO: 29:

MDKKYSIGLDIGTNSVGWAVITDEYKVPSKKFKVLGNTDRHSIKKNLIGALL

FDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHF
LIEGDLNPDNSDVDKLFIQLVQTYNQLFEENPINASGVDAKAILSARLSKSRRLENLI
AQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQ
LPEKYKEIFFDQSKNGYAGYIDGGASQEEFYKFIKPILEKMDGTEELLVKLNREDLLR
KQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIEC
FDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQL
QNEKLYLYYLQNGRDMYVDQELDINRLSDYDVDAIVPQSFLKDDSIDNKVLTRSDKNR
GKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINN
YHHAHDAYLNAVVGTALIKKYPKLESEFVYGDYKVYDVRKMIAKSEQEIGKATAKYFF
YSNIMNFFKTEITLANGEIRKRPLIETNGETGEIVWDKGRDFATVRKVLSMPQVNIVK
KTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFD
TTIDRKRYTSTKEVLDATLIHQSITGLYETRIDLSQLGGD (SEQ ID NO: 29).

In some embodiments, a wild-type SpCas9 variant including R221K and N394K variations may contain the following amino acid sequence of SEQ ID NO: 30:

MDKKYSIGLDIGTNSVGWAVITDEYKVPSKKFKVLGNTDRHSIKKNLIGALL
FDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHF
LIEGDLNPDNSDVDKLFIQLVQTYNQLFEENPINASGVDAKAILSARLSKSRKLENLI
AQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQ
LPEKYKEIFFDQSKNGYAGYIDGGASQEEFYKFIKPILEKMDGTEELLVKLKREDLLR
KQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIEC
FDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQL
QNEKLYLYYLQNGRDMYVDQELDINRLSDYDVDHIVPQSFLKDDSIDNKVLTRSDKNR
GKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINN
YHHAHDAYLNAVVGTALIKKYPKLESEFVYGDYKVYDVRKMIAKSEQEIGKATAKYFF
YSNIMNFFKTEITLANGEIRKRPLIETNGETGEIVWDKGRDFATVRKVLSMPQVNIVK
KTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII
EQISEFSKRVILADANLDKVLSAYNKHRDKPIREQAENIIHLFTLTNLGAPAAFKYFD
TTIDRKRYTSTKEVLDATLIHQSITGLYETRIDLSQLGGD (SEQ ID NO: 30).

In some embodiments, a wild-type SpCas9 variant including R221K, N394K, and H840A variations may contain the following amino acid sequence of SEQ ID NO: 31:

MDKKYSIGLDIGTNSVGWAVITDEYKVPSKKFKVLGNTDRHSIKKNLIGALL
FDSGETAEATRLKRTARRRYTRRKNRICYLQEIFSNEMAKVDDSFFHRLEESFLVEED
KKHERHPIFGNIVDEVAYHEKYPTIYHLRKKLVDSTDKADLRLIYLALAHMIKFRGHF
LIEGDLNPDNSDVDKLFIQLVQTYNQLFEENPINASGVDAKAILSARLSKSRKLENLI
AQLPGEKKNGLFGNLIALSLGLTPNFKSNFDLAEDAKLQLSKDTYDDDLDNLLAQIGD
QYADLFLAAKNLSDAILLSDILRVNTEITKAPLSASMIKRYDEHHQDLTLLKALVRQQ
LPEKYKEIFFDQSKNGYAGYIDGGASQEEFYKFIKPILEKMDGTEELLVKLKREDLLR
KQRTFDNGSIPHQIHLGELHAILRRQEDFYPFLKDNREKIEKILTFRIPYYVGPLARG
NSRFAWMTRKSEETITPWNFEEVVDKGASAQSFIERMTNFDKNLPNEKVLPKHSLLYE
YFTVYNELTKVKYVTEGMRKPAFLSGEQKKAIVDLLFKTNRKVTVKQLKEDYFKKIEC
FDSVEISGVEDRFNASLGTYHDLLKIIKDKDFLDNEENEDILEDIVLTLTLFEDREMI
EERLKTYAHLFDDKVMKQLKRRRYTGWGRLSRKLINGIRDKQSGKTILDFLKSDGFAN
RNFMQLIHDDSLTFKEDIQKAQVSGQGDSLHEHIANLAGSPAIKKGILQTVKVVDELV
KVMGRHKPENIVIEMARENQTTQKGQKNSRERMKRIEEGIKELGSQILKEHPVENTQL
QNEKLYLYYLQNGRDMYVDQELDINRLSDYDVDAIVPQSFLKDDSIDNKVLTRSDKNR
GKSDNVPSEEVVKKMKNYWRQLLNAKLITQRKFDNLTKAERGGLSELDKAGFIKRQLV
ETRQITKHVAQILDSRMNTKYDENDKLIREVKVITLKSKLVSDFRKDFQFYKVREINN
YHHAHDAYLNAVVGTALIKKYPKLESEFVYGDYKVYDVRKMIAKSEQEIGKATAKYFF
YSNIMNFFKTEITLANGEIRKRPLIETNGETGEIVWDKGRDFATVRKVLSMPQVNIVK
KTEVQTGGFSKESILPKRNSDKLIARKKDWDPKKYGGFDSPTVAYSVLVVAKVEKGKS
KKLKSVKELLGITIMERSSFEKNPIDFLEAKGYKEVKKDLIIKLPKYSLFELENGRKR
MLASAGELQKGNELALPSKYVNFLYLASHYEKLKGSPEDNEQKQLFVEQHKHYLDEII

EQISEFSKRVILADANLDKVLSAYNKHRDKPIREQAENIIHLFTLTNLGAPAAFKYFD

TTIDRKRYTSTKEVLDATLIHQSITGLYETRIDLSQLGGD (SEQ ID NO: 31).

### Prime editor protein element 2 - Polymerase

### Overview of polymerase used in prime editing

The prime editor protein includes the Cas protein and the polymerase. The polymerase refers to an enzyme or protein that synthesizes nucleotide strands and may be used in relation to the prime editing system or prime editing-based system described herein. The polymerase is a "template-dependent polymerase" (that is, a polymerase synthesizing a nucleotide strand on the basis of the order of the nucleotide bases in a template strand). The polymerase may also be a "template-independent" polymerase. The polymerase may also be categorized as a "DNA polymerase" or "RNA polymerase".

In various embodiments, the prime editing system or the prime editor protein may include the DNA polymerase synthesizing a DNA strand.

In some embodiments, the DNA polymerase may be a DNA-dependent DNA polymerase, in which case the pegRNA may include a DNA template serving as a template of polymerization by the DNA-dependent DNA polymerase. In this case, the pegRNA may be called a hybrid pegRNA or chimera including an RNA portion (guide RNA component including a spacer and a gRNA core) and a DNA portion (DNA template).

In various embodiments, the DNA polymerase may be an "RNA-dependent DNA polymerase". In this case, the pegRNA may include an RNA template serving as a template of polymerization by the RNA-dependent DNA polymerase. In other words, the pegRNA may be composed of RNA components and includes an RNA extension region.

The polymerase may also refer to an enzyme catalyzing the polymerization of nucleotides. Typically, polymerization by the polymerase will start at the 3'-end of a polynucleotide template sequence-annealed primer (such as primer sequences annealed to the primer binding site of pegRNAs in prime editing) and be performed toward the 5'-end of the template strand. The DNA polymerase may catalyze the polymerization of deoxynucleotides. As used herein, the term polymerase may be used to include meanings of enzymes, proteins, variants thereof, and fragments thereof that catalyze and/or perform the polymerization of nucleotides. In this case, the fragment of the polymerase refers to any portion of a wild-type or mutant (variant) DNA polymerase that contains an amino acid sequence of a length smaller than the entire length of the wild-type polymerase and has the ability to catalyze and/or perform the polymerization of deoxynucleotides under at least one condition. Such a fragment may exist as a separate entity or constitute larger polypeptides, such as fusion proteins.

### Examples of polymerase: reverse transcriptase

For example, the polymerase, one element used in prime editing, may be a reverse transcriptase (RT). The reverse transcriptase refers to a class of polymerases characterized as an RNA-dependent DNA polymerase. All known reverse transcriptases require a primer for synthesizing a DNA transcript from an RNA template. As used herein, the term reverse transcriptase may be used as a term including meanings of variants and fragments thereof. For example, the variant may contain an amino acid sequence having about 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, 99.5%, or 99.9% sequence identity compared to the amino acid sequence of a wild-type reverse transcriptase or parent reverse transcriptase. For example, the variant may include one or more insertions, one or more deletions, one or more substitutions, or a combination thereof compared to the amino acid sequence of a wild-type reverse transcriptase or parent reverse transcriptase.

The reverse transcriptase may be derived from many different sources. Examples of the reverse transcriptase sources include, but are not limited to, Moloney murine leukemia virus (M-MLV or MLVRT), human T-cell leukemia virus type 1 (HTLV-1), bovine leukemia virus (BLV), Rous sarcoma virus (RSV), human immunodeficiency virus (HIV), yeasts such as *Saccharomyces, Neurospora,* and *Drosophila,* primates, and rodents.

Examples of the reverse transcriptase may include an avian myeloblastosis virus (AMV) reverse transcriptase, a reverse transcriptase originating from Moloney murine leukemia virus (M-MLV) (see [GERARD, GARY F., et al. "Influence on stability in Escherichia coli of the carboxy-terminal structure of cloned Moloney murine leukemia virus reverse transcriptase." Dna 5.4 (1986): 271-279.; and Kotewicz, Michael L., et al. "Cloning and overexpression of Moloney murine leukemia virus reverse transcriptase in Escherichia coli." Gene 35.3 (1985): 249-258.]), an M-MLV reverse transcriptase substantially lacking RNase H activity (see Application No. US 07/671,156, Publication No. US5244797A), a human immunodeficiency virus (HIV) reverse transcriptase, an avian sarcoma-leukosis virus (ASLV) reverse transcriptase, a Rous sarcoma virus (RSV) reverse transcriptase, an avian erythroblastosis virus (AEV) helper virus MCAV reverse transcriptase, an avian myelocytomatosis virus MC29 helper virus MCAV reverse transcriptase, an avian reticuloendotheliosis virus (REV-T) helper virus REV-A reverse transcriptase, an avian sarcoma virus UR2 helper virus UR2AV reverse transcriptase, an avian sarcoma virus Y73 helper virus YAV reverse transcriptase, a Rous-associated virus (RAV) reverse transcriptase, a myeloblastosis-associated virus (MAV) reverse transcriptase, variants thereof, or fragments thereof, but are not limited thereto. In some embodiments, the reverse transcriptase may be a retroviral reverse transcriptase. In some embodiments, the reverse transcriptase may be an error-prone reverse transcriptase. The "error-prone" reverse transcriptase (or any polymerase in a broader sense) refers to a reverse transcriptase derived from another reverse transcriptase that has an error rate lower than that of naturally occurring or wild-type M-MLV reverse transcriptases. The error-prone reverse transcriptase may have a higher error rate than the wild-type reverse transcriptases being compared. For example, an error rate of 6.7 × 10⁻⁵, 7.14 × 10⁻⁵, 7.7 × 10⁻⁵, 9.1 × 10⁻⁵, or 1 × 10⁻⁴ may be obtained. Reference may be made to [Bebenek, K., et al. "Error-prone polymerization by HIV-1 reverse transcriptase. Contribution of template-primer misalignment, miscoding, and termination probability to mutational hot spots." Journal of Biological Chemistry 268.14 (1993): 10324-10334.; and Sebastian-Martin, Alba, Veronica Barrioluengo, and Luis Menendez-Arias. "Transcriptional inaccuracy threshold attenuates differences in RNA-dependent DNA synthesis fidelity between retroviral reverse transcriptases." Scientific Reports 8.1 (2018) : 1-13.], the entire contents of each of which are incorporated herein by reference.

In some embodiments, the reverse transcriptase may be the M-MLV reverse transcriptase. The term M-MLV reverse transcriptase may be used to include variants and fragments thereof. Examples of the M-MLV reverse transcriptase may include wild-type M-MLV reverse transcriptases, M-MLV reverse transcriptase variants, wild-type M-MLV reverse transcriptase fragments, or fragments of wild-type M-MLV reverse transcriptase variants. For example, the M-MLV reverse transcriptase variant may have a form in which one or more amino acid residues selected from P51, S67, E69, L139, T197, D200, H204, F209, E302, E302, T306, F309, W313, T330, L345, L435, N454, D524, E562, D583, H594, L603, E607, and D653 of the wild-type M-MLV reverse transcriptases or other wild-type reverse transcriptases are substituted with another amino acid residue. The amino acid sequence of the wild-type M-MLV reverse transcriptase is disclosed in SEQ ID NO: 26. For example, the M-MLV reverse transcriptase variant may include any one or more amino acid variations selected from P51L, S67K, E69K, L139P, T197A, D200N, H204R, F209N, E302K, E302R, T306K, F309N, W313F, T330P, L345G, L435G, N454K, D524G, E562Q, D583N, H594Q, L603W, E607K, and D653N (where the sequence on which the amino acid variation is based is the amino acid sequence of the wild-type M-MLV reverse transcriptase of SEQ ID NO: 26). In certain embodiments, the reverse transcriptase may be an M-MLV reverse transcriptase variant including amino acid variations of D200N, T306K, W313F, T330P, and L603W (such as M-MLV reverse transcriptase pentamutant). In certain embodiments, the reverse transcriptase may be a terminally truncated M-MLV reverse transcriptase. In this case, the terminally truncated M-MLV reverse transcriptase may include four mutations (D200N, T306K, W313F, and T330P). In this case, the L603W mutation present in the above-described M-MLV reverse transcriptase pentamutant no longer exists due to terminal truncation. In some embodiments, the polymerase or reverse transcriptase may be codon optimized.

Reverse transcriptase (RT) genes (or the genetic information included therein) may be obtained from many different sources. For example, the genes may be obtained from retrovirus-infected eukaryotic cells or a variety of plasmids containing a part or all of the retroviral genome. Additionally, messenger RNA-like RNAs containing the RT gene may be obtained from retroviruses. Various examples of the reverse transcriptase that may be included in the prime editor protein are described in [U.S. Patent Application No. 17/219,672] in detail.

In some embodiments, the wild-type M-MLV reverse transcriptase may contain the following amino acid sequence of SEQ ID NO: 26:

TLNIEDEHRLHETSKEPDVSLGSTWLSDFPQAWAETGGMGLAVRQAPLIIPL
KATSTPVSIKQYPMSQEARLGIKPHIQRLLDQGILVPCQSPWNTPLLPVKKPGTNDYR
PVQDLREVNKRVEDIHPTVPNPYNLLSGLPPSHQWYTVLDLKDAFFCLRLHPTSQPLF
AFEWRDPEMGISGQLTWTRLPQGFKNSPTLFDEALHRDLADFRIQHPDLILLQYVDDL
LLAATSELDCQQGTRALLQTLGNLGYRASAKKAQICQKQVKYLGYLLKEGQRWLTEAR
KETVMGQPTPKTPRQLREFLGTAGFCRLWIPGFAEMAAPLYPLTKTGTLFNWGPDQQK
AYQEIKQALLTAPALGLPDLTKPFELFVDEKQGYAKGVLTQKLGPWRRPVAYLSKKLD
PVAAGWPPCLRMVAAIAVLTKDAGKLTMGQPLVILAPHAVEALVKQPPDRWLSNARMT
HYQALLLDTDRVQFGPVVALNPATLLPLPEEGLQHNCLDILAEAHGTRPDLTDQPLPD
ADHTWYTDGSSLLQEGQRKAGAAVTTETEVIWAKALPAGTSAQRAELIALTQALKMAE
GKKLNVYTDSRYAFATAHIHGEIYRRRGLLTSEGKEIKNKDEILALLKALFLPKRLSI
IHCPGHQKGHSAEARGNRMADQAARKAAITETPDTSTLLI (SEQ ID NO: 26).

In some embodiments, the wild-type M-MLV reverse transcriptase including D200N, T306K, W313F, T330P, and L603W variations may contain the following amino acid sequence of SEQ ID NO: 27:

TLNIEDEYRLHETSKEPDVSLGSTWLSDFPQAWAETGGMGLAVRQAPLIIPL
KATSTPVSIKQYPMSQEARLGIKPHIQRLLDQGILVPCQSPWNTPLLPVKKPGTNDYR
PVQDLREVNKRVEDIHPTVPNPYNLLSGLPPSHQWYTVLDLKDAFFCLRLHPTSQPLF

AFEWRDPEMGISGQLTWTRLPQGFKNSPTLFNEALHRDLADFRIQHPDLILLQYVDDL
LLAATSELDCQQGTRALLQTLGNLGYRASAKKAQICQKQVKYLGYLLKEGQRWLTEAR
KETVMGQPTPKTPRQLREFLGKAGFCRLFIPGFAEMAAPLYPLTKPGTLFNWGPDQQK
AYQEIKQALLTAPALGLPDLTKPFELFVDEKQGYAKGVLTQKLGPWRRPVAYLSKKLD
PVAAGWPPCLRMVAAIAVLTKDAGKLTMGQPLVILAPHAVEALVKQPPDRWLSNARMT
HYQALLLDTDRVQFGPVVALNPATLLPLPEEGLQHNCLDILAEAHGTRPDLTDQPLPD
ADHTWYTDGSSLLQEGQRKAGAAVTTETEVIWAKALPAGTSAQRAELIALTQALKMAE
GKKLNVYTDSRYAFATAHIHGEIYRRRGWLTSEGKEIKNKDEILALLKALFLPKRLSI
IHCPGHQKGHSAEARGNRMADQAARKAAITETPDTSTLLI (SEQ ID NO: 27).

### Elements that may be additionally included in prime editor protein

The prime editor protein includes the Cas protein and the polymerase (such as reverse transcriptases). In some embodiments, the prime editor protein may further include additional elements such as one or more linkers (such as linkers for linking elements included in prime editor proteins) and one or more nuclear localization sequences (NLSs), in addition to the two elements.

The prime editor protein may include one or more linkers. For example, the linkers may be used for linking the Cas protein to another structure included in the prime editor protein. The linker may be any linker known in the art. For example, the linker may be used for linking the polymerase to another structure included in the prime editor protein. For example, the linker may be used for linking the NLS to another structure included in the prime editor protein. For example, the linker may be used for linking the Cas protein and the polymerase. For example, the linker may be used for linking the linker to another linker selected independently. In some embodiments, the linker may be a covalent bond, organic molecule, group, polymer, or chemical moiety. In some embodiments, each linker may independently be selected. The linker may have a length of 3 to 100 or more amino acids. For example, the linker may be about 3, 4, 5, 6, 7, 8, 9, 10, 11, 12, 13, 14, 15, 16, 17, 18, 19, 20, 21, 22, 23, 24, 25, 26, 27, 28, 29, 30, 32, 34, 36, 38, 40, 42, 44, 46, 48, 50, 55, 60, 65, 70, 75, 80, 85, 90, 95, 100, 110, 120, 130, 140, 150, 160, 170, 180, 190, or 200 amino acids in length or may be of a length within a range set by any two values selected from the above-described values. In some embodiments, the linker may contain the following amino acid sequences: one or more G, one or more XP (where X is any amino acid), one or more EAAAK (SEQ ID NO: 35), one or more GGS (SEQ ID NO: 36), one or more SGGS (SEQ ID NO: 37), or one or more GGGGS (SEQ ID NO: 38). In some embodiments, the linker may contain, but are not particularly limited to, an amino acid sequence of SGSETPGTSESATPES (SEQ ID NO: 39) or SGGSSGGSSGSETPGTSESATPESSGGSSGGS (SEQ ID NO: 40). In some embodiments, the linker may be XTEN linker (for example, linker XTEN16). As described above, the prime editor protein may include one or more linkers, which may each independently be selected or determined. Various examples of the linker are described in [U.S. Patent Application No. 17/219,672] in detail.

The prime editor protein may include one or more NLSs. In some embodiments, the prime editor protein may include two or more NLSs. When the prime editor protein includes a plurality of NLSs, each NLS may independently be selected or determined. The NLS may be any NLS known in the art. The NLS may be any later discovered NLS for nuclear localization. The NLS may be any naturally occurring NLS or any unnaturally occurring NLS (for example, having one or more mutations). In some embodiments, the NLS may be, including but not limited to, : an SV40 virus large T-antigen NLS having an amino acid sequence of PKKKRKV (SEQ ID NO: 1); a bipartite SV40 NLS containing an amino acid sequence of KRTADGSEFESPKKKRKVE (SEQ ID NO: 18) (or a bipartite SV40 NLS including one amino acid deletion in a portion other than PKKKRKV); an NLS from nucleoplasmin (such as a nucleoplasmin bipartite NLS having a sequence of KRPAATKKAGQAKKKK (SEQ ID NO: 2)); a c-myc NLS having an amino acid sequence of PAAKRVKLD (SEQ ID NO: 3) or RQRRNELKRSP (SEQ ID NO: 4) ; a hRNPA1 M9 NLS having a sequence of NQSSNFGPMKGGNFGGRSSGPYGGGGQYFAKPRNQGGY (SEQ ID NO: 5); an IBB domain sequence of RMRIZFKNKGKDTAELRRRRVEVSVELRKAKKDEQILKRRNV (SEQ ID NO: 6) from importin-alpha; myoma T protein sequences of VSRKRPRP (SEQ ID NO: 7) and PPKKARED (SEQ ID NO: 8) a human p53 sequence of PQPKKKPL (SEQ ID NO: 9); a mouse c-abl IV sequence of SALIKKKKKKMAP (SEQ ID NO: 10); influenza virus NS sequences of DRLRR (SEQ ID NO: 11) and PKQKKRK (SEQ ID NO: 12); a hepatitis virus delta antigen sequence of RKLKKKIKKL (SEQ ID NO: 13); a mouse Mx1 protein sequence of REKKKFLKRR (SEQ ID NO: 14); a human poly(ADP-ribose) polymerase sequence of KRKGDEVDGVDEVAKKKSKK (SEQ ID NO: 15) ; or an NLS sequence derived from a steroid hormone receptor (human) glucocorticoid sequence of RKCLQAGMNLEARKTKK (SEQ ID NO: 16). In some embodiments, the NLS may be codon optimized.

Various examples of the NLS are described in [U.S. Patent Application No. 17/219,672] in detail.

### Prime editing guide RNA (pegRNA)

### Overview of pegRNA

As used herein, the term "prime editing guide RNA", "pegRNA", or "extended guide RNA" refers to the guide RNA in a form specialized to contain one or more additional sequences so that a prime editing method and a composition disclosed herein are implemented. The pegRNA is used with the prime editor protein in the prime editing system. As described herein, the pegRNA includes an extension arm or extension region. The extension arm may contain a single-stranded RNA sequence and/or DNA sequence, but is not limited thereto. As described above, guide RNAs used in traditional CRISPR/Cas systems (that is, guide RNAs free of the extension arm of pegRNAs) are called traditional guide RNAs and thus may be distinguished from the pegRNA. For example, the extension arm may be formed at the 3' end of a traditional guide RNA. For another example, the extension arm may be formed at the 5' end of the traditional guide RNA. In some embodiments, the pegRNA may include a spacer region, a gRNA core, and the extension arm formed at the 3' or 5' end of the traditional guide RNA.

### Extension arm

The term "extension arm" refers to a pegRNA nucleotide sequence portion that provides various functions, the portion including a DNA synthesis template (for example, including editing templates) and a primer binding site (PBS) for the polymerase (such as reverse transcriptases). In the pegRNA, the extension arm may be described as an extension region. In some embodiments, the extension arm may be located at the 3' end of the guide RNA. In some embodiments, the extension arm located at the 3' end of the guide RNA may be called a 3' extension arm. In other embodiments, the extension arm may be located at the 5' end of the guide RNA. In some embodiments, the extension arm located at the 5' end of the guide RNA may be called a 5' extension arm. In some embodiments, the extension arm may include a homology arm. In some embodiments, the extension arm may include an editing template. In some embodiments, the extension arm may include the primer binding site. In various embodiments, the extension arm (such as the 3' extension arm) may include the following elements in the 5' to 3' directions: the DNA synthesis template and the primer binding site. In other words, for the description based on the entire pegRNA, the pegRNA may include the following elements in the 5' to 3' directions: the spacer, the gRNA core, the DNA synthesis template, and the primer binding site. The DNA synthesis template may include the homology region and the editing template. In various embodiments, the extension arm may include the following elements in the 5' to 3' direction: the homology region, the editing template, and the primer binding site. In other words, for the description based on the entire pegRNA, the pegRNA may include the following elements in the 5' to 3' directions: the spacer, the gRNA core, the homology region, the editing template, and the primer binding site. In some embodiments, the 5' extension arm may include the following elements in the 5' to 3' direction: the DNA synthesis template and the primer binding site.

The polymerization activity of the reverse transcriptase, one example of the polymerase, is present in the 5' to 3' direction, relative to a strand to be ultimately bound to a template strand. Once a primer is annealed to the primer binding site (PBS), the reverse transcriptase polymerizes a single strand of DNA using a complementary template strand (DNA synthesis template) as a template of reverse transcription. Various embodiments of the extension arm used in prime editing are described in [U.S. Patent Application No. 17/219,672] in detail.

Typically, the extension arm of the pegRNA may, for example, be described as including two regions: the primer binding site (PBS) and the DNA synthesis template (such as reverse transcription templates). For example, in PE2, the primer binding site binds to a primer sequence formed from an endogenous DNA strand at a nicking target site resulting from the prime editor protein, thereby exposing the 3' end of the strand subjected to nicking. As described herein, the binding of the primer sequence to the primer binding site in the extension arm of the pegRNA generates a duplex region having the exposed 3' end (that is, the 3' end of the primer sequence), which subsequently provides a matrix enabling the reverse transcriptase to polymerize the single strand of DNA from the exposed 3' end along the length of the DNA synthesis template. The sequence of the resulting single-stranded DNA product is a complement of the DNA synthesis template. Polymerization continues toward the 5' of the DNA synthesis template (or extension arm) until polymerization terminates. Accordingly, the DNA synthesis template is encoded into the resulting single-stranded DNA product (that is, a 3' single-stranded DNA flap containing the desired gene editing information) by the polymerase of the prime editor protein. As a result, the 3' single-stranded DNA flap (for example, complementary to the DNA synthesis template) to replace the endogenous DNA strand corresponding to the target region located immediately downstream of the PE-induced nick site is formed. The polymerization of the DNA synthesis template may continue toward the 5' end of the extension arm until polymerization terminates, but is not limited thereto. The polymerization may terminate through various manners including, but not limited to, (a) reaching the 5' end of the pegRNA, (b) reaching an impassable RNA secondary structure (such as hairpins or stem/loops), or (c) reaching a replication termination signal, such as specific nucleotides sequence to block or inhibit polymerases, or a nucleic acid phase signal, such as supercoiled DNA or RNA. However, termination of the polymerization is not limited thereto. Reports have been made in some prime editing-related documents that sequences homologous to a portion of the gRNA core of the pegRNA are found in the 3' DNA flap or editing sites. On this basis, it will be understood by those skilled in the art that the above-described embodiments are merely examples, and termination of polymerization is not limited to the above-described embodiments.

### Primer binding site (PBS)

In the prime editing system, information present in the DNA synthesis template included in the pegRNA is delivered to the endogenous strand through polymerization by the polymerase. Polymerization by the polymerase requires a primer to be bound to a template strand, and binding or annealing of the primer enables DNA polymerization. In the prime editing system, a portion of the region where the DSB or nick is made, induced by the Cas protein, is used as the primer. For example, for the description based on PE2, a portion of the region located upstream of the nick in the spacer non-binding strand, induced by the Cas protein of the prime editor protein, is used as the primer. In this case, the region designed to complementarily bind to a sequence of the region located upstream of the nick is called the primer binding site, and the primer binding site is located in the extension region of the pegRNA. Hereinbelow, the prime editing process in PE2 is to be additionally described. Once the primer binding site and the region used as the primer of the endogenous DNA (such as genomes) are bound, reverse transcription is performed by the reverse transcriptase using the primer as the template of reverse transcription. In this case, it will be apparent to those skilled in the art that the reverse transcription is performed in the 3' to 5' direction relative to the reverse transcription template strand (that is, the pegRNA). When reverse transcription is performed, a sequence complementary to a sequence of the DNA template is contained in the 3' flap of the genome DNA, meaning that information on the DNA template is delivered to the 3' flap by reverse transcription. Then, through processes including 5' flap removal and a process including cellular DNA repair and/or replication, the information on the DNA template is ultimately delivered to another desired strand of DNA to be edited. The desired result of prime editing is to deliver or install the information on the DNA template into a first strand (in which case the first strand is the spacer non-binding strand) and/or a second strand (in which case the second strand is the spacer binding strand) at the desired site to be edited. In other words, as a result of the exemplary PE2 prime editing, the DNA sequence complementary to the sequence of the DNA template is present at the desired site in the first strand, and the DNA sequence same as the sequence of the DNA template sequence is present at the desired site in the second strand.

In some embodiments, the primer binding site of the pegRNA may be designed as a sequence complementary to the sequence of the region located upstream of the site where the nick or DSB is made (such as genome DNA) in a DNA molecule. In some embodiments, the primer binding site may be designed as a sequence complementary to the sequence of the region located upstream of the site where the nick or DSB is made in the spacer non-binding strand of a DNA molecule. In other words, the sequence of the region located upstream of the site where the nick or DBS is made in the spacer non-binding strand of the DNA molecule may function as the primer in the prime editing process. As described above, in one example of PE2, the sequence located in the 5' direction of the nick functions as the primer, and the nick end of the DNA molecule is exposed to the reverse transcriptase through the binding of the primer to the primer binding site.

In some embodiments, the primer may have a length of 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt or greater, or may have a length within a range formed by two values selected from the above-described values. In certain embodiments, the primer may have a length of 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, or 25 nt, or may have a length within a range formed by two values selected from the above-described values.

In some embodiments, the primer binding site may have a length of 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt or greater, or may have a length within a range formed by two values selected from the above-described values. In certain embodiments, the primer binding site may have a length of 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, or 25 nt, or may have a length within a range formed by two values selected from the above-described values. The length of the primer binding site may be selected appropriately depending on the purpose and is not particularly limited.

### DNA synthesis template

As used herein, the term "DNA synthesis template" in prime editing refers to a region or portion used as the template strand by the polymerase of the prime editor protein to encode the 3' single-stranded DNA flap including the desired editing. Furthermore, the term DNA synthesis template refers to a region or portion included in the extension region of the pegRNA, the region or portion replacing the corresponding endogenous DNA strand at a target site through a prime editing mechanism. Various embodiments of the DNA synthesis template and the extension region of the pegRNA are described in [U.S. Patent Application No. 17/219,672] in detail, the entire contents of which are incorporated herein by reference.

The extension region including the DNA synthesis template may be composed of DNA, RNA, or a DNA/RNA hybrid. In the case of RNA, the polymerase of the prime editor protein may be an RNA-dependent DNA polymerase (such as reverse transcriptases). The DNA synthesis template may be called a DNA polymerization template or reverse transcription template (RT template), in which case the RT template is intended for use of the reverse transcriptase in the prime editing system. In the case of DNA, the polymerase of the prime editor may be a DNA-dependent DNA polymerase. In various embodiments, the DNA synthesis template (such as RT templates) may include the "editing template" and the "homology region".

In some embodiments, the DNA synthesis template may include a part or all of the optional 5' end modifier region e2, in addition to the editing template and the homology region. Depending on the nature of region e2 (for example, whether secondary structures such as hairpins, T-loops, or stem/loops are included), the polymerase may encode none, some, or all of the e2 region. In some embodiments, in the case of the 3' extension arm, the DNA synthesis template may include a portion of the extension arm, covering from the 5' end of the primer binding site (PBS) to the 3' end of the gRNA core. In other embodiments, in the case of the 5' extension arm, the DNA synthesis template may include a portion of the extension arm, covering from the 5' end of the pegRNA molecule to the 3' end of the primer binding site. Preferably, the DNA synthesis template excludes of the primer binding site (PBS) of the pegRNA having the 3' extension arm or 5' extension arm.

In certain embodiments described herein, the DNA synthesis template may be called the "reverse transcription template (RT template)" including the editing template and the homology arm. The RT template may refer to a portion of the sequence of the pegRNA extension arm used as a template in DNA synthesis. The term "RT template" may be used equivalently to the DNA synthesis template.

In the case of trans prime editing, the primer binding site (PBS) and the DNA synthesis template may be engineered using individual molecules called trans prime editing RNA templates (tPERT) (see [U.S. Patent Application No. 17/219, 672]) .

### DNA synthesis template element 1 - Editing template

The term "editing template" refers to a portion of the extension arm, encoding the desired editing into the single-stranded 3' DNA flap synthesized by the polymerase, such as DNA-dependent DNA polymerases and RNA-dependent DNA polymerases (such as reverse transcriptases). In other words, the editing template may be complementary to the desired editing. In some embodiments, the DNA synthesis template may include the editing template and the homology arm. In some embodiments, the RT template may include the editing template and the homology arm. The term "RT template" is equivalent to the DNA synthesis template. However, the RT template herein is based on the use of the prime editor protein having the polymerase, that is, the reverse transcriptase, and the DNA synthesis template is further widely based on the use of a prime editor protein having any polymerase.

The desired editing to be installed in the target region of an editing-subject DNA molecule (such as genomes) may include any one or a combination of insertions of one or more nucleotides, deletions of one or more nucleotides, and substitutions of one or more nucleotides with other nucleotides. For example, the editing may include insertions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be inserted may or may not be located in succession in a nucleic acid. For example, the editing may include deletions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be deleted may or may not be located in succession in a nucleic acid. For example, the editing may include substitutions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be substituted may or may not be located in succession in a nucleic acid. For another example, the editing may include the above-described insertions and substitutions. For another example, the editing may include the above-described deletions and substitutions. For another example, the editing may include the above-described insertions and deletions. For another example, the editing may include the above-described insertions, deletions, and substitutions.

### DNA synthesis template element 2 - Homology arm (or homology region)

The term "homology arm" refers to a portion of the extension arm, to be integrated into a target DNA region through the replacement of the endogenous strand. For example, in PE2 prime editing, the term "homology arm" may refer to a portion of the extension arm encoding the single-stranded DNA flap portion encoded by the reverse transcriptase. For example, in the PE2 system, a portion of the single-stranded DNA flap to be encoded by the homology arm is complementary to the unedited strand (such as spacer binding strands) of the target DNA. In other words, in PE2, a sequence of the homology arm has a sequence complementary to a corresponding sequence located in the spacer non-binding strand of the target DNA and has a sequence that is practically the same as a corresponding DNA sequence located in the spacer binding strand. The homology arm replaces the endogenous strand and facilitates annealing of the single-stranded DNA flap, thus helping install the editing in the target DNA molecule. The homology arm, by definition, is encoded by the polymerase of the prime editor disclosed herein and thus is a portion of the DNA synthesis template.

### Additional elements that may be included in pegRNA and engineered pegRNA (epegRNA)

An engineered pegRNA (epegRNA) is described with reference to [Nelson, James W., et al. "Engineered pegRNAs improve prime editing efficiency." Nature biotechnology 40.3 (2022): 402-410.], the entire contents of which are incorporated herein by reference. The epegRNA, one type of pegRNA, may be used to refer to an improved pegRNA. Specifically, the epegRNA refers to a pegRNA having a form in which an RNA motif is added to the 3' or 5' end of the pegRNA. In some embodiments, the epegRNA may be a pegRNA having a form in which the RNA motif (or an engineered RNA motif) is added to the 3' end. The epegRNA may, for example, include the following elements in the 5' to 3' direction: the spacer, the gRNA core, the DNA synthesis template, the primer binding site, and the RNA motif.

David R. Liu et al. developed the engineered pegRNA (epegRNAs) with the RNA motif added to the 3' end of the pegRNA to improve the stability of the pegRNA and prevent the 3' extension region of the pegRNA from degradation. Specifically, in the above document, David R. Liu et al. disclosed an epegRNA in which a stable pseudoknot is additionally integrated into the 3' end of a traditional pegRNA. Examples of the pseudoknot include evopreQ₁ (modified prequeosine1-1 riboswitch aptamer) and mpknot (frameshifting pseudoknot from Moloney murine leukemia virus) described in [Nelson, James W., et al. "Engineered pegRNAs improve prime editing efficiency." Nature biotechnology 40.3 (2022): 402-410.], but are not limited thereto.

The epegRNA may be used regardless of the type of prime editor protein used. For example, the epegRNA may be used with the prime editor protein including an SpCas9 nickase of Prime Editing Version 2 (PE2). For another example, the epegRNA may be used with PE-nuclease containing Cas9 having nuclease activity (that is, DSB activity) to edit a DNA molecule (such as genomes). The term pegRNA used herein includes embodiments of the epegRNA, and descriptions for the pegRNA may be interpreted as including content related to the epegRNA unless otherwise specified.

In some embodiments, the pegRNA may further include a 3' engineering region at the 3' end. The pegRNA including the 3' engineering region may be called the epegRNA. In other words, the epegRNA may further include the 3' engineering region, in addition to the elements of the pegRNA. In some embodiments, the 3' engineering region may include an RNA protection motif. In certain embodiments, the RNA protection motif may contain an RNA sequence. In certain embodiments, the RNA protection motif may contain a DNA sequence. In certain embodiments, the RNA protection motif may contain a DNA/RNA hybrid sequence. In certain embodiments, the RNA protection motif may include evopreQ1 or mpknot, but is not limited thereto, and may include any other structure to prevent RNA from degradation and increase stability.

In some embodiments, the 3' engineering region may include the RNA protection motif and a linker for linking the RNA protection motif. The linker serves to link the RNA protection motif and the primer binding site in the epegRNA. In some embodiments, the linker for linking the RNA protection motif may contain an RNA sequence. In some embodiments, the linker for linking the RNA protection motif may contain a DNA sequence. In some embodiments, the linker for linking the RNA protection motif may be 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40 nt or greater in length, or may be of a length within a range set by any two values selected from the above-described values. In some embodiments, the linker for linking the RNA protection motif may be designed to avoid base pairing interactions between the linker and the PBS or between the linker and the spacer of the pegRNA. In some embodiments, the sequence of the linker for linking the RNA protection motif may be designed in consideration of the sequence in the target region of the target DNA molecule.

Hereinbelow, various prime editing versions developed on the basis of the prime editor protein and the pegRNA, the basic elements of prime editing, are to be illustrated. Prime editing is not limited to the versions illustrated below.

### Examples of prime editing versions

### Overview of examples of prime editing versions

Various prime editing versions have been developed on the basis of the above-described core mechanism of prime editing. For the benefit of technologists in the art, examples of prime editing versions are to be explained. A method for finding off-targets in prime editing, provided by the present application, may use prime editor proteins in many prime editing versions illustrated below, many types of pegRNAs including epegRNAs, and/or additional elements such as a dnMLH1, but are not particularly limited. Furthermore, the method for finding off-targets in prime editing, provided by the present application, may also be applied to the prime editing versions illustrated below and to new prime editing versions to be developed later. Thus, it should be noted that the prime editing versions illustrated below do not limit the range of application of methods provided by the present application.

### Prime Editing Version 1 (PE1)

Prime Editing Version 1 (PE1) describes a prime editing system version including the use of the following elements:
a prime editor protein including SpCas9 (H840A) and a wild-type Moloney murine leukemia virus reverse transcriptase (MMLV RT); and
a pegRNA.

In other words, the PE1 prime editor protein includes the Cas protein having nickase activity and the wild-type MMLV RT. The PE1 prime editor protein has a form of a fusion protein in which the Cas protein and the reverse transcriptase are linked via a linker.

The PE1 prime editor protein and the pegRNA form a complex, thereby directing or performing the editing of a DNA molecule (such as genome editing) in the target region. PE1 is described in [Anzalone, Andrew V., et al. "Search-and-replace genome editing without double-strand breaks or donor DNA." Nature 576.7785 (2019): 149-157.] in detail.

### Prime Editing Version 2 (PE2)

Prime Editing Version 2 (PE2) describes a prime editing system version including the use of the following elements:
a prime editor protein including SpCas9 (H840A) and an MMLV RT (D200N + L603W + T330P + T306K + W313F); and
a pegRNA.

In other words, the PE2 prime editor protein includes the Cas protein having nickase activity and the MMLV RT pentamutant. The PE2 prime editor protein has a form of a fusion protein in which the Cas protein and the reverse transcriptase are linked via a linker. Specifically, the PE2 prime editor protein has the following structure:

[bpNLS(SV40)]-[SpCas9 H840A]-[SGGSX2-XTEN16-SGGSX2]-[MMLV RT pentamutant]-[bpNLS(SV40)].

In this case, bpNLS(SV40) refers to a bipartite SV40 NLS. The MMLV RT pentamutant refers to an MMLV RT variant including amino acid variations of D200N, L603W, T330P, T306K, and W313F, compared to the wild-type MMLV RT.

The PE2 prime editing system is described in [Anzalone, Andrew V., et al. "Search-and-replace genome editing without double-strand breaks or donor DNA." Nature 576.7785 (2019): 149-157.; and Chen, Peter J., et al. "Enhanced prime editing systems by manipulating cellular determinants of editing outcomes." Cell 184.22 (2021): 5635-5652.] in detail. In some embodiments, the PE2 prime editor protein may contain an amino acid sequence of SEQ ID NO: 32.

The amino acid sequence of SEQ ID NO: 32 is as follows:

MKRTADGSEFESPKKKRKVDKKYSIGLDIGTNSVGWAVITDEYKVPSKKFKVLGNTDR
HSIKKNLIGALLFDSGETAEATRLKRTARRRYTRRKNRICYLQEIFSNEMAKVDDSFF
HRLEESFLVEEDKKHERHPIFGNIVDEVAYHEKYPTIYHLRKKLVDSTDKADLRLIYL
ALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYD
DDLDNLLAQIGDQYADLFLAAKNLSDAILLSDILRVNTEITKAPLSASMIKRYDEHHQ
DLTLLKALVRQQLPEKYKEIFFDQSKNGYAGYIDGGASQEEFYKFIKPILEKMDGTEE
LLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIV
LTLTLFEDREMIEERLKTYAHLFDDKVMKQLKRRRYTGWGRLSRKLINGIRDKQSGKT

ILDFLKSDGFANRNFMQLIHDDSLTFKEDIQKAQVSGQGDSLHEHIANLAGSPAIKKG
ILQTVKVVDELVKVMGRHKPENIVIEMARENQTTQKGQKNSRERMKRIEEGIKELGSQ
ILKEHPVENTQLQNEKLYLYYLQNGRDMYVDQELDINRLSDYDVDAIVPQSFLKDDSI
DNKVLTRSDKNRGKSDNVPSEEVVKKMKNYWRQLLNAKLITQRKFDNLTKAERGGLSE
LDKAGFIKRQLVETRQITKHVAQILDSRMNTKYDENDKLIREVKVITLKSKLVSDFRK
DFQFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPK
YSLFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLT
NLGAPAAFKYFDTTIDRKRYTSTKEVLDATLIHQSITGLYETRIDLSQLGGDSGGSSG
GSSGSETPGTSESATPESSGGSSGGSSTLNIEDEYRLHETSKEPDVSLGSTWLSDFPQ
AWAETGGMGLAVRQAPLIIPLKATSTPVSIKQYPMSQEARLGIKPHIQRLLDQGILVP
CQSPWNTPLLPVKKPGTNDYRPVQDLREVNKRVEDIHPTVPNPYNLLSGLPPSHQWYT
VLDLKDAFFCLRLHPTSQPLFAFEWRDPEMGISGQLTWTRLPQGFKNSPTLFNEALHR
DLADFRIQHPDLILLQYVDDLLLAATSELDCQQGTRALLQTLGNLGYRASAKKAQICQ
KQVKYLGYLLKEGQRWLTEARKETVMGQPTPKTPRQLREFLGKAGFCRLFIPGFAEMA
APLYPLTKPGTLFNWGPDQQKAYQEIKQALLTAPALGLPDLTKPFELFVDEKQGYAKG
VLTQKLGPWRRPVAYLSKKLDPVAAGWPPCLRMVAAIAVLTKDAGKLTMGQPLVILAP
HAVEALVKQPPDRWLSNARMTHYQALLLDTDRVQFGPVVALNPATLLPLPEEGLQHNC
LDILAEAHGTRPDLTDQPLPDADHTWYTDGSSLLQEGQRKAGAAVTTETEVIWAKALP
AGTSAQRAELIALTQALKMAEGKKLNVYTDSRYAFATAHIHGEIYRRRGWLTSEGKEI
KNKDEILALLKALFLPKRLSIIHCPGHQKGHSAEARGNRMADQAARKAAITETPDTST
LLIENSSPSGGSKRTADGSEFEPKKKRKV (SEQ ID NO: 32).

### Prime Editing Version 3 (PE3)

A PE3 prime editing system refers to a prime editing version developed for increasing prime editing efficiency by nicking an unedited strand (that is, a strand binding to a spacer of a pegRNA) using a second strand nicking guide RNA. The second strand guide RNA may be designed in the form of a traditional gRNA (such as sgRNAs) to nick the unedited strand at a nearby site of an editing site or target site. In some embodiments, PE3 may include the use of a separate Cas9 nickase in addition to the prime editing protein.

Although PE3b refers to PE3, the second strand nicking guide RNA is designed for temporal control such that the second strand nick is not introduced until the desired editing is installed. This is achieved by designing a gRNA having a sequence of the spacer, which matches only the edited strand and not the original allele. PE3 and PE3b are described in [Anzalone, Andrew V., et al. "Search-and-replace genome editing without double-strand breaks or donor DNA." Nature 576.7785 (2019): 149-157.] in detail.

### Prime Editing Version 4 (PE4)

Prime Editing Version 4 (PE4) includes the use of the same machinery as PE2 but further includes the use of a plasmid encoding dominant negative MLH1 (dnMLH1) or the use of the dnMLH1. For example, PE4 may be recognizable as including the use of the following elements:
the PE2 prime editing protein;
a pegRNA; and
a dominant negative MLH1 (dnMLH1).

[Chen, Peter J., et al. "Enhanced prime editing systems by manipulating cellular determinants of editing outcomes." Cell 184.22 (2021): 5635-5652.] describes that the dominant negative MLH1 may knock out endogenous MLH1 by inhibition, thereby reducing cellular measles-mumps-rubella (MMR) responses and increasing prime editing efficiency.

### Prime Editing Version 5 (PE5)

Prime Editing Version 5 (PE5) includes the use of the same machinery as PE3 but further includes the use of a plasmid encoding dominant negative MLH1 (dnMLH1) or the use of the dnMLH1. PE5 is described in [Chen, Peter J., et al. "Enhanced prime editing systems by manipulating cellular determinants of editing outcomes." Cell 184.22 (2021): 5635-5652.] in detail.

### PEmax

PEmax is an improved prime editing version developed to increase editing efficiency. A PEmax prime editor protein includes a SpCas9 variant and an MMLV RT variant. Specifically, the PEmax prime editor protein has the following structure:

[bpNLS(SV40)]-[SpCas9 R221K N394K H840A]-[SGGSX2-bpNLS(SV40)-SGGSX2]-[MMLV RT pentamutant (codon opt.)]-[bpNLS(SV40)]-[NLS(c-Myc)].

In this case, bpNLS(SV40) refers to a bipartite SV40 NLS. The MMLV RT pentamutant (codon opt.) refers to a human codon-optimized MMLV RT variant including amino acid variations of D200N, L603W, T330P, T306K, and W313F, compared to the wild-type MMLV RT. "SpCas9 R221K N394K H840A" refers to a SpCas9 variant including amino acid variations of R221K, N394K, and H840A, compared to wild-type SpCas9. NLS(c-Myc) refers to a c-Myc NLS. PEmax is described in [Chen, Peter J., et al. "Enhanced prime editing systems by manipulating cellular determinants of editing outcomes." Cell 184.22 (2021): 5635-5652.] in detail. Furthermore, the above document discloses various versions of prime editor proteins, such as a PE2* prime editor protein, a CMP-PE-V1 prime editor protein, and a CMP-PEmax prime editor protein, all of which may be used in the off-target prediction system in prime editing provided by the present application.

### Nuclease-based prime editing

Nuclease-based prime editing, one version of prime editing, uses a Cas protein (such as wild-type SpCas9 or SpCas9 variants other than nickases) having nuclease activity (that is, DSB activity) instead of Cas9 (H840A) nickases. A prime editor protein for Nuclease-based prime editing may be called a PE nuclease. Unlike PE3, designed to cause nick in the strand binding to the spacer of the pegRNA, the use of two types of gRNA is not essential. Through the prime editing protein including one type of pegRNA and Cas nuclease (other than nickases), a DSB is made at a desired site, thus inducing editing. Nuclease-based prime editing is described in [Adikusuma, Fatwa, et al. "Optimized nickase-and nuclease-based prime editing in human and mouse cells." Nucleic acids research 49.18 (2021): 10785-10795.] in detail, the entire contents of which are incorporated herein by reference. One example of the PE nuclease is PE2-nuclease. PE2-nuclease has the following structure: [bpNLS(SV40)]-[SpCas9 (WT)]-[SGGSx2-XTEN16-SGGSx2]-[MMLV RT]-[bpNLS(SV40)].

In some embodiments, PE2-nuclease may contain an amino acid sequence of SEQ ID NO: 33.

The amino acid sequence of SEQ ID NO: 33 is as follows:

MKRTADGSEFESPKKKRKVDKKYSIGLDIGTNSVGWAVITDEYKVPSKKFKVLGNTDR

HSIKKNLIGALLFDSGETAEATRLKRTARRRYTRRKNRICYLQEIFSNEMAKVDDSFF
HRLEESFLVEEDKKHERHPIFGNIVDEVAYHEKYPTIYHLRKKLVDSTDKADLRLIYL
ALAHMIKFRGHFLIEGDLNPDNSDVDKLFIQLVQTYNQLFEENPINASGVDAKAILSA
RLSKSRRLENLIAQLPGEKKNGLFGNLIALSLGLTPNFKSNFDLAEDAKLQLSKDTYD
DDLDNLLAQIGDQYADLFLAAKNLSDAILLSDILRVNTEITKAPLSASMIKRYDEHHQ
DLTLLKALVRQQLPEKYKEIFFDQSKNGYAGYIDGGASQEEFYKFIKPILEKMDGTEE
LLVKLNREDLLRKQRTFDNGSIPHQIHLGELHAILRRQEDFYPFLKDNREKIEKILTF
RIPYYVGPLARGNSRFAWMTRKSEETITPWNFEEVVDKGASAQSFIERMTNFDKNLPN
EKVLPKHSLLYEYFTVYNELTKVKYVTEGMRKPAFLSGEQKKAIVDLLFKTNRKVTVK
QLKEDYFKKIECFDSVEISGVEDRFNASLGTYHDLLKIIKDKDFLDNEENEDILEDIV
LTLTLFEDREMIEERLKTYAHLFDDKVMKQLKRRRYTGWGRLSRKLINGIRDKQSGKT
ILDFLKSDGFANRNFMQLIHDDSLTFKEDIQKAQVSGQGDSLHEHIANLAGSPAIKKG
ILQTVKVVDELVKVMGRHKPENIVIEMARENQTTQKGQKNSRERMKRIEEGIKELGSQ
ILKEHPVENTQLQNEKLYLYYLQNGRDMYVDQELDINRLSDYDVDHIVPQSFLKDDSI
DNKVLTRSDKNRGKSDNVPSEEVVKKMKNYWRQLLNAKLITQRKFDNLTKAERGGLSE
LDKAGFIKRQLVETRQITKHVAQILDSRMNTKYDENDKLIREVKVITLKSKLVSDFRK
DFQFYKVREINNYHHAHDAYLNAVVGTALIKKYPKLESEFVYGDYKVYDVRKMIAKSE
QEIGKATAKYFFYSNIMNFFKTEITLANGEIRKRPLIETNGETGEIVWDKGRDFATVR
KVLSMPQVNIVKKTEVQTGGFSKESILPKRNSDKLIARKKDWDPKKYGGFDSPTVAYS
VLVVAKVEKGKSKKLKSVKELLGITIMERSSFEKNPIDFLEAKGYKEVKKDLIIKLPK
YSLFELENGRKRMLASAGELQKGNELALPSKYVNFLYLASHYEKLKGSPEDNEQKQLF
VEQHKHYLDEIIEQISEFSKRVILADANLDKVLSAYNKHRDKPIREQAENIIHLFTLT
NLGAPAAFKYFDTTIDRKRYTSTKEVLDATLIHQSITGLYETRIDLSQLGGDSGGSSG
GSSGSETPGTSESATPESSGGSSGGSSTLNIEDEYRLHETSKEPDVSLGSTWLSDFPQ
AWAETGGMGLAVRQAPLIIPLKATSTPVSIKQYPMSQEARLGIKPHIQRLLDQGILVP

CQSPWNTPLLPVKKPGTNDYRPVQDLREVNKRVEDIHPTVPNPYNLLSGLPPSHQWYT
VLDLKDAFFCLRLHPTSQPLFAFEWRDPEMGISGQLTWTRLPQGFKNSPTLFNEALHR
DLADFRIQHPDLILLQYVDDLLLAATSELDCQQGTRALLQTLGNLGYRASAKKAQICQ
KQVKYLGYLLKEGQRWLTEARKETVMGQPTPKTPRQLREFLGKAGFCRLFIPGFAEMA
APLYPLTKPGTLFNWGPDQQKAYQEIKQALLTAPALGLPDLTKPFELFVDEKQGYAKG
VLTQKLGPWRRPVAYLSKKLDPVAAGWPPCLRMVAAIAVLTKDAGKLTMGQPLVILAP
HAVEALVKQPPDRWLSNARMTHYQALLLDTDRVQFGPVVALNPATLLPLPEEGLQHNC
LDILAEAHGTRPDLTDQPLPDADHTWYTDGSSLLQEGQRKAGAAVTTETEVIWAKALP
AGTSAQRAELIALTQALKMAEGKKLNVYTDSRYAFATAHIHGEIYRRRGWLTSEGKEI
KNKDEILALLKALFLPKRLSIIHCPGHQKGHSAEARGNRMADQAARKAAITETPDTST
LLIENSSPSGGSKRTADGSEFEPKKKRKV (SEQ ID NO: 33).

### PEmax-nuclease

PEmax-nuclease, a nuclease-based prime editor protein developed on the basis of the PEmax prime editor protein (that is, one type of PE-nuclease), is a prime editor protein including a Cas protein having nuclease activity (that is, DSB activity) other than nickase activity. PEmax-nuclease has the following structure:

[bpNLS(SV40)]-[SpCas9 R221K N394K]-[SGGSX2-bpNLS(SV40)-SGGSX2]-[MMLV RT pentamutant (codon opt.)]-[bpNLS(SV40)]-[NLS(c-Myc)].

In some embodiments, PEmax-nuclease may contain an amino acid sequence of SEQ ID NO: 34.

The amino acid sequence of SEQ ID NO: 34 is as follows:

MKRTADGSEFESPKKKRKVDKKYSIGLDIGTNSVGWAVITDEYKVPSKKFKV
LGNTDRHSIKKNLIGALLFDSGETAEATRLKRTARRRYTRRKNRICYLQEIFSNEMAK
VDDSFFHRLEESFLVEEDKKHERHPIFGNIVDEVAYHEKYPTIYHLRKKLVDSTDKAD
LRLIYLALAHMIKFRGHFLIEGDLNPDNSDVDKLFIQLVQTYNQLFEENPINASGVDA
KAILSARLSKSRKLENLIAQLPGEKKNGLFGNLIALSLGLTPNFKSNFDLAEDAKLQL
SKDTYDDDLDNLLAQIGDQYADLFLAAKNLSDAILLSDILRVNTEITKAPLSASMIKR
YDEHHQDLTLLKALVRQQLPEKYKEIFFDQSKNGYAGYIDGGASQEEFYKFIKPILEK
MDGTEELLVKLKREDLLRKQRTFDNGSIPHQIHLGELHAILRRQEDFYPFLKDNREKI
EKILTFRIPYYVGPLARGNSRFAWMTRKSEETITPWNFEEVVDKGASAQSFIERMTNF
DKNLPNEKVLPKHSLLYEYFTVYNELTKVKYVTEGMRKPAFLSGEQKKAIVDLLFKTN
RKVTVKQLKEDYFKKIECFDSVEISGVEDRFNASLGTYHDLLKIIKDKDFLDNEENED
ILEDIVLTLTLFEDREMIEERLKTYAHLFDDKVMKQLKRRRYTGWGRLSRKLINGIRD
KQSGKTILDFLKSDGFANRNFMQLIHDDSLTFKEDIQKAQVSGQGDSLHEHIANLAGS
PAIKKGILQTVKVVDELVKVMGRHKPENIVIEMARENQTTQKGQKNSRERMKRIEEGI
KELGSQILKEHPVENTQLQNEKLYLYYLQNGRDMYVDQELDINRLSDYDVDHIVPQSF
LKDDSIDNKVLTRSDKNRGKSDNVPSEEVVKKMKNYWRQLLNAKLITQRKFDNLTKAE
RGGLSELDKAGFIKRQLVETRQITKHVAQILDSRMNTKYDENDKLIREVKVITLKSKL
VSDFRKDFQFYKVREINNYHHAHDAYLNAVVGTALIKKYPKLESEFVYGDYKVYDVRK
MIAKSEQEIGKATAKYFFYSNIMNFFKTEITLANGEIRKRPLIETNGETGEIVWDKGR
DFATVRKVLSMPQVNIVKKTEVQTGGFSKESILPKRNSDKLIARKKDWDPKKYGGFDS
PTVAYSVLVVAKVEKGKSKKLKSVKELLGITIMERSSFEKNPIDFLEAKGYKEVKKDL
IIKLPKYSLFELENGRKRMLASAGELQKGNELALPSKYVNFLYLASHYEKLKGSPEDN
EQKQLFVEQHKHYLDEIIEQISEFSKRVILADANLDKVLSAYNKHRDKPIREQAENII
HLFTLTNLGAPAAFKYFDTTIDRKRYTSTKEVLDATLIHQSITGLYETRIDLSQLGGD

SGGSSGGSKRTADGSEFESPKKKRKVSGGSSGGSTLNIEDEYRLHETSKEPDVSLGST
WLSDFPQAWAETGGMGLAVRQAPLIIPLKATSTPVSIKQYPMSQEARLGIKPHIQRLL
DQGILVPCQSPWNTPLLPVKKPGTNDYRPVQDLREVNKRVEDIHPTVPNPYNLLSGLP
PSHQWYTVLDLKDAFFCLRLHPTSQPLFAFEWRDPEMGISGQLTWTRLPQGFKNSPTL
FNEALHRDLADFRIQHPDLILLQYVDDLLLAATSELDCQQGTRALLQTLGNLGYRASA
KKAQICQKQVKYLGYLLKEGQRWLTEARKETVMGQPTPKTPRQLREFLGKAGFCRLFI
PGFAEMAAPLYPLTKPGTLFNWGPDQQKAYQEIKQALLTAPALGLPDLTKPFELFVDE
KQGYAKGVLTQKLGPWRRPVAYLSKKLDPVAAGWPPCLRMVAAIAVLTKDAGKLTMGQ
PLVILAPHAVEALVKQPPDRWLSNARMTHYQALLLDTDRVQFGPVVALNPATLLPLPE
EGLQHNCLDILAEAHGTRPDLTDQPLPDADHTWYTDGSSLLQEGQRKAGAAVTTETEV
IWAKALPAGTSAQRAELIALTQALKMAEGKKLNVYTDSRYAFATAHIHGEIYRRRGWL
TSEGKEIKNKDEILALLKALFLPKRLSIIHCPGHQKGHSAEARGNRMADQAARKAAIT
ETPDTSTLLIENSSPSGGSKRTADGSEFESPKKKRKVGSGPAAKRVKLD (SEQ ID
NO: 34).

### Use of epegRNA

As described above, an epegRNA is an improved pegRNA version. The pegRNA used in the above-described prime editing systems may be the epegRNA or the pegRNA other than epegRNA, but is not particularly limited.

### Genome editing process by prime editing system

For the benefit of technologists in the art, a process of editing the genome of a cell by a prime editing system is to be explained through the example of PE2. One example of the process of editing the genome of a cell by the prime editing system in the cell is as follows. The PE2 prime editor protein and the pegRNA form a complex, followed by contacting the complex with the genome of a cell. A spacer of the pegRNA binds to a sequence of the corresponding target region. A nick is made in a strand not binding to the spacer of the genome DNA. The nick is made in between the third and fourth nucleotides located upstream relative to the 5' end of the PAM sequence. The sequence located upstream of the nick site functions as a primer to form complementary binding to a primer binding site of the pegRNA, thereby exposing the 3' end of the cleaved strand to a reverse transcription process. On the basis of the primer that forms the complementary binding to the primer binding site, the reverse transcriptase performs a reverse transcription process to form a 3' DNA flap. The template of reverse transcription in this reverse transcription process is an RT template of the pegRNA. Through cell-specific mechanisms of 5' flap removal, 3' flap ligation, and DNA mismatch repair, information on the 3' flap is installed into the genome DNA. As a result of prime editing, the information on the RT template of the pegRNA is delivered to the desired site of both strands of the genome DNA. The RT template includes a template for the desired editing (that is, an editing template), and information contained in the editing template is ultimately delivered to the target site of the genome DNA.

Hereinbelow, a method for predicting or confirming off-targets in prime editing provided by the present application, the method developed targeting prime editing to be widely usable or applicable when confirming off-targets that may occur in prime editing described above or to be developed in the future, is to be described in detail. In the following method for predicting or confirming the off-targets in prime editing, prime editor proteins used in many prime editing versions described above may be used and are not particularly limited. Furthermore, additional elements used in the prime editing versions described above may also be used in the method for predicting or confirming the off-targets in prime editing of the present application. It will be apparent to those skilled in the art that the prime editor proteins, pegRNAs, and/or prime editing systems, developed on the basis of the technical features of prime editing characterized by using Cas proteins and polymerases, may be used in the method for predicting the off-targets of the present application.

### Off-target prediction method provided by present application

### Off-target

In the field of DNA editing (such as gene editing or genome editing), an off-target refers to a genetic modification occurring at an unintended site. Genetic modifications caused by off-targets may be non-specific. Genome editing tools that have been developed to date include traditional CRISPR/Cas systems, base editing systems, prime editing systems, transcription activator-like effector nucleases (TALENs), meganucleases, zinc finger nucleases, and the like. Such genome editing tools or genome editing systems are designed such that editing in target regions may be performed through each of the specific mechanisms that enable binding to predetermined sequences (such as sequences in target regions). For example, in a CRISPR/Cas gene editing system, a guide RNA (gRNA) directs the movement of a Cas/gRNA complex to an intended target site. Movement to the target site may also involve a PAM sequence in the genome. However, the Cas/gRNA complex is still likely to bind to a sequence at an unintended site rather than a sequence in the target region. As described above, when the Cas/gRNA complex binds to the sequence in the unintended site and makes a nick or DSB therein, unintended genetic modifications occur. An off-target effect induces unintended genetic modifications such as unintended point mutations, deletions, insertions, inversions, and translocations. Similarly, in the process of editing a DNA molecule (such as genome DNA) by prime editing, there have also been issues with off-targets despite involving at least the PAM sequence and a sequence of a spacer of pegRNAs in targeting. It is known that the binding of genome editing tools to an unwanted region results from a partial but sufficient match to a target sequence in the unwanted regions. Regarding off-target binding mechanisms, reference may be made to a known document [Lin, Yanni, et al. "CRISPR/Cas9 systems have off-target activity with insertions or deletions between target DNA and guide RNA sequences." Nucleic acids research 42.11 (2014): 7473-7485.].

The off-target binding mechanisms are described as being grouped into two main forms: base mismatch tolerance and bulge mismatch. For example, an off-target region may include 0, 1, 2, 3, 4, 5, 6, 7, 8, 9, or 10 or more mismatches with the guide RNA sequence, but is not limited thereto. For example, an off-target region may include 0, 1, 2, 3, 4, 5, 6, 7, 8, 9, or 10 or more mismatches with a sequence of a target site corresponding to a sequence of each region of the pegRNA, but is not limited thereto. In other words, in prime editing, mismatches in the off-target region may exist in one or more of the regions corresponding to a spacer region of the pegRNA, a PBS of the pegRNA, a DNA synthesis template (such as homology arms) of the pegRNA, and the PAM sequence.

Issues with off-targets are likely to cause disruption of important coding regions, leading to serious problems such as cancer. Furthermore, issues with off-targets may lead to confusion of variables in biological research, which are further likely to lead to unreproducible results (see [Eid, Ayman, and Magdy M. Mahfouz. "Genome editing: the road of CRISPR/Cas9 from bench to clinic." Experimental & Molecular Medicine 48.10 (2016): e265-e265.], the entire contents of which are incorporated herein by reference).

As described above, such issues with off-targets still exist not only in CRISPR/Cas gene editing systems but also in base editing and prime editing developed on the basis of the CRISPR/Cas gene editing systems. The off-target herein may be used as a counterpart concept of an on-target and may be used to refer to a genetic modification at an unintended site.

### Need for off-target prediction method suitable for prime editing

### Overview of need for off-target prediction method suitable for prime editing

As described above, off-targets cause strong side effects (such as irreversible side effects and/or undetectable side effects) in various aspects. Accordingly, confirming off-targets that may occur when using systems for editing DNA molecules (such as genome editing systems) is critical in the research and development of therapeutics. Additionally, confirming bona-fide off-targets occurring in designed editing systems (such as CRISPR/Cas systems or prime editing systems) is costly and time-consuming. For this reason, research and development are in progress on various methods capable of confirming off-target candidates, that is, capable of predicting off-targets. However, existing methods for predicting off-targets that may occur in gene editing processes (such as genome editing processes by genome editing systems), developed before the filing date of the present application, were developed targeting traditional CRISPR/Cas systems or base editing. Off-target prediction methods to target prime editing, that is, developed targeting genome editing by prime editing, have not yet been developed. Despite unique editing mechanisms differentiated from those in traditional CRISPR/Cas systems, prime editing still uses off-target prediction systems having been developed targeting traditional CRISPR/Cas systems to predict off-targets that may occur in DNA editing processes by prime editing (see [Anzalone, Andrew V., et al. "Search-and-replace genome editing without double-strand breaks or donor DNA." Nature 576.7785 (2019) : 149-157.; Kim, Do Yon, et al. "Unbiased investigation of specificities of prime editing systems in human cells." Nucleic acids research 48.18 (2020): 10576-10589.; Bae, Sangsu, Jeongbin Park, and Jin-Soo Kim. "Cas-OFFinder: a fast and versatile algorithm that searches for potential off-target sites of Cas9 RNA-guided endonucleases." Bioinformatics 30.10 (2014): 1473-1475.; and Jin, Shuai, et al. "Genome-wide specificity of prime editors in plants." Nature Biotechnology 39.10 (2021): 1292-1299.], the entire contents of each of which are incorporated herein by reference). There are many disadvantages in the application of such traditional off-target prediction systems to prime editing. Hereinbelow, the need for the off-target prediction method suitable for prime editing is to be explained in more detail.

### Off-target prediction system used for predicting off-targets in genome editing by traditional CRISPR/Cas systems

As described above, various methods for predicting off-targets in genome editing by CRISPR/Cas systems have been developed. Traditional off-target prediction and/or confirmation methods (such as systems, platforms, and the like) may be classified into the following three categories based on the mechanism of action (MOA) of the above methods: a cell-based off-target prediction system, an *in vitro* off-target prediction system, and an *in silico* off-target prediction system. Examples of the prediction systems included in each category are as follows.
- Cell-based off-target prediction system: GUIDE-seq, GUIDE-tag, BLISS, BLESS, DISCOVER-seq, integrase-defective lentiviral vector-mediated DNA break capture, HTGTS, CReVIS-seq, ITR-seq, TAG-seq, INDUCE-seq, and the like.
- *In vitro* off-target prediction system: Digenome-seq, DIG-seq, CHANGE-seq, CIRCLE-seq, SITE-seq, and the like.
- *In silico* off-target prediction system: Cas-OFFinder, CRISPOR, CHOPCHOP, and the like.

Each of the above-described off-target prediction systems has different advantages and disadvantages. Typically, two or three systems are combined and used to predict genome-wide off-target activity during CRISPR-based genome editing.

### Application of CRISPR/Cas-based off-target prediction system to base editing

The above-described systems were expected to be used for predicting off-target activities of base editor systems, such as cytidine base editors and adenine base editors, developed using Cas proteins. However, the above-described systems, developed for predicting off-targets that may occur in genome editing by CRISPR/Cas systems, were unsuitable to be applied to base editing, where the operating mechanisms of the systems are different. Off-target prediction systems more suitable for base editing were required and, accordingly, off-target activity prediction systems or methods that are more suitable and sophisticated, such as One-seq (cell-based), CBE Digenome-seq (in vitro), ABE Digenome-seq (in vitro), and the like, were developed.

### Traditional off-target prediction method used in prime editing

The first step of a genome editing mechanism by prime editing, such as Prime Editing Version 2 (PE2), is the Cas9-induced nicking of a spacer non-binding strand. Thus, the off-target activity in PE2 was predicted to be similar to that of Cas9 or Cas9 nickases (nCas9). Accordingly, attempts were made to predict the off-target activity in prime editing by using systems for predicting and/or confirming off-targets in CRISPR/Cas genome editing, such as GUIDE-seq, nDignome-seq, and CAS-OFFinder *(in silico).* However, according to the experiments disclosed herein, it is confirmed that using such methods for predicting and/or confirming off-targets in traditional CRISPR/Cas genome editing is unsuitable for predicting off-targets in prime editing.

### Demand for off-target prediction system suitable for prime editing

Genome editing using prime editor proteins and pegRNAs is performed on the basis of a mechanism differentiated from those of genome editing by traditional CRISPR/Cas systems. Additionally, unlike traditional CRISPR/Cas systems, prime editing involves numerous factors (for example, a primer binding site, reverse transcription template, reverse transcriptase, or the like), in addition to a guide sequence, and is performed through processes using numerous enzymes (flap endonuclease, exonuclease, ligase, and the like). Even though prime editing has been developed on the basis of traditional CRISPR/Cas systems, the genome editing mechanism of prime editing differs from that of traditional CRISPR/Cas systems in various aspects. Thus, traditional off-target prediction methods, developed targeting genome editing by traditional CRISPR/Cas systems, are unsuitable for off-target prediction in prime editing. Furthermore, due to being performed through multiple processes involving numerous factors, as described above, in vitro-based off-target analysis methods capable of closely mimicking such complex intracellular processes are challenging to develop. For this reason, traditional off-target prediction methods are inapplicable to prime editing or are expected to cause inaccurate results.

Actually, the inventors of the present application have confirmed through experiments that not only a mismatch in the spacer region of a pegRNA but also the primer binding site, the homology arm, and/or the editing template affects off-targets in prime editing (see the "Editing patterns at validated off-target sites" and "Mismatch analysis by region" sections of Experimental Examples in the present application).

No methods have yet been reported to predict the off-target activity developed to target prime editing in consideration of the prime editing mechanism. In other words, there are still no reliable off-target prediction methods to confirm off-target candidates in prime editing.

### Overview of off-target prediction method provided by present application

The present application provides a new off-target prediction method suitable for prime editing. The inventors of the present application have confirmed that the application of off-target prediction systems targeting traditional CRISPR/Cas systems causes inaccurate predictions (false positives and/or false negatives). Therefore, the inventors of the present application have developed a new method or system for predicting off-targets in prime editing. The inventors of the present application have developed a new system or method for predicting off-targets, the system or method suitable for prime editing using a new prime editing guide RNA (pegRNA) including a tag template for tag insertion by focusing on the ability or effect of prime editing that enables a desired sequence to be inserted (installed or written) into a desired site. Furthermore, the inventors of the present application have confirmed that the prediction reliability and/or accuracy of the newly developed off-target prediction system in prime editing is better than those of traditional off-target prediction systems developed targeting traditional CRISPR/Cas genome editing systems.

The off-target prediction system developed to target prime editing (that is, developed to be suitable for prime editing), provided by the present application, may be called TAgmentation of Prime Editor sequencing (TAPE-seq). Furthermore, the new pegRNA including the tag template for installing a tag into the genome, used in TAPE-seq, may be called a tagmentation pegRNA (tpegRNA).

The present application provides a method or system for predicting off-targets that may occur in the process of editing a DNA molecule by the prime editing system. The present application provides the method for predicting off-targets that may occur in the genome editing process by the prime editing system. The method for predicting off-targets may, for example, be called a method for confirming off-target candidates, a method for confirming information on off-targets, a method for confirming candidate off-target sites, and the like. Additionally, any descriptions related to methods or systems for predicting off-targets that may occur in the process of editing a DNA molecule (such as genomes) or confirming information on off-targets may be used without limitation. As used herein, the term "off-target" includes the concept of an off-target region. For example, the off-target region or site may be described as the off-target. The off-target prediction herein may mean confirming the off-target candidate. The off-target prediction herein may mean confirming an off-target candidate region. The descriptions for the "off-target", "off-target prediction", and "off-target candidate" herein should not be interpreted as limited. In other words, the method for predicting off-targets in prime editing may be described as, but is not limited to, any of the following, and may be used interchangeably as long as the description relates to the prediction or confirmation of off-targets that may occur in prime editing: predicting off-targets that may occur in prime editing; confirming (or screening) off-target candidates in prime editing (or that may occur in prime editing); confirming (or screening) off-targets in prime editing (or that may occur in prime editing); confirming information on off-targets in prime editing (or that may occur in prime editing); confirming regions where off-targets may occur; confirming off-target sites; and the like.

Regarding off-target prediction, the terms false positive and/or false negative may be used. Locating regions other than a bona-fide off-target as the off-target candidate may be expressed as a false-positive result. A high false positive rate may be associated with a low validation rate. In this case, the bona-fide off-target is a validated off-target, which is used to refer to an off-target that occurs actually, other than the off-target candidate merely found by the prediction system. For example, an off-target occurring when editing the genome of a cell by the prime editing system may be called a bona-fide off-target. In contrast, a region related to the off-target found by the off-target prediction system is called an "off-target candidate", "off-target predicted", or the like and thus may be distinguished from the bona-fide off-target. The off-target candidate found by the off-target prediction system may or may not be the bona-fide off-target. For example, the bona-fide off-target may be found by validating each off-target candidate. It is important that the off-target prediction system exhibits a low false positive rate. This is because finding bona-fide off-targets is challenging when a large number of off-target candidates are derived from the off-target prediction system.

In another aspect, a group of off-target candidates found by the off-target prediction system may be free of all bona-fide off-targets. This case is associated with a miss rate. For example, failure to locate a bona-fide off-target region as the off-target candidate results in a higher miss rate.

As described above, the system for predicting the off-targets occurring in the process of editing a DNA molecule in prime editing of the present application is characterized by tagmentation based on the prime editing mechanism using the tpegRNA. Hereinbelow, tools for predicting the off-targets of the present application (such as prime editor proteins and tpegRNAs) are to be explained in detail.

### Tools for predicting off-targets in prime editing

### Overview of tools for predicting off-targets in prime editing (elements used for TAPE-seq)

The method for predicting the off-targets in prime editing of the present application requires two elements:
a prime editor protein; and
a tagmentation pegRNA (tpegRNA) including a tag template.

The tools for predicting the off-targets in prime editing of the present application may, at least, include the prime editor protein and the tpegRNA.

The method for predicting the off-targets of the present application may be called TAPE-seq. TAPE-seq, designed on the basis of the prime editing mechanism and relating to methods developed targeting prime editing for predicting off-targets, may use the prime editing mechanism. Accordingly, the method for predicting the off-targets, provided by the present application, includes the use of the prime editor protein used in prime editing. In other words, the various prime editor proteins described above may be used in the off-target prediction system of the present application. The prime editor protein used in the off-target prediction system in prime editing of the present application includes a Cas protein and a polymerase (such as reverse transcriptases). However, such a description does not require the use of the same type of prime editor protein as a prime editor protein in a specific prime editing system subject to off-target prediction (for example, specific prime systems to be targeted for off-target prediction by TAPE-seq). In the off-target prediction system of the present application, prime editor proteins that are the same or differ in type from the prime editor protein in the prime editing system subject to off-target prediction may be used.

Similarly, in the off-target prediction system of the present application, the use of the same type of pegRNA as that in a specific prime editing system subject to off-target prediction is not necessarily required. In the off-target prediction system of the present application, a pegRNA-based tpegRNA that is the same type as a pegRNA used in a specific prime editing system subject to off-target prediction may be used. Alternatively, tpegRNAs based on different types of pegRNA (such as epegRNAs), other than typical pegRNAs, may be used.

For example, even when a first prime editing system specified to confirm information on off-targets through the off-target prediction system is the PE2 prime editing system, prime editor proteins having nuclease activity (such as PE2-nuclease and PEmax-nuclease) may be used in TAPE-seq performed to confirm the information on off-targets in the first prime editing system. For another example, when the first prime editing system specified to confirm the information on off-targets through the off-target prediction system is the PE2 prime editing system, the PE2 prime editor protein may be used in TAPE-seq. Similarly, even when the first prime editing system subject to off-target prediction is the PE2 prime editing system, an engineered tpegRNA (etpegRNA) may be used in TAPE-seq. For another example, when the first prime editing system subject to off-target prediction is the PE2 prime editing system, the tagmentation pegRNA (tpegRNA) other than the engineered tpegRNA (etpegRNA) may be used.

### Prime editor protein

The off-target prediction system in prime editing of the present application includes the use of the prime editor protein. The prime editor protein includes the Cas protein and the polymerase (such as reverse transcriptases). The prime editor protein is described in the "Prime editing system" section of the present application in detail. Examples of the prime editor protein that may be used in the off-target prediction system of the present application include the prime editor protein described above, but are not limited thereto. It will be appreciated by those skilled in the art that fusion proteins or complexes for prime editing, developed for prime editing after the filing date of the present application (or inventions that inherit the inventive idea of prime editing), may also be used in the off-target prediction system of the present application.

Similarly, examples of the tpegRNA that may be used in the off-target prediction system of the present application include embodiments of various tpegRNAs developed on the basis of the pegRNA described above, but are not limited thereto. It will be appreciated by those skilled in the art that tpegRNAs based on the pegRNA for prime editing, developed for prime editing after the filing date of the present application (or inventions that inherit the inventive idea of prime editing), may also be used in the off-target prediction system of the present application.

In one embodiment, the prime editor protein used in the off-target prediction system in prime editing of the present application may include the Cas protein and the polymerase. In one embodiment, the Cas protein may be Cas12a, Cas12b1 (C2c1), Cas12c (C2c3), Cas12e (CasX), Cas12d (CasY), Cas12g, Cas12h, Cas12i, Cas1, Cas1B, Cas2, Cas3, Cas4, Cas5, Cas6, Cas7, Cas8, Cas9 (also known as Csn1 and Csx12), Cas10, Csy1, Csy2, Csy3, Cse1, Cse2, Csc1, Csc2, Csa5, Csn2, Csm2, Csm3, Csm4, Csm5, Csm6, Cmr1, Cmr3, Cmr4, Cmr5, Cmr6, Csb1, Csb2, Csb3, Csx17, Csx14, Csx10, Csx16, CsaX, Csx3, Csx1, Csx15, Csf1, Csf2, Csf3, Csf4, Cas13a (C2c2), Cas13b, Cas13c, Cas13d, Cas14, xCas9, circularly permuted Cas9, an Argonaute (Ago) domain, or fragments, homologs, or variants thereof, but is not particularly limited thereto. In certain embodiments, the Cas protein may have nickase activity. In certain embodiments, the Cas protein may be nCas9. In certain embodiments, the Cas protein may be an SpCas9 nickase. In certain embodiments, the Cas protein may have nuclease activity. In certain embodiments, the Cas protein may be a Cas protein having nuclease activity. In certain embodiments, the Cas protein may be a Cas9 variant having nuclease activity. In certain embodiments, the Cas protein may be SpCas9 or a variant thereof. For example, the SpCas9 variant may have a form in which any one or more amino acid residues selected from D10, R221, L244, N394, H840, K1211, and L1245 of wild-type SpCas9 are substituted with another amino acid residue. In certain embodiments, the Cas protein may contain an amino acid sequence including a H840A variation in the amino acid sequence (SEQ ID NO: 28) of wild-type SpCas9. In certain embodiments, the Cas protein may contain an amino acid sequence including amino acid variations of R221K and N394K in the amino acid sequence (SEQ ID NO: 28) of wild-type SpCas9. In certain embodiments, the Cas protein may contain an amino acid sequence including amino acid variations of R221K and N394K in the amino acid sequence (SEQ ID NO: 28) of wild-type SpCas9. In certain embodiments, the Cas protein may contain an amino acid sequence of SEQ ID NO: 29, 30, or 31.

In certain embodiments, the polymerase may be a reverse transcriptase. In certain embodiments, the reverse transcriptase may be a wild-type M-MLV reverse transcriptase. In certain embodiments, the reverse transcriptase may be a wild-type M-MLV reverse transcriptase variant. In certain embodiments, the wild-type M-MLV reverse transcriptase variant may contain an amino acid sequence including any one or more amino acid variations selected from D200N, T306K, W313F, T330P, and L603W in the amino acid sequence (SEQ ID NO: 26) of the wild-type M-MLV reverse transcriptase. In certain embodiments, the wild-type M-MLV reverse transcriptase variant may include amino acid variations of D200N, T306K, W313F, T330P, and L603W based on the amino acid sequence of SEQ ID NO: 26 of the wild-type M-MLV reverse transcriptase. In certain embodiments, the wild-type M-MLV reverse transcriptase variant may include amino acid variations of D200N, T306K, W313F, and T330P based on the amino acid sequence of SEQ ID NO: 26 of the wild-type M-MLV reverse transcriptase. In certain embodiments, the reverse transcriptase may contain an amino acid sequence of SEQ ID NO: 26 or 27.

As described above, the prime editor protein may further include additional elements, such as one or more linkers and/or one or more NLSs.

Examples of the prime editor protein that may be used in the off-target prediction system of the present application may include the prime editor proteins in the above-described prime editing versions (such as PE1 to PE5, PEmax, Nuclease-based prime editing, PEmax-nuclease, and the like). In some embodiments, the prime editor protein may be the PE2 prime editor protein, PE2-nuclease, PEmax prime editor protein, or PEmax-nuclease. In certain embodiments, the prime editor protein may be PEmax-nuclease.

### Tagmentation pegRNA (tpegRNA)

### Overview of tpegRNA

The tagmentation pegRNA (tpegRNA) is a guide nucleic acid developed from the pegRNA, designed for inserting a tag sequence into a DNA molecule, and used in the off-target prediction method (that is, the off-target prediction method in prime editing), provided by the present application. The tpegRNA, developed from the pegRNA, may be called one type of pegRNA. The tpegRNA provided by the present application includes a tag template and may be used for delivering the information contained in the tag template (such as tag sequences) into a DNA molecule (such as genomes) on the basis of the prime editing mechanism.

In some embodiments, the tpegRNA may be a single-stranded nucleic acid molecule (such as single-stranded RNAs). In some embodiments, the tpegRNA may be a nucleic acid complex composed of two or more strands (such as complexes of single-stranded RNA and double-stranded RNA). When the tpegRNA is formed to include two strands, a portion of the sequence of the two strands may form complementary binding to a gRNA core portion, thus forming a double-stranded tpegRNA. In certain embodiments, the tpegRNA may be a single-stranded RNA molecule.

Some embodiments of the present application provide the tpegRNA. Hereinbelow, elements included in the tpegRNA are to be disclosed.

The tpegRNA includes a spacer, a gRNA core, and an extension region. As described above, the pegRNA used in prime editing has a form in which the extension arm is added to the 3' or 5' end of a traditional gRNA. Typically, pegRNAs have a form in which an extension arm is added to the 3' end of a traditional gRNA. Similarly, the tpegRNA has a form in which an extension arm is added to the 3' or 5' end of a traditional gRNA, and the extension arm may include the extension region.

In some embodiments, the tpegRNA may have a form in which the extension arm is added to the 3' end of a traditional gRNA. In some embodiments, the spacer, the gRNA core, and the extension region may be located in the tpegRNA in the 5' to 3' direction. In some embodiments, the tpegRNA may further include any one or more independently selected additional functional elements (such as linkers, transcription terminators, RNA protection motifs, and the like) at one or more regions selected from in between the 5' end and the spacer, in between the spacer and the gRNA core, in between the gRNA core and the extension region, and in between the extension region and the 3' end, but is not limited thereto. In other words, in the tpegRNA, such independently selected additional functional elements may or may not exist in between each element described above, and are not particularly limited.

In some embodiments, the extension region of the tpegRNA includes the tag template. In some embodiments, the tag template may be separately described from a DNA synthesis template (such as RT templates). For example, the extension region of the tpegRNA may be described as including a primer binding site (PBS), a tag template, and a DNA synthesis template. In this case, the tag template and the DNA synthesis template are described separately, which is to describe the tag template separately from the DNA synthesis template of a traditional pegRNA. In another aspect, the tag template is encoded into an edited DNA molecule by the reverse transcriptase of the prime editor protein and may thus be described as one element of the DNA synthesis template. For example, the extension region of the tpegRNA may be described as including the primer binding site and the DNA synthesis template (in which case the DNA synthesis template includes the tag template). In the following description, the tag template and the DNA synthesis template are to be separately described. Unless otherwise described, the tpegRNA will be recognized as including the tag template.

Furthermore, the extension region of the tpegRNA may further include one or more independently selected additional functional regions, in addition to the PBS, the tag template, and the DNA synthesis template.

For example, the extension region of the tpegRNA may further include a 3' engineering region including an RNA protective motif. When the extension region of the tpegRNA further includes the 3' engineering region including the RNA protective motif, the tpegRNA may be called an engineered tpegRNA (etpegRNA). For example, the RNA protection motif may contain a sequence CGCGGUUCUAUCUAGUUACGCGUUAAACCAACUAGAA (SEQ ID NO: 41). In some embodiments, the 3' engineering region may further include a linker for linking the RNA protection motif, in addition to the RNA protection motif. In this case, the linker for linking the RNA protection motif may serve to link the RNA protection motif and the PBS. The term tpegRNA herein is used as a concept including embodiments of the etpegRNA, and descriptions for the tpegRNA may be interpreted as including content related to the etpegRNA unless otherwise specified. Certain embodiments limited to the use of the etpegRNA will be described with the context regarding the etpegRNA.

In some embodiments, the 3' engineering region may have a length of 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 51 nt, 52 nt, 53 nt, 54 nt, 55 nt, 56 nt, 57 nt, 58 nt, 59 nt, 60 nt, 61 nt, 62 nt, 63 nt, 64 nt, 65 nt, 66 nt, 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, or 100 nt or greater, or may have a length within a range set by two values selected from the above-described values, but is not limited thereto. In certain embodiments, the 3' engineering region may have a length of 10 to 70 nt. In certain embodiments, the 3' engineering region may have a length of 20 to 60 nt.

In some embodiments, the tpegRNA may have a length of about 30 nt, 40 nt, 50 nt, 60 nt, 70 nt, 80 nt, 90 nt, 100 nt, 110 nt, 120 nt, 130 nt, 140 nt, 150 nt, 160 nt, 170 nt, 180 nt, 190 nt, 200 nt, 210 nt, 220 nt, 230 nt, 240 nt, 250 nt, 260 nt, 270 nt, 280 nt, 290 nt, 300 nt, 310 nt, 320 nt, 330 nt, 340 nt, 350 nt, 360 nt, 370 nt, 380 nt, 390 nt, 400 nt, 410 nt, 420 nt, 430 nt, 440 nt, 450 nt, 460 nt, 470 nt, 480 nt, 490 nt, 500 nt, 520 nt, 540 nt, 560 nt, 580 nt, or 600 nt or greater, or may have a length within a range set by two values selected from the above-described values, but is not limited thereto. In certain embodiments, the tpegRNA may have a length of 100 to 300 nt or 100 to 400 nt.

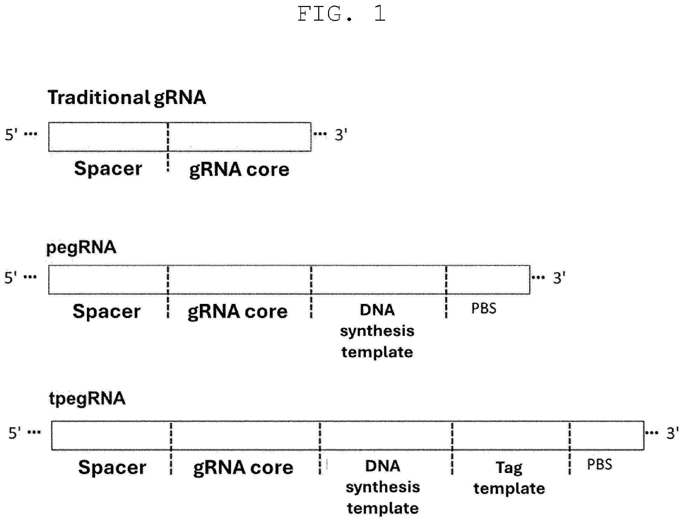

It is noted that unlike typical pegRNAs (tag template-free pegRNAs), the tpegRNA of the present application includes the tag template for inserting a tag sequence into a DNA molecule. For the benefit of technologists in the art, examples of traditional gRNA, pegRNA, and tpegRNA are disclosed in FIG. 1. Examples of the gRNA, pegRNA, and tpegRNA disclosed in FIG. 1. are illustrated on the basis of essential elements included in each guide RNA, and it will be apparent to those skilled in the art that additional elements may be further included in between each element or at the terminal end.

Hereinbelow, each element of the tpegRNA is to be explained in detail.

### Traditional gRNA portion - Spacer

As described above, the tpegRNA may include the spacer, the gRNA core, and the extension region. In this case, the spacer and the gRNA core are elements derived from a traditional gRNA. The spacer and the gRNA core are fully described in the "CRISPR/Cas system" and "Prime editing system" sections herein. The spacer contains a sequence of the spacer. The sequence of the spacer may be designed according to a target sequence without limitation. In this case, the region of the PAM sequence may be taken into account. The sequence of the spacer may be designed as a sequence complementary to the target sequence in a spacer binding strand of the genome DNA. The sequence of the spacer may be designed as the same sequence as (or substantially the same as or corresponding to) the target sequence in a spacer non-binding strand of the genome DNA. The sequence of the spacer may be an RNA sequence, DNA sequence, or RNA/DNA hybrid sequence. Typically, the sequence of the spacer is the RNA sequence. Like traditional gRNAs, the sequence of the spacer is involved in directing the Cas protein (Cas proteins included in the prime editor) to a target region. In other words, the sequence of the spacer and the target sequence form complementary binding, thus locating the prime editor protein/tpegRNA complex in the target region, and the prime editor protein makes a nick or DSB in the target region.

In some embodiments, the sequence of the spacer may have a length of about 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt or greater, or may have a length within a range of two values selected from the above-described values, but is not limited thereto. In certain embodiments, the sequence of the spacer may have a length of 10 to 35 nt. In certain embodiments, the sequence of the spacer may have a length of 13 to 30 nt. In certain embodiments, the sequence of the spacer may have a length of 15 to 25 nt.

### Traditional gRNA portion - gRNA core

As described above, the tpegRNA may include the spacer, the gRNA core, and the extension region. In this case, the spacer and the gRNA core are elements derived from a traditional gRNA. The gRNA core, a portion that interacts with the Cas protein, binds to the Cas protein to form a complex. The gRNA core may be called a scaffold region. The gRNA core or scaffold may be designed differently depending on the type of Cas protein used, which may, for example, be different depending on the type of microorganism from which the Cas protein is derived and the type of CRISPR system.

In one embodiment, the gRNA core may contain a scaffold sequence. The scaffold sequence may be an RNA sequence, DNA sequence, or RNA/DNA hybrid sequence. A portion of the sequence of the gRNA core may interact with a portion of other sequences of the gRNA core, thus forming structures such as stem/loops or hairpins.

In some embodiments, the scaffold sequence may have a length of about 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 55 nt, 60 nt, 65 nt, 70 nt, 75 nt, 80 nt, 85 nt, 90 nt, 95 nt, 100 nt, 110 nt, 120 nt, 130 nt, 140 nt, 150 nt, 160 nt, 170 nt, 180 nt, 190 nt, 200 nt, 210 nt, 220 nt, 230 nt, 240 nt, 250 nt, 260 nt, 270 nt, 280 nt, 290 nt, or 300 nt or greater, or may have a length within a range set by two values selected from the above-described values. In certain embodiments, the length of the scaffold sequence may be 30 to 200 nt, but is not particularly limited thereto. In certain embodiments, the length of the scaffold sequence may be 50 to 150 nt. In certain embodiments, the length of the scaffold sequence may be 60 to 100 nt.

### Overview of tpegRNA extension region

As described above, the tpegRNA may include the extension region (or extension arm). The extension region of the tpegRNA is characterized by including the tag template. The extension region of the pegRNA may be located at the 3' or 5' end of a traditional gRNA. For example, the tpegRNA may have the following structure in the 5' to 3' direction: "[traditional gRNA portion]-[extension region]" or "[extension region]-[traditional gRNA portion]". The [traditional gRNA portion] may include the above-described spacer and scaffold (gRNA core). Preferably, the extension region is located at the 3' end of a traditional gRNA portion. For example, the tpegRNA may include the spacer, the gRNA core, and the extension region. In some embodiments, the spacer, the gRNA core, and the extension region may be located in the tpegRNA in the 5' to 3' direction. In some embodiments, the extension region, the spacer, and the gRNA core may be located in the tpegRNA in the 5' to 3' direction.

In some embodiments, the extension region of the tpegRNA may contain an RNA sequence, DNA sequence, or DNA/RNA hybrid sequence. Preferably, the extension region contains the RNA sequence, but is not limited thereto.

The extension region of the tpegRNA is characterized by including the tag template. In other words, the extension region includes the primer binding site (PBS), the tag template, and the DNA synthesis template (such as RT templates). The extension region may further include one or more independently selected additional elements (such as linkers, RNA protection motifs, or the like) in between the above-described elements or at the terminal element.

### Additional elements that may be included

In some embodiments, the tpegRNA may include one or more independently selected additional elements, in addition to the extension region, the gRNA core, and the spacer. Additional elements may, for example, be any one from a linker, a poly U tail, a poly A tail, and an RNA protection motif, but are not particularly limited. For example, the tpegRNA may contain a U-rich, A-rich, or AU-rich sequence at the 3' end. In certain embodiments, the tpegRNA may contain a (U)n sequence at the 3' end, in which case n may be an integer of 3 to 20. In certain embodiments, the tpegRNA may contain a (U)₇ sequence at the 3' end.

### tpegRNA extension region (1)

### Overview of tpegRNA extension region (1)

As described above, the tpegRNA includes the extension region. The extension region may include the tag template and the primer binding site described with regard to the pegRNA in detail.

In some embodiments, the extension region of the tpegRNA may be described as including: a first region including the DNA synthesis template, a second region including the tag template, and a third region including the primer binding site. In this case, a part or all of the first region may be the DNA synthesis template. In this case, a part or all of the second region may be the tag template. In this case, a part or all of the third region may be the primer binding site. Hereinbelow, the elements included in the extension region are to be explained in detail.

### Tag template

The extension region of the tpegRNA may include the tag template. The tag template refers to a portion of the extension region, being complementary to the tag sequence to be installed in the spacer non-binding strand of a DNA molecule or single-stranded DNA flap (such as 3' DNA flaps) synthesized by the polymerase such as reverse transcriptases. The tag template may be complementary to the tag sequence to be installed in the spacer non-binding strand of the DNA molecule or the DNA flap (such as 3' DNA flaps). The off-target prediction method of the present application may achieve the objectives of off-target prediction in prime editing by confirming information on a tag containing the tag sequence to be installed in a DNA molecule and a sequence complementary to the tag sequence (for example, information on a site where the tag sequence is inserted, whether the tag sequence or the sequence complementary to the tag sequence is present, a chromosome where the tag sequence is inserted, and/or the like). Examples of the tag sequence corresponding to the tag template of the tpegRNA may be described in [Tsai, Shengdar Q., et al. "GUIDE-seq enables genome-wide profiling of off-target cleavage by CRISPR-Cas nucleases." Nature biotechnology 33.2 (2015): 187-197.], the entire contents of which are incorporated herein by reference.

The tag template of the tpegRNA and the tag sequence to be inserted into DNA are not particularly limited and may be selected appropriately depending on the purpose of using the tpegRNA. For example, a sequence of the tag template may include a sequence of AUACCGUUAUUAACAUAUGACAACUCAAUUAAAC (SEQ ID NO: 42), GUUAUUAACAUAUGACAACUCAAUUAAAC (SEQ ID NO: 43), UAUGACAACUCAAUUAAAC (SEQ ID NO: 44), AUUAACAUAUGAC (SEQ ID NO: 45), GACAACUCA (SEQ ID NO: 46), or CUCAAUUA (SEQ ID NO: 47). For example, the tag sequence may include a sequence of GTTTAATTGAGTTGTCATATGTTAATAACGGTAT (SEQ ID NO: 48), GTTTAATTGAGTTGTCATATGTTAATAAC (SEQ ID NO: 49), or GTTTAATTGAGTTGTCATA (SEQ ID NO: 50).

In some embodiments, the tag template may be an RNA sequence, DNA sequence, or RNA/DNA hybrid sequence. Preferably, the tag template is the RNA sequence.

In some embodiments, the tag template may have a length of 1 nt to 500 nt. In some embodiments, the tag template may have a length of 1 nt, 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 51 nt, 52 nt, 53 nt, 54 nt, 55 nt, 56 nt, 57 nt, 58 nt, 59 nt, 60 nt, 61 nt, 62 nt, 63 nt, 64 nt, 65 nt, 66 nt, 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, or 100 nt or greater, or may have a length within a range set by two values selected from the above-described values. In certain embodiments, the tag template may have a length of 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt or greater, or may have a length within a range set by two values selected from the above-described values. In certain embodiments, the tag template may have a length of 10 to 70 nt. In certain embodiments, the tag template may have a length of 10 to 50 nt. In certain embodiments, the tag template may have a length of 15 to 40 nt. In certain embodiments, the tag template may have a length of 25 to 40 nt. In certain embodiments, the tag template may have a length of 30 to 40 nt. In certain embodiments, the tag template may have a length of 19, 24, 29, or 34 nt.

The length of the tag template may be designed appropriately depending on the purpose of the disclosure disclosed in the present application, which is to analyze off-targets in prime editing through the tag sequence to be installed. When the length of the tag template is too short, information on the tag sequence inserted into a DNA molecule may be challenging to obtain. When the length of the tag template is too long, the tag sequence may be unlikely to be installed into a DNA molecule, making it impossible to achieve the purpose of off-target prediction fails to be achieved.

Furthermore, the tag template may be designed depending on the purpose of using the tpegRNA without limitation. In the off-target prediction method of the present application, the tag template is the basis for the tag sequence to be inserted into the genome DNA. In other words, the tag template is used as a template of reverse transcription, thus installing the tag sequence in the genome DNA. Using the tag sequence installed in the genome DNA in such a manner or the sequence complementary to the tag sequence, a tagging site in the genome DNA may be specified. Using the tagging site, regions where off-targets may occur (such as candidate off-target regions or off-target candidates) may be located. Whether the same sequence is present in the genome DNA may be taken into account when designing the tag sequence or the tag template of the tpegRNA used for off-target prediction. This is because when the same sequence as the tag sequence or the sequence of the tag template is, for example, present in the genome DNA, off-target prediction results may be affected. For another example, even when the same sequence is present, once the site where the same sequence is present is known in advance, the results for the corresponding site may be excluded from off-target prediction results. As described above, the sequence of the tag template or the tag sequence may be designed depending on the purpose or plan of using the tpegRNA.

### Primer binding site (PBS)

The extension region of the tpegRNA may include the primer binding site (PBS). The PBS of the tpegRNA may perform a role that is the same as or similar to the primer binding site of the pegRNA in prime editing. The polymerization activity of the polymerase (such as reverse transcriptases) in the prime editing protein may be present in the 5' to 3' direction, relative to a strand to be bound to a template strand. Once a primer (such as regions in the spacer non-binding strand) is annealed to the primer binding site, the polymerase (such as reverse transcriptases) may polymerize a single strand of DNA using the template strand as a template. For example, when using the prime editing protein in Prime Editing Version 2, the primer binding site (PBS) of the tpegRNA binds to a primer sequence formed from an endogenous DNA strand at a nicking target site resulting from the prime editing protein, thereby exposing the 3' end of the strand subjected to nicking. The binding of the primer binding site in the extension region of the tpegRNA to the primer sequence provides a matrix enabling the reverse transcriptase to polymerize the single strand of DNA. The primer binding site may have a sequence complementary to the primer sequence located upstream (toward the 5' direction) of a cleavage site (resulting from the nick or DSB) in the spacer non-binding strand. In some embodiments, the primer sequence may be a portion of a sequence located in a region ranging from -0 to -200 relative to the cleavage site. In certain embodiments, the primer sequence may be a portion of a sequence located in a region ranging from -0 to -50 relative to the cleavage site. In certain embodiments, the primer sequence may be a portion of a sequence located in a region ranging from -0 to -30 relative to the cleavage site. In certain embodiments, the primer sequence may be a portion of a sequence located in a region ranging from -0 to -20 relative to the cleavage site. In this case, - refers to the 5' direction, and numbers such as 30 refer to the nucleotide number. For example, -30 refers to the 30th nucleotide located upstream from the cleavage site. However, 0 refers to the cleavage site.

In some embodiments, the primer binding site may be an RNA sequence, DNA sequence, or RNA/DNA hybrid sequence. Preferably, the primer binding site is the RNA sequence.

In some embodiments, the primer binding site or the primer may have a length of 1 nt to 500 nt. In some embodiments, the primer binding site or the primer may have a length of 1 nt, 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 51 nt, 52 nt, 53 nt, 54 nt, 55 nt, 56 nt, 57 nt, 58 nt, 59 nt, 60 nt, 61 nt, 62 nt, 63 nt, 64 nt, 65 nt, 66 nt, 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, or 100 nt or greater, or may have a length within a range set by two values selected from the above-described values, but is not limited thereto. In certain embodiments, the primer binding site or the primer may have a length of 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt or greater, or may have a length within a range set by two values selected from the above-described values. In certain embodiments, the primer binding site or primer may have a length of 3 to 30 nt. In certain embodiments, the primer binding site or primer may have a length of 5 to 20 nt. In certain embodiments, the primer binding site or primer may have a length of 5 to 15 nt.

### DNA synthesis template

The extension region of the tpegRNA may include the DNA synthesis template. The DNA synthesis template may be the reverse transcription template (RT template). The DNA synthesis template of the tpegRNA may perform a role that is the same as or similar to the DNA synthesis template of the pegRNA. The DNA synthesis template of the tpegRNA may optionally include an editing template. Typical pegRNAs used in prime editing necessarily include the editing template because prime editing aims to perform editing. On the other hand, the tpegRNA used in the off-target prediction system of the present application preferentially aims to install the tag rather than editing and thus selectively includes the editing template. In other words, in some embodiments, the DNA synthesis template may include the editing template or may be editing-template free. Preferably, the DNA synthesis template includes the editing template, but is not limited thereto.

In some embodiments, the DNA synthesis template may be an RNA sequence, DNA sequence, or DNA/RNA hybrid sequence. Preferably, the DNA synthesis template (such as RT templates) is the RNA sequence.

In some embodiments, a sequence of the DNA synthesis template may correspond to a portion of a sequence present in a region ranging from +0 to +500 of the cleavage site (resulting from the nick or DSB) in the spacer non-binding strand. In this case, + refers to the 3' direction, and numbers such as 500 refer to an order from the cleavage site of the nucleotide. For example, 1 refers to the first nucleotide located downstream from the cleavage site. For example, 500 refers to the 500th nucleotide located downstream from the cleavage site. However, 0 refers to the cleavage site. In some embodiments, the sequence of the DNA synthesis template may correspond to a portion of a sequence in a region of <+100, <+90, <+80, <+70, <+60, <+50, <+40, <+30, <+20, or <+10 relative to the cleavage site (resulting from the nick or DSB) in the spacer non-binding strand. For example, the sequence of the DNA synthesis template other than the editing template may be a sequence complementary to a portion of a sequence in a region of < +100, < +90, < +80, < +70, < +60, < +50, < +40, < +30, < +20, or < +10 relative to the cleavage site in the spacer non-binding strand, and/or may be a sequence that is practically the same as the above-described portion of the sequence of the spacer non-binding strand.

In some embodiments, the DNA synthesis template may have a length of 1 nt to 500 nt. In some embodiments, the DNA synthesis template may have a length of 1 nt, 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 51 nt, 52 nt, 53 nt, 54 nt, 55 nt, 56 nt, 57 nt, 58 nt, 59 nt, 60 nt, 61 nt, 62 nt, 63 nt, 64 nt, 65 nt, 66 nt, 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, 100 nt, 110 nt, 120 nt, 130 nt, 140 nt, 150 nt, 160 nt, 170 nt, 180 nt, 190 nt, or 200 nt or greater, or may have a length within a range set by two values selected from the above-described values, but is not limited thereto. In certain embodiments, the DNA synthesis template may have a length of 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, or 40 nt. In certain embodiments, the DNA synthesis template may have a length of 3 to 40 nt. In certain embodiments, the DNA synthesis template may have a length of 5 to 30 nt. In certain embodiments, the DNA synthesis template may have a length of 7 to 30 nt.

In some embodiments, the DNA synthesis template may include the editing template and a homology region (or homology arm). In some embodiments, the DNA synthesis template may include the homology region. Hereinbelow, the homology region included in the DNA synthesis template is to be explained.

The homology region is a region corresponding to the above-described homology arm or homology region of the pegRNA used in prime editing.

In some embodiments, the homology region is complementary to a portion of the sequence of the spacer non-binding strand of the target DNA. In some embodiments, the homology region has a sequence homologous to a portion of the sequence of the spacer binding strand of the target DNA.

The sequence of the homology region is complementary to a portion of a sequence of a region located downstream (toward the 3' direction) of the resulting cleavage site (resulting from the DSB or nick) in the spacer non-binding strand of a DNA molecule. For example, in Prime Editing Version 2, the homology region may have a sequence complementary to a sequence located downstream of a site where the nick is made in the spacer non-binding strand. From another viewpoint, in Prime Editing Version 2, the homology region may have a sequence homologous to a portion of a sequence located upstream of the corresponding region to a site where the nick is made in the spacer binding strand.

On the other hand, the homology region replaces a sequence of an endogenous strand of a DNA molecule and facilitates the annealing of a single-stranded DNA flap (such as 3' DNA flaps), thus helping the editing and/or the tag sequence to be installed in the DNA molecule. The homology region is encoded by the polymerase (such as reverse transcriptases) of the prime editing protein and thus may be described as a portion of the DNA synthesis template.

In some embodiments, the homology region may contain an RNA sequence, DNA sequence, or DNA/RNA hybrid sequence. Preferably, the homology region contains the RNA sequence.

In some embodiments, the homology region may have a length of 1 nt to 500 nt. In some embodiments, the homology region may have a length of 1 nt, 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, 50 nt, 51 nt, 52 nt, 53 nt, 54 nt, 55 nt, 56 nt, 57 nt, 58 nt, 59 nt, 60 nt, 61 nt, 62 nt, 63 nt, 64 nt, 65 nt, 66 nt, 67 nt, 68 nt, 69 nt, 70 nt, 71 nt, 72 nt, 73 nt, 74 nt, 75 nt, 76 nt, 77 nt, 78 nt, 79 nt, 80 nt, 81 nt, 82 nt, 83 nt, 84 nt, 85 nt, 86 nt, 87 nt, 88 nt, 89 nt, 90 nt, 91 nt, 92 nt, 93 nt, 94 nt, 95 nt, 96 nt, 97 nt, 98 nt, 99 nt, or 100 nt or greater, or may have a length within a range set by two values selected from the above-described values. In certain embodiments, the homology region may have a length of 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, or 40 nt. In certain embodiments, the homology region may have a length of 3 to 40 nt. In certain embodiments, the homology region may have a length of 5 to 30 nt. In certain embodiments, the homology region may have a length of 7 to 30 nt.

In some embodiments, the DNA synthesis template may include the editing template. The editing template refers to a portion of the extension region encoding the editing to be installed in the spacer non-binding strand or the single-stranded DNA flap (such as 3' DNA flaps) synthesized by the polymerase (such as reverse transcriptases).

The editing template may be complementary to the editing to be installed in the spacer non-binding strand of a DNA molecule or DNA flap (such as 3' DNA flaps). For example, as a result of prime editing, the editing to be installed in the spacer non-binding strand is located downstream of the resulting cleavage site.

In some embodiments, the RT template may include the editing template, the homology region, and the like. In this case, the RT template is equivalent to the DNA synthesis template. However, the RT template herein is based on the use of the prime editing protein having the polymerase, that is, the reverse transcriptase, and the DNA synthesis template is further widely based on the use of prime editing proteins having any polymerase.

For example, the editing template of the tpegRNA may have the same sequence as the editing template corresponding to the desired editing for encoding the desired editing in a DNA molecule (In this case, the desired editing may be a pre-designed desired editing in prime editing subject to off-target analysis through the off-target prediction system of the present application).

For example, the editing template of the tpegRNA may have a sequence complementary to a sequence of the desired editing to be installed in a DNA molecule (such as genomes) or DNA flap (such as 3' DNA flaps). For another example, the editing template of the tpegRNA may have a sequence that differs from the editing template corresponding to the desired editing to be encoded in a DNA molecule. For another example, the editing template of the tpegRNA may have a sequence that differs from a part or all of the sequence complementary to the sequence of the desired editing to be installed in a DNA molecule (such as genomes) or DNA flap (such as 3' DNA flaps). In some embodiments, two types of tpegRNAs may be used for off-target prediction in prime editing, in which case the sequence of the editing template included in each tpegRNA may differ from a part or all of the sequence of the editing template of the desired editing.

In some embodiments, one type of tpegRNA may be used for off-target prediction in prime editing, in which case the sequence of the editing template included in the tpegRNA may have the same sequence as the editing template corresponding to the desired editing. In some embodiments, one type of tpegRNA may be used for TAPE-seq, in which case the sequence of the editing template included in the tpegRNA may have a sequence that differs from a part or all of the editing template corresponding to the desired editing.

As described above, prime editing technology is a system designed to insert the desired sequence into the desired site (that is, a system designed to "write" a desired sequence), and the editing is not particularly limited. For example, the editing may have a length of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 52, 54, 56, 58, or 60 nt (or bp) or greater.

In some embodiments, compared to the original sequence (that is, a sequence before editing) located in a region corresponding to the editing of an editing-subject DNA molecule, the editing to be installed in the editing-subject DNA molecule may include insertions of one or more nucleotides, deletions of one or more nucleotides, substitution of one or more nucleotides with other nucleotides, or a combination thereof. Furthermore, the editing to be installed in the editing-subject DNA molecule may have a region designed to insert the same sequence as a portion of the sequence of the endogenous DNA strand to be replaced. For example, the editing may include insertions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be inserted may or may not be located in succession in a nucleic acid. For example, the editing may include deletions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be deleted may or may not be located in succession in a nucleic acid. For example, the editing may include substitutions of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, or 50 or more nucleotides, in which case the nucleotides to be substituted may or may not be located in succession in a nucleic acid. For another example, the editing may include the above-described insertions and substitutions. For another example, the editing may include the above-described deletions and substitutions. For another example, the editing may include the above-described insertions and deletions. For another example, the editing may include the above-described insertions, deletions, and substitutions. Any one or more of the above-described insertions, deletions, and substitutions may occur in a region corresponding to a site where the editing is to be installed in the "editing-subject DNA molecule".

Furthermore, the editing to be installed in the editing-subject DNA molecule may have a region designed to insert the same sequence as the portion of the sequence of the endogenous DNA strand to be replaced, and a region present in the editing template coding the same may be called the "homology region of the editing template". There may be one or more homology regions in the editing template. In other words, the editing template may include one or more homology regions in the editing template.

For the benefit of technologists in the art, a structure that the editing template may have is to be illustrated. The editing template may be designed according to the purpose without limitation, so possible embodiments thereof should not be interpreted as being limited to the following examples. For example, the editing template may have the following structure: [first homology region of editing template]-[nucleotide for G to T substitution]-[second homology region of editing template]-[nucleotide for the A to T substitution]-[third homology region of editing template]. For another example, the editing template may have the following structure: [first homology region of editing template]-[nucleotide for A to C substitution]-[second homology region of editing template]. For a further example, the editing template may have the following structure: [first homology region of editing template]-[nucleotide for TAA insertion]. For a further example, the editing template may have the following structure: [first homology region of editing template]-[nucleotide for TGG insertion]-[second homology region of editing template]-[nucleotide for A to G substitution]. For a further example, the editing template may have the following structure: [nucleotide for AGG insertion]-[first homology region of editing template] .

In some embodiments, a site where the editing occurs may be within a region ranging from +0 to +100 relative to the cleavage site of the spacer non-binding strand. In certain embodiments, the site where the editing occurs may be within a region ranging from +0 to +60. In certain embodiments, the site where the editing occurs may be within a region ranging from +1 to +30. In certain embodiments, the site where the editing occurs may be within a region ranging from +0 to +20. In certain embodiments, the site where the editing occurs may be within a region ranging from +0 to +10. In some embodiments, when inserting the tag, the site where the editing occurs may be located downstream of the installed tag sequence. For example, the editing may occur within a region ranging from +10 to +50 relative to the cleavage site.

In some embodiments, the editing template may be composed of RNA. In certain embodiments, the editing template may be composed of DNA. In certain embodiments, the editing template may be composed of an RNA/DNA hybrid. In certain embodiments, the editing template may be composed of RNA.

In some embodiments, the editing template may have a length of 1 nt to 200 nt. In some embodiments, the editing template may have a length of 1 nt, 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, 20 nt, 21 nt, 22 nt, 23 nt, 24 nt, 25 nt, 26 nt, 27 nt, 28 nt, 29 nt, 30 nt, 31 nt, 32 nt, 33 nt, 34 nt, 35 nt, 36 nt, 37 nt, 38 nt, 39 nt, 40 nt, 41 nt, 42 nt, 43 nt, 44 nt, 45 nt, 46 nt, 47 nt, 48 nt, 49 nt, or 50 nt or greater. In certain embodiments, the editing template may have a length of 1 nt, 2 nt, 3 nt, 4 nt, 5 nt, 6 nt, 7 nt, 8 nt, 9 nt, 10 nt, 11 nt, 12 nt, 13 nt, 14 nt, 15 nt, 16 nt, 17 nt, 18 nt, 19 nt, or 20 nt or greater.

### Relative locational relationship in extension region

Hereinbelow, the relative locational relationship between the above-described elements in the extension region is to be explained. Each of the tag template, the PBS, and the DNA synthesis may be directly linked to other elements (for example, through covalent bonds), or may be linked via additional elements such as linkers.

In the case of a 3' extension region (that is, tpegRNAs having a form in which the extension region is added to the 3' end of a traditional gRNA) may have a locational relationship as follows.

In some embodiments, these elements may be located in the order of the DNA synthesis template, the tag template, and the primer binding site in the 5' to 3' direction of the tpegRNA in the extension region. In this case, in a DNA molecule (such as genome DNA), for the description in the 5' to 3' direction relative to the resulting cleavage site in the spacer non-binding strand, a tag sequence delivered by the tag template may be located at the first nucleotide, and a sequence delivered by the DNA synthesis template may be located at the second nucleotide. In other words, the locational relationship between the tag sequence installed in the DNA molecule and the sequence complementary to the DNA synthesis template in the spacer non-binding strand may be structured as follows: v-[tag sequence]-[sequence complementary to DNA synthesis template]. In this case, v refers to the resulting cleavage site.

In other embodiments, these elements may be located in the order of the tag template, the DNA synthesis template, and the primer binding site in the extension region in the 5' to 3' direction of the tpegRNA. In this case, in a DNA molecule (such as genome DNA), for the description in the 5' to 3' direction relative to the resulting cleavage site in the spacer non-binding strand, a sequence delivered by the DNA synthesis template may be located at the first nucleotide, and a tag sequence delivered by the tag template may be located at the second nucleotide. In other words, the locational relationship between the tag sequence installed in the DNA molecule and the sequence complementary to the DNA synthesis template in the spacer non-binding strand may be structured as follows: v-[sequence complementary to DNA synthesis template]-[tag sequence]. In this case, v refers to the resulting cleavage site.

Preferably, the DNA synthesis template, the tag template, and the primer binding site are located in such an order in the 5' to 3' direction of the tpegRNA, but are not limited thereto.

In the case of a 5' extension region (that is, tpegRNAs having a form in which the extension region is added to the 5' end of a traditional gRNA) may have a locational relationship as follows. In some embodiments, the tag template, the DNA synthesis template, and the primer binding site may be located in such a described order in the 5' to 3' direction of the tpegRNA. In some embodiments, the DNA synthesis template, the tag template, and the primer binding site may be located in such a described order in the 5' to 3' direction of the tpegRNA.

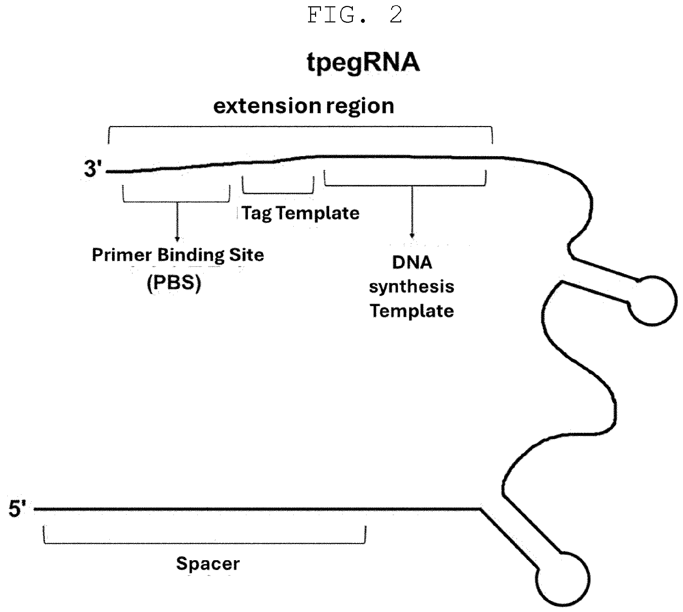

In some embodiments, the tag template may be located in between the DNA synthesis template and the primer binding site. In some embodiments, the tag template may be located in between the gRNA core and the DNA synthesis template. In some embodiments, the tag template may be located in between the spacer and the DNA synthesis template. In some embodiments, the DNA synthesis template may be located in between the tag template and the primer binding site. In some embodiments, the DNA synthesis template may be located in between the tag template and the gRNA core. In some embodiments, the DNA synthesis template may be located in between the tag template and the spacer. An exemplary embodiment of the tpegRNA described as including the DNA synthesis template, the tag template, and the primer binding site is disclosed in FIG. 2.

### tpegRNA extension region (2)

In some embodiments, the tpegRNA may be described as including the homology region, the editing template, the tag template, and the primer binding site, which enables the case where the tag template is located in between the editing template and the homology region to be described. In some embodiments, the tpegRNA may include: a first region including the homology region, a second region including the editing template, a third region including the tag template, and a fourth region including the primer binding site. In this case, a part or all of the first region may be the homology region. In this case, a part or all of the second region may be the editing template. In this case, a part or all of the third region may be the tag template. In this case, a part or all of the fourth region may be the primer binding site.

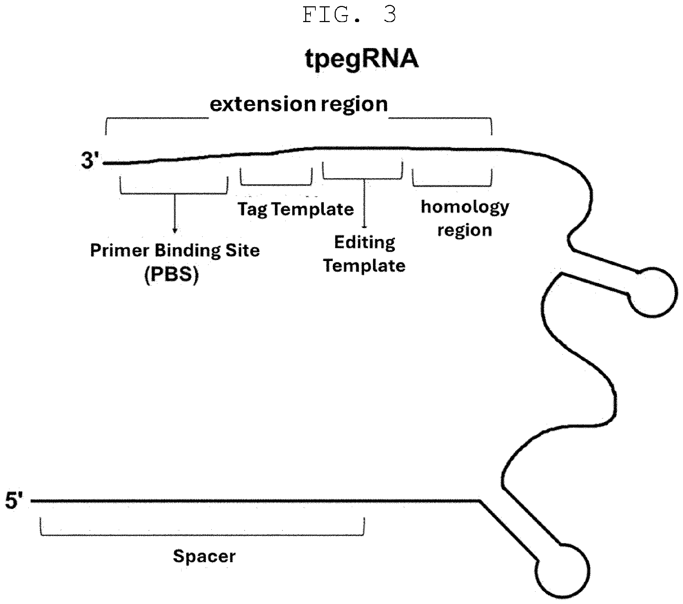

The locational relationship between each of the elements based on the primer binding site, the tag template, and the DNA synthesis template is described in detail in the previous section. Thus, the locational relationship between the homology region, the editing template, and the tag template is to be described below. As described above, the tag template is installed in the genome DNA by the polymerase and thus may be described as a portion of the DNA synthesis template. In several embodiments including the following embodiments of the "tpegRNA extension region (2)" section herein, the tag template may be described as being included in the DNA synthesis template, and this will not mislead those skilled in the art. The tpegRNA including the 3' extension region is to be illustrated. In some embodiments, the tag template may be located downstream of the editing template, that is, in between the primer binding site and the editing template. In some embodiments, the tag template may be located downstream of the homology region, that is, in between the homology region and the primer binding site. In some embodiments, the tag template may be located in between the editing template and the homology region. In some embodiments, the tag template may be located upstream of the homology region, that is, in between the homology region and the gRNA core. In some embodiments, the tag template may be located upstream of the editing template, that is, in between the editing template and the gRNA core. An exemplary embodiment of the tpegRNA described as including the homology region, the editing template, the tag template, and the primer binding site is disclosed in FIG. 3.

### Engineered tpegRNA

Some embodiments of the present application provide the engineered tpegRNA (etpegRNA). The etpegRNA, developed from the pegRNA, epegRNA, and tpegRNA, may be called the tpegRNA. In other words, the term "tpegRNA" in the present application will be recognized as including the embodiments of the etpegRNA. The etpegRNA refers to the pegRNA in the case where the extension region of the tpegRNA further includes the 3' engineering region, one element of the epegRNA. In other words, the etpegRNA includes the extension region including the tag template, the DNA synthesis template, the primer binding site, and the 3' engineering region. In some embodiments, the 3' engineering region may include the RNA protection motif. In some embodiments, the 3' engineering region may further include the linker for linking the RNA protection motif, in addition to the RNA protection motif. For example, each of the above-described elements of the etpegRNA may be located in the order of the DNA synthesis template, the tag template, the primer binding site, and the 3' engineering region in the 5' to 3' direction in the extension region.

It is noted that unlike typical pegRNAs (tag template-free pegRNAs), the tpegRNA includes the tag template for inserting a tag sequence into a DNA molecule.

### Examples of tools for predicting off-targets in prime editing

Tools for predicting off-targets in prime editing of the present application include at least the following two elements, as described above:
a prime editor protein; and a tpegRNA.

In some embodiments, the tools for predicting off-targets in prime editing may further include additional elements. For example, any one or more from a dominant negative MLH1 (dnMLH1), Cas proteins, guide RNAs (such as traditional sgRNAs), additional prime editing proteins, pegRNAs, and additional tpegRNAs (such as tpegRNAs including an editing template having a sequence that differs from that of the tpegRNA used) may be further included in the tools for predicting off-targets in prime editing, but are not limited thereto. Those skilled in the art will be able to improve or optimize the off-target prediction system in prime editing of the present application using appropriate additional elements.

### Tag insertion mechanism using tpegRNA in off-target prediction in prime editing

The off-target prediction method of the present application, designed on the basis of the prime editing mechanism, is a method for confirming or analyzing information on off-targets in prime editing. The prime editing mechanism is characterized by including the use of the pegRNA including the DNA synthesis template (such as RT templates) used as a template in a polymerization process (such as reverse transcription) to install a desired editing into an editing-subject DNA molecule. The off-target prediction method of the present application confirms or analyzes off-targets in prime editing by inserting a tag sequence into an analyzing-subject DNA molecule and confirming information on the inserted tag sequence, on the basis of the characteristic mechanism of prime editing. Hence, the off-target prediction method of the present application uses the characteristic mechanism of prime editing described above in the process of inserting the tag sequence.

Hereinbelow, an example of the mechanism of inserting a tag into the editing-subject DNA molecule in the off-target prediction method of the present application is to be disclosed. This disclosure is for the benefit of those skilled in the art viewing this specification, and the scope of this specification should not be limited by the following description.

Hereinbelow, an example of the mechanism of inserting a tag into a DNA molecule using the tpegRNA and the prime editor protein in Prime Editing Version 2 is to be disclosed.

The prime editing protein (including nCas9 and a reverse transcriptase MMLV_RT(D200N) (T330P) (L603W) (T306K) (W313F)) and the tpegRNA form a complex. The gRNA core of the tpegRNA may be called a gRNA scaffold or backbone sequence and refers to a sequence in the gRNA, pegRNA, or tpegRNA responsible for binding to Cas9 or equivalents thereof. The tpegRNA may bind to the Cas protein included in the prime editing protein via the gRNA core.

The prime editor protein/tpegRNA complex is localized to a site where off-targets may occur, on the basis of the sequence of the spacer and the PAM sequence. The sequence of the spacer of the tpegRNA may form complementary binding to the target (on-target or off-target) sequence in a DNA molecule complementary thereto. In this case, the complementary binding may be free of mismatches or include one or more mismatches. The mismatch may be any one or more selected from bulge mismatches and base mismatches known to cause off-targets, but is not limited thereto. Furthermore, off-targets may result from a mismatch of a portion of a sequence contained in the extension region with a sequence of the genome DNA. Furthermore, the site where the prime editing protein/tpegRNA complex is localized may not be limited to the PAM sequence. When compared to a sequence of an on-target, the sequence of off-targets predicted (such as off-target candidates) may include any one or more mismatches selected from one or more PAM mismatches, one or more spacer mismatches (that is, mismatches existing in a protospacer being a sequence corresponding to the sequence of the spacer), one or more PBS mismatch (that is, mismatches existing in a primer sequence being a sequence corresponding to a PBS sequence), and one or more DNA synthesis template mismatches (that is, mismatches existing in a sequence corresponding to the DNA synthesis template).

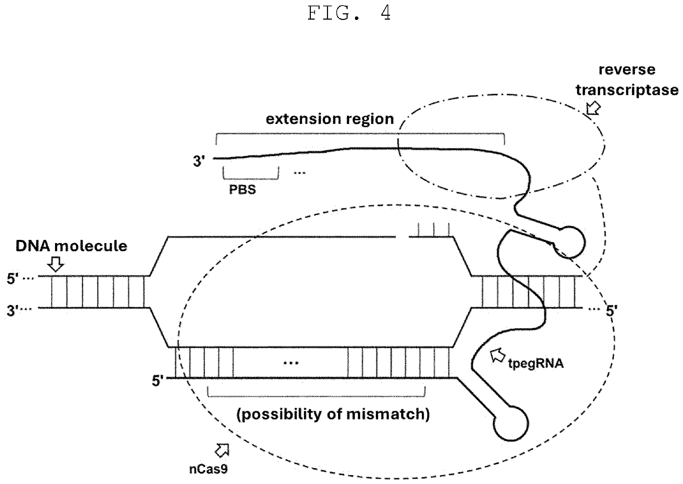

Due to the Cas protein (nCas9 in PE2) of the prime editor protein, a nick is made in between the -3 and -4 nucleotides relative to the 5' of the PAM sequence located upstream of the PAM sequence (5'-NGG-3') in the spacer non-binding strand. As a result, the tag sequence may be inserted into a window of 1 to 100 nucleotides located downstream of the nick site. The tag sequence may be inserted into a region ranging from about -4 to +100 of the PAM sequence. FIG. 4 illustrates an example of a DNA molecule where a nick is made at the resulting off-target site and a prime editor protein/tpegRNA complex that induces the nick.

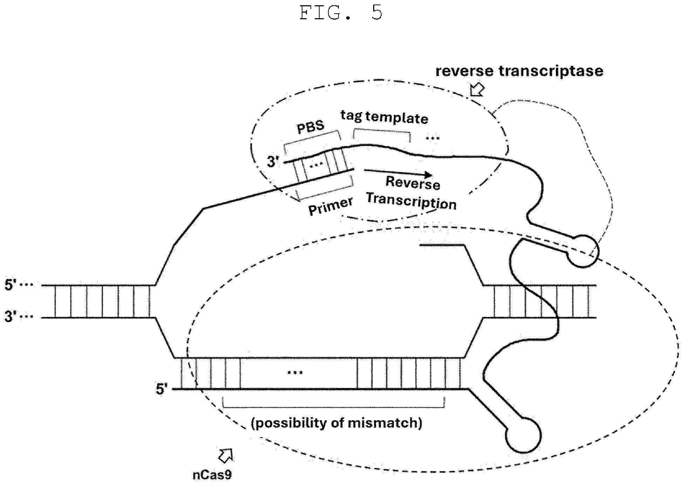

In the upstream of the site where the nick is made, the PBS is annealed to a region functioning as the primer (a portion of a region existing in the spacer non-binding strand of the DNA molecule, which may be called the primer) . The annealing of the PBS to the primer is illustrated in FIG. 5.

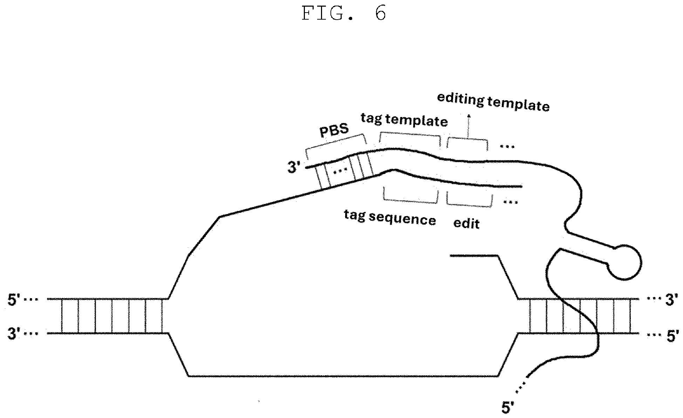

After annealing, reverse transcription by the reverse transcriptase is performed using the tag template and the DNA synthesis template as the templates of reverse transcription. Reverse transcription is performed in the 5' to 3' direction relative to a strand to which the nucleotides are polymerized, which is, in other words, performed in the 5' to 3' direction relative to the spacer non-binding strand. A sequence (tag sequence) having a complementary sequence to the tag template is added to the endogenous DNA strand by reverse transcription, followed by adding a sequence with a complementary sequence to the DNA synthesis template to the endogenous DNA strand. The tag sequence, the editing, and the like, added to the endogenous DNA strand by reverse transcription, are illustrated in FIG. 6.

The tag sequence added to the endogenous DNA strand and the sequence corresponding to the DNA synthesis template (the editing, a sequence complementary to a homology region, and the like) construct a 3' DNA flap. A 5' flap is removed, and the tag sequence and the editing are ultimately integrated into the DNA molecule through a repair system.

Through the processes described above, the tag sequence is inserted into a region where the editing may be inserted by prime editing. On this ground, the tag sequence may be inserted into sites where not only on-targets but also off-targets may occur. Accordingly, the site and/or the possibility of off-targets may be verified by confirming the presence and/or site of the tag sequence. Subsequently, analysis is performed on the tag sequence by methods capable of specifically analyzing the tag sequence, such as tag-specific amplification, sequencing, and the like. Through the analysis of the tag sequence, information on the tag sequence, such as types of DNA molecules where the tag sequence is inserted (for example, types of chromosomes), sites where the tag sequence is inserted (for example, sites in DNA molecules where the tag sequence is inserted), the insertion rates of the tag sequence by sites, and/or the like, may be obtained. Information on off-targets that may occur in prime editing may be obtained based on the information on the tag sequence.

Scenarios for inserting the tag sequence into the target DNA molecule (such as genome DNA) are not particularly limited. In some embodiments, tag insertion may not disturb the remaining pattern of prime editing. In this case, when removing the tag sequence from the resulting product of prime editing, the resulting product of prime editing from which the tag sequence is removed may be the same as a prime editing pattern induced by a tag-template-free pegRNA. For example, the tag sequence may be installed with the editing at one or more off-target candidate sites and/or on-target sites. In some embodiments, tag insertion may disturb the remaining pattern of prime editing. For example, the tag sequence may be installed without the editing at one or more off-target candidate sites and/or on-target sites. For another example, the editing may be installed without the tag sequence at one or more off-target candidate sites and/or on-target sites. In certain embodiments, the tag sequence may be installed with the editing at one or more off-target candidate sites and/or on-target sites. The off-target prediction system of the present application includes a process of contacting the genome DNA of the cell with the prime editor protein and the tpegRNA and then analyzing the genome DNA. Hereinbelow, the process of the off-target prediction system of the present application is to be described in detail.

### Contact of genome DNA of cell with prime editor protein and tpegRNA

### Overview of contact with genome DNA

The off-target prediction method in prime editing of the present application relates to confirming off-targets that may occur during a DNA editing process by prime editing. In other words, as a result of the off-target prediction method in prime editing of the present application, information on off-target candidates that may occur during the DNA editing process by prime editing may be derived. For example, whether the off-target candidates exist, off-target candidate sites, off-target candidate scores related to bona-fide off-targets, and/or like may be derived through the off-target prediction method of the present application. To obtain information on the off-target occurring during the DNA editing process, the prime editor protein and the tpegRNA must be in contact with the target DNA. Once contact with the target DNA is achieved, the mechanism of inserting the tag, including a DNA cleavage process, may be performed. The target DNA may, for example, be the genome DNA of the cell. As described above, the off-target prediction method of the present application may be classified as one of the cell-based off-target prediction methods, and the contact of the genome DNA of the cell with the prime editor protein and the tpegRNA may be performed intracellularly.

The cell used in the off-target prediction method in prime editing are not particularly limited. In some embodiments, the cell may be an animal cell or plant cell. In some embodiments, the cell may be a human cell or a cell of non-human animals (such as mice, rats, monkeys, chimpanzees, dogs, cats, cows, pigs, horses, sheep, and the like), but is not particularly limited. In some embodiments, the cells used in the off-target prediction method of the present application may be a cell derived from a patient. In some embodiments, the cell used in the off-target prediction method of the present application may be a cell from a cell line (such as human, mouse, monkey, or rat cell lines). In certain embodiments, the cell may be a human cell or a human cell line. Examples of the cells from the cell line may include 3T3 cells, A549 cells, HeLa cells, HEK 293 cells, K562 cells, Huh7 cells, Jurkat cells, OK cells, Ptk2 cells, or Vero cells, but are not limited thereto.

One embodiment of the off-target prediction system of the present application may include contacting the genome DNA of the cell with the prime editor protein and the tpegRNA (or the prime editor protein/tpegRNA complex). The contacting of the genome DNA with the prime editor protein and the tpegRNA may be performed in a cell or in the nucleus of a cell, but is not particularly limited. To contact the genome DNA with the prime editor protein and the tpegRNA, a cell containing the prime editor protein and the tpegRNA must be prepared. Hereinbelow, the cell containing the prime editor protein and the tpegRNA and a method for preparing the same are to be described in detail.

### Cell containing tools for predicting off-targets in prime editing

In some embodiments, the off-target prediction method of the present application may include preparing the cell containing tools for predicting off-targets in prime editing.

Some embodiments of the present application provide the cell containing the tools for predicting off-targets in prime editing.

The tools for predicting off-targets in prime editing include the prime editor protein and the tpegRNA. In some embodiments, the tools for predicting off-targets in prime editing may further include additional elements. For example, any one or more from a dominant negative MLH1 (dnMLH1), Cas proteins, guide RNAs (such as traditional sgRNAs), additional prime editing proteins, pegRNAs, and additional tpegRNAs (such as tpegRNAs including an editing template having a sequence that differs from that of the tpegRNA used) may be further included in the tools for predicting off-targets in prime editing, but are not limited thereto.

### Method for preparing cell containing tools for predicting off-targets in prime editing

Preparing the cell containing the tools for predicting off-targets in prime editing may be achieved by introducing each element of the prime editing tools into the cell (for example, by electroporation and the like) or by introducing a nucleic acid encoding each element of the prime editing tools into the cell. The process of preparing the cell containing the tools for predicting off-targets in prime editing is to be described in detail.

In some embodiments, preparing the cell containing the tools for predicting off-targets in prime editing may include: contacting the cell with the prime editor protein or a nucleic acid encoding the prime editor protein and the tpegRNA or a nucleic acid encoding the tpegRNA.

In some embodiments, preparing the cell containing the tools for predicting off-targets in prime editing may include: introducing the prime editor protein or the nucleic acid encoding the prime editor protein and the tpegRNA or the nucleic acid encoding the tpegRNA into the cell. The cell being in contact with the prime editor protein or the nucleic acid encoding the prime editor protein and the tpegRNA or the nucleic acid encoding the tpegRNA, or the cell where the prime editor protein or the nucleic acid encoding the prime editor protein and the tpegRNA or the nucleic acid encoding the tpegRNA are introduced in such a manner may be called an analyzing-subject cell.

The contact of the cell with each element of the tools for predicting off-targets in prime editing may be performed simultaneously (for example, in a single composition or using an all-in-one vector) or may be performed at different times. For example, these tools may be introduced into the cell by contacting the cell with a composition including the prime editor protein or the nucleic acid encoding the prime editor protein and the tpegRNA or the nucleic acid encoding the tpegRNA. For another example, these tools may be introduced into the cell by contacting the cell with a first composition including the prime editor protein or the nucleic acid encoding the prime editor protein, followed by (or following) contacting the cell with a second composition including the tpegRNA or the nucleic acid encoding the tpegRNA. As described above, the process of introducing the tools for predicting off-targets in prime editing into the cell is not particularly limited.

In some embodiments, the prime editor protein or the nucleic acid encoding the prime editor protein and/or the tpegRNA or the nucleic acid encoding the tpegRNA may be introduced into the cell in a vector or non-vector form.

In some embodiments, the prime editor protein may be a fusion protein composed of a single molecule or in the form of a complex containing two or more molecules. For example, when the prime editor protein is a fusion protein in a single molecule form, the prime editor protein or the nucleic acid encoding the prime editor protein may be introduced into the cell. For another example, when the prime editor protein is in the form of a complex containing two or more molecules, each element constituting the prime editor protein or each nucleic acid encoding each element may be introduced or delivered into the cell simultaneously (for example, in the form of an assembled complex or by being encoded in a single vector) or separately (for example, in the form of separated elements, by being encoded in separate vectors, or at appropriate time intervals).

In some embodiments, the prime editor protein or the nucleic acid encoding the prime editor protein and the tpegRNA or the nucleic acid encoding the tpegRNA may be introduced into the cell simultaneously (for example, in the form of an assembled complex or by being encoded in a single vector) or separately (for example, in the form of separated elements, by being encoded in separate vectors, or at appropriate time intervals). In some embodiments, the prime editor protein may be delivered or introduced into the cell in a protein form. In some embodiments, the prime editor protein may be delivered or introduced into the cell in the form of the nucleic acid encoding the prime editor protein. In some embodiments, the tpegRNA may be delivered or introduced into the cell in an RNA form. In some embodiments, the tpegRNA may be delivered or introduced into the cell in the form of the nucleic acid encoding the tpegRNA.

In some embodiments, the prime editor protein or the nucleic acid encoding the prime editor protein (such as prime editor protein-encoding DNA) and/or the tpegRNA or the nucleic acid encoding the tpegRNA (such as tpegRNA-encoding DNA) may be introduced into the cell in the form of a liposome, plasmid, viral vector, nanoparticle, or protein translation domain (PTD).

In some embodiments, the prime editor protein or the nucleic acid encoding the prime editor protein and/or the tpegRNA or the nucleic acid encoding the tpegRNA may be delivered or introduced into the cell by any one selected from electroporation, lipofection, microinjection, a gene gun, a virosome, a liposome, an immunoliposome, and lipid-mediated transfection.

In some embodiments, the nucleic acid encoding the prime editor protein (such as prime editor protein-encoding DNA, RNA, or hybrid of DNA or RNA) and/or the nucleic acid encoding the tpegRNA (such as tpegRNA-encoding DNA, RNA, or hybrid of DNA or RNA) may be delivered or introduced into the cell by methods known in the art. Alternatively, the nucleic acid encoding the prime editor protein and/or the nucleic acid encoding the tpegRNA may be delivered into the target by a vector, a non-vector, or a combination thereof. The vector may be a viral vector or a non-viral vector (such as plasmids). The non-vector may be naked DNA, a DNA complex, or mRNA.

### Vector-based introduction

In some embodiments, the prime editor protein or the nucleic acid encoding the prime editor protein and/or the tpegRNA and the nucleic acid encoding the tpegRNA may be introduced into the cell in a vector form, which may, in other words, be delivered or introduced into the target by the vector.

In some embodiments, the vector may contain the nucleic acid encoding the prime editor protein and/or the nucleic acid encoding the tpegRNA. In some embodiments, the nucleic acid encoding the prime editor protein may be contained in a single vector or may be split and contained in several vectors. For example, the nucleic acid encoding the prime editor protein may be introduced or delivered into the cell by one, two, three, four, or five or more vectors. In some embodiments, the nucleic acid encoding the tpegRNA may be contained in a single vector or may be split and contained in several vectors. For example, the nucleic acid encoding the tpegRNA may be introduced or delivered into the cell by one, two, three, four, or five or more vectors. In some embodiments, the nucleic acid encoding the prime editor protein and the nucleic acid encoding the tpegRNA may be contained in a single vector or may be split and contained in several vectors. For example, the nucleic acid encoding the prime editor protein and the nucleic acid encoding the tpegRNA may be introduced or delivered into the cell by one, two, three, four, or five or more vectors.

In some embodiments, the vector may include one or more regulatory/control elements. In this case, the regulatory/control elements may be any one or more selected from a promoter, an enhancer, an intron, a polyadenylation signal, a Kozak consensus sequence, an internal ribosome entry site (IRES), a nuclear localization signal (NLS) or a nucleic acid encoding the same, Poly A, a splice acceptor, and a 2A sequence. The promoter may be a promoter recognized by RNA polymerase II. The promoter may be a promoter recognized by RNA polymerase III. The promoter may be an inducible promoter. The promoter may be a target-specific promoter. The promoter may be a viral or non-viral promoter. As the promoter, a suitable promoter may be selected depending on a control region.

In some embodiments, the vector may be a viral vector or a recombinant viral vector. The virus may be a DNA virus or an RNA virus. In this case, the DNA virus may be a double-stranded DNA (dsDNA) virus or a single-stranded DNA (ssDNA) virus. In this case, the RNA virus may be the single-stranded RNA (ssRNA) virus. The virus may be a retrovirus, lentivirus, adenovirus, adeno-associated virus (AAV), vaccinia virus, pox virus, or herpes simplex virus, but is not limited thereto. The AAV vector may, for example, be any one selected from AAV1, AAV2, AAV5, AAV6, AAV8, AAV9, AAVrh.10, AAVrh.74, and AAVhu.37, but is not limited thereto. Examples of the AAV vector used in research or clinical practice are disclosed in [Wang, Dan, Phillip WL Tai, and Guangping Gao. "Adeno-associated virus vector as a platform for gene therapy delivery." Nature reviews Drug discovery 18.5 (2019): 358-378.] in detail, the entire contents of which are incorporated hereby reference. Typically, a virus may infect a host (such as cells) to introduce a nucleic acid encoding the genetic information on the virus into the host or to insert a nucleic acid encoding the genetic information into the host genome. Such characterized viruses may be used to introduce a nucleic acid encoding a target protein or a target sequence into a target (such as cells). Furthermore, the target protein and the target sequence may be expressed within the host.

### Introduction based on non-vector

In one embodiment, the prime editor protein or the nucleic acid encoding the prime editor protein and/or the tpegRNA and the nucleic acid encoding the tpegRNA may be introduced into the cell by introduction based on non-vector.

In some embodiments, one or more from the prime editor protein or the nucleic acid encoding the prime editor protein and/or the tpegRNA and the nucleic acid encoding the tpegRNA may be introduced into the cell by introduction based on non-vector.

In some embodiments, one or more from the prime editor protein or the nucleic acid encoding the prime editor protein and/or the tpegRNA and the nucleic acid encoding the tpegRNA may be introduced into the cell by one or more non-vectors. For example, the protein editor protein or the nucleic acid encoding the protein editor protein and the tpegRNA or the nucleic acid encoding the tpegRNA may be introduced or delivered into the cell by one, two, three, four, or five or more non-vectors.

The non-vector may contain the protein editor protein or the nucleic acid encoding the protein editor protein and the tpegRNA or the nucleic acid encoding the tpegRNA. The non-vector may be naked DNA, a DNA complex, mRNA, or a mixture thereof. The non-vector may be delivered or introduced into the target by electroporation, a gene gun, ultrasonic perforation, magnetofection, temporary cell compression or squeezing (disclosed in Lee, et al, (2012) Nano Lett., 12, 6322-6327), lipid-mediated transfection, a dendrimer, a nanoparticle, calcium phosphate, silica, silicate (ormosil), or a combination thereof. For example, delivery by electroporation may be performed by mixing a cell with a nucleic acid encoding a desired element in a cartridge, chamber, or cuvette and then applying electrical stimulation of a defined duration and amplitude. For another example, the non-vector may be delivered using a nanoparticle. The nanoparticle is an inorganic nanoparticle (such as magnetic nanoparticles, silica, and the like) or an organic nanoparticle (such as polyethylene glycol (PEG)-coated lipids and the like). The outer surface of the nanoparticle may be conjugated with a positively charged polymer (such as polyethylene imine, polylysine, polyserine, and the like) to enable attachment.

### Delivery or introduction in form of peptide, polypeptide, protein, or RNA

In one embodiment, the prime editor protein and/or the tpegRNA may be delivered or introduced into the target by methods known in the art. Forms of peptides, polypeptides, proteins, or RNA may be delivered or introduced into the cell by electroporation, trace injection, temporary cell compression or squeezing (disclosed in Lee, et al, (2012) Nano Lett., 12, 6322-6327), lipid-mediated transfection, a nanoparticle, a liposome, a peptide-mediated delivery, or a combination thereof.

As described above, the cell containing the prime editor protein and the tpegRNA is obtained. The prime editor protein and the tpegRNA (or prime editor protein/tpegRNA complex) in the cell may contact with the genome DNA of the cell. Hereinbelow, the results achievable by the contact of the genome DNA of the cell with the prime editor protein and the tpegRNA are to be described in detail.

### Result of contacting genome DNA with prime editor protein and tpegRNA (tagmentation)

As a result of contacting the genome DNA with the prime editor protein and the tpegRNA, the tag sequence and the sequence complementary to the tag sequence may be inserted into the genome DNA. In other words, the tag may be installed in the genome DNA. Such a process of installing the tag in the genome DNA may be called tagmentation. As a result of the contact, the tag may be installed at an off-target candidate region and/or an on-target region. After contacting the genome DNA with the prime editor protein and the tpegRNA, the resulting genome DNA may be called analyzing-subject genome DNA. In some embodiments, the analyzing-subject genome DNA may be tag-free. This applies to the case in the absence of off-target candidates, failure to tag sequence installation in the genome DNA, and the like. In some embodiments, the analyzing-subject genome DNA may contain the tag. The analyzing-subject genome DNA containing the tag may be called tagged DNA (or tagmented DNA). The tag exists at an off-target candidate site (that is, candidate off-target regions) and/or an on-target region. The analysis of the tag inserted into the genome DNA may enable a potential candidate off-target region that may be a bona-find off-target to be found. For example, the analyzing-subject genome DNA may contain one or more tags. One or a plurality of off-target candidates may be found by analyzing the presence or absence of each tag, each tagging site, and the like. For example, the off-target prediction method of the present application may be performed on a population of cells. The analyzing-subject genome DNA of some cells in the population of cells may contain one or more tags. The analyzing-subject genome DNA of some cells in the population of cells may be tag-free. The analysis of the genome DNA of each cell in the population of cells may enable one or multiple off-target candidates to be found. When the tag is inserted into the off-target candidate region, a tagmentation rate for each candidate off-target region may be obtained. Furthermore, the tag may also be inserted into the on-target region, so a tagmentation rate for the on-target region may be obtained. The tagmentation rate may, for example, be about 0.001%, 0.01%, 0.1%, 0.5%, 1%, 2%, 3%, 4%, 5%, 6%, 7%, 8%, 9%, 10%, 11%, 12%, 13%, 14%, 15%, 16%, 17%, 18%, 19%, 20%, 25%, 30%, 35%, 40%, 45%, 50%, 60%, 70%, 80%, 90%, or 100%, or may fall within a range of two values selected from the above-described values.

### Manipulated cell containing manipulated genome DNA

In some embodiments, a manipulated cell containing a manipulated genome may be provided. For example, when the analyzing-subject genome DNA contains the tag, that is, successfully installing the tag in the analyzing-subject genome DNA, the analyzing-subject genome DNA may be called the manipulated genome. For example, when the analyzing-subject genome DNA contains the editing, that is, successfully installing the editing in the analyzing-subject genome DNA, the analyzing-subject genome DNA may be called manipulated genome DNA. In some embodiments, the manipulated genome DNA may contain any one or more from the tag and the editing. In some embodiments, a population of cells containing the manipulated cell may be provided.

### Analyzing analyzing-subject DNA

### Overview of analysis of analyzing-subject DNA

The off-target prediction system in prime editing of the present application includes analyzing the analyzing-subject DNA. When the off-target prediction system in prime editing of the present application is performed on a cell, the analyzing-subject DNA may be the analyzing-subject genome DNA. The analysis of the analyzing-subject DNA is described using the analysis of the analyzing-subject genome DNA as an example. The analyzing-subject genome DNA may be the DNA of one or a plurality of genomes. The analysis of the analyzing-subject genome DNA may include analyzing the DNA of one or the plurality of genomes, but is not particularly limited. The analysis of the analyzing-subject genome DNA enables tagmentation information on the genome DNA to be obtained. For example, the tagmentation information may include, but is not particularly limited to, whether the tag sequence is contained in the analyzing-subject genome DNA, a site of each tag sequence for one or more tag sequences in the genome DNA (such as tagging sites), tagmentation rates at one or more tagging sites, and the like. Information on the off-target candidates may be obtained based on the tagmentation information. For example, the information on the off-target candidates may include information on one or more off-target candidates, off-target scores for one or more off-target candidates, and the like, but is not particularly limited.

### Analysis method

The analyzing-subject genome DNA may be analyzed to obtain the tagmentation information. The analyzing-subject genome DNA may be manipulated genome DNA. The off-target prediction system of the present application is characterized in that information on sites where off-targets may occur is confirmed based on the tag sequence integrated into the manipulated genome. Information on one or more tag sequences contained in the manipulated genome may be confirmed by methods known in the art or methods to be developed, but is not particularly limited. The information on the tag sequence may include, but is not limited to, any one or more from: whether each tag sequence is inserted, a chromosome where each tag sequence is inserted, a site where each tag sequence is inserted (for example, sites in chromosomes), the insertion rate of the tag sequence, and the insertion rate by each site where the tag sequence is inserted. For example, the information on the tag sequence may be confirmed by tag sequence analysis methods including tag-specific amplification, sequencing, and/or the like, but is not particularly limited. For analysis methods of the information on the tag sequence, reference may be made to [Tsai, Shengdar Q., et al. "GUIDE-seq enables genome-wide profiling of off-target cleavage by CRISPR-Cas nucleases." Nature biotechnology 33.2 (2015): 187-197.; Kim, Daesik, et al. "Digenome-seq: genome-wide profiling of CRISPR-Cas9 off-target effects in human cells." Nature methods 12.3 (2015): 237-243.; and Kim, Do Yon, et al. "Unbiased investigation of specificities of prime editing systems in human cells." Nucleic acids research 48.18 (2020): 10576-10589.] and the like, the entire content of each of which are incorporated herein by reference.

In some embodiments, the analysis of the analyzing-subject genome DNA may be tag-specific analysis (for example, analysis to find sites where tag sequences are present). In some embodiments, the analysis method of the analyzing-subject genome DNA may include tag-specific amplification. In some embodiments, the analysis method of the analyzing-subject genome DNA may include sequencing. In some embodiments, the analysis of the analyzing-subject genome DNA may include tag-specific amplification and sequencing.

In some embodiments, the analysis of the analyzing-subject genome DNA may be performed by DNA analysis methods well known to those skilled in the art. In some embodiments, the analysis of the analyzing-subject genome DNA may be performed by a process including any one or more selected from PCR-based analysis (see [Cameron, Peter, et al. "Mapping the genomic landscape of CRISPR-Cas9 cleavage." Nature methods 14.6 (2017): 600-606.]) and sequencing (see [Metzker, Michael L. "Sequencing technologies-the next generation." Nature reviews genetics 11.1 (2010): 31-46.; and Kumar, Kishore R., Mark J. Cowley, and Ryan L. Davis. "Next-generation sequencing and emerging technologies." Seminars in thrombosis and hemostasis. Vol. 45. No. 07. Thieme Medical Publishers, 2019.]) (such as DNA sequencing).

For example, any one or more sequencing methods referred to as whole-genome sequencing (WGS), deep sequencing, high-throughput sequencing (HTS), de-novo sequencing, second-generation sequencing, next-generation sequencing, third-generation sequencing, large-scale sequencing, shotgun sequencing, long-read sequencing, and short-read sequencing may be used, but the sequencing is not particularly limited. For example, a sequencing method of Hi-seq may be used. For example, a sequencing method of Mi-seq may be used. For example, two or more sequencing methods may be used to analyze the analyzing-subject DNA. For a specific example, a process including Hi-seq and Mi-seq may be included in analyzing the analyzing-subject DNA. In one embodiment, a sequencing depth in the sequencing method used to analyze the analyzing-subject genome DNA may be about 1, 2, 3, 4, 5, 6, 7, 8, 9, 10, 11, 12, 13, 14, 15, 16, 17, 18, 19, 20, 22, 24, 26, 28, 30, 32, 34, 36, 38, 40, 42, 44, 46, 48, 50, 55, 60, 65, 70, 75, 80, 85, 90, 95, 100, 200, 300, 400, 500, 600, 700, 800, 900, or 1000 x. In one embodiment, the sequencing depth may be within a range of two values selected from the above-described values. In one embodiment, the sequencing depth may be smaller than, equal to, or greater than the above-described values. In certain embodiments, the sequencing depth in the sequencing used for analysis may be about 10 to 40 x. The sequencing depth is not particularly limited, and a sequencing depth enabling the presence or absence and/or the site of the tag sequence in the analyzing-subject genome DNA to be confirmed is acceptable.

In some embodiments, the analysis of the analyzing-subject genome DNA may include a tag-specific amplification process. Through tag-specific amplification, an amplified tag-specific library may be generated. In some embodiments, the analysis of the analyzing-subject genome DNA may include sequencing the amplified tag-specific library.

Through the analysis of the analyzing-subject genome DNA, the tagmentation information may be obtained. In some embodiments, the analysis of the analyzing-subject genome DNA may include: generating a tag-specific library from the analyzing-subject genome DNA; and sequencing the tag-specific library. In some embodiments, the analysis of the analyzing-subject genome DNA may include: generating an amplified tag-specific library from the analyzing-subject genome DNA; and sequencing the amplified tag-specific library. In some embodiments, the analysis of the analyzing-subject genome DNA may include: generating a tag-specific library from the analyzing-subject genome DNA; amplifying the tag-specific library; and sequencing the amplified tag-specific library. For example, a tag-specific primer and/or an adapter-specific primer may be used in the tag-specific amplification. For example, the tag-specific amplification may be performed by PCR.

In some embodiments, generating the tag-specific library from the analyzing-subject genome DNA may include one or more processes selected from shearing the analyzing-subject genome DNA and ligating the sheared genome DNA using an adapter to generate the tag-specific library. For the amplification process of the tag-specific library, reference may be made to [Tsai, Shengdar Q., et al. "GUIDE-seq enables genome-wide profiling of off-target cleavage by CRISPR-Cas nucleases." Nature biotechnology 33.2 (2015): 187-197.; and Liang, Shun-Qing, et al. "Genome-wide detection of CRISPR editing in vivo using GUIDE-tag." Nature communications 13.1 (2022): 1-14.].

In some embodiments, any one or more processes from cell disruption, incubation, RNA removal, and DNA purification may be further included for the analysis of the analyzing-subject genome DNA. The above-described process may, for example, be performed after contacting the genome DNA with the prime editor protein and the tpegRNA.

### Obtainment of tagmentation information

Through the analysis of the above-described analyzing-subject DNA, the tagmentation information may be obtained. The tagmentation information is information obtained based on the information on the tag sequence and/or the tag sequence present in the analyzing-subject genome DNA. For example, the tagmentation information may be information obtained based on information on the tag sequence present in the DNA of one analyzing-subject genome. For another example, the tagmentation information may be information obtained based on information on the tag sequence present in the DNA of a plurality of analyzing-subject genomes. It will be appreciated that the analysis of the analyzing-subject genome DNA includes all embodiments of the analysis of the DNA of one or the plurality of analyzing-subject genomes.

For example, the tagmentation information may include, but is not limited to, any one or more from: whether each tag sequence is inserted, the chromosome where each tag sequence is inserted, the site where each tag sequence is inserted (for example, sites in chromosomes), the insertion rate of the tag sequence, and the insertion rate by each site where the tag sequence is inserted.

In some embodiments, the tagmentation information may include any one or more from:
whether the tag sequence is contained in the analyzing-subject genome DNA;
a site in the genome DNA of each tag sequence for one or more tag sequences; and
tagmentation rates for one or more tag sequences.

For example, when the analyzing-subject genome DNA contains the tag sequence, the presence of the tag sequence may be related to the presence of the on-target or the candidate off-target region. As described above, one or more tag sequences may be contained in the DNA of one genome, or one or more tag sequences may be contained in the DNA of the plurality of analyzing-subject genomes for the analysis. As a result, whether the tag sequence is contained in the analyzing-subject DNA is information on whether one or more tag sequences are present in the DNA of one or the plurality of analyzing-subject genomes. For example, in the case of the DNA of the plurality of analyzing-subject genomes, the tag sequence may be determined as being present in the analyzing-subject genome DNA when first analyzing-subject genome DNA is tag sequence-free, but second analyzing-subject genome DNA contains the tag sequence.

For example, the site in the genome DNA of each tag sequence for one or more tag sequences may be derived through the analysis of a site where the tag sequence is present, which may be called a "tagged site". For example, when the DNA of one analyzing-subject genome (first analyzing-subject genome DNA) among the DNA of the plurality of analyzing-subject genomes contains a first tag sequence, and another analyzing-subject genome DNA (second analyzing-subject genome DNA) contains a second tag sequence, a site of the first tag sequence may be called a first site, and a site of the second tag sequence may be called a second site. For another example, a plurality of tag sequences may be present in the DNA of one analyzing-subject genome, in which case one tag sequence may be called the first tag sequence, and another tag sequence may be called the second tag sequence. In this case, the site in the genome DNA of each tag sequence for one or more tag sequences may include the first site, the second site, or both the first and second sites. Here, the first and second sites are related to target sites (on-target sites and/or candidate off-target sites). The first and second sites other than on-target sites may both be candidate off-target sites. The first and second sites may refer to the same or different sites. In this case, information on such sites as the first and second sites includes information on the chromosome number and information on sites at specific chromosomes.

For example, the tagmentation rates for one or more tag sequences may be derived from the frequency of discovery by tagged sites. For example, in the analysis of the analyzing-subject genome DNA, when the tag sequences are discovered 10 times at the first site and five times at the second site, the tagmentation rate at the first site is twice the tagmentation rate at the second site. The tagmentation rate may be associated with a possibility that the corresponding off-target candidate is a bona-fide off-target, but is not particularly limited.

In some embodiments, the process of obtaining the tagmentation information by analyzing the analyzing-subject gnome DNA may further include an additional process for obtaining the tagmentation information. For example, this process may further include processing the information (or data) and/or normalizing the obtained information (or data) . For example, a process including comparing cleavage information obtained with information on predetermined on-targets may be further included. The process of obtaining the cleavage information may further include additional processes, as described above, and is not particularly otherwise limited.

In some embodiments, the tagmentation information may further include other information obtainable through the analysis of the analyzing-subject genome DNA (such as DNA sequencing), but is not particularly limited.

### Obtaining of information on off-targets

Based on the tagmentation information, information on off-targets may be obtained. Those skilled in the art to which the present application is related may obtain the information on the off-targets based on the cleavage information without particular difficulty. Accordingly, the disclosure herein does not limit the process of the off-target prediction system of the present application. Those skilled in the art to which the present application is related may obtain information on the off-targets using the tagmentation information obtained by analyzing the analyzing-subject genome DNA while undergoing an appropriate process or not undergoing a separate process.

In some embodiments, the off-target prediction method of the present application may include a process of confirming information on off-target candidates based on the tagmentation information.

In some embodiments, the information on the off-target candidates may include information on sites in the genome DNA of one or more off-target candidates (such as information on candidate off-target regions). For example, the information on the off-target candidate sites may include information on each site of all off-target candidates (sites in the genome DNA). For example, the information on the off-target candidate sites may include information on each site of one or more off-target candidates. In other words, information on all candidate off-target regions may be obtained. Alternatively, information on one or more candidate off-target regions, but not all candidate off-target regions, may be obtained. Of all off-target candidates, bona-fide off-targets (such as actual off-targets occurring from the use of prime editing systems) may exist. The information on the off-target candidate sites may be obtained based on the tagmentation information described above.

In one embodiment, the information on the off-target candidates may include off-target scores of one or more off-target candidates (for example, off-target predicting scores). For example, the information on the off-target candidates may include an off-target score of each off-target candidate for all off-target candidates. For example, the information on the off-target candidates may include an off-target score of each off-target candidate for one or more off-target candidates. In other words, the off-target scores for all candidate off-target regions may be obtained. Alternatively, off-target scores for one or more candidate off-target regions, but not all candidate off-target regions, may be obtained. Information on the off-target scores of the off-target candidates may be obtained based on the tagmentation information described above (such as information on tagmentation rates). In one embodiment, each rank of the off-target candidates may be calculated based on the obtained off-target scores. For example, off-target candidates (such as candidate off-target regions) with high off-target scores may rank higher. For example, the off-target candidate with the highest off-target score may rank first. For example, high off-target scores of the off-target candidates may be related to bona-fide off-targets, but are not particularly limited.

In one embodiment, the information on the off-target candidates may include information on the number of off-target candidates. For example, the total number of off-target candidates may be calculated. For example, in the calculation of the number of off-target candidates, overlapping sites may be counted as one. For another example, in the calculation of the number of off-target candidates, overlapping sites may be counted as the plural. For example, when the number of candidate off-target region X found is five, the total number thereof may be counted as 1 or 5. Through the information on the number of off-target candidates, the total number of off-target candidates that may occur during the genome editing process by prime editing may be confirmed. In other words, the total number of off-targets predicted may be confirmed.

In one embodiment, the information on the off-targets or off-target candidates may include, but is not particularly limited to, any one or more from:
the off-target candidate sites in the genome DNA for one or more off-target candidates;
the off-target score of each off-target candidate for one or more off-target candidates; and
the number of off-target candidates predicted.

In some embodiments, the process of obtaining the information on the off-target candidates may further include an additional process for obtaining the information on the off-target candidates. For example, this process may further include processing the information (or data) and/or normalizing the obtained information (or data). For example, a process of comparing the obtained information on off-target candidates with information on a predetermined on-target may be further included. The process of obtaining the information on the off-target candidates may further include additional processes, as described above, and is not particularly otherwise limited.

In some embodiments, the information on the off-target candidates may further include additional information that helps predict off-targets that may occur from the use of the prime editing system, but is not particularly limited.

### Comparison of off-target candidates with tpegRNA

As described above, the tag may be inserted into the off-target candidate sites (that is, candidate off-target regions). In traditional CRISPR/Cas systems, it is known that off-targets may result from a partial but sufficient match between a guide sequence and a target sequence. Similarly, in the prime editing system, although off-targets are expected to result from a partial but sufficient match between the sequence of each element of the tpegRNA and the target sequence, the reason why the off-targets occur is not limited herein. In some embodiments, off-targets may result from one or more mismatches between the sequence of the tpegRNA and the sequence of the off-target. In this case, the mismatch includes a base mismatch (for example, a difference in one or more nucleotides) and a bulge mismatch (for example, additions of one or more nucleotides or deletions of one or more nucleotides). In some embodiments, the sequence of the off-target (or off-target candidates) may include 1, 2, 3, 4, 5, 6, 7, 8, 9, 10, 11, 12, 13, 14, 15, 16, 17, 18, 19, or 20 or more mismatches, compared to the corresponding sequence of the tpegRNA. In some embodiments, the sequence of the off-target (off-target candidates) may have a 60%, 65%, 70%, 75%, 80%, 85%, 90%, 95%, 98%, 99%, or 100% sequence identity, or may have a sequence identity within a range set by two values selected from the above-described values, compared to the corresponding sequence of the tpegRNA. For example, the sequence of the spacer of the tpegRNA and a sequence corresponding to a spacer of the off-target (or off-target candidate) may include 1, 2, 3, 4, 5, 6, 7, 8, 9, or 10 or more mismatches. For another example, the PAM sequence and a sequence corresponding to a PAM sequence of the off-target (or off-target candidates) may include 1, 2, 3, 4, or 5 or more mismatches. For example, the DNA synthesis template of the tpegRNA and a sequence corresponding to a DNA synthesis template of the off-target (or off-target candidates) may include 1, 2, 3, 4, 5, 6, 7, 8, 9, or 10 or more mismatches. For example, the homology region of the tpegRNA and a sequence corresponding to a homology region of the off-target (or off-target candidates) may include 1, 2, 3, 4, 5, 6, 7, 8, 9, or 10 or more mismatches. For example, the primer binding site of the tpegRNA and a sequence (such as sequences functioning as primers) corresponding to a primer binding site of the off-target (or off-target candidates) may include 1, 2, 3, 4, 5, 6, 7, 8, 9, or 10 or more mismatches. For example, one or more mismatches may exist in one or more of the sequences corresponding to the spacer of the off-target (or off-target candidates), corresponding to the PAM sequence of the off-target (or off-target candidates), corresponding to the DNA synthesis template of the off-target (or off-target candidates), and corresponding to the primer binding site of the off-target (or off-target candidates), but is not limited thereto.

### Comparison of off-target candidates with on-target

As described above, the tag may be inserted into the off-target candidate sites (that is, candidate off-target regions). The off-target candidate refers to an off-target predicted through a prediction system, which may or may not be a bona-fide off-target. In some embodiments, the off-target candidate region may refer to a specified site. In some embodiments, an on-target site or on-target region, or the off-target candidate site or off-target candidate region may be understood as a specific region, in which case this specific region may refer to a region composed of about 1, 2, 3, 4, 5, 6, 7, 8, 9, 10, 20, 30, 40, 50, 60, 70, 80, 90, 100, 110, 120, 130, 140, 150, 160, 170, 180, 190, 200, 220, 240, 260, 280, 300, 320, 340, 360, 380, 400, 450, or 500 consecutive nucleotides, or may refer to a region composed of the number of consecutive nucleotides exceeding the above-described value. In some aspects, the greater the number of consecutive nucleotides, the more accurately the off-target or on-target region may be designated. This is because the greater the number of nucleotides, the lower the possibility that the same sequences (duplicate sequences) are present in the genome DNA.

The off-target or off-target candidate may be compared with a sequence of an on-target. In some embodiments, the off-target candidate or a bona-fide off-target may include 1, 2, 3, 4, 5, 6, 7, 8, 9, 10, 11, 12, 13, 14, 15, 16, 17, 18, 19, or 20 or more mismatches (on-target mismatches) compared to the sequence of the on-target. In some embodiments, the sequence of the off-target (off-target candidates) may have a 60%, 65%, 70%, 75%, 80%, 85%, 90%, 95%, 98%, 99%, or 100% sequence identity, or may have a sequence identity within a range set by two values selected from the above-described values, compared to the corresponding sequence of the on-target. The mismatch, used for the comparison of the on-target with the off-target, is used to explain the difference in the sequences between the off-target and the on-target. Furthermore, the mismatch is used as including both a nucleotide mismatch (for example, a difference in nucleotides) and a bulge mismatch (for example, additions of one or more nucleotides or deletions of one or more nucleotides). For example, when a sequence corresponding to a spacer of the off-target candidate is GGCACTGaGGgTGGAGGTGG (SEQ ID NO: 51), and a sequence corresponding to a spacer of the on-target is GGCACTGCGGCTGGAGGTGG (SEQ ID NO: 52), the sequence corresponding to the spacer of the off-target candidate may be described as having two nucleotide mismatches (written in lowercase letters) compared to the sequence of the on-target. For another example, when the sequence corresponding to the spacer of the off-target candidate is GGCACTGC--CTGGAGGTGG (SEQ ID NO: 53), and the sequence corresponding to the spacer of the on-target is GGCACTGCGGCTGGAGGTGG (SEQ ID NO: 54), the sequence corresponding to the spacer of the off-target candidate may be described as having two bulge mismatches (for example, two bulge on-target mismatches) compared to the sequence of the on-target. For a further example, when the sequence corresponding to the spacer of the off-target candidate is GGCACTGCGGCTGGAGgTGG (SEQ ID NO: 55), and the sequence corresponding to the spacer of on-target is GGCACT-GGCTGGAGGTGG (SEQ ID NO: 56), the sequence corresponding to the spacer of the off-target candidate may be described as having one nucleotide mismatch and two bulge mismatches (total of three mismatches) compared to the sequence of the on-target. Hereinbelow, the sequence of off-targets (or off-target candidates) is to be compared with the sequence of the on-target and described.

In some embodiments, the sequence corresponding to the spacer of the off-target (or off-target candidates) may include 0, 1, 2, 3, 4, 5, 6, 7, 8, 9, or 10 or more mismatches (such as on-target mismatches). In some embodiments, the sequence corresponding to the PAM sequence of the off-target (or off-target candidates) may include 0, 1, 2, 3, 4, or 5 or more mismatches. In some embodiments, the sequence corresponding to the DNA synthesis template of the off-target (or off-target candidates) may include 0, 1, 2, 3, 4, 5, 6, 7, 8, 9, or 10 or more mismatches. In some embodiments, the sequence corresponding to the homology region of the off-target (or off-target candidates) may include 0, 1, 2, 3, 4, 5, 6, 7, 8, 9, or 10 or more mismatches. In some embodiments, the sequence corresponding to the primer binding site of the off-target (or off-target candidates) may include 0, 1, 2, 3, 4, 5, 6, 7, 8, 9, or 10 or more mismatches. In some embodiments, one or more mismatches may exist in any one or more of the sequences corresponding to the spacer of the off-target (or off-target candidates), corresponding to the PAM sequence of the off-target (or off-target candidates), corresponding to the DNA synthesis template of the off-target (or off-target candidates), and the primer binding site of the off-target (or off-target candidates).

In some embodiments, in any one or more of the regions corresponding to the spacer, the PAM, the PBS, and the DNA synthesis template, the off-target candidate (or off-target) region may include 0, 1, 2, 3, 4, 5, 6, 7, 8, 9, 10, 11, 12, 13, 14, 15, 16, 17, 18, 19, 20, 21, 22, 23, 24, or 25 or more on-target mismatches, or may include an on-target mismatch within a range set by two values selected from the above-described values. In some embodiments, in the regions corresponding to the spacer and the DNA synthesis template, the off-target candidate (or off-target) region may include 0, 1, 2, 3, 4, 5, 6, 7, 8, 9, 10, 11, 12, 13, 14, 15, 16, 17, 18, 19, 20, 21, 22, 23, 24, or 25 or more on-target mismatches, or may include an on-target mismatch within a range set by two values selected from the above-described values. In certain embodiments, the off-target candidate (or off-target) region may include 0 to 20 on-target mismatches in the regions corresponding to the spacer and the DNA synthesis template. In certain embodiments, the off-target candidate (or off-target) region may include 1 to 15 on-target mismatches in the regions corresponding to the spacer and the DNA synthesis template. In certain embodiments, the off-target candidate (or off-target) region may include 1 to 10 on-target mismatches in the regions corresponding to the spacer and the DNA synthesis template. In this case, the on-target mismatch refers to a mismatch determined through the comparison with the sequence of the corresponding region of the on-target region. The on-target mismatch may be counted in a single strand or in both strands. For example, the off-target candidate (or off-target) region may include 0 to 10 on-target mismatches in the following regions of a spacer non-binding strand: (i) a region corresponding to a protospacer and (ii) a region composed of 5 to 20 nucleotides located downstream of the region corresponding to the protospacer. For example, the off-target candidate (or off-target) region may include 0 to 10 on-target mismatches in a region ranging from -30 to +10 or -20 to +10 relative to the cleavage site (nick or DSB).

### Relationship with prime editing system subject to prediction

The off-target prediction system of the present application may be associated with the prime editing system subject to prediction. In this case, the prime editing system subject to prediction may refer to a prime editing system used in research or determined to be used in therapeutics, but is not particularly limited. In other words, the prime editing system subject to prediction refers to a prime editing system (or genome editing processes by prime editing systems) for which off-targets need to be predicted.

For example, when using specific cells in the prime editing system subject to prediction, these specific cells may also be used in the method for predicting the off-targets of the present application. For another example, when using specific cells in the prime editing system subject to prediction, cells other than the above specific cells may be used in the method for predicting the off-targets of the present application. For example, cells derived from patients may be used in the prime editing system subject to prediction, and cells used in the off-target prediction system of the present application may be a human cell line.

For example, when using a tpegRNA having a specific sequence in the prime editing system subject to prediction, a tpegRNA having a sequence that is the same as or partially differs from the above sequence may be used in the method for predicting the off-targets of the present application. Similarly, when using a specific prime editor protein in the prime editing system subject to prediction, a prime editor protein that is the same or differs from the above protein in type may be used in the method for predicting the off-targets of the present application. For another example, in the method for predicting the off-targets of the present application, additional elements (such as a dnMLH1, sgRNAs, additional tpegRNAs, and/or the like) may be used, in addition to the elements in the prime editing system subject to prediction, and are not particularly limited.

In this aspect, the method for predicting the off-targets, according to one embodiment of the present application, may further include a process of confirming the prime editing system subject to prediction. The prime editing system subject to prediction may be called a predetermined prime editing system. The predetermined prime editing system may include the use of any one or more or a combination of a predetermined cell (such as cells subject to genome editing by prime editing systems), a predetermined prime editor protein, and a predetermined pegRNA.

In one embodiment, the method for predicting the off-targets of the present application may further include confirming or designing the predetermined prime editing system. The predetermined prime editing system may be confirmed, thereby enabling elements to be appropriately used in the off-target prediction system to be designed. In this case, the process of confirming the predetermined gene editing system may be performed before contacting the genome DNA of the cell with the prime editor protein and the tpegRNA. Hereinbelow, one example of confirming the predetermined prime editing system (that is, subject to prediction) is to be described.

In one embodiment, the method for predicting off-targets of the present application may include confirming the predetermined prime editing system. In this case, confirming the predetermined prime editing system may include confirming any one or more from information on the predetermined cell, the predetermined prime editor protein, and the predetermined pegRNA. The predetermined prime editing system, the predetermined cell, the predetermined prime editor protein, the predetermined pegRNA, and the like may be used with ordinal determiners, such as a first prime editing system, a first cell, a first prime editor protein, a first pegRNA, and the like.

In certain embodiments, confirming the predetermined prime editing system may include confirming the predetermined cell. In certain embodiments, a cell that is the same as the predetermined cell may be used in the off-target prediction system of the present application. In certain embodiments, a cell that differs from the predetermined cell may be used in the off-target prediction system of the present application. For example, the predetermined cell may be a human cell rather than a cell line, and in the off-target prediction system of the present application, a human cell line may be used. In some embodiments, the predetermined cell may be an animal cell or plant cell. In some embodiments, the predetermined cell may be a human cell or a cell of non-human animals (such as mice, rats, monkeys, chimpanzees, dogs, cats, cows, pigs, horses, sheep, and the like), but is not particularly limited. In some embodiments, the predetermined cell may be a cell derived from a patient. In some embodiments, the predetermined cell may be a cell from a cell line (such as human, mouse, monkey, or rat cell lines). Examples of the cells from the cell line may include 3T3 cells, A549 cells, HeLa cells, HEK 293 cells, K562 cells, Huh7 cells, Jurkat cells, OK cells, Ptk2 cells, or Vero cells, but are not limited thereto.

In certain embodiments, confirming the predetermined prime editing system may include confirming the predetermined prime editor protein. In certain embodiments, a prime editor protein that is the same as the predetermined prime editor protein may be used in the off-target prediction system of the present application. In certain embodiments, a prime editor protein that differs from the predetermined prime editor protein may be used in the off-target prediction system of the present application. For example, although the predetermined prime editor protein may be the PE2 prime editor protein, the prime editor protein used in the off-target prediction system of the present application may be the PE2-nuclease prime editor protein or PEmax-nuclease prime editor protein. Other types of prime editor proteins may be used to increase tagmentation rates.

In certain embodiments, confirming the predetermined prime editing system may include confirming the predetermined pegRNA. In certain embodiments, a tpegRNA that is the same as the predetermined pegRNA may be used in the off-target prediction system of the present application (in which case, the tpegRNA same as the predetermined pegRNA shows that all sequences are the same except for the tag template). In certain embodiments, a tpegRNA that differs from the predetermined pegRNA may be used in the off-target prediction system of the present application. Hereinbelow, the relationship between the predetermined pegRNA and the tpegRNA used in the off-target prediction system of the present application is to be described.

The predetermined pegRNA may be called a first pegRNA, and the first pegRNA includes a first spacer, a first DNA synthesis template, and a first primer binding site. The tpegRNA used in the off-target prediction system of the present application is called a second tpegRNA for convenience. The second tpegRNA includes a second spacer, a second DNA synthesis template, a second tag template, and a second primer binding site. Furthermore, the second tpegRNA may further include a 3' engineering region. In this case, an etpegRNA developed on the basis of an epegRNA, other than the type of first pegRNA, may be used in the off-target prediction method of the present application.

In some embodiments, the second spacer may have the same sequence as a sequence of the first spacer, or may have a sequence having about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity to the sequence of the first spacer.

In some embodiments, the second primer binding site may have the same sequence as a sequence of the first primer binding site, or may have a sequence having about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity to the sequence of the first primer binding site.

In some embodiments, the second DNA synthesis template may have the same sequence as a sequence of the first DNA synthesis template, or may have a sequence having about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity to the sequence of the first DNA synthesis template.

In some embodiments, a second extension region may have the same sequence as a sequence of a first extension region except for the tag template, or may have a sequence having about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity to the sequence of the first extension region except for the tag template.

In some embodiments, although the first pegRNA is not the epegRNA but the pegRNA, the tpegRNA used in the off-target prediction method of the present application may further include the 3' engineering region (for example, using the etpegRNA) .

In some embodiments, the first DNA synthesis template may include a first editing template, but the second DNA synthesis template may be editing template-free. In some embodiments, the first DNA synthesis template may include the first editing template, and the second DNA synthesis template may include a second editing template. In this case, the second editing template may have the same sequence as a sequence of the first editing template, or may have a sequence having about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity to the sequence of the first editing template. In some embodiments, the second editing template may have a sequence that differs from the sequence of the first editing template.

In some embodiments, the first DNA synthesis template may include a first homology region, and the second DNA synthesis template may include a second homology region. In some embodiments, the second homology region may have the same sequence as a sequence of the first homology region, or may have a sequence having about 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99%, or 99.9% sequence identity to the sequence of the first homology region.

### May be used with additional off-target prediction systems

In some embodiments, the off-target system of the present application may be used with one or more additional off-target prediction systems. For example, the additional off-target systems may be off-target prediction systems in prime editing using different types of prime editor proteins and/or tpegRNAs. In other words, the additional off-target system may be TAPE-seq using different types of prime editor proteins and/or tpegRNAs. For example, a first off-target prediction system using a first prime editor protein and a first tpegRNA may be used with a second off-target prediction system using a second prime editor protein and a second tpegRNA. In certain embodiments, the first prime editor protein may be the same as or differ from the second prime editor protein in type. In certain embodiments, the first tpegRNA may have a sequence that is the same as or partially differs from that of the second tpegRNA. For example, a first editing template of the first tpegRNA may differ from a second editing template of the second tpegRNA. For example, tools for predicting off-targets in first prime editing may be used to perform a first TAPE-seq, tools for predicting off-targets in second prime editing may be used to perform a second TAPE-seq, and the results of the first TAPE-seq and the second TAPE-seq may be combined to be used as the results for predicting off-targets. In this case, any one or more of the tools for predicting off-targets in the second prime editing may differ from the corresponding elements included in the tools for predicting off-targets in the first prime editing. For example, a sequence of an editing template of a tpegRNA in the tools for predicting the off-targets in the first prime editing may differ from a sequence of an editing template of a tpegRNA in the tools for predicting the off-targets in the second prime editing. For another example, the tools for predicting the off-targets in the first prime editing may include PEmax-nuclease and the tepegRNA, but the tools for predicting the off-targets in the second prime editing may include PE2 and the tpegRNA, not the tepegRNA (that is, the 3' engineering region-free tpegRNA). For another example, the first TAPE-seq and the second TAPE-seq may use the same tools for predicting the off-targets in the prime editing, but sequencing platforms used for tagmentation analysis in each TAPE-seq may differ. For another example, the first TAPE-seq, the second TAPE-seq, and a third TAPE-seq may be performed, and the results of the first TAPE-seq, the second TAPE-seq, and the third TAPE-seq may be combined to be used as the results for predicting off-targets. As described above, the number of off-target prediction systems in prime editing that may be additionally used and the configuration of each off-target prediction system are not particularly limited.

In some embodiments, the additional off-target prediction system may be other off-target prediction systems. For example, the off-target prediction system of the present application may be used with any one or more selected from Cas-OFFinder, CHOPCHOP, CRISPOR, Digenome-seq, nDigenome-seq, DIG-seq, SITE-seq, CIRCLE-seq, CHANGE-seq, GUIDE-seq, GUIDE-tag, DISCOVER-seq, BLISS, BLESS, integrase-defective lentiviral vector-mediated DNA break capture, HTGTS, ONE-seq, CReVIS-Seq, ITR-seq, and TAG-seq. For the purpose of more efficiently finding bona-fide off-target regions, other off-target prediction systems may be used with the off-target prediction system of the present application. Additionally, other off-target prediction systems may be off-target prediction systems developed before or after the filing date of the present application, and are not particularly limited.

### Advantages of off-target prediction system of present application

The inventors of the present application have thoroughly tested the off-target prediction method provided by the present application. It was confirmed by comparing the off-target prediction method of the present application with other off-target prediction methods, that the off-target prediction method of the present application showed better performance than other off-target prediction methods (see experimental examples herein). The off-target prediction method of the present application uses a molecular mechanism of the prime editing system and thus shows numerous advantages in predicting off-targets in the prime editing system compared to other off-target prediction methods.

The off-target prediction method of the present application may exhibit a lower false positive rate than known off-target prediction methods. The off-target prediction method of the present application may exhibit a lower miss rate than known off-target prediction methods. The miss rate may mean missing a bona-fide off-target. For example, failure to locate a bona-fide off-target region as an off-target candidate results in a higher false negative rate. For example, the miss rate may be the number of off-target regions validated by the off-target prediction system divided by the total number of validated off-target regions. The off-target prediction method of the present application may be performed using a surrogate cell. For example, genome editing by the prime editing system involves various cell-specific processes, making it challenging to predict off-targets through in vitro-based off-target prediction. Accordingly, cell-based off-target prediction methods are required to be used. Known cell-based prediction methods cause inaccurate results when using surrogate cells. However, the off-target prediction method of the present application may derive further accurate results even when using surrogate cells (such as human cell lines).

The inventors of the present application confirmed through a large number of and many types of experiments that the off-target prediction method of the present application is actually operable in relation to the off-target prediction in prime editing. Furthermore, the performance of the off-target prediction method of the present application was tested through various experiments. The validation results of the performance of the off-target prediction method of the present application are confirmed through the experimental examples of the present application.

In one embodiment, the validation rate of the off-target prediction method of the present application may be 10%, 15%, 20%, 25%, 30%, 35%, 40%, 45%, 50%, 55%, 60%, 65%, 70%, 75%, 80%, 85%, 90%, 95%, or 100% or higher, but is not particularly limited. The validation rate may refer to a ratio of bona-fide off-targets among off-target candidates predicted through the off-target prediction method. In one embodiment, the validation rate calculated on the basis of the off-target candidates confirmed by the off-target prediction method of the present application may fall within a range formed by two values of the above-described values, but is not particularly limited. The validation rate may be affected by the types of prime editing systems used in the off-target prediction system (such as types of prime editor proteins and/or tpegRNAs), type of cells, and the like.

In one embodiment, the miss rate of the off-target prediction method of the present application may be 0, or 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, or 40% or lower, but is not particularly limited. In one embodiment, the miss rate of the off-target prediction method of the present application may fall within a range formed by two values selected from the above-described values, but is not particularly limited. The miss rate may be affected by the types of prime editing systems used in the off-target prediction system, the types of cells, and the like. In one embodiment, the number of bona-fide off-targets missed by the off-target prediction method of the present application may be 0, or 1, 2, 3, 4, 5, 6, 7, 8, 9, or 11 or less, but is not particularly limited.

In one embodiment, a receiver operating characteristic (ROC) curve may be drawn for the off-target prediction method of the present application. In one embodiment, the area under the receiver operating characteristic curve (AUC) may be calculated for the off-target prediction method of the present application. The ROC curve and the area under the ROC curve are powerful tools that may show the diagnostic ability of a binary classifier system. Typically, ROC curves are plotted by comparing a true positive rate (TPR) with a false positive rate (FPR) or by comparing sensitivity with specificity. For example, a TPR may be shown on the y-axis, and an FPR may be shown on the x-axis to plot an ROC curve. For example, sensitivity may be shown on the y-axis, and specificity may be shown on the x-axis to plot an ROC curve. An area under the ROC curve closer to 1 (an area under the ROC curve with a larger width) indicates that the model performs better. In one embodiment, the area under the ROC curve for the off-target prediction method of the present application may be calculated. In this case, the area under the ROC curve may be about 0.4, 0.42, 0.44, 0.46, 0.48, 0.5, 0.52, 0.54, 0.56, 0.58, 0.6, 0.62, 0.64, 0.66, 0.68, 0.7, 0.72, 0.74, 0.75, 0.76, 0.77, 0.78, 0.79, 0.8, 0.81, 0.82, 0.83, 0.84, 0.85, 0.86, 0.87, 0.88, 0.89, 0.9, 0.91, 0.92, 0.93, 0.94, 0.95, 0.96, 0.97, 0.98, or 0.99 or higher, or 1, but is not limited thereto. In one embodiment, the area under the ROC curve calculated for the off-target prediction method of the present application may fall within a range of two values selected from the above-described values, but is not particularly limited. The area under the ROC curve may be affected by the types of prime editing systems used in the off-target prediction system, the types of cells, and the like.

### Composition for predicting off-target

Some embodiments of the present application provide a composition including the tpegRNA or the nucleic acid encoding the tpegRNA. Some embodiments of the present application provide a composition including the tpegRNA or the nucleic acid encoding the tpegRNA and the prime editor protein or the nucleic acid encoding the prime editor protein. In some embodiments, the composition including the tpegRNA or the nucleic acid encoding the tpegRNA and/or the prime editor protein or the nucleic acid encoding the prime editor protein of the present application may be used for off-target prediction. In other words, some embodiments of the present application provide a composition for predicting the off-targets. The composition for predicting the off-targets may be used to confirm information on off-targets occurring in the process of editing a DNA molecule (such as genome DNA) by prime editing or to predict off-targets. For example, the composition for predicting the off-targets of the present application may be used in the off-target prediction method in the prime editing system of the present application.

### Exemplary embodiment of off-target prediction method of present application (1)

Hereinbelow, exemplary embodiments of the off-target prediction method of the present application are to be provided. In some embodiments, the off-target prediction method of the present application may be called an off-target prediction system in prime editing. In some embodiments, the off-target prediction method of the present application may be a method for confirming information on an off-target that may occur in the process of editing a DNA molecule (such as genome DNA) by prime editing. In some embodiments, the off-target prediction method of the present application may be called a method or system for confirming an off-target that may occur in the process of editing genome DNA by prime editing. A description in the meaning of "for predicting an off-target occurring in the process of editing genome DNA by prime editing" may be used to describe the off-target prediction method of the present application without limitation. The off-target prediction may be achieved by obtaining information on an off-target candidate or information on a candidate off-target region.

Throughout this specification, terms used herein may be interpreted as including plural concepts even when written in singular forms. In other words, terms written in singular forms will be understood as including plural concepts as necessary. In the case of being described as "analyzing analyzing-subject genome DNA", this may be interpreted as meaning analyzing the DNA of one or a plurality of analyzing-subject genomes.

In some embodiments, the present application provides a method for predicting an off-target occurring in a process of editing genome DNA of a cell by a prime editing system, the method including:
(a) obtaining analyzing-subject genome DNA by contacting genome DNA with a prime editor protein and a tpegRNA,
   wherein the prime editor protein includes a Cas protein and a reverse transcriptase,
   wherein the tpegRNA includes a spacer, a gRNA core, and an extension region, wherein the extension region includes a reverse transcription template (RT template), a tag template, and a primer binding site (PBS); and
(b) obtaining tagmentation information on the analyzing-subject DNA by analyzing the analyzing-subject genome DNA.

In certain embodiments, contacting the genome DNA of the cell with the prime editor protein and the tpegRNA may be performed in a cell.

In certain embodiments, contacting the genome DNA of the cell with the prime editor protein and the tpegRNA may be performed in the nucleus of a cell.

In certain embodiments, the prime editor protein and the tpegRNA may be in contact with the genome DNA in a prime editor protein/tpegRNA complex form.

In certain embodiments, after contacting the genome DNA with the prime editor protein and the tpegRNA, whether the genome DNA of the cell is modified may be confirmed.

In certain embodiments, as a result of contacting the genome DNA with the prime editor protein and the tpegRNA, a tag sequence may be inserted into the genome DNA, and the analyzing-subject genome DNA containing the tag sequence may be obtained.

In certain embodiments, the tag sequence may be inserted into the analyzing-subject genome DNA through a reverse transcription process performed by the reverse transcriptase using the tag template of the tpegRNA as a template.

In certain embodiments, the RT template may include an editing template and a homology region.

In certain embodiments, the analyzing-subject genome DNA may contain one or more tag sequences.

In certain embodiments, the one or more tag sequences may independently be inserted into sites corresponding to each on-target and/or off-target candidate in the genome DNA through the reverse transcription process by the reverse transcriptase. In this case, the analyzing-subject genome DNA may contain the one or more tag sequences.

In certain embodiments, the tag sequence may be inserted into an on-target site.

In certain embodiments, the tag sequence may be inserted into a candidate off-target site.

In certain embodiments, analyzing the analyzing-subject genome DNA may include: analyzing the analyzing-subject DNA genome by sequencing.

In certain embodiments, analyzing the analyzing-subject genome DNA may include: generating an amplified tag-specific library by amplifying the analyzing-subject genome DNA genome tag-specifically; and analyzing the tag-specific library by sequencing.

In certain embodiments, analyzing the analyzing-subject genome DNA may include: generating a tag-specific library from the analyzing-subject genome DNA; and sequencing the tag-specific library.

In certain embodiments, analyzing the analyzing-subject genome DNA may include: generating a tag-specific library from the analyzing-subject genome DNA; generating an amplified tag-specific library by amplifying the tag-specific library; and sequencing the tag-specific library.

In certain embodiments, the tagmentation information may include: information on whether the tag sequence is contained in the analyzing-subject genome DNA.

In certain embodiments, the tagmentation information may include: a site in the genome DNA of each tag sequence for one or more tag sequences.

In certain embodiments, the tagmentation information may include: a tagmentation rate for each tagged region.

In certain embodiments, the tagmentation information may include one or more from: the information on whether the tag sequence is contained in the analyzing-subject genome DNA; the site in the genome DNA of each tag sequence for one or more tag sequences; and the tagmentation rate for each tagged region.

In certain embodiments, the method for predicting the off-target of the present application may further include: confirming information on the off-target based on the tagmentation information.

In certain embodiments, the method for predicting the off-target of the present application may further include: confirming information on the on-target.

In certain embodiments, the information on the off-target may include: whether the off-target candidate exists. In this case, whether the off-target candidate exists may be obtained from the information on whether the tag sequence is contained in the analyzing-subject genome DNA.

In certain embodiments, the information on the off-target may include: a site in the genome DNA of each off-target candidate for one or more off-target candidates (candidate off-target region). In this case, the site in the genome DNA of each off-target candidate for one or more off-target candidates may be obtained from the site in the genome DNA of each tag sequence for one or more tag sequences.

In certain embodiments, the information on the off-target may include: an off-target predicting score of each off-target candidate for one or more off-target candidates. In this case, the off-target predicting score of each off-target candidate for one or more off-target candidates may be obtained from the tagmentation rate for each tagged region.

In certain embodiments, the Information on the off-target may include: the number of off-target candidates predicted.

In certain embodiments, the information on the off-target may include one or more from: whether the off-target candidate exists; the site in the genome DNA of each off-target candidate for one or more off-target candidates; the off-target predicting score of each off-target candidate for one or more off-target candidates; and the number of the off-target candidates predicted.

In certain embodiments, the method for predicting the off-target of the present application may further include: confirming information on a predetermined prime editing system. In certain embodiments, the predetermined prime editing system includes the use of a first pegRNA, wherein the first pegRNA may include a first primer binding site, a first DNA synthesis template, and a first spacer. In this case, a sequence of a DNA synthesis template of the tpegRNA may be the same as a sequence of the first DNA synthesis template. In this case, a sequence of the primer binding site of the tpegRNA may be the same as a sequence of the first primer binding site. In this case, a sequence of the spacer of the tpegRNA may be the same as a sequence of the first spacer. In certain embodiments, the first pegRNA may be 3' engineering region-free, and the tpegRNA may further include a 3' engineering region. In certain embodiments, the predetermined prime editing system includes the use of a first prime editor protein, wherein the prime editor protein used in the off-target prediction method of the present application may be a prime editor protein that differs from the first prime editor protein. In certain embodiments, the sequence of the spacer of the tpegRNA may have an 80% or higher sequence identity to a sequence of the spacer of a predetermined pegRNA. In certain embodiments, the sequence of the primer binding site of the tpegRNA may have an 80% or higher sequence identity to a sequence of the primer binding site of the predetermined pegRNA. In certain embodiments, a sequence of the homology region of the tpegRNA may have an 80% or higher sequence identity to a sequence of the homology region of the predetermined pegRNA.

In certain embodiments, the Cas protein may have nuclease activity. In certain embodiments, the Cas protein may be Cas9. In certain embodiments, the Cas protein may be wild-type SpCas9. In certain embodiments, the Cas protein may be a SpCas9 variant. In certain embodiments, the Cas protein may be a SpCas9 variant including R221K and N394K variations.

In certain embodiments, the Cas protein may have nickase activity. In certain embodiments, the Cas protein may be a Cas9 nickase. In certain embodiments, the Cas protein may be a SpCas9 variant including an H840A variation. In certain embodiments, the Cas protein may be a SpCas9 variant including R221K, N394K, and H840A variations. In certain embodiments, the Cas protein may be a SpCas9 variant including a D10A variation. In certain embodiments, the Cas protein may be a SpCas9 variant including R221K, N394K, and D10A variations.

In certain embodiments, the reverse transcriptase may be a wild-type Moloney murine leukemia virus reverse transcriptase (MMLV reverse transcriptase). In certain embodiments, the reverse transcriptase may be an MMLV reverse transcriptase variant. In certain embodiments, the reverse transcriptase may be an MMLV reverse transcriptase including D200N, T306K, W313F, T330P, and L603W variations.

In certain embodiments, the Cas protein and/or the reverse transcriptase may be codon optimized.

In certain embodiments, the prime editor protein may be a PE2 prime editor protein. In certain embodiments, the prime editor protein may be a PE2-nuclease prime editor protein. In certain embodiments, the prime editor protein may be a PEmax prime editor protein. In certain embodiments, the prime editor protein may be a PEmax-nuclease prime editor protein.

In certain embodiments, the tpegRNA may include the 3' engineering region. In certain embodiments, the tpegRNA may be an etpegRNA.

In certain embodiments, a dnMLH1 may be involved in the process of obtaining the analyzing-subject DNA.

In certain embodiments, the tag template may be located in between the primer binding site and the RT template. In certain embodiments, the RT template, the tag template, and the primer binding site of the tpegRNA may be located in such an order in the 5' to 3' direction in the extension region of the tpegRNA.

In certain embodiments, the tpegRNA may be a pegRNA designed so that the tag sequence is enabled to be inserted into the genome DNA by the reverse transcriptase of the prime editor protein.

In certain embodiments, the length of the tag template may be 5 to 60 nt. In certain embodiments, the length of the tag template may be 15 to 40 nt. In certain embodiments, the length of the tag template may be about 19 nt, 24 nt, 29 nt, or 34 nt.

In certain embodiments, the method for predicting the off-target may further include: contacting the prime editor protein or a nucleic acid encoding the prime editor and the tpegRNA or a nucleic acid encoding the tpegRNA with a cell.

In some embodiments, the present application provides a method for obtaining information on one or more off-targets that may occur in a process of prime editing, the method including:
(a) preparing a manipulated cell,
   wherein preparing the manipulated cell includes: manipulating a genome of a cell using a tpegRNA and a prime editing protein including a Cas protein and a reverse transcriptase,
   wherein the tpegRNA includes a spacer region, a gRNA core, and an extension region,
   wherein the extension region includes a reverse transcription template, a tag template, and a primer binding site (PBS),
   wherein the manipulated cell contains the manipulated genome,
   wherein the manipulated genome contains one or more tag sequences, wherein each of the tag sequences is inserted into the genome of the cell through a reverse transcription process performed by the reverse transcriptase using the tag template of the tpegRNA as a template of the reverse transcription process;
(b) analyzing the manipulated cell obtained through (a) to obtain analysis data on the manipulated cell;
(c) obtaining tagmentation information based on the analysis data obtained through (b); and
(d) confirming information on one or more off-targets that may occur in the process of genome editing by prime editing, based on the tagmentation information obtained through (c).

In certain embodiments, the RT template may include an editing template and a homology region.

In certain embodiments, the tag sequence may be inserted into an on-target region.

In certain embodiments, the tag sequence may be inserted into one or more candidate off-target regions.

In certain embodiments, the tagmentation information obtained through (c) may include any one or more from: information on whether the tag sequence is inserted into the genome of the cell; information on chromosomes where one or more tag sequences are inserted; information on sites where one or more tag sequences are inserted; information on the chromosomes where one or more tag sequences are inserted and sites in the chromosomes; and information on insertion rates by the sites where one or more tag sequences are inserted.

In certain embodiments, the off-target that may occur in the process of genome editing by the prime editing is an off-target candidate, and the information on one or more off-targets that may occur in the process of genome editing by the prime editing, confirmed through (d), may include any one or more from: information on whether the off-target candidate exists; information on chromosomes where one or more off-target candidates occur; information on sites where one or more off-target candidates occur; information on the chromosomes where one or more off-target candidates occur and sites in the chromosomes; and information on possibilities of one or more off-target candidates by the sites thereof.

In certain embodiments, the tagmentation information may include: information on whether the tag sequence is contained in manipulated genome DNA.

In certain embodiments, the tagmentation information may include: a site in the genome DNA of each tag sequence for one or more tag sequences.

In certain embodiments, the tagmentation information may include: a tagmentation rate for each tagged region.

In certain embodiments, the tagmentation information may include one or more from: the information on whether the tag sequence is contained in the manipulated genome DNA; the site in the genome DNA of each tag sequence for one or more tag sequences; and the tagmentation rate for each tagged region.

In certain embodiments, the method for predicting the off-target of the present application may further include: confirming information on the on-target.

In certain embodiments, the information on the off-target may include: whether the off-target candidate exists. In this case, whether the off-target candidate exists may be obtained from the information on whether the tag sequence is contained in the analyzing-subject genome DNA.

In certain embodiments, the information on the off-target may include: a site in the genome DNA of each off-target candidate for one or more off-target candidates (candidate off-target region). In this case, the site in the genome DNA of each off-target candidate for one or more off-target candidates may be obtained from the site in the genome DNA of each tag sequence for one or more tag sequences.

In certain embodiments, the information on the off-target may include: an off-target predicting score of each off-target candidate for one or more off-target candidates. In this case, the off-target predicting score of each off-target candidate for one or more off-target candidates may be obtained from the tagmentation rate for each tagged region.

In certain embodiments, the Information on the off-target may include: the number of off-target candidates predicted.

In certain embodiments, the information on the off-target may include one or more from: whether the off-target candidate exists; the site in the genome DNA of each off-target candidate for one or more off-target candidates; the off-target predicting score of each off-target candidate for one or more off-target candidates; and the number of the off-target candidates predicted.

In certain embodiments, the length of the tag template herein may be 5 to 60 nt.

In certain embodiments, the length of the tag template herein may be 15 to 40 nt.

In certain embodiments, the length of the tag template herein may be about 19 nt.

In certain embodiments, the length of the tag template herein may be about 24 nt.

In certain embodiments, the length of the tag template herein may be about 29 nt.

In certain embodiments, the length of the tag template herein may be about 34 nt.

In certain embodiments, the prime editing protein may induce nicking of an editing-subject DNA molecule.

In certain embodiments, the prime editing protein may induce a DSB in the editing-subject DNA molecule.

In certain embodiments, the prime editing protein may be a PE2 prime editing protein.

In certain embodiments, the prime editing protein may be a PE2-nuclease.

In certain embodiments, the prime editing protein may be a PEmax prime editing protein.

In certain embodiments, the prime editing protein may be a PEmax-nuclease.

In certain embodiments, the prime editing protein may be the same or differ from the predetermined prime editing protein.

In certain embodiments, the tpegRNA may be an etpegRNA.

In certain embodiments, the tag template may be located in between the editing template and the primer binding site in the extension region of the tpegRNA.

In certain embodiments, a sequence contained in the spacer region of the tpegRNA may have a 90% or higher sequence identity to a sequence in a region of the spacer of the predetermined pegRNA.

In certain embodiments, a sequence contained in the primer binding site of the tpegRNA may have a 90% or higher sequence identity to the sequence of the primer binding site of the predetermined pegRNA.

In certain embodiments, a sequence contained in the homology region of the tpegRNA may have a 90% or higher sequence identity to the sequence of the homology region of the predetermined pegRNA.

In certain embodiments, (a) may further include: preparing the prime editing protein and the tpegRNA in a cell.

In certain embodiments, (a) may further include: introducing the prime editing protein, a fragment thereof, or a nucleic acid encoding the prime editing protein and the tpegRNA, a fragment thereof, or a nucleic acid encoding the tpegRNA into a cell. In certain embodiments, the prime editing protein, the fragment thereof, or the nucleic acid encoding the prime editing protein and the tpegRNA, the fragment thereof, or the nucleic acid encoding the tpegRNA herein may be introduced into the cell by one or more vectors.

In certain embodiments, the method for obtaining the information on the off-target may further include: destroying the manipulated cell. In this case, destroying the manipulated cell may be performed prior to (b).

In certain embodiments, the method for obtaining the information on the off-target may further include: extracting DNA from the manipulated cell. In this case, extracting the DNA from the manipulated cell may be performed prior to (b) analyzing.

In certain embodiments, (b) may further include: amplifying a region containing the tag sequence by tag-specific amplification.

In certain embodiments, (b) may further include: sequencing the manipulated cell for analysis.

In certain embodiments, (b) may include: analyzing the manipulated genome by sequencing.

In certain embodiments, (b) may include: generating an amplified tag-specific library by amplifying the manipulated genome tag-specifically; and analyzing the tag-specific library by sequencing.

In certain embodiments, (b) may include: generating a tag-specific library from the manipulated genome; and sequencing the tag-specific library.

In certain embodiments, (b) may include: generating a tag-specific library from the manipulated genome; generating an amplified tag-specific library by amplifying the tag-specific library; and sequencing the tag-specific library.

Some embodiments of the present application provide a method for obtaining information on one or more off-targets that may occur in a process of prime editing, the method including:
(a) preparing a population of cells including one or more manipulated cells,
   wherein preparing the population of cells including the one or more manipulated cells includes: manipulating genomes of one or more cells by treating the population of cells with a prime editing protein including a Cas protein and a reverse transcriptase or a nucleic acid encoding the prime editing protein and a tpegRNA and a nucleic acid encoding the tpegRNA,
   wherein the tpegRNA includes a spacer, a gRNA core, and an extension region,
   wherein the extension region includes a reverse transcription template (RT template), a tag template, and a primer binding site (PBS),
   wherein each of the manipulated cells contains the manipulated genome,
   wherein the manipulated genome contains one or more tag sequences, wherein each of the tag sequences is inserted into the genome of the cell through a reverse transcription process performed by the reverse transcriptase using the tag template of the tpegRNA as a reverse transcription template;
(b) analyzing the population of cells obtained through (a) to obtain analysis data on one or more of the manipulated cells;
(c) obtaining tagmentation information on one or more of the manipulated cells based on the analysis data obtained through (b); and
(d) confirming information on one or more off-targets that may occur in the process of genome editing by prime editing, based on the tagmentation information obtained through the (c) obtaining.

In certain embodiments, the tag sequence may be inserted into an on-target region.

In certain embodiments, the tag sequence may be inserted into one or more candidate off-target regions.

In certain embodiments, the tagmentation information may include any one or more from: information on whether the tag sequence is inserted into the genome of the cell; information on chromosomes where one or more tag sequences are inserted; information on sites where one or more tag sequences are inserted; information on the chromosomes where one or more tag sequences are inserted and sites in the chromosomes; and information on insertion rates by the sites where one or more tag sequences are inserted.

In certain embodiments, the off-target that may occur in the process of genome editing by the prime editing is an off-target candidate, wherein the information on one or more off-targets that may occur in the process of genome editing by the prime editing may include any one or more from: information on whether the off-target candidate exists; information on chromosomes where one or more off-target candidates occur; information on sites where one or more off-target candidates occur; information on the chromosomes where one or more off-target candidates occur and sites in the chromosomes; and information on possibilities of one or more off-target candidates by the sites thereof.

In certain embodiments, the tagmentation information may include: information on whether the tag sequence is contained in the DNA of one or more manipulated genomes.

In certain embodiments, the tagmentation information may include: a site in the genome DNA of each tag sequence for one or more tag sequences.

In certain embodiments, the tagmentation information may include: a tagmentation rate for each tagged region.

In certain embodiments, the tagmentation information may include one or more from: the information on whether the tag sequence is contained in the DNA of one or more manipulated genomes; the site in the genome DNA of each tag sequence for one or more tag sequences; and the tagmentation rate for each tagged region.

In certain embodiments, the method for predicting the off-target of the present application may further include: confirming information on an on-target.

In certain embodiments, the information on the off-target may include: whether the off-target candidate exists. In this case, whether the off-target candidate exists may be obtained from the information on whether the tag sequence is contained in the analyzing-subject genome DNA.

In certain embodiments, the information on the off-target may include: a site in the genome DNA of each off-target candidate for one or more off-target candidates (candidate off-target region). In this case, the site in the genome DNA of each off-target candidate for one or more off-target candidates may be obtained from the site in the genome DNA of each tag sequence for one or more tag sequences.

In certain embodiments, the information on the off-target may include: an off-target predicting score of each off-target candidate for one or more off-target candidates. In this case, the off-target predicting score of each off-target candidate for one or more off-target candidates may be obtained from the tagmentation rate for each tagged region.

In certain embodiments, the Information on the off-target may include: the number of off-target candidates predicted.

In certain embodiments, the information on the off-target may include one or more from: whether the off-target candidate exists; the site in the genome DNA of each off-target candidate for one or more off-target candidates; the off-target predicting score of each off-target candidate for one or more off-target candidates; and the number of the off-target candidates predicted.

In certain embodiments, the RT template herein may include an editing template and a homology region.

In certain embodiments, the length of the tag template herein may be 5 to 60 nt.

In certain embodiments, the length of the tag template herein may be 15 to 40 nt.

In certain embodiments, the length of the tag template herein may be about 19 nt.

In certain embodiments, the length of the tag template herein may be about 24 nt.

In certain embodiments, the length of the tag template herein may be about 29 nt.

In certain embodiments, the length of the tag template herein may be about 34 nt.

In certain embodiments, the prime editing protein may induce nicking of an editing-subject DNA molecule.

In certain embodiments, the prime editing protein may induce a DSB in the editing-subject DNA molecule.

In certain embodiments, the prime editing protein may be a PE2 prime editing protein.

In certain embodiments, the prime editing protein may be a PE2-nuclease.

In certain embodiments, the prime editing protein may be a PEmax prime editing protein.

In certain embodiments, the prime editing protein may be a PEmax-nuclease.

In certain embodiments, the method for obtaining the information on the off-target may include confirming the predetermined prime editing system.

In certain embodiments, the prime editing protein may be the same as or different from the predetermined prime editing protein.

In certain embodiments, the tpegRNA may be an etpegRNA.

In certain embodiments, the tag template may be located in between the reverse transcription template and the primer binding site in the extension region of the tpegRNA.

In certain embodiments, a sequence contained in a region of the spacer of the tpegRNA may have a 90% or higher sequence identity to the sequence in the region of the spacer of the predetermined pegRNA.

In certain embodiments, a sequence contained in the primer binding site of the tpegRNA may have a 90% or higher sequence identity to the sequence of the primer binding site of the predetermined pegRNA.

In certain embodiments, a sequence contained in the homology region of the tpegRNA may have a 90% or higher sequence identity to the sequence of the homology region of the predetermined pegRNA.

In certain embodiments, (a) may include: preparing the prime editing protein and the tpegRNA in a cell.

In certain embodiments, (a) may further include: introducing the prime editing protein, a fragment thereof, or a nucleic acid encoding the prime editing protein and the tpegRNA, a fragment thereof, or a nucleic acid encoding the tpegRNA into a cell. In certain embodiments, the prime editing protein, the fragment thereof, or the nucleic acid encoding the prime editing protein and the tpegRNA, the fragment thereof, or the nucleic acid encoding the tpegRNA herein may be introduced into the cell by one or more vectors.

In certain embodiments, the method for obtaining the information on the off-target may further include: destroying the manipulated cell. In this case, destroying the manipulated cell may be performed prior to (b).

In certain embodiments, the method for obtaining the information on the off-target may further include: extracting DNA from the population of cells including one or more manipulated cells. In this case, the extracting the DNA may be performed prior to (b).

In certain embodiments, (b) may further include: amplifying a region containing the tag sequence by tag-specific amplification performed on the manipulated genome contained in one or more manipulated cells.

In certain embodiments, (b) may further include: sequencing the manipulated genome contained in one or more manipulated cells to analyze the manipulated cell.

In certain embodiments, (b) may include: analyzing the one or more manipulated genomes by sequencing.

In certain embodiments, (b) may include: generating an amplified tag-specific library by amplifying the one or more manipulated genomes tag-specifically; and analyzing the amplified tag-specific library by sequencing.

In certain embodiments, (b) may include: generating a tag-specific library from the one or more manipulated genomes; and sequencing the tag-specific library.

In certain embodiments, (b) may include: generating a tag-specific library from the one or more manipulated genomes; generating an amplified tag-specific library by amplifying the tag-specific library; and sequencing the amplified tag-specific library.

In certain embodiments, the manipulated genome contained in each of the manipulated cells may be the same or different from each other.

### Exemplary embodiment (2)

Hereinbelow, exemplary embodiments (non-limiting embodiments) described in a manner different from the above-described "Exemplary embodiment of off-target prediction method of present application (1)" are to be disclosed. Throughout this specification, elements used for describing the present disclosure may be interpreted as including plural concepts even when written in singular forms, as described above. In other words, terms written in singular forms will be understood as including plural concepts as necessary. For example, in the case of being described as "analyzing manipulated genome DNA", the manipulated genome DNA herein may be interpreted as meaning the DNA of one or a plurality of manipulated genomes.

A01. A method for predicting an off-target occurring in a process of genome editing by a prime editing system, the method including:
(a) obtaining a manipulated cell,
   wherein the manipulated cell contains manipulated genome DNA, wherein the manipulated genome DNA contains a tag sequence, wherein the manipulated genome DNA is generated through the following process involving a prime editor protein and a tagmentation pegRNA (tpegRNA):
   (i) contacting the genome DNA with the prime editor protein and the tpegRNA, wherein the prime editor protein includes a Cas protein and a reverse transcriptase, and wherein the tpegRNA includes a spacer and an extension region including a tag template,
   (ii) inserting the tag sequence into the genome DNA through a reverse transcription process performed by the reverse transcriptase using the tag template of the tpegRNA as a template of reverse transcription;
(b) analyzing the manipulated genome DNA to obtain tagmentation information,
   wherein the tagmentation information includes information on a region of the genome DNA where the tag sequence is inserted.

A02. The prediction method of A01,
further including:
obtaining information on the off-target based on the tagmentation information, wherein the information on the off-target includes: information on whether an off-target candidate exists and information on an off-target candidate region when the off-target candidate exists.

A03. The prediction method of A02,
wherein the off-target candidate is a potential off-target being a bona-fide off-target occurring in the process of genome editing by the prime editing system.

A04. The prediction method of any one of A01 to A03,
further including:
confirming information on an on-target and comparing the information on the on-target with the tagmentation information.

A05. The prediction method of any one of A01 to A03,
further including:
confirming information on whether the off-target candidate exists by confirming information on an on-target and comparing the information on the on-target with the tagmentation information.

A06. The prediction method of any one of A01 to A05,
wherein the tag sequence is inserted into a region in the genome DNA specified by the spacer of the tpegRNA.

A07. The prediction method of any one of A01 to A06,
wherein the region where the tag sequence is inserted is associated with the off-target candidate region or an on-target region.

A08. The prediction method of any one of A01 to A07,
wherein information on the region where the tag sequence is inserted includes information on a chromosome where the tag sequence is located and information on a region in the chromosome where the tag sequence is present.

A09. The prediction method of any one of A01 to A08,
wherein the information on the off-target candidate region includes information on a chromosome where each off-target candidate is located and a region in the chromosome where the off-target candidate is located.

A10. The prediction method of any one of A01 to A09,
wherein the tagmentation information further includes:
information on an insertion rate of the tag sequence by the region where the tag sequence is inserted.

A11. The prediction method of any one of A01 to A10,
wherein the information on the off-target further includes:
an off-target predicting score for the off-target candidate.

A12. The prediction method of any one of A01 to A11,
wherein the information on the off-target further includes:
the number of the off-target candidates predicted.

A13. The prediction method of any one of A01 to A12,
wherein the manipulated cell is obtained by a method including:
contacting the protein editor protein or a nucleic acid encoding the prime editor protein and the tpegRNA or a nucleic acid encoding the tpegRNA with a cell.

A14. The prediction method of any one of A01 to A13,
wherein the manipulated cell is obtained by a method including:
introducing the protein editor protein or the nucleic acid encoding the prime editor protein and the tpegRNA or the nucleic acid encoding the tpegRNA into a cell.

A15. The prediction method of any one of A01 to A14,
further including:
extracting DNA from the manipulated cell, wherein the extraction of the DNA from the one or more manipulated cells is performed prior to (b).

A16. The prediction method of any one of A01 to A15,
wherein the tpegRNA includes:
the spacer; a gRNA core; and the extension region including a primer binding site, the tag template, and a reverse transcription template.

A17. The prediction method of A16,
wherein the reverse transcription template of the tpegRNA includes an editing template and a homology region.

A18. The prediction method of A17,
wherein the manipulated genome DNA includes an editing.

A19. The prediction method of any one of A16 to A18,
wherein the spacer, the gRNA core, and the extension region are located in the order of the spacer, the gRNA core, and the extension region in the 5' to 3' direction.

A20. The prediction method of any one of A16 to A19,
wherein the tag template is located in between the primer binding site and the reverse transcription template in the extension region.

A21. The prediction method of any one of A16 to A20,
wherein the tpegRNA further includes a 3' engineering region including an RNA protection motif.

A22. The prediction method of any one of A01 to A21,
further including:
confirming a predetermined prime editing system,
wherein the confirmation of the predetermined prime editing system includes confirming any one or more from: information on a predetermined cell, information on a predetermined pegRNA, and information on a predetermined prime editor protein.

A23. The prediction method of A22,
wherein the predetermined cell is different from the cell used in the prediction method.

A24. The prediction method of A22 or A23,
wherein a sequence of the spacer of the tpegRNA is the same as a sequence of a spacer of the predetermined pegRNA, and
a sequence of the primer binding site of the tpegRNA is the same as a sequence of a primer binding site of the predetermined pegRNA.

A25. The prediction method of any one of A22 to A24,
wherein the sequence of the spacer of the tpegRNA is the same as the sequence of the spacer of the predetermined pegRNA,
the sequence of the primer binding site of the tpegRNA is the same as the sequence of the primer binding site of the predetermined pegRNA, and
a sequence of the reverse transcription template of the tpegRNA is the same as a sequence of a reverse transcription template of the predetermined pegRNA.

A26. The prediction method of any one of A22 to A25,
wherein the prime editor protein used in the prediction method is the same as or different from the predetermined prime editor protein.

A27. The prediction method of any one of A01 to A26,
wherein the length of the tag template is 5 to 60 nt.

A28. The prediction method of any one of A01 to A27,
wherein the length of the tag template is 10 to 50 nt.

A29. The prediction method of any one of A01 to A28,
wherein the prime editor protein is a PE-nuclease including the Cas protein having double-strand break activity.

A30. The prediction method of any one of A01 to A29,
wherein the prime editor protein is a PEmax-nuclease.

A31. The prediction method of any one of A01 to A28,
wherein the Cas protein included in the prime editor protein is a nickase.

A32. The prediction method of any one of A01 to A28 and A31,
wherein the prime editor protein is a PE2 prime editor protein.

A33. The prediction method of any one of A01 to A32,
wherein the manipulation of the genome DNA additionally involves any one or more from a dominant negative MLH1 (dnMLH1), a gRNA, and an additional Cas protein, and an additional prime editor protein.

A34. The prediction method of any one of A01 to A33,
wherein (b) includes:
analyzing the manipulated genome DNA tag-specifically.

A35. The prediction method of any one of A01 to A34,
wherein (b) includes:
sequencing the manipulated genome DNA.

A36. The prediction method of any one of A01 to A35,
wherein (b) includes:
generating a tag-specific library from the manipulated genome DNA; generating an amplified tag-specific library by amplifying the tag-specific library; and sequencing the amplified tag-specific library.

B01. A method for predicting an off-target occurring in a process of genome editing by a prime editing system, the method including:
(a) preparing a population of cells including one or more manipulated cells,
   wherein the manipulated cell contains manipulated genome DNA, wherein the manipulated genome DNA contains one or more tag sequences, wherein the manipulated genome DNA is generated through the following process involving a prime editor protein and a tagmentation pegRNA (tpegRNA):
   (i) contacting the genome DNA of the cell with the prime editor protein and the tpegRNA, wherein the prime editor protein includes a Cas protein and a reverse transcriptase, and wherein the tpegRNA includes a spacer and an extension region including a tag template, and
   (ii) inserting the tag sequence into the genome DNA, wherein the insertion of the tag sequence into the genome DNA is achieved through a reverse transcription process performed by the reverse transcriptase using the tag template of the tpegRNA as a template of reverse transcription;
(b) obtaining tagmentation information by analyzing the manipulated genome DNA through a process including sequencing the manipulated genome DNA of the one or more manipulated cells,
   wherein the tagmentation information includes information on each region where one or more tag sequences are inserted.

B02. The prediction method of B01,
further including:
obtaining information on the off-target based on the tagmentation information, wherein the information on the off-target includes: information on whether an off-target candidate exists and information on one or more off-target candidate regions.

B03. The prediction method of B02,
wherein the off-target candidate is a potential off-target being a bona-fide off-target occurring in the process of genome editing by the prime editing system.

B04. The prediction method of any one of B01 to B03,
further including:
confirming information on an on-target and comparing the information on the on-target with the tagmentation information.

B05. The prediction method of any one of B01 to B03,
further including:
confirming information on whether the off-target candidate exists and information on one or more off-target candidate regions by confirming information on an on-target and comparing the information on the on-target with the tagmentation information.

B06. The prediction method of any one of B01 to B05,
wherein the tag sequence is inserted into a region in the genome DNA specified by the spacer of the tpegRNA.

B07. The prediction method of any one of B01 to B06,
wherein each region in the genome DNA where the tag sequence is inserted is associated with the off-target candidate region or an on-target region.

B08. The prediction method of any one of B01 to B07,
wherein any one of the regions in the genome DNA where the tag sequence is inserted is the on-target region, and all the others are the off-target candidate regions.

B09. The prediction method of any one of B01 to B08,
wherein information on each region where the tag sequence is inserted includes information on a chromosome where each tag sequence is located and information on a region in the chromosome where the tag sequence is present.

B10. The prediction method of any one of B01 to B09,
wherein the information on the off-target candidate region includes information on a chromosome where each off-target candidate is located and a region in the chromosome where the off-target candidate is located.

B11. The prediction method of any one of B01 to B10,
wherein the tagmentation information further includes:
information on an insertion rate of the tag sequence by the region where each tag sequence is inserted.

B12. The prediction method of any one of B01 to B11,
wherein the information on the off-target further includes:
an off-target predicting score of each off-target candidate for one or more off-target candidates.

B13. The prediction method of any one of B01 to B12,
wherein the information on the off-target further includes:
the total number of off-target candidates predicted.

B14. The prediction method of any one of B01 to B13,
wherein the population of cells including the one or more manipulated cells is prepared by a method including:
contacting a population of cells including one or more cells with a composition including the prime editor protein or a nucleic acid encoding the prime editor protein and the tpegRNA or a nucleic acid encoding the tpegRNA.

B15. The prediction method of any one of B01 to B14,
wherein the population of cells including the one or more manipulated cells is prepared by a method including:
contacting a composition including the protein editor protein or the nucleic acid encoding the prime editor protein and a composition including the tpegRNA or the nucleic acid encoding the tpegRNA with a population of cells including one or more cells.

B16. The prediction method of any one of B01 to B15,
further including:
extracting DNA from the population of cells including the one or more manipulated cells, wherein the extraction of the DNA from the population of cells including the one or more manipulated cells is performed prior to (b).

B17. The prediction method of any one of B01 to B16,
wherein the tpegRNA includes:
the spacer; a gRNA core; and the extension region including a primer binding site, the tag template, and a reverse transcription template.

B18. The prediction method of B17,
wherein the reverse transcription template of the tpegRNA includes an editing template and a homology region.

B19. The prediction method of B18,
wherein the manipulated genome DNA includes one or more editings.

B20. The prediction method of any one of B17 to B19,
wherein the spacer, the gRNA core, and the extension region are located in the order of the spacer, the gRNA core, and the extension region in the 5' to 3' direction.

B21. The prediction method of any one of B17 to B20,
wherein the tag template is located in between the primer binding site and the reverse transcription template in the extension region.

B22. The prediction method of any one of B17 to B21,
wherein the tpegRNA further includes a 3' engineering region including an RNA protection motif.

B23. The prediction method of any one of B01 to B22,
further including:
confirming a predetermined prime editing system,
wherein the confirmation of the predetermined prime editing system includes confirming any one or more from: information on a predetermined cell, information on a predetermined pegRNA, and information on a predetermined prime editor protein.

B24. The prediction method of B23,
wherein the predetermined cell is different from the cell used in the prediction method.

B25. The prediction method of B23 or B24,
wherein a sequence of the spacer of the tpegRNA is the same as a sequence of a spacer of the predetermined pegRNA,
a sequence of the reverse transcription template of the tpegRNA is the same as a sequence of a reverse transcription template of the predetermined pegRNA, and
a sequence of the primer binding site of the tpegRNA is the same as a sequence of a primer binding site of the predetermined pegRNA.

B26. The prediction method of any one of B23 to B25,
wherein the prime editor protein used in the prediction method is the same as or different from the predetermined prime editor protein.

B27. The prediction method of any one of B01 to B26,
wherein the length of the tag template is 5 to 60 nt.

B28. The prediction method of any one of B01 to B27,
wherein the length of the tag template is 10 to 50 nt.

B29. The prediction method of any one of B01 to B28,
wherein the prime editor protein is a PE-nuclease including the Cas protein having double-strand break activity.

B30. The prediction method of any one of B01 to B29,
wherein the prime editor protein is a PEmax-nuclease.

B31. The prediction method of any one of B01 to B28,
wherein the Cas protein included in the prime editor protein is a nickase.

B32. The prediction method of any one of B01 to B28 and B31,
wherein the prime editor protein is a PE2 prime editor protein.

B33. The prediction method of any one of B01 to B32,
wherein the manipulation of the genome DNA additionally involves any one or more from a dominant negative MLH1 (dnMLH1), a gRNA, and an additional Cas protein, and an additional prime editor protein.

B34. The prediction method of any one of B01 to B32,
wherein (b) includes:
generating a tag-specific library from the manipulated genome DNA; generating an amplified tag-specific library by amplifying the tag-specific library; and sequencing the amplified tag-specific library.

C01. A tagmentation pegRNA (tpegRNA) including:
a spacer; a gRNA core; and an extension region including a tag template.

C02. The tpegRNA of C01,
wherein the spacer, the gRNA core, and the extension region including the tag template are located in the order of the spacer, the gRNA core, and the extension region including the tag template in the 5' to 3' direction in the tpegRNA.

C03. The tpegRNA of C01 or C02,
wherein the extension region includes the tag template, a primer binding site, and a reverse transcription template.

C04. The tpegRNA of any one of C01 to C03,
wherein the tag template is located in between the primer binding site and the reverse transcription template.

C05. The tpegRNA of any one of C01 to C03,
wherein the reverse transcription template is located in between the tag template and the primer binding site.

C06. The tpegRNA of any one of C01 to C04,
wherein the primer binding site, the tag template, and the reverse transcription template are located in the order of the reverse transcription template, the tag template, and the primer binding site in the 5' to 3' direction in the extension region.

C07. The tpegRNA of any one of C01 to C06,
wherein the reverse transcription template includes an editing template and a homology region.

C08. The tpegRNA of any one of C01 to C07,
wherein the tag template has a length of 5 to 60 nt.

C09. The tpegRNA of any one of C01 to C08,
wherein the tag template has a length of 10 to 50 nt.

C10. The tpegRNA of any one of C01 to C09,
wherein the tpegRNA further includes a 3' engineering region including an RNA protection motif.

C11. The tpegRNA of C10,
wherein the RNA protection motif has a length of 10 to 60 nt.

C12. The tpegRNA of any one of C01 to C11,
wherein the tpegRNA has a length of 100 to 350 nt.

D01. A composition including a tpegRNA.

D02. The composition of D01,
wherein the tpegRNA is the tpegRNA of any one of C01 to C12.

D03. The composition of D01 or D02,
further including a prime editor protein.

D04. The composition of any one of D01 to D03,
wherein the composition is used to predict an off-target occurring in a process of editing a DNA molecule by a prime editing system.

D05. The composition of any one of D01 to D04,
wherein the composition is used in the prediction method of any one of A01 to A36 and B01 to B34.

### Expected usage of off-target prediction method of present application (non-limiting description)

In some embodiments, an off-target prediction system of the present application may be called TAPE-seq. TAPE-seq is a new system related to an off-target screening system, in which information on an off-target that may occur in prime editing is confirmed by inserting a tag sequence into a DNA molecule on the basis of a specific mechanism of prime editing and analyzing information on the tag sequence. Accordingly, all off-target confirmation methods that use the above-described features of TAPE-seq and are performed to achieve the purpose of confirming off-targets that may occur in prime editing are included as one embodiment of using or applying TAPE-seq.

For example, TAPE-seq may be used by technologists or researchers using prime editing to edit the genomes of cells.

For example, the researchers select a prime editing system for use in editing the genomes of cells. For example, the researchers select a cell subject to genome editing. For example, the researchers select one pegRNA for use in prime editing. In the process of selecting a prime editing system for use in editing the genomes of cells, an in silico-based off-target prediction method may be used to design a suitable sequence of the pegRNA. The researchers intend to develop therapeutics that include the use of the selected pegRNA. In developing the therapeutics, information on the presence of off-targets of the selected pegRNA must be confirmed. Based on the selected pegRNA and the selected prime editing system, details of the TAPE-seq system are designed depending on the purpose. TAPE-seq is performed to confirm information on off-targets that may occur in the selected prime editing system. The information confirmed here is information on an off-target candidate or candidate off-target. Next, using the confirmed information on the off-target candidate, information on a problematic off-target in the selected prime editing. Specifically, from the information on the off-target candidate confirmed through TAPE-seq, a site where the off-target candidate occurs (chromosomes where the off-target occurs, sites in the chromosomes, and the like) is confirmed. Then, genome editing is performed on desired cells using the selected prime editing system, and a bona-fide off-target is ultimately confirmed by validation focused on the site where the off-target candidate occurs. In such a process, known off-target prediction methods may be used in combination to find a bona-fide off-target region.

For another example, TAPE-seq may be used in the selection process of a pegRNA. To design a prime editing system, the researchers create a pegRNA library including various types of pegRNAs. TAPE-seq is performed on one or more pegRNAs included in the pegRNA library. A pegRNA for use in the prime editing system is selected. In this case, the selection of the pegRNA may be based on one or more of the following criteria: pegRNAs with no or few off-target candidates confirmed by TAPE-seq; and pegRNAs capable of performing desired editing.

As described above, TAPE-seq may be used in various scenes, and the usage of TAPE-seq is not limited to the examples described above.

### Mode for Invention

Hereinbelow, the present disclosure provided by the present disclosure will be described in more detail through experimental examples or examples. These experimental examples are only for illustrating the content disclosed by the present application, and it is apparent that the scope of the content disclosed hereby should not be construed as being limited by these experimental examples.

### Experimental Example

### Overview of Experimental Examples

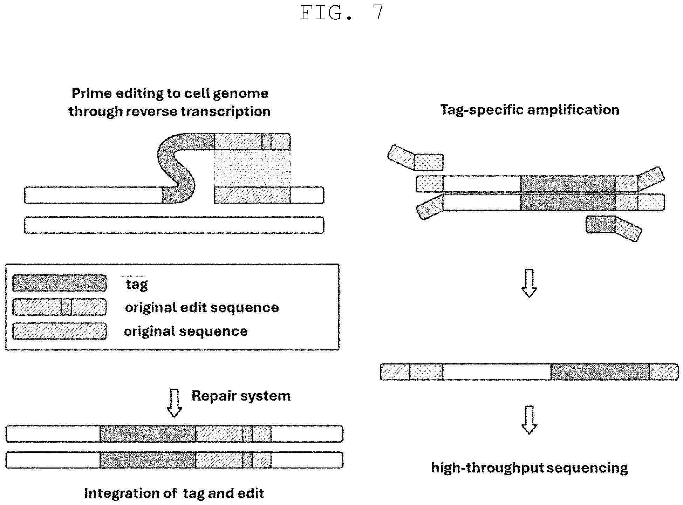

The inventors of the present application developed TAPE-seq, an off-target prediction method further directly related to prime editing based on a molecular mechanism of prime editing. Furthermore, the inventors of the present application confirmed the performance of TAPE-seq, newly developed as a prediction method of off-targets that may occur in the process of genome editing by prime editing, through comparison with known GUIDE-seq, nDigenome-seq, and the like. Hereinbelow, experiments conducted to develop TAPE-seq by the inventors of the present application and the results derived from the experiments are to be explained in detail. By way of example, a schematic diagram of TAPE-seq is disclosed in FIG. 7. Specifically, FIG. 7 illustrates a partial process of TAPE-seq using PE2. A schematic diagram for processes of inserting a tag and an editing into a strand where a nick is made, integrating the tag and the editing ultimately into genome DNA, and analyzing a tag is illustrated in FIG. 7.

### Description of terms used in experimental examples

Terms used in the experimental examples herein are to be explained.

In the experimental examples herein, not only a typical prime editing guide RNA but also a pegRNA containing a tag sequence and/or further including an additional engineering element at the 3' end may be called a pegRNA.

That is, a tagmentation pegRNA (tpegRNA) including a tag template may be called the pegRNA. Those skilled in the art may confirm whether the pegRNA contains the tag sequence or is tag sequence-free through the contents of the relevant paragraph or related data. Furthermore, in some experimental examples, an engineered pegRNA (epegRNA) was used (in particular, the epegRNA is used with PEmax-nuclease), and in some descriptions or results, the epegRNA was called a pegRNA for convenience. Those skilled in the art may confirm whether the epegRNA is used through the contents of the relevant paragraph or related data.

The pegRNA used is, for example, called *HEK4* (+2 G to T) pegRNA or *HEK4* targeted (+2 G to T) pegRNA. The *HEK4* herein represents a gene targeted by the pegRNA used. In other words, *HEK4* (+2 G to T) pegRNA targets a certain region of the *"HEK4* gene" (specifically, an on-target sequence specified by a sequence of a spacer) relative to an on-target. In this case, (+2 G to T) represents a pegRNA-induced editing. For example, *HEK4* (+2 G to T) pegRNA refers to a pegRNA designed to induce an editing that "substitutes G at the second nucleotide from a region where a nick or a DSB is made (or a region where a DSB is made), with T". + indicates the 3' direction relative to a site where a nick or DSB is made. More specifically, in one example of PE2, when described relative to a nicking strand (that is, a spacer non-binding strand), *HEK4* (+2 G to T) pegRNA refers to a pegRNA designed to induce an editing that substitutes G located in the 3' direction from a site where a nick is made (present in the nicking strand), with T. According to a prime editing mechanism, a spacer binding strand is also integrated into the genome by the effect of an editing complementary to the editing in the spacer non-binding strand. In the experimental examples herein, pegRNAs, other than the above example, were also named using the same or similar criteria.

### Experimental method

### Plasmid construction

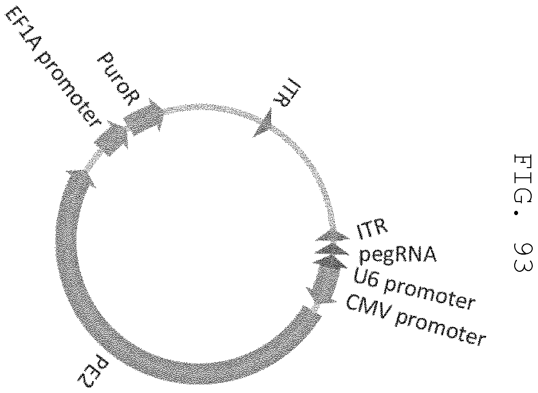

Plasmid pRG2 (addgene #104174) expressing a sgRNA was modified to prepare a pegRNA-expressing plasmid (pRG2-pegRNA). Specifically, Gibson assembly was performed after cleavage at the BsmBI restriction site at the 3' end of a sgRNA scaffold. The plasmid was modified to include a BsaI site (for integration of a sequence of a spacer) and a BsmBI site (for integration of a 3' extension region of the pegRNA). To prepare PiggyBac PE2 all-in-one plasmid (pAllin1-PE2) (an example of pAllin1-PE2 is shown in FIG. 93), the piggyBac PE2-expressing plasmid DNA was synthesized and cloned, thereby preparing a vector (piggy-PE2). Then, the resulting product was digested with Mlu I. A sequence encoding the pegRNA was amplified from the pRG2-pegRNA by PCR, thereby preparing an insert fragment. The insertion fragment was cloned into the piggyBac PE2 vector digested by Gibson assembly. Other PE all-in-one plasmids (pAllin1-PE4, pAllin1-PE2-nuclease, and pAllin1-PEmax-nuclease) were constructed by the same procedure as that used to construct pAllin1-PE2. Additionally, a pRG2-epegRNA vector was constructed by the same procedure as that used to construct pRG2-pegRNA. DNA sequences of all constructed vectors (pRG2-pegRNA, pAllin1-PE2, piggy-PE2, pRG2-epegRNA, pAllin1-PE4, pAllin1-PE2-nuclease, and pAllin1-PEmax-nuclease) are disclosed in the "Vector sequence" section of the experimental Examples.

### Human cell incubation and transfection

HEK293T (ATCC CRL-1268), HeLa (ATCC CCL-2), and K562 (Sigma 89121407) cells were kept in an appropriate medium containing 10% fetal bovine serum (FBS) and 1% (penicillin-streptomycin [Dulbecco's Modified Eagle Medium (DMEM) for the HEK293T and HeLa cell lines; Roswell Park Memorial Institute 1640 Medium (RPMI 1640) for the K562 cell line], at 37°C under 5% CO₂. Transfection was prepared by seeding 1 x 10⁵ HEK293T cells or 4 x 10⁴ HeLa cells in a 24-well plate. One day after seeding, cells were transfected with the appropriate amount of plasmid (see below) and 2 µl of Lipofectamine 2000 (Thermo Fisher Scientific).

For transient PE2 expression, 500 ng of piggy-PE2 and 500 ng of pRG2-pegRNA were used. For stable PE2 expression, 850 ng of pAllin1-PE2 and 150 ng of a piggyBac transposase expression vector (System Biosciences) were used. For stable PE4 expression, 880 ng of pAllin1-PE4 and 120 ng of a piggyBac transposase expression vector were used. For stable PE2-EGFP expression, 865 ng of pAllin1-PE2-EGFP and 135 ng of a piggyBac transposase expression vector were used. For transient PE2-nuclease expression, 1000 ng of pAllin1-PE2-nuclease was used. For transient PEmax-nuclease and epegRNA expression, 1000 ng of pAllin1-PEmax-nuclease-epegRNA was used.

A transposon and the piggyBac plasmid were used in a molar ratio of about 2.5:1 (transposon: transposase plasmid). Through a Neon transfection system, 1 x 10⁵ K562 cells were electroporated with the above-mentioned amount of plasmid (electroporation conditions: 1450 V, 10 ms, and 3 pulses). One day after transfection (or electroporation), antibiotic selection was performed using puromycin (InvivoGen) at a concentration of 2 mg/ml. Puromycin selection was performed over two weeks (for TAPE-seq and fluorescence-activated cell sorting (FACs)), four weeks (for targeted deep sequencing), or two days (for TAPE-seq using PE2-nuclease or PEmax-nuclease; additional cell incubation in a regular medium over four days after puromycin selection). Genome DNA was purified with the Blood Genomic DNA Extraction Mini Kit (Favorgen) according to the manufacturer's instructions.

### DNA analysis in TAPE-seq

A full description of a DNA analysis method in TAPE-seq is to be further disclosed in the "DNA analysis in TAPE-seq: Specific method" section described below. The genome DNA was sheared to an average length of 325 bp by a Covaris M220 instrument and isolated using 1X AMPure XP beads (Beckman coulter). A next-generation sequencing (NGS) library was prepared with minor modifications to the specific reaction time (adaptor ligation, 1 h; treatment with Uracil-Specific Excision Reagent, 30 min) based on the manufacturer's protocol, using the NEBNext^{®} Ultra^{™} II DNA Library Prep Kit. According to the GUIDE-seq method (see [Tsai, Shengdar Q., et al. "GUIDE-seq enables genome-wide profiling of off-target cleavage by CRISPR-Cas nucleases." Nature biotechnology 33.2 (2015): 187-197.; and Liang, Shun-Qing, et al. "Genome-wide detection of CRISPR editing in vivo using GUIDE-tag." Nature communications 13.1 (2022): 1-14.]) previously described, tag-specific library amplification was performed using tag- and adapter-specific primers. The amplified library was analyzed using MiSeq or HiSeq platforms (Illumina).

A paired-end FASTQ file was processed using the following steps:
1. Tag-containing sequences were collected using the BBDuk program (Tag sequences for sense library (+), 5'-GTTTAATTGAGTTGTCATATGT-3' (SEQ ID NO: 57) and 5'-ACATATGACAACTCAATTAAAC-3' (SEQ ID NO: 58); Tag sequences for antisense library (-), 5'-TTGAGTTGTCATATGTTAATAACGGTA-3' (SEQ ID NO: 59) and 5'- TACCGTTATTAACATATGACAACTCAA-3' (SEQ ID NO: 60)).
2. The filtered FASTQ file was mapped to the reference genome (hg19), and read depth was calculated using the BWA, Picard tools, and SAMtools programs.
3. Off-target candidates (including up to 4 mismatches and/or 2 bulges compared to on-target sites) were identified using Cas-OFFinder3 (http://www.rgenome.net).
4. The read depth of the site, identified by Cas-OFFinder, was extracted from a region ranging from -150 bp to +150 bp around the site using an in-house script.
5. A short mapped sequence (smaller than 30 bp in length) and a false tagmentation sequence (where tagmentation occurs outside the PE nick site) were excluded.

### DNA analysis in TAPE-seq: specific method

### Reagent used

Blood Genomic DNA Extraction Mini Kit (Favorgen, Cat No. FABGK 001);
AMPure XP beads (Beckman coulter, Cat No. A63881);
NEBNext^{®} Ultra^{™} II DNA Library Prep Kit for Illumina (NEB, Cat No. E7645L);
NEBNext adaptor (from NEBNext Singleplex kit) (NEB, Cat No. E7350);
Ethyl Alcohol 99.9% GR grade (DUKSAN, Cat No. UN1170);
TMAC Buffer, 5 M (Sigma Aldrich, Cat No. T3411);
1X TE Buffer (Invitrogen, Cat No. 12090015);
Platinum^{®} Tag DNA Polymerase (Invitrogen, Cat No. 10966026);
dNTP Mix, 2.5 mM each (Elpis biotech, Cat No. EBN-1006);
Nuclease-Free Water (Ambion, Cat No. AM9932);
Q5 High-Fidelity DNA Polymerase (NEB, Cat No. M0491L) ;
microTUBE-50 AFA Fiber Screw-Cap (Covaris, Cat No. 520166) .

### Equipment used

M220 Focused-ultrasonicator (Covaris);
T100 Thermocycler (Bio rad);
NEBNext^{®} Magnetic Separation Rack (NEB);
Nanodrop One C (Thermo);
MiSeq (Illumina);
HiSeq (Illumina);

### Oligonucleotide used

GSP1+: ATACCGTTATTAACATATGACA (SEQ ID NO: 61);
GSP1-: GTTTAATTGAGTTGTCATATGTTAATAAC (SEQ ID NO: 62);
GSP2+:
   GTGACTGGAGTTCAGACGTGTGCTCTTCCGATCTACATATGACAACTCAATTAAAC (SEQ ID NO: 63);
GSP2-:

GTGACTGGAGTTCAGACGTGTGCTCTTCCGATCTTTGAGTTGTCATATGTTAATAACG
GTA (SEQ ID NO: 64);

Index Forward (Illumina D501-508 index, N's denote Index sequences): AATGATACGGCGACCACCGAGATCTACACNNNNNNNNACACTCTTTCCCTACACGACG (SEQ ID NO: 65);

Index Reverse (Illumina D701-712 index): CAAGCAGAAGACGGCATACGAGATTANNNNNNNNGACTGGAGTTCAGACGTGTGCTC (SEQ ID NO: 66).

### Genome DNA shearing

1. Genome DNA (gDNA) was isolated using the Blood Genomic DNA Extraction Mini Kit. Then, the purified gDNA was eluted using 1X TE buffer, and the concentration was determined using NanoDrop.
2. Using the M220 Focused-ultrasonicator, 5 µg of the gDNA was sheared to an average length of 325 bp. Detailed conditions for sonication conditions are disclosed below:
   - Tube: microTUBE-50 AFA Fiber Screw-Cap
   - Sample volume: 55 ul
   - Temperature (°C): 20
   - Peak Incident Power (W): 75
   - Duty Factor (%): 10
   - Cycles per Burst (cpb): 200
   - Processing time (sec): 90
3. According to the manufacturer's protocol, the sheared gDNA was washed with 55 µl of AMPure XP beads (1X ratio) and eluted in 50 µl of the 1X TE buffer.

### End repair, A-tailing, and NEBNext adaptor ligation

4. Using the NEBNext^{®} Ultra^{™} II DNA Library Prep Kit for Illumina, 1 µg of sheared gDNA was ligated with an NEBNext adapter (one TAPE-seq reaction requires two vials of adapter-ligated gDNA for + (sense) and - (antisense) library construction).

5. All steps were performed using the manufacturer's protocol (adapter ligation, 1 h/USER enzyme treatment, 30 min), except for modifying USER enzyme processing reaction times and adapter connection.

6. The resulting product was purified with 0.9X AMPure XP beads and eluted in 12 µl of nuclease-free water.

### PCR for tag-specific library amplification

Modified GUIDE-seq Discovery PCR was used in tag-specific library amplification. For + (sense) and - (antisense) library construction, GSP+ and GSP- primers were used separately in + and - PCR reactions, respectively.

7. Two primary PCR reaction vials for + and - libraries were prepared, and the primary PCR was performed under the following conditions.

Conditions of a primary PCR mixture are disclosed below.
- Nuclease-free water: 10.1 ul
- Buffer for Taq Polymerase, 10X (MgCl2 free): 3.0 µl
- dNTP Mix, 2.5 mM each: 2.4 ul
- MgCl2, 50 mM: 1.2 ul
- Platinum Tag polymerase, 5 U/ul: 0.3 µl
- GSP1+ or GSP1- primer (10 uM) (for separate PCR reactions): 0.75 µl
- D5_#(Index forward D501-D508): 0.75 ul
- TMAC (0.5 M): 1.5 ul
- DNA sample (from Step 6): 10.0 ul
- Total: 30.0 µl

Thermal cycler conditions for the primary PCR are disclosed below:
- 95°C, 5 min;
- 15 cycles of [95°C for 30 s, 70°C (-1°C/cycle) for 2 min, 72°C for 30 s];
- 10 cycles of [95°C for 30 s, 55°C for 1 min, 72°C for 30 s];
- 72°C, 5 min; and
- 4°C hold.

8. The resulting PCR product was washed with 0.9X AMPure XP beads and eluted in 20 µl of nuclease-free water.

9. A secondary PCR vial was prepared under the conditions below, and secondary PCR was performed.

Conditions of a secondary PCR mixture are disclosed below.
- Nuclease-free water: 3.6 ul
- Buffer for Taq Polymerase, 10X (MgCl2 free): 3.0 ul
- dNTP Mix, 2.5 mM each: 2.4 µl
- MgCl2, 50 mM: 1.2 µl
- Platinum Tag polymerase, 5 U/ul: 0.3 µl
- GSP2+ or GSP2- primer (10 uM) (for separate PCR reactions): 1.5 µl
- D5_#(Index forward D501-D508): 1.5 µl
- TMAC (0.5 M): 1.5 µl
- DNA sample (from Step 8): 15.0 µl
- Total: 30.0 µl

Thermal cycler conditions for the secondary PCR are disclosed below:
- 95°C, 5 min;
- 15 cycles of [95°C for 30 s, 70°C (-1°C/cycle) for 2 min, 72°C for 30 s];
- 10 cycles of [95°C for 30 s, 55°C for 1 min, 72°C for 30 s];
- 72°C, 5 min; and
- 4°C hold.

10. The resulting PCR product was washed with 0.7X AMPure XP beads and eluted in 15 µl of nuclease-free water.

11. A tertiary PCR vial was prepared under the conditions below, and tertiary PCR was performed.

Conditions of a tertiary PCR mixture are disclosed below.
- Nuclease-free water: 20.5 µl
- 5X Q5 Reaction Buffer: 10.0 µl
- dNTP Mix, 2.5 mM each: 4.0 µl
- Q5 High-Fidelity DNA Polymerase: 0.5 µl
- D7_#(Index reverse D701-712): 2.5 µl
- D5_#(Index forward D501-D508): 2.5 µl
- DNA sample (from Step 10): 10.0 µl
- Total: 50.0 µl

Thermal cycler conditions for the tertiary PCR are disclosed below:
- 98°C, 30 s;
- 30 cycles of [98°C for 10 s, 58°C for 20 s, 72°C for 30 s];
- 72°C, 5 min; and
- 4°C hold.

12. The resulting PCR product was washed with 0.7X AMPure XP beads and eluted in 60 µl of nuclease-free water.

13. The resulting PCR product purified by 2x150-bp paired-end Mi-seq or Hi-seq was analyzed.

### Targeted deep sequencing and validation of off-target region

After pegRNA and PE2 expression, target regions were analyzed through targeted deep sequencing. Deep sequencing libraries were generated by PCR. The TruSeq HT Dual Index primer was used for labeling each sample. Pooled libraries were subjected to paired-end sequencing using Miseq (Illumina). A paired-end FASTQ file was analyzed using PE-Analyzer (http://www.rgenome.net).

Candidates who satisfy the two conditions below were designated "validated off-targets".
1. The frequency of at least one of the events of mutations, insertions, deletions, substitutions, or primary editings is higher than that in wild-type samples.
2. Variant sequences (primarily edited sequences) capable of being only generated by prime editing exist.

Validation experiments were performed using cells in which PE2 was stably expressed over four weeks to overcome problems caused by PCR errors and detection limitations in NGS, and biologically independent genome DNA was used to repeatedly perform the experiments. A validation rate was calculated by dividing the number of validated targets by the "total of the number of validated targets and the number of false positive targets". Unanalyzed targets were excluded from the calculation of the validation rate.

### Prime editing tagmentation analysis

The presence of the tag sequence was defined by tagmentation. Information on the tag sequence integrated into an edited strand (spacer non-binding strand) of the genome DNA is as follows:
34-bp full-length tag: GTTTAATTGAGTTGTCATATGTTAATAACGGTAT (SEQ ID NO: 48);
29-bp tag: GTTTAATTGAGTTGTCATATGTTAATAAC (SEQ ID NO: 49) ;
19-bp tag: GTTTAATTGAGTTGTCATA (SEQ ID NO: 50).

PE-Analyzer (http://www.rgenome.net) was used to identify reads where tagmentation occurred (see [Hwang, Gue-Ho, et al. "PE-Designer and PE-Analyzer: web-based design and analysis tools for CRISPR prime editing." Nucleic acids research 49.W1 (2021): W499-W504.]). Tagmentation Case 1 and Case 2 were distinguished by sequence analysis. After analyzing the TAPE-seq reads by NGS, only reads containing the full-length tag sequence were selected. The tag sequence was then removed from the sequence for analysis, and the remaining sequence was compared with NGS reads obtained from targeted deep sequencing of cells subjected to prime editing using a tag sequence-free pegRNA.

Case 1 indicates that an editing pattern obtained after removing the tag sequence is the same as an editing pattern generated using the tag sequence-free pegRNA. When no such patterns were detectable, the sequence was classified as Case 2.

### PiggyBac copy number analysis

To quantify the average copy number of the integrated piggyBac transposon, a set of primers in the direction of the 5' inverted repeat (IR) of the piggyBac vector was used. The sequences of the forward and reverse primers used to amplify the 5' IR were 5'-CTAAATAGCGCGAATCCGTC-3' (SEQ ID NO: 67) and 5-'TCATTTTGACTCACGCGG-3' (SEQ ID NO: 68), respectively. The copy numbers were calculated using a standard curve generated using a mixture of untransfected HEK293T genome DNA and serially diluted piggyBac plasmids whose copy numbers were known. Real-time PCR was performed using the QuantStudio 3 Real-Time PCR System (Applied Biosystems) with PowerUp SYBR Green Master Mix (Applied Biosystems).

### GFP-expressing cell FACS

Two weeks after puromycin selection, cells were washed with phosphate-buffered saline and detached from the plate using trypsin-ethylenediaminetetraacetic acid (EDTA). The cells were centrifuged at 500 xg for 5 min at room temperature and resuspended in phosphate-buffered saline containing 2% FBS. GFP-positive cells were isolated using the Attune NxT Acoustic Focusing Cytometer (Thermo Scientific). Raw data were analyzed using Attune NxT software v4.2.0.

### Statistics & reproducibility

The 10 sample sites studied in the previous nDigenome-seq paper (see [Kim, Do Yon, et al. "Unbiased investigation of specificities of prime editing systems in human cells." Nucleic acids research 48.18 (2020): 10576-10589.]) were analyzed. No data were excluded from the analysis. Statistical analysis was performed using Prism (version 9.4.1). The results of a two-sided unpaired student t-test are disclosed.

### Data availability

The deep sequencing data supporting the results of this study were deposited in the NCBI Bioproject (https://www.ncbi.nlm.nih.gov/bioproject/) with the accession code PRJNA802977. Source data are provided in a source data file.

### Code availability

The code supporting the results of this study was archived in an online archive (https://github.com/PhyzenInc/TAPE-seq flanking depth).

### Analysis data

The analysis data obtained by the experimental method herein are provided through tables in the "Table for analysis data" section disclosed below the following "Result" section.

### Result

### Optimization of tagmentation rate

Experimental genome-wide off-target prediction methods may be divided into cell-based methods or in vitro-based methods. Prime editing is a multi-step process involving many cellular enzymes, such as flap endonucleases, exonucleases, and ligases, so in vitro-based methods closely mimicking such complex cellular processes are challenging to develop. On the other hand, most existing cell-based methods allow tag sequences to be introduced into on-target and off-target loci so that the tag sequences may be amplified by PCR in a later step. However, PE2, Prime Editing Version 2, does not cause a double-strand break (DSB) but makes a nick at a target, making it impossible to introduce double-stranded oligonucleotide or viral DNA fragments as tags.

PE2 itself has the ability to insert any short sequences into target sites. Therefore, the inventors of the present application designed a pegRNA in which a tag template (may be referred to as a tag sequence or tag for convenience) is located in between sequences of a primer binding site (PBS) and a reverse transcriptase template (RT template). For tags, the same sequence used in GUIDE-seq (see [Tsai, Shengdar Q., et al. "GUIDE-seq enables genome-wide profiling of off-target cleavage by CRISPR-Cas nucleases." Nature biotechnology 33.2 (2015): 187-197.]) was selected. This is because such a sequence was proven to work in cells of various origins.

The inventors of the present application used the sequences of the PBS and the RT template used in the validation experiments of the previous study in experiments related to TAPE-seq. In the previous study, validation experiments were conducted using GUIDE-seq and nDigenome-seq as prediction tools (see [Anzalone, Andrew V., et al. "Search-and-replace genome editing without double-strand breaks or donor DNA." Nature 576.7785 (2019): 149-157.; and Kim, Do Yon, et al. "Unbiased investigation of specificities of prime editing systems in human cells." Nucleic acids research 48.18 (2020): 10576-10589.]). Hereinbelow, the pegRNA containing the tag used in this TAPE-seq study and the sequence of each element included in the pegRNA, and the epegRNA (the epegRNA is used with PEmax-nuclease) containing the tag and the sequence of each element included in the epegRNA are to be disclosed.

### HEK4 (+2 G to T) pegRNA

- Entire sequence: 
GGCACUGCGGCUGGAGGUGGGUUUUAGAGCUAGAAAUAGCAAGUUAAAAUAAGGCUAG

UCCGUUAUCAACUUGAAAAAGUGGCACCGAGUCGGUGCUUAACCCCAAAUACCGUUAU

UAACAUAUGACAACUCAAUUAAACCCUCCAGCCUUUUUU (SEQ ID NO: 69)
- Sequence of spacer: GGCACUGCGGCUGGAGGUGG (SEQ ID NO: 70)
- Sequence of RT template: UUAACCCCAA (SEQ ID NO: 71)
- Sequence of tag template: AUACCGUUAUUAACAUAUGACAACUCAAUUAAAC (SEQ ID NO: 72)
- PBS: CCUCCAGCC (SEQ ID NO: 73)

### HEK4 (+3 TAA ins) pegRNA

- Entire sequence of pegRNA: 
GGCACUGCGGCUGGAGGUGGGUUUUAGAGCUAGAAAUAGCAAGUUAAAAUAAGGCUAG

UCCGUUAUCAACUUGAAAAAGUGGCACCGAGUCGGUGCUUAACCCCUUACAAUACCGU

UAUUAACAUAUGACAACUCAAUUAAACCCUCCAGCCUUUUUU (SEQ ID NO: 74)
- Sequence of spacer: GGCACUGCGGCUGGAGGUGG (SEQ ID NO: 75)
- Sequence of RT template: UUAACCCCUUACA (SEQ ID NO: 76)
- Sequence of tag template: AUACCGUUAUUAACAUAUGACAACUCAAUUAAAC (SEQ ID NO: 77)
- Sequence of PBS: CCUCCAGCC (SEQ ID NO: 78)

### HBB (+4 A to T) pegRNA

- Entire sequence of pegRNA: 
CAUGGUGCACCUGACUCCUGGUUUUAGAGCUAGAAAUAGCAAGUUAAAAUAAGGCUAG
UCCGUUAUCAACUUGAAAAAGUGGCACCGAGUCGGUGCAGACUUCUCCACAGAUACCG
UUAUUAACAUAUGACAACUCAAUUAAACGAGUCAGGUGCACUUUUUU (SEQ ID NO:
79)
- Sequence of spacer: CAUGGUGCACCUGACUCCUG (SEQ ID NO: 80)
- Sequence of RT template: AGACUUCUCCACAG (SEQ ID NO: 81)
- Sequence of tag template: AUACCGUUAUUAACAUAUGACAACUCAAUUAAAC (SEQ ID NO: 82)
- Sequence of PBS: GAGUCAGGUGCAC (SEQ ID NO: 83)

### HEK3 (+1 CTT ins) pegRNA

- Entire sequence of pegRNA: 
GGCCCAGACUGAGCACGUGAGUUUUAGAGCUAGAAAUAGCAAGUUAAAAUAAGGCUAG
UCCGUUAUCAACUUGAAAAAGUGGCACCGAGUCGGUGCUCUGCCAUCAAAGAUACCGU
UAUUAACAUAUGACAACUCAAUUAAACCGUGCUCAGUCUGUUUUUU (SEQ ID NO:
84)
- Sequence of spacer: GGCCCAGACUGAGCACGUGA (SEQ ID NO: 85)
- Sequence of RT template: UCUGCCAUCAAAG (SEQ ID NO: 86)
- Sequence of tag template: AUACCGUUAUUAACAUAUGACAACUCAAUUAAAC (SEQ ID NO: 87)
- Sequence of PBS: CGUGCUCAGUCUG (SEQ ID NO: 88)

### FANCF (+6 G to C) pegRNA

- Entire sequence of pegRNA: 
GGAAUCCCUUCUGCAGCACCGUUUUAGAGCUAGAAAUAGCAAGUUAAAAUAAGGCUAG
UCCGUUAUCAACUUGAAAAAGUGGCACCGAGUCGGUGCGGAAAAGCGAUGCAGGUAUA
CCGUUAUUAACAUAUGACAACUCAAUUAAACGCUGCAGAAGGGAUUUUUUU (SEQ
ID NO: 89)
- Sequence of spacer: GGAAUCCCUUCUGCAGCACC (SEQ ID NO: 90)
- Sequence of RT template: GGAAAAGCGAUGCAGGU (SEQ ID NO: 91)
- Sequence of tag template: AUACCGUUAUUAACAUAUGACAACUCAAUUAAAC (SEQ ID NO: 92)
- Sequence of PBS: GCUGCAGAAGGGAU (SEQ ID NO: 93)

### EMX1 (+5 G to T) pegRNA

- Entire sequence of pegRNA: 
GAGUCCGAGCAGAAGAAGAAGUUUUAGAGCUAGAAAUAGCAAGUUAAAAUAAGGCUAG
UCCGUUAUCAACUUGAAAAAGUGGCACCGAGUCGGUGCAUGGGAGCACUUCAUACCGU
UAUUAACAUAUGACAACUCAAUUAAACUUCUUCUGCUCGGACUUUUUU (SEQ ID
NO: 94)
- Sequence of spacer: GAGUCCGAGCAGAAGAAGAA (SEQ ID NO: 95)
- Sequence of RT template: AUGGGAGCACUUC (SEQ ID NO: 96)
- Sequence of tag template: AUACCGUUAUUAACAUAUGACAACUCAAUUAAAC (SEQ ID NO: 97)
- Sequence of PBS: UUCUUCUGCUCGGAC (SEQ ID NO: 98)

### DNMT1 (+6 G to C) pegRNA

- Entire sequence of pegRNA: 
GAUUCCUGGUGCCAGAAACAGUUUUAGAGCUAGAAAUAGCAAGUUAAAAUAAGGCUAG
UCCGUUAUCAACUUGAAAAAGUGGCACCGAGUCGGUGCGUCACGCCUGUAUACCGUUA
UUAACAUAUGACAACUCAAUUAAACUUCUGGCACCAGGUUUUUU (SEQ ID NO:
99)
- Sequence of spacer: GAUUCCUGGUGCCAGAAACA (SEQ ID NO: 100)
- Sequence of RT template: GUCACGCCUGU (SEQ ID NO: 101)
- Sequence of tag template: AUACCGUUAUUAACAUAUGACAACUCAAUUAAAC (SEQ ID NO: 102)
- Sequence of PBS: UUCUGGCACCAGG (SEQ ID NO: 103)

### RUNX1 (+6 G to C) pegRNA

- Entire sequence of pegRNA: 
GCAUUUUCAGGAGGAAGCGAGUUUUAGAGCUAGAAAUAGCAAGUUAAAAUAAGGCUAG
UCCGUUAUCAACUUGAAAAAGUGGCACCGAGUCGGUGCUGUCUGAAGGCAUCGAUACC
GUUAUUAACAUAUGACAACUCAAUUAAACCUUCCUCCUGAAAAUUUUUUU (SEQ ID
NO: 104)
- Sequence of spacer: GCAUUUUCAGGAGGAAGCGA (SEQ ID NO: 105)
- Sequence of RT template: UGUCUGAAGGCAUCG (SEQ ID NO: 106)
- Sequence of tag template: AUACCGUUAUUAACAUAUGACAACUCAAUUAAAC (SEQ ID NO: 107)
- Sequence of PBS: CUUCCUCCUGAAAAU (SEQ ID NO: 108)

### VEGFA (+5 G to T) pegRNA

- Entire sequence of pegRNA: 
GAUGUCUGCAGGCCAGAUGAGUUUUAGAGCUAGAAAUAGCAAGUUAAAAUAAGGCUAG
UCCGUUAUCAACUUGAAAAAGUGGCACCGAGUCGGUGCAAUGUGCCAUCUGGAGCACU
 
CAAUACCGUUAUUAACAUAUGACAACUCAAUUAAACUCUGGCCUGCAGAUUUUUU

(SEQ ID NO: 109)
- Sequence of spacer: GAUGUCUGCAGGCCAGAUGA (SEQ ID NO: 110)
- Sequence of RT template: AAUGUGCCAUCUGGAGCACUCA (SEQ ID NO: 111)
- Sequence of tag template: AUACCGUUAUUAACAUAUGACAACUCAAUUAAAC (SEQ ID NO: 112)
- Sequence of PBS: UCUGGCCUGCAGA (SEQ ID NO: 113)

### RNF2 (+6 G to A) pegRNA

- Entire sequence of pegRNA: 
GUCAUCUUAGUCAUUACCUGGUUUUAGAGCUAGAAAUAGCAAGUUAAAAUAAGGCUAG
UCCGUUAUCAACUUGAAAAAGUGGCACCGAGUCGGUGCAACGAACACCGCAGAUACCG
UUAUUAACAUAUGACAACUCAAUUAAACGUAAUGACUAAGAUGUUUUUU (SEQ ID
NO: 114)
- Sequence of spacer: GUCAUCUUAGUCAUUACCUG (SEQ ID NO: 115)
- Sequence of RT template: AACGAACACCGCAG (SEQ ID NO: 116)
- Sequence of tag template: AUACCGUUAUUAACAUAUGACAACUCAAUUAAAC (SEQ ID NO: 117)
- Sequence of PBS: GUAAUGACUAAGAUG (SEQ ID NO: 118)

### HEK4 (+2 G to T) epegRNA

- Entire sequence of pegRNA: 
GGCACUGCGGCUGGAGGUGGGUUUUAGAGCUAGAAAUAGCAAGUUAAAAUAAGGCUAG

UCCGUUAUCAACUUGAAAAAGUGGCACCGAGUCGGUGCUUAACCCCAAAUACCGUUAU

UAACAUAUGACAACUCAAUUAAACCCUCCAGCCAUCUUAACCGCGGUUCUAUCUAGUU

ACGCGUUAAACCAACUAGAAUUUUUU (SEQ ID NO: 119)
- Sequence of spacer: GGCACUGCGGCUGGAGGUGG (SEQ ID NO: 70)
- Sequence of RT template: UUAACCCCAA (SEQ ID NO: 71)
- Sequence of tag template: AUACCGUUAUUAACAUAUGACAACUCAAUUAAAC (SEQ ID NO: 72)
- PBS: CCUCCAGCC (SEQ ID NO: 73)
- Sequence of linker: AUCUUAAC (SEQ ID NO: 120)
- Sequence of RNA motif: CGCGGUUCUAUCUAGUUACGCGUUAAACCAACUAGAA (SEQ ID NO: 121)

### HBB (+4 A to T) epegRNA

- Entire sequence of pegRNA: 
CAUGGUGCACCUGACUCCUGGUUUUAGAGCUAGAAAUAGCAAGUUAAAAUAAGGCUAG

UCCGUUAUCAACUUGAAAAAGUGGCACCGAGUCGGUGCAGACUUCUCCACAGAUACCG

UUAUUAACAUAUGACAACUCAAUUAAACGAGUCAGGUGCACAAAUAAAGCGCGGUUCU

AUCUAGUUACGCGUUAAACCAACUAGAAUUUUUU (SEQ ID NO: 122)
- Sequence of spacer: CAUGGUGCACCUGACUCCUG (SEQ ID NO: 80)
- Sequence of RT template: AGACUUCUCCACAG (SEQ ID NO: 81)
- Sequence of tag template: AUACCGUUAUUAACAUAUGACAACUCAAUUAAAC (SEQ ID NO: 82)
- Sequence of PBS: GAGUCAGGUGCAC (SEQ ID NO: 83)
- Sequence of linker: AAAUAAAG (SEQ ID NO: 123)
- Sequence of RNA motif: CGCGGUUCUAUCUAGUUACGCGUUAAACCAACUAGAA (SEQ ID NO: 124)

### HEK3 (+1 CTT ins) epegRNA

- Entire sequence of pegRNA: 
GGCCCAGACUGAGCACGUGAGUUUUAGAGCUAGAAAUAGCAAGUUAAAAUAAGGCUAG

UCCGUUAUCAACUUGAAAAAGUGGCACCGAGUCGGUGCUCUGCCAUCAAAGAUACCGU

UAUUAACAUAUGACAACUCAAUUAAACCGUGCUCAGUCUGUUAAACUUCGCGGUUCUA

UCUAGUUACGCGUUAAACCAACUAGAAUUUUUU (SEQ ID NO: 125)
- Sequence of linker: UUAAACUU (SEQ ID NO: 126)
- Sequence of spacer: GGCCCAGACUGAGCACGUGA (SEQ ID NO: 85)
- Sequence of RT template: UCUGCCAUCAAAG (SEQ ID NO: 86)
- Sequence of tag template: AUACCGUUAUUAACAUAUGACAACUCAAUUAAAC (SEQ ID NO: 87)
- Sequence of PBS: CGUGCUCAGUCUG (SEQ ID NO: 88)
- Sequence of RNA motif: CGCGGUUCUAUCUAGUUACGCGUUAAACCAACUAGAA (SEQ ID NO: 127)

### FANCF (+6 G to C) epegRNA

- Entire sequence of pegRNA: 
GGAAUCCCUUCUGCAGCACCGUUUUAGAGCUAGAAAUAGCAAGUUAAAAUAAGGCUAG

UCCGUUAUCAACUUGAAAAAGUGGCACCGAGUCGGUGCGGAAAAGCGAUGCAGGUAUA

CCGUUAUUAACAUAUGACAACUCAAUUAAACGCUGCAGAAGGGAUAUAGAACGCGCGG

UUCUAUCUAGUUACGCGUUAAACCAACUAGAAUUUUUU (SEQ ID NO: 128)
- Sequence of spacer: GGAAUCCCUUCUGCAGCACC (SEQ ID NO: 90)
- Sequence of RT template: GGAAAAGCGAUGCAGGU (SEQ ID NO: 91)
- Sequence of tag template: AUACCGUUAUUAACAUAUGACAACUCAAUUAAAC (SEQ ID NO: 92)
- Sequence of PBS: GCUGCAGAAGGGAU (SEQ ID NO: 93)
- Sequence of linker: AUAGAACG (SEQ ID NO: 129)
- Sequence of RNA motif: CGCGGUUCUAUCUAGUUACGCGUUAAACCAACUAGAA (SEQ ID NO: 130)

### EMX1 (+5 G to T) epegRNA

- Entire sequence of pegRNA: 
GAGUCCGAGCAGAAGAAGAAGUUUUAGAGCUAGAAAUAGCAAGUUAAAAUAAGGCUAG
UCCGUUAUCAACUUGAAAAAGUGGCACCGAGUCGGUGCAUGGGAGCACUUCAUACCGU
UAUUAACAUAUGACAACUCAAUUAAACUUCUUCUGCUCGGACAAUAUUACCGCGGUUC
UAUCUAGUUACGCGUUAAACCAACUAGAAUUUUUU (SEQ ID NO: 131)
- Sequence of spacer: GAGUCCGAGCAGAAGAAGAA (SEQ ID NO: 95)
- Sequence of RT template: AUGGGAGCACUUC (SEQ ID NO: 96)
- Sequence of tag template: AUACCGUUAUUAACAUAUGACAACUCAAUUAAAC (SEQ ID NO: 97)
- Sequence of PBS: UUCUUCUGCUCGGAC (SEQ ID NO: 98)
- Sequence of linker: AAUAUUAC (SEQ ID NO: 132)
- Sequence of RNA motif: CGCGGUUCUAUCUAGUUACGCGUUAAACCAACUAGAA (SEQ ID NO: 133)

### DNMT1 (+6 G to C) epegRNA

- Entire sequence of pegRNA: 
GAUUCCUGGUGCCAGAAACAGUUUUAGAGCUAGAAAUAGCAAGUUAAAAUAAGGCUAG

UCCGUUAUCAACUUGAAAAAGUGGCACCGAGUCGGUGCGUCACGCCUGUAUACCGUUA

UUAACAUAUGACAACUCAAUUAAACUUCUGGCACCAGGCUAACUACCGCGGUUCUAUC

UAGUUACGCGUUAAACCAACUAGAAUUUUUU (SEQ ID NO: 134)
- Sequence of spacer: GAUUCCUGGUGCCAGAAACA (SEQ ID NO: 100)
- Sequence of RT template: GUCACGCCUGU (SEQ ID NO: 101)
- Sequence of tag template: AUACCGUUAUUAACAUAUGACAACUCAAUUAAAC (SEQ ID NO: 102)
- Sequence of PBS: UUCUGGCACCAGG (SEQ ID NO: 103)
- Sequence of linker: CUAACUAC (SEQ ID NO: 135)
- Sequence of RNA motif: CGCGGUUCUAUCUAGUUACGCGUUAAACCAACUAGAA (SEQ ID NO: 136)

### RUNX1 (+6 G to C) epegRNA

- Entire sequence of pegRNA: 
GCAUUUUCAGGAGGAAGCGAGUUUUAGAGCUAGAAAUAGCAAGUUAAAAUAAGGCUAG
UCCGUUAUCAACUUGAAAAAGUGGCACCGAGUCGGUGCUGUCUGAAGGCAUCGAUACC
GUUAUUAACAUAUGACAACUCAAUUAAACCUUCCUCCUGAAAAUAUCCUACCCGCGGU
UCUAUCUAGUUACGCGUUAAACCAACUAGAAUUUUUU (SEQ ID NO: 137)
- Sequence of spacer: GCAUUUUCAGGAGGAAGCGA (SEQ ID NO: 105)
- Sequence of RT template: UGUCUGAAGGCAUCG (SEQ ID NO: 106)
- Sequence of tag template: AUACCGUUAUUAACAUAUGACAACUCAAUUAAAC (SEQ ID NO: 107)
- Sequence of PBS: CUUCCUCCUGAAAAU (SEQ ID NO: 108)
- Sequence of linker: CUAACUAC (SEQ ID NO: 138)
- Sequence of RNA motif: CGCGGUUCUAUCUAGUUACGCGUUAAACCAACUAGAA (SEQ ID NO: 139)

### VEGFA (+5 G to T) epegRNA

- Entire sequence of pegRNA: 
GAUGUCUGCAGGCCAGAUGAGUUUUAGAGCUAGAAAUAGCAAGUUAAAAUAAGGCUAG

UCCGUUAUCAACUUGAAAAAGUGGCACCGAGUCGGUGCAAUGUGCCAUCUGGAGCACU

CAAUACCGUUAUUAACAUAUGACAACUCAAUUAAACUCUGGCCUGCAGAAAGAAAGGC

GCGGUUCUAUCUAGUUACGCGUUAAACCAACUAGAAUUUUUU (SEQ ID NO: 140)
- Sequence of spacer: GAUGUCUGCAGGCCAGAUGA (SEQ ID NO: 110)
- Sequence of RT template: AAUGUGCCAUCUGGAGCACUCA (SEQ ID NO: 111)
- Sequence of tag template: AUACCGUUAUUAACAUAUGACAACUCAAUUAAAC (SEQ ID NO: 112)
- Sequence of PBS: UCUGGCCUGCAGA (SEQ ID NO: 113)
- Sequence of linker: AAGAAAGG (SEQ ID NO: 141)
- Sequence of RNA motif: CGCGGUUCUAUCUAGUUACGCGUUAAACCAACUAGAA (SEQ ID NO: 142)

### RNF2 (+6 G to A) epegRNA

- Entire sequence of pegRNA: 
GUCAUCUUAGUCAUUACCUGGUUUUAGAGCUAGAAAUAGCAAGUUAAAAUAAGGCUAG

UCCGUUAUCAACUUGAAAAAGUGGCACCGAGUCGGUGCAACGAACACCGCAGAUACCG

UUAUUAACAUAUGACAACUCAAUUAAACGUAAUGACUAAGAUGUAAUAUACCGCGGUU

CUAUCUAGUUACGCGUUAAACCAACUAGAAUUUUUU (SEQ ID NO: 143)
- Sequence of spacer: GUCAUCUUAGUCAUUACCUG (SEQ ID NO: 115)
- Sequence of RT template: AACGAACACCGCAG (SEQ ID NO: 116)
- Sequence of tag template: AUACCGUUAUUAACAUAUGACAACUCAAUUAAAC (SEQ ID NO: 117)
- Sequence of PBS: GUAAUGACUAAGAUG (SEQ ID NO: 118)
- Sequence of linker: UAAUAUAC (SEQ ID NO: 145)
- Sequence of RNA motif: CGCGGUUCUAUCUAGUUACGCGUUAAACCAACUAGAA (SEQ ID NO: 146)

The signal-to-noise ratio of the developed off-target prediction method may be proportional to the efficiency of tag insertion at on-target and off-target sites. The inventors of the present application first optimized experimental conditions for tagmentation into the on-target sites. When transiently transfecting a plasmid encoding the HEK4-targeting pegRNA (+2 G to T editing, numbered relative to the nick) containing PE2 and the tag sequence into HEK293T cells, 0.011% of a tagmentation rate was observed. To improve such ratios, the inventors of the present application constructed an all-in-one vector encoding the pegRNA and PE2 in the piggyBac system (see [Li, Xianghong, et al. "piggyBac transposase tools for genome engineering." Proceedings of the National Academy of Sciences 110.25 (2013): E2279-E2287.]) . This vector was transfected with transposase to construct a stable cell line.

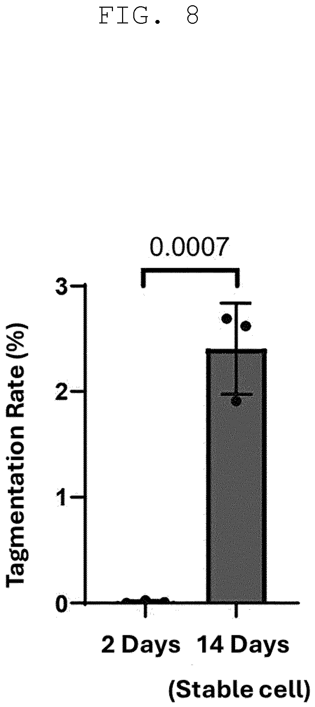

In this situation, the tagmentation rate increased by 2% or more after 14 days of puromycin selection. The results thereof are disclosed in FIG. 8. Specifically, the tagmentation rates after 2 or 14 days are disclosed in FIG. 8 (bars represent means; error bars represent standard deviations; n = 3, for independent transfections; two-sided unpaired student t-test).

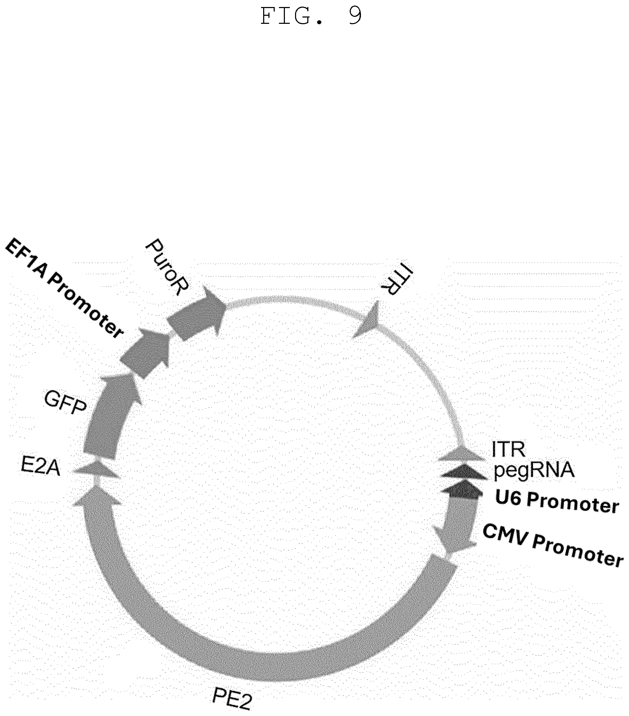

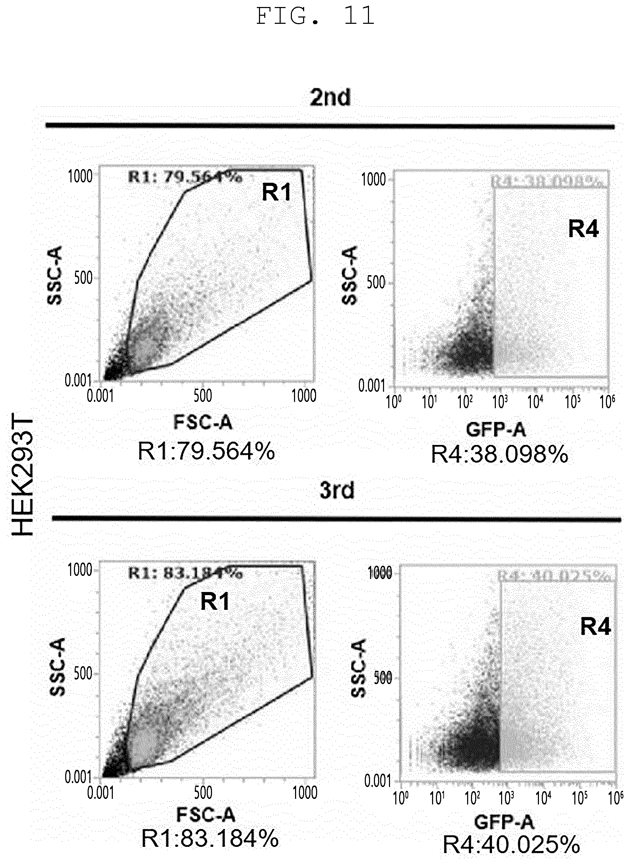

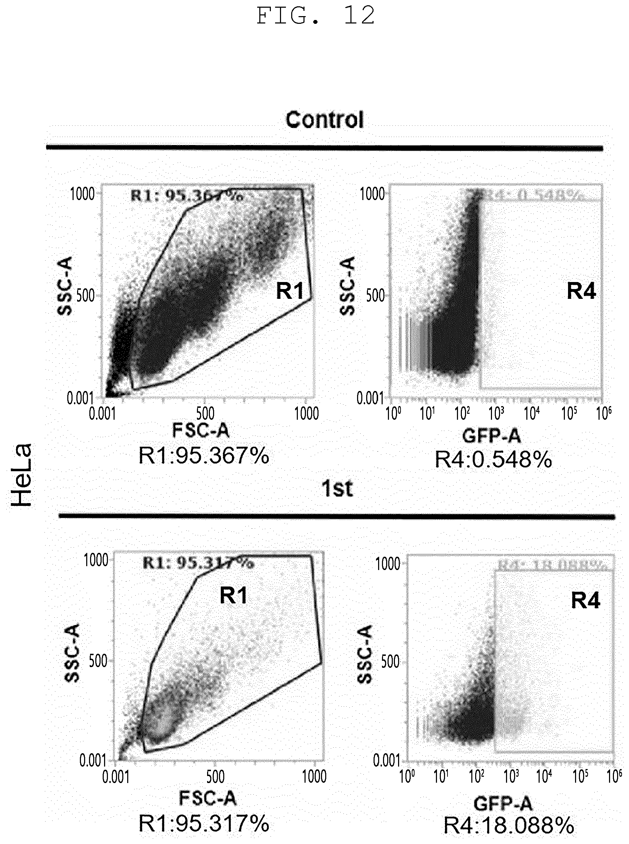

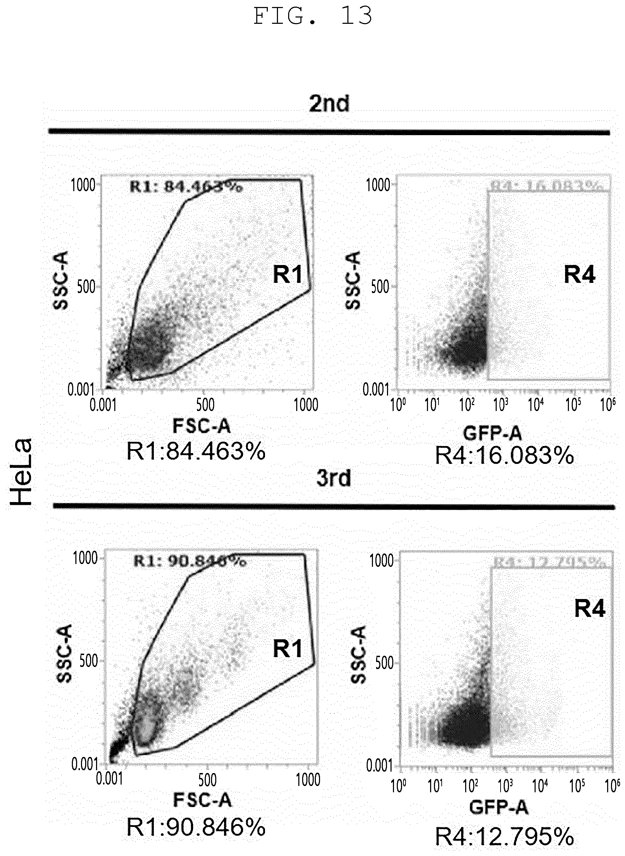

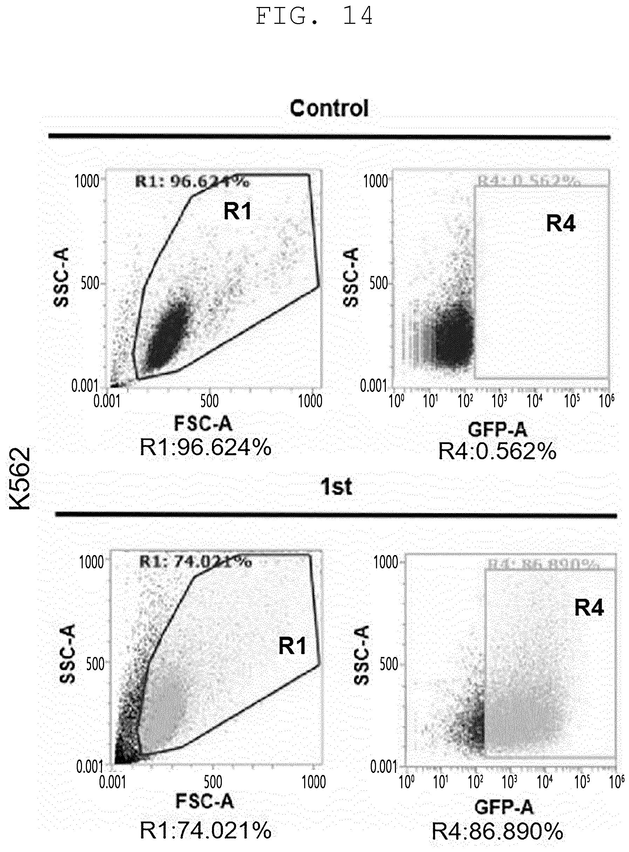

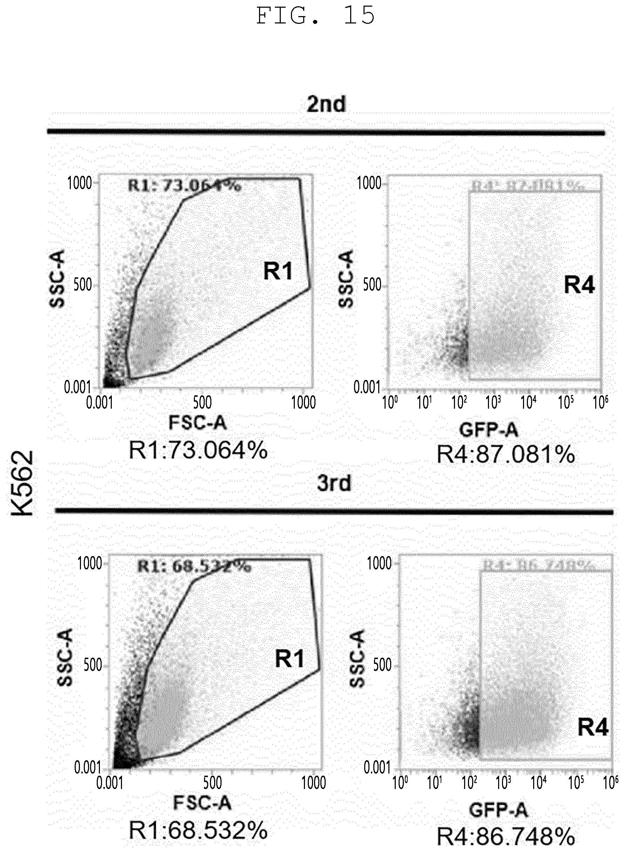

After transfection with the GFP-piggyBac construct, 14 days of puromycin selection successfully led to the enrichment of green fluorescent protein (GFP)-positive cells (see FIGS. 9 to 15). The 14 days of puromycin selection enriched the cells transfected with the GFP-piggyBac construct. FIG. 9 shows a map of the GFP-piggyBac vector. The GFP is linked to PE2 through the E2A sequence.

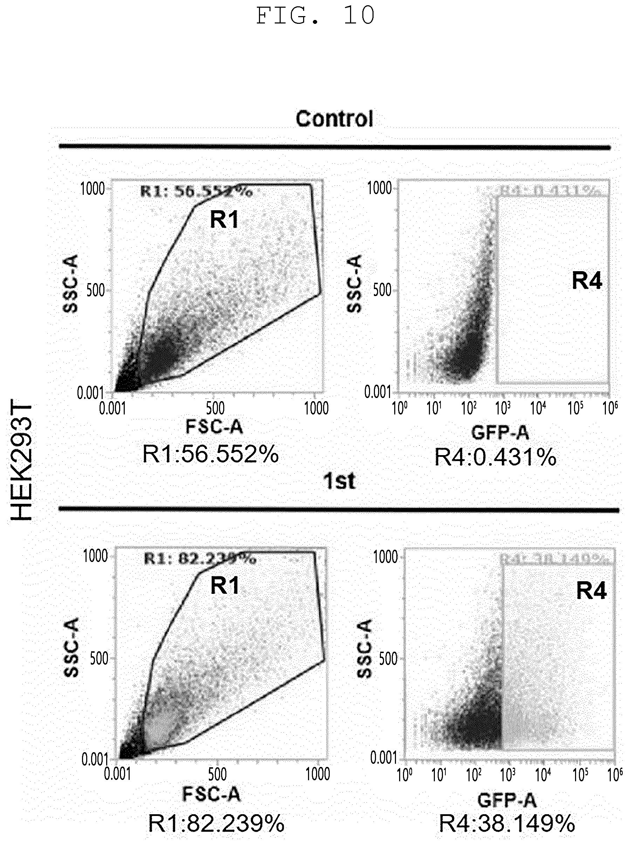

The enrichment results of the positive cells are disclosed in FIGS. 10 to 15. Specifically, FIGS. 10 to 15 show the FACS-based analysis results to detect GFP expression in three different transfected populations of cells (HEK293T, HeLa, and K562). The populations of cells were gated on SSC-A vs. FSC-A, and the GFP+ population was plotted against SSC-A. The experiment was performed three times (primary, secondary, and tertiary).

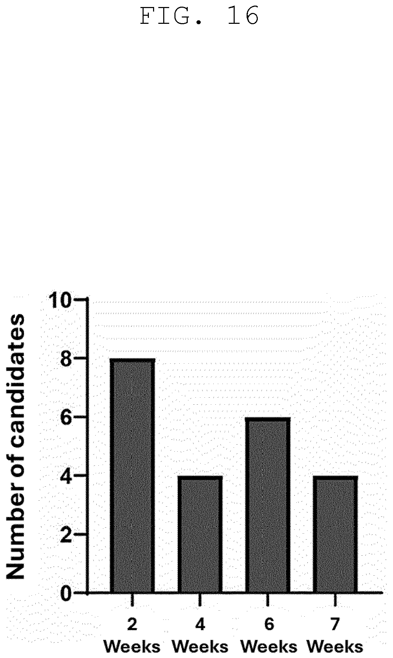

Even when the incubation period was extended from two weeks to seven weeks, the improvement in the number of targets discovered was insignificant. The results for the number of targets by incubation periods are disclosed in FIG. 16. Specifically, FIG. 16 shows the number of candidate off-target sites discovered by TAPE-seq, by the incubation periods after transfecting *HEK4* (+2 G to T) pegRNA into HEK294T cells.

The inventors of the present application assigned the number of Mi-seq reads similar to those of samples from Week 2 (5329899), Week 4 (5313548), Week 6 (2324242), and Week 7 (4021702) (see Analysis Data 3 and Table 11). Compared to the samples of Week 4 (2369), Week 6 (1060), and Week 7 (1594), the sample of Week 2 (62565) showed a greater number of on-target reads (see Analysis Data 2 and Tables 2 to 10). This indicates that the signal-to-noise ratio obtained by TAPE-seq analysis performed on the sample of Week 2 is higher than those of other samples. Therefore, in the following study, puromycin selection was performed over two weeks.

The inventors of the present application further optimized the tagmentation rate by finding the optimal amount of piggyBac vector for co-transfection with the transposase plasmid. The tested amounts ranged from 50 ng to 1000 ng. The results thereof are disclosed in FIGS. 17 to 19. In FIGS. 17 to 19, PB refers to PiggyBac.

Specifically, FIG. 17 is a graph showing the copy number of piggyBac constructs found in cells by quantitative PCR, by the amount (ng) of the piggyBac plasmid (PB plasmid) used for HEK293T cell transfection (error bars represent standard deviations; n = 3, for independent transfections; Two-sided unpaired student-test).

FIG. 18 is a graph showing tagmentation rates at an on-target site by the amount (ng) of the piggyBac plasmid used for HEK293T transfection (error bars represent standard deviations; n = 3, for independent transfections, two-sided unpaired student-t test). In this case, the on-target site refers to an on-target site for *HEK4* (+2 G to T) pegRNA.

FIG. 19 is a graph showing tagmentation rates at Off-target site 1 by the amount (ng) of the piggyBac plasmid used for HEK293T transfection (bars represent means; n = 3, for independent transfections, two-sided unpaired student-t test). In this case, Off-target site 1 refers to an off-target site for *HEK4* (+2 G to T) pegRNA. When measuring the copy numbers of the piggyBac vector, the highest values were shown at 1000 ng. Furthermore, at 1000 ng, the consistently high tagmentation rates at both on-target and off-target sites were shown. Therefore, the inventors of the present application transfected 1000 ng of the piggyBac vector in the following TAPE-seq.

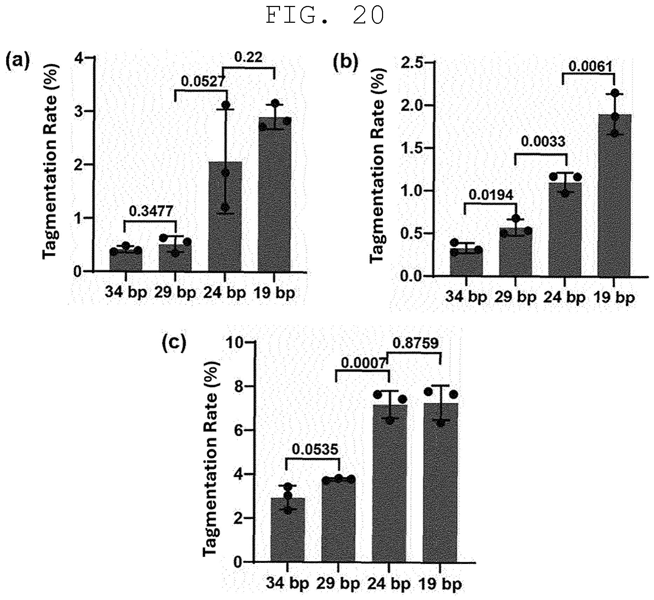

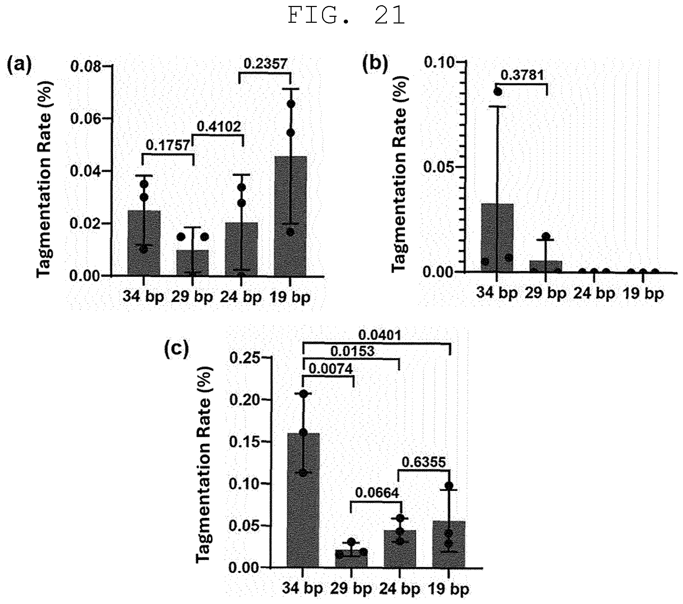

Next, because the tagmentation rate may vary with the length of the probe sequence (tag template or tag), tests were conducted on various probe sequence lengths. The tests were conducted on 19 to 34-bp long probe sequences. As a result of the tests, in the on-target region of *HEK4-*targeting pegRNA (+2 G to T), the 19-bp long probe sequence exhibited a much higher integration rate than the 34-bp long probe sequence (see FIG. 20). The opposite tendency was observed in one of the off-target regions (see FIG. 21). The results thereof are disclosed in FIGS. 20 to 21 (bars represent means; error bars represent standard deviations; n = 3, for independent transfections; two-sided unpaired student-t test).

Specifically, in FIG. 20, graphs of the tagmentation rates at the on-target site of *HEK4* (+2 G to T) pegRNA by the probe sequence length are disclosed. FIG. 20A shows the results for HEK293T cells. FIG. 20B shows the results for HeLa cells. FIG. 20C shows the results for K562 cells.

Specifically, in FIG. 21, graphs of the tagmentation rates at Off-target site 1 of *HEK4* (+2 G to T) pegRNA by the probe sequence length are disclosed. FIG. 21A shows the results for HEK293T cells. FIG. 21B shows the results for HeLa cells. FIG. 21C shows the results for K562 cells.

A part of the purpose of TAPE-seq is the tagmentation of off-target sites, and GUIDE-seq experiments and analysis were optimized using 34-bp long tag sequences. For this reason, the inventors of the present application decided to use the 34-bp long sequence in the following analysis. Methods of GUIDE-seq (see [Tsai, Shengdar Q., et al. "GUIDE-seq enables genome-wide profiling of off-target cleavage by CRISPR-Cas nucleases." Nature biotechnology 33.2 (2015): 187-197.; and Malinin, Nikolay L., et al. "Defining genome-wide CRISPR-Cas genome-editing nuclease activity with GUIDE-seq." Nature Protocols 16.12 (2021): 5592-5615.]) and anchored multiplex PCR (AMP), the previous model, (see [Zheng, Zongli, et al. "Anchored multiplex PCR for targeted next-generation sequencing." Nature medicine 20.12 (2014): 1479-1484.]) include nested PCR steps to ensure high specificity, which is achieved using two unidirectional primers. When primers were optimized for 17 different targets for AMP analysis (see [U.S. Patent No. 9,487,828 B2; inv. Iafrate, A.J., et al.]), the use of two tandem primers enabled 35 to 71-bp long target priming sites, in which the average thereof was 46 bp and the median value thereof was 44 bp. The inventors of the present application presumed that a decrease in the length of the target priming site from 34 bp to 19 bp would remove the high specificity obtained by nested PCR in the AMP and GUIDE-seq methods. Actually, when reducing the probe sequence length from 34 bp to 19 bp, the number of Nucleotide basic local alignment search tool (BLAST) (see [Altschul, Stephen F., et al. "Basic local alignment search tool." Journal of molecular biology 215.3 (1990): 403-410.; and Zhang, Zheng, et al. "A greedy algorithm for aligning DNA sequences." Journal of Computational biology 7.1-2 (2000): 203-214.] hits drastically increased from 1 to about 4000, suggesting a 4000 times higher chance of genome-wide mis-priming. As a result, a lower signal-to-noise ratio is exhibited. The 34-bp probe sequence used in GUIDE-seq was successfully tagged at on- and off-target sites in GUIDE-seq for six different cases, so the inventors of the present application decided to use the 34-bp sequence in the following analysis.

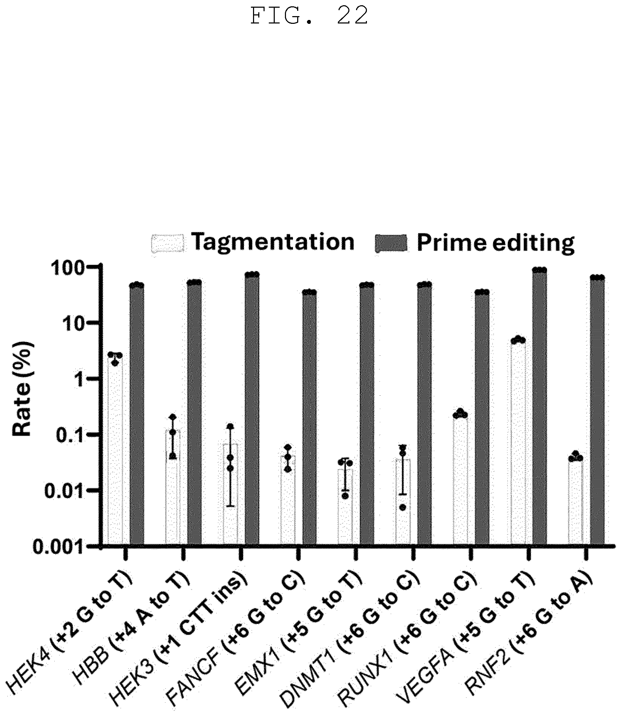

When measuring the tagmentation rates for samples incubated under optimized conditions with nine different individual pegRNAs containing tag sequences and targeting different genes, tagmentation was observed in all targets. The results thereof are disclosed in FIG. 22. Specifically, FIG. 22 shows prime editing rates and tagmentation rates for nine different pegRNAs at on-target sites (error bars represent standard deviations; n = 3, for independent transfections; two-sided unpaired student-t test). Results were measured for each of the pegRNAs containing the tag sequence and the corresponding pegRNAs free of the tag sequence.

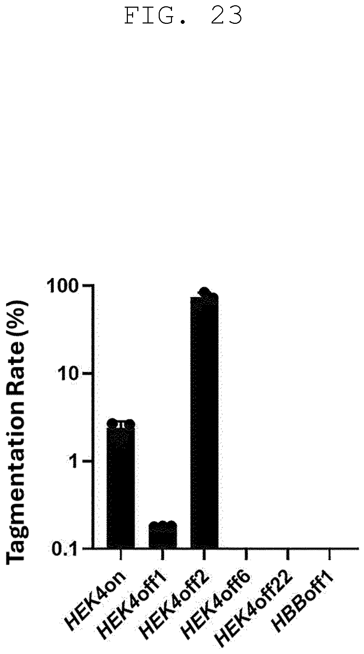

Furthermore, the inventors of the present application compared the tagmentation rates of five off-target loci and one on-target locus identified previously by the nDigenome-seq. The results thereof are shown in FIG. 23. Specifically, FIG. 23 shows a graph of the tagmentation rates for six target sites of *HEK4* (+2 G to T) pegRNA and *HBB* (+4 A to T) pegRNA (bars represent means; error bars represent standard deviations; n = 3, for independent transfections).

One of the off-target loci exhibited a tagmentation rate close to 100%, so the following step was conducted under the conditions of the tagmentation step mentioned above.

### Pattern analysis of on-target and off-target tagmentation

The inventors of the present application compared prime editing patterns in the on-target locus for each prime-edited sample obtained using the tag sequence-containing pegRNAs. The addition of the tag sequence to the pegRNA may lead to two alternative integration scenarios.

A first case (Case 1) is as follows. The 34-bp tag sequence is added without disturbing the remaining prime editing pattern. Accordingly, when removing the 34-bp probe sequence from this pattern, the resulting pattern is the same as a prime editing pattern induced by the tag-free pegRNA.

A second case (Case 2) is as follows. The tagmentation disturbs the remaining prime editing pattern. Accordingly, when removing the 34-bp tag sequence from this pattern, the resulting pattern differs from the prime editing pattern induced by the tag-free pegRNA.

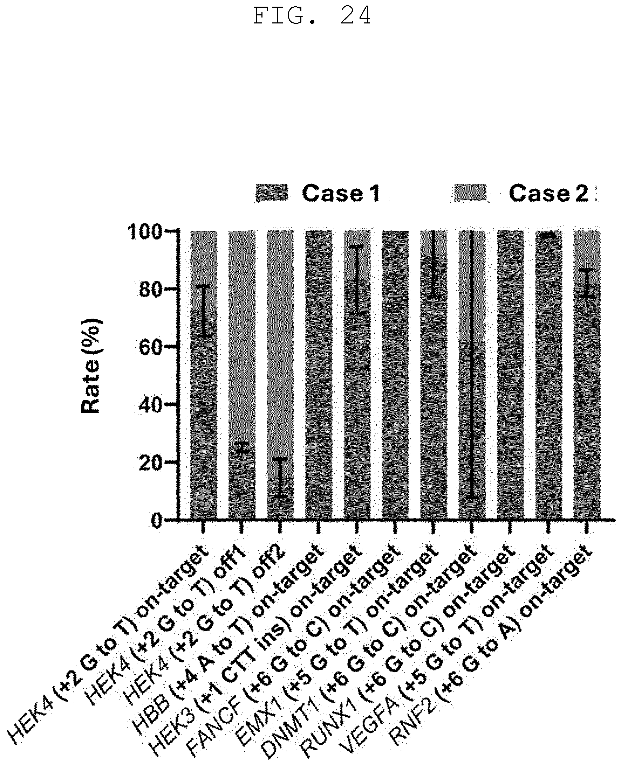

The tagmentation patterns at on- and off-target loci for nine different pegRNAs were analyzed by targeted deep sequencing analysis and a PE-analyzer (see [Hwang, Gue-Ho, et al. "PE-Designer and PE-Analyzer: web-based design and analysis tools for CRISPR prime editing." Nucleic acids research 49.W1 (2021): W499-W504.]). Most of the tagged samples corresponded to the Case 1 scenario. The results thereof are shown in FIG. 24. Specifically, FIG. 24 shows editing ratios in Case 1 to Case 2 determined using targeted deep sequencing and the PE-analyzer.

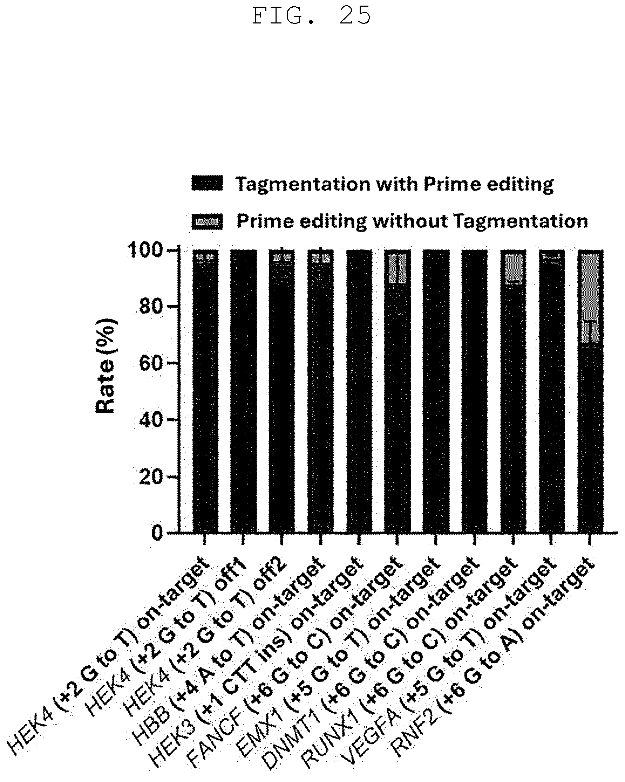

Furthermore, additional analysis of the samples of Case 1 revealed that both the tag and the prime editing (editing) were contained in most cases. Only a small portion was tagged without the prime editing (see Analysis Data 1 and Table 1). The results thereof are disclosed in FIG. 25. Specifically, FIG. 25 shows the analysis results for 10 different on- and off-target sites and discloses the tagmentation results with and without the prime editing (error bars represent standard deviation; n = 3, for independent transfections).

From these results, the inventors of the present application confirmed that the presence of the tag sequence had minimal effect on (that is, being hardly affected) the prime editing patterns at the on- and off-target sites.

### Analysis of tag-inserted (tagged) genome DNA for predicting genome-wide off-target effect of PE2

The inventors of the present application purified tag-integrated (that is, tagmented) genome DNA (in other words, tagmented genome DNA) and processed the resulting purified product using protocols from GUIDE-seq (see [Tsai, Shengdar Q., et al. "GUIDE-seq enables genome-wide profiling of off-target cleavage by CRISPR-Cas nucleases." Nature biotechnology 33.2 (2015): 187-197.; and Malinin, Nikolay L., et al. "Defining genome-wide CRISPR-Cas genome-editing nuclease activity with GUIDE-seq." Nature Protocols 16.12 (2021): 5592-5615.]) for tag-specific amplification to generate TAPE-seq libraries. In the previous analysis (see [Kim, Do Yon, et al. "Unbiased investigation of specificities of prime editing systems in human cells." Nucleic acids research 48.18 (2020): 10576-10589.]), *HEK4-*targeting pegRNAs were related to many validated non-target regions compared to pegRNAs targeting other regions. Hence, the inventors of the present application optimized the TAPE-seq protocol using the *HEK4* region as a case study.

The inventors of the present application first transfected cells with PE2 and plasmids encoding and *HEK4* (+2 G to T) pegRNA and PE2 using MiSeq and HiSeq and then analyzed a TAPE-seq library generated using the same resulting genome DNA pool. The results thereof are summarized using a Venn diagram (FIG. 26). Specifically, FIG. 26 discloses a Venn diagram showing the comparison results for validated regions and off-target sites of *HEK4-*targeted pegRNA predicted by TAPE-seq. In FIG. 26, disclosed are the comparison results for the validated sites of *HEK4* (+2 G to T), the off-target sites of *HEK4* (+2 G to T) predicted by TAPE-seq using Mi-seq, and the off-target sites of *HEK4* (+2 G to T) predicted by TAPE-seq using Hi-seq.

HiSeq (53,771,178 reads) failed to reveal more off-target sites, indicating that the read number for MiSeq (2,251,379 reads) is large enough for this analysis.

Furthermore, the inventors of the present application compared the results for *HEK4* (+2 G to T) pegRNA with *HEK4* (+3 TAA ins) pegRNA. The results thereof are disclosed in FIGS. 27 to 28. Specifically, FIGS. 27 to 28 disclose Venn diagrams showing the comparison results for validated regions and off-target sites of HEK4-targeted pegRNA predicted by TAPE-seq.

Regarding FIG. 27, disclosed are the comparison results for the validated sites of *HEK4* (+3 TAA ins), combination of the off-target sites of *HEK4* (+2 G to T) predicted by TAPE-seq using Mi-seq and the off-target sites of *HEK4* (+2 G to T) predicted by TAPE-seq using Hi-seq, and the off-target sites of *HEK4* (+3 TAA ins) predicted by TAPE-seq (Mi-seq) .

Regarding FIG. 28, disclosed are the comparison results for the validated sites of *HEK4* (+2 G to T), combination of the off-target sites of *HEK4* (+2 G to T) predicted by TAPE-seq using Mi-seq and the off-target sites of *HEK4* (+2 G to T) predicted by TAPE-seq using Hi-seq, and the off-target sites of *HEK4* (+3 TAA ins) predicted by TAPE-seq (Mi-seq) .

The results, summarized in the Venn diagram, show that the TAPE-seq analysis of *HEK4* (+2 G to T) pegRNA-treated samples precisely predicted the off-target validated for *HEK4* (+3 TAA ins) pegRNA missed by the TAPE-seq analysis of *HEK4* (+3 TAA ins) pegRNA. The results, summarized in the Venn diagram, also show that the TAPE-seq analysis of *HEK4* (+3 TAA ins) pegRNA-treated samples precisely predicted the off-target validated for HEK4 (+2 G to T) pegRNA missed by the TAPE-seq analysis of *HEK4* (+2 G to T) pegRNA.

The inventors of the present application presumed that the off-target profile of *HEK4* (+2 G to T) pegRNA would be similar to that of *HEK4* (+3 TAA ins) pegRNA. Thus, it was presumed that a difference in the TAPE-seq results between these two samples might have resulted from common replication issues (that is, low tagmentation rates at off-target sites) found in the HiSeq and MiSeq samples obtained after being treated with *HEK4* (+2 G to T) pegRNA. Therefore, the inventors of the present application combined all three sets of the TAPE-seq results for *HEK4* pegRNA for the following analysis: the *HEK4* (+2 G to T) pegRNA MiSeq TAPE-seq results, the *HEK4* (+2 G to T) pegRNA HiSeq TAPE-seq results, and the *HEK4*(+3 TAA ins) pegRNA TAPE-seq results.

### Comparison of TAPE-seq prediction results with GUIDE-seq and nDigenome-seq

TAPE-seq analysis was performed using the optimized protocol for 10 different pegRNAs, and the results thereof were compared with the previous predictions by GUIDE-seq and nDigenome-seq (see Analysis Data 2 and 3 and Tables 1 to 11). Validation experiments were performed on all off-target candidates predicted by TAPE-seq. TAPE-seq was performed on the HEK293T cell line stably expressing the appropriate pegRNA and PE2 (see Analysis Data 4 and Tables 12 to 16). Some targets identified as false positives in nDigenome-seq were confirmed to be validated in the experiments disclosed in the present application (see Analysis Data 5 and Tables 17 to 26). These results may be attributable to the extended incubation period (4 weeks) of this protocol compared to the transient transfection (96 h) used in the nDigineme-seq validation experiments.

The inventor of the present application also performed validation experiments on the validated target loci identified by the method in the previous paper, even when missed in TAPE-seq. The Venn diagrams summarize the results for the off-target sites and validated sites predicted by each method of TAPE-seq, GUIDE-seq, and nDigenome-seq (FIGS. 29 to 38). The comparison results of TAPE-seq with other off-target prediction methods are shown in FIGS. 29 to 38. Specifically, FIGS. 29 to 38 show the comparison results for the validated off-target sites and the off-target sites predicted by nDigenome-seq, GUIDE-seq, and TAPE-seq (in FIGS 29 to 38, some numbers are underlined to be distinguished from neighboring numbers). FIG. 29 shows the results for *HEK4* (+2 G to T) pegRNA. FIG. 30 shows the results for *HEK4* (+3 TAA ins) pegRNA. FIG. 31 shows the results for *EMX1* (+5 G to T) pegRNA. FIG. 32 shows the results for *FANCF* (+6 G to C) pegRNA. FIG. 33 shows the results for *HEK3* (+1 CTT ins) pegRNA. FIG. 34 shows the results for *RNF2* (+6 G to A) pegRNA. FIG. 35 shows the results for *DNMT1* (+6 G to C) pegRNA. FIG. 36 shows the results for *HBB* (+4 A to T) pegRNA. FIG. 37 shows the results for *RUNX1* (+6 G to C) pegRNA. FIG. 38 shows the results for *VEGFA* (+5 G to T) pegRNA.

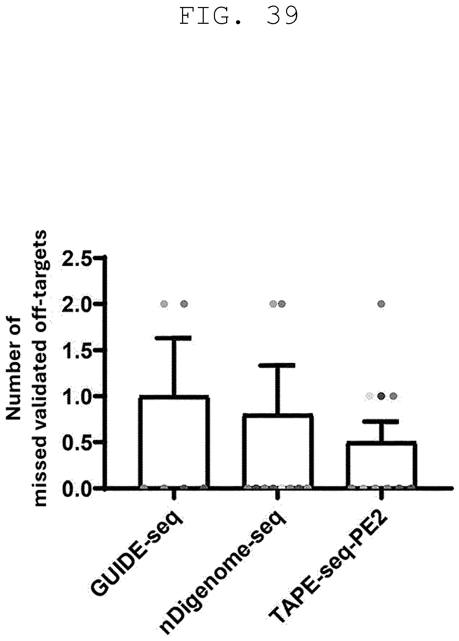

The results showing the validated off-targets missed by each prediction method, related to the results in FIGS. 29 to 38, are disclosed using a graph in FIG. 39. Specifically, FIG. 39 shows the results for the validated off-targets missed by nDigenome-seq (n = 10, for independent experiments), GUIDE-seq (n = 6, for independent experiments), and TAPE-seq (n = 10, for independent experiments) (bars represent means, and error bars represent standard deviations).

TAPE-seq predicted much fewer off-targets than GUIDE-seq and nDigenome-seq. Furthermore, TAPE-seq missed fewer validated off-target sites than other methods. This suggests that predictions by TAPE-seq show higher accuracy.

### TAPE-seq analysis using PE2 and PE4, and TAPE-seq analysis in different cell lines

A later version of PE has been developed and reported to have higher prime editing efficiency than the previous version. PE4, a modified version of PE2, has been reported to exhibit higher prime editing efficiency by containing a plasmid encoding a dominant negative MLH1 to inhibit mismatch repair (see [Chen, Peter J., et al. "Enhanced prime editing systems by manipulating cellular determinants of editing outcomes." Cell 184.22 (2021): 5635-5652.]).

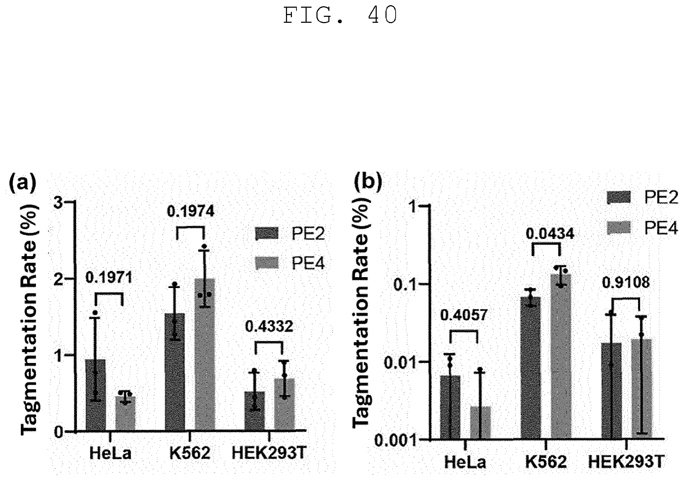

The inventors of the present application performed TAPE-seq using PE2 and PE4 in HEK293T, HeLa, and K562 cells (see Analysis Data 2 and Tables 2 to 10). In the three cell lines, no significant differences were found between the tagmentation rates at one of the off-target loci and the on-target of *HEK4* (+2 G to T) pegRNA. The results thereof are disclosed in FIG. 40. Specifically, FIG. 40A discloses the tagmentation rates at the on-target site analyzed by TAPE-seq performed on HeLa, K562, and HEK293T cells and *HEK4* (+2 G to T) pegRNA (using PE2 and using PE4, respectively). FIG. 40B discloses the tagmentation rates at Off-target site 1 analyzed by TAPE-seq performed on HeLa, K562, and HEK293T cells and HEK4 (+2 G to T) pegRNA (using PE2 and using PE4, respectively).

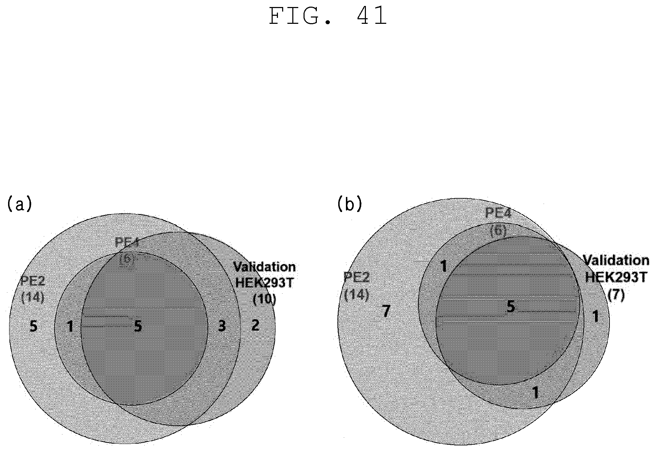

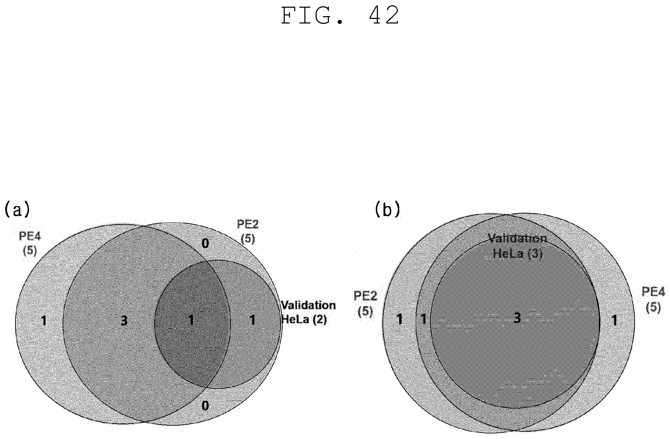

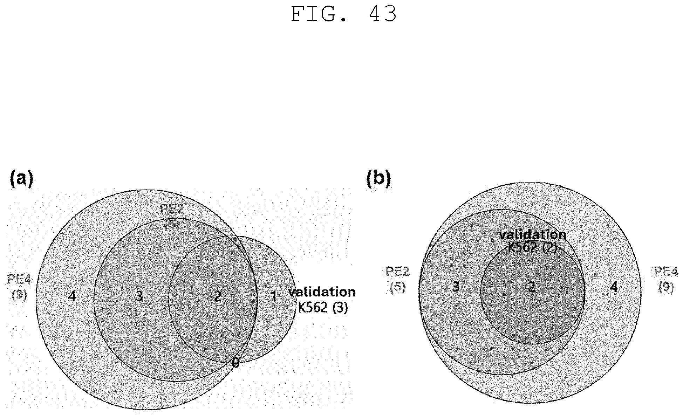

Validation was performed on the off-target regions predicted by targeted deep sequencing. The results thereof were expressed using Venn diagrams. The Venn diagrams are disclosed in FIGS. 41 to 43. Specifically, FIGS. 41 to 43 disclose the comparison results for the off-target sites validated in the PE2-transfected cell lines, the off-target sites predicted by TAPE-seq using PE2, and the off-target sites predicted by TAPE-seq using PE4. FIG. 41A shows the results for *HEK4* (+2 G to T) pegRNA and HEK293T cells. FIG. 41B shows the results for *HEK4* (+3 TAA ins) pegRNA and HEK293T cells. FIG. 42A shows the results for *HEK4* (+2 G to T) pegRNA and HeLa cells. FIG. 42B shows the results for *HEK4* (+3 TAA ins) pegRNA and HeLa cells. FIG. 43A shows the results for *HEK4* (+2 G to T) pegRNA and K562 cells. FIG. 43B shows the results for HEK4 (+3 TAA ins) pegRNA and K562 cells.

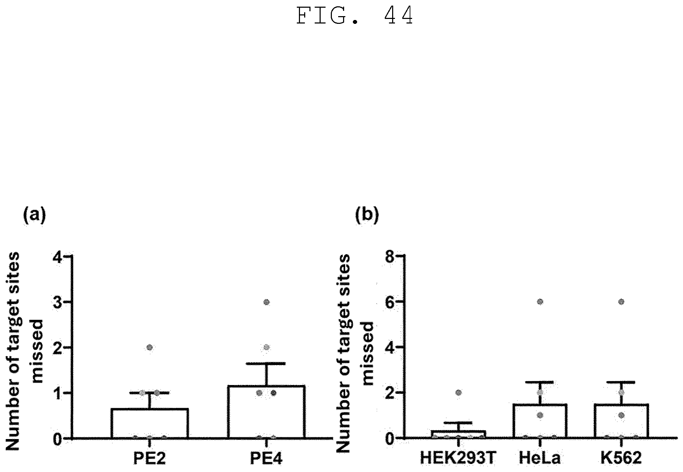

FIG. 44A discloses a summary of the number of target sites missed by each sample related to FIGS. 41 to 43, using a graph (bars represent means; error bars represent standard deviations; n = 6, for independent experiments). In other words, FIG. 44A shows the number of targets missed for the sample using PE2 and the sample using PE4.

FIG. 44B discloses a summary of the number of target sites missed by each cell related to FIGS. 41 to 43, using a graph (bars represent means; error bars represent standard deviations; n = 6, for independent experiments). In other words, FIG. 44B shows the number of targets missed for HEK293T, HeLa, and K562. TAPE-seq performed in HEK293T cells missed fewer validated off-target sites than analysis in the other two cell lines.

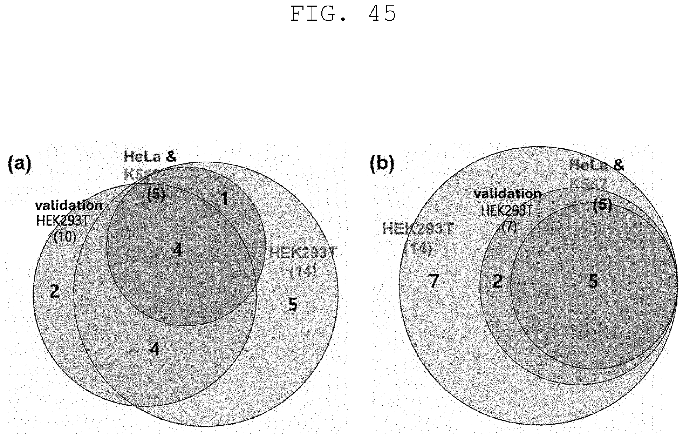

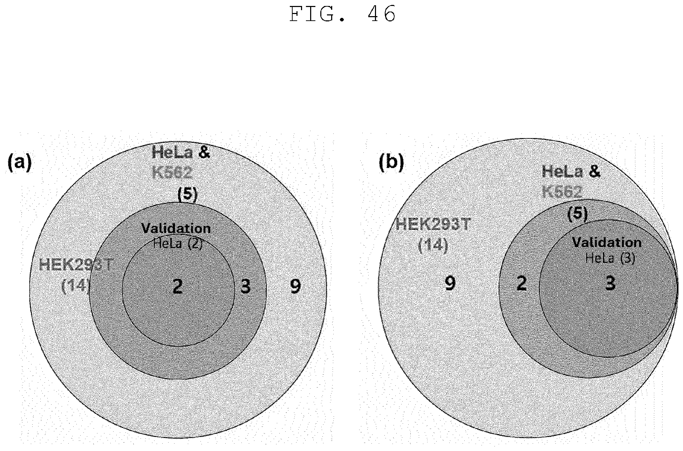

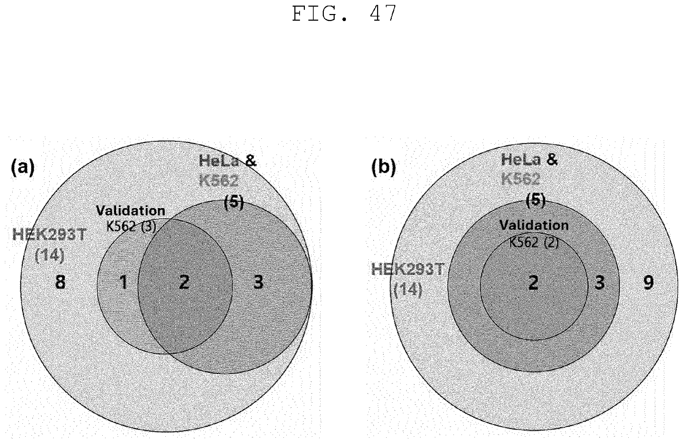

Next, the inventors of the present application determined whether candidate off-target sites were enabled to be validated in the HEK293T, HeLa, and K562 cell lines, and compared the validation results with TAPE-seq predictions for each cell line using Venn diagrams. The results thereof are disclosed in FIGS. 45 to 47. Specifically, FIGS. 45 to 47 show the results of comparing the off-target sites predicted by TAPE-seq with the validated sites. FIG. 45A shows the validation results for *HEK4* (+2 G to T) pegRNA in HEK293T cells and the prediction results in each cell by TAPE-seq using the PE2 prime editor protein. FIG. 45B shows the validation results for *HEK4* (+3 TAA ins) pegRNA in HEK293T cells and the prediction results in each cell by TAPE-seq. FIG. 46A shows the validation results for *HEK4* (+2 G to T) pegRNA in HeLa and the prediction results in each cell by TAPE-seq. FIG. 46B shows the validation results for *HEK4* (+3 TAA ins) pegRNA in HeLa cells and the prediction results in each cell by TAPE-seq. FIG. 47A shows the validation results for *HEK4* (+2 G to T) pegRNA in K562 cells and the prediction results in each cell by TAPE-seq. FIG. 47B shows the validation results for *HEK4* (+3 TAA ins) pegRNA in K562 cells and the prediction results in each cell by TAPE-seq. The valid (validated) off-target sites were much fewer in HeLa and K562 cells than in HEK293T cells.

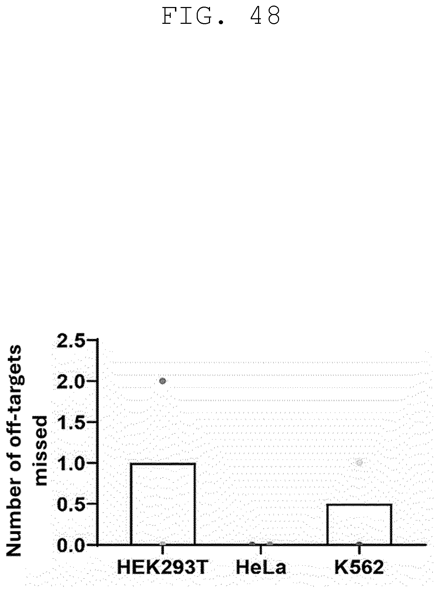

Furthermore, only a few off-target sites in some cell lines were missed by TAPE-seq. The results thereof are disclosed in FIG. 48. Specifically, FIG. 48 shows the results for the number of off-target sites missed in each cell type (bars represent means; error bars represent standard deviations; n = 2, for independent experiments, for individual experiments indicated by dots within the graph).

The inventors of the present application presumed that the TAPE-seq predictions were accurate. Additionally, the TAPE-seq prediction made by using the HEK293T cell line was found in all validated off-target sites for HeLa and K562 cells. Accordingly, in the following experiment, HEK293T cells were used. It will be understood that HEK293T cells are used in the experiments described below unless otherwise stated regarding cell type.

### TAPE-seq analysis using PE2-nuclease and PEmax-nuclease using epegRNA

A report has also been made that a prime editor nuclease containing a wild-type Cas9 nuclease instead of a Cas9 nickase shows higher prime editing efficiency than PE2 (see [Adikusuma, Fatwa, et al. "Optimized nickase-and nuclease-based prime editing in human and mouse cells." Nucleic acids research 49.18 (2021): 10785-10795.]). The inventors of the present application reasoned that the use of such a prime editor nuclease would result in higher tagmentation rates at off-target loci, leading to an increase in the success rate of TAPE-seq for identifying off-target loci. The inventors of the present application performed TAPE-seq using PE2-nuclease and PEmax-nuclease, which are modified versions of the PE2 prime editor, and confirmed these results. PEmax-nuclease was used with epegRNAs. PE2-nuclease and PEmax-nuclease, used in the following experiments, have the following structures, respectively.

### PE2-nuclease:

[bpNLS(SV40)]-[SpCas9 (WT)]-[SGGSx2-XTEN16-SGGSx2]-[MMLV RT]-[bpNLS(SV40)].

### PEmax-nuclease:

[bpNLS(SV40)]-[SpCas9 (R221K)(N394K)]-[SGGSx2-bpNLS(SV40)-SGGSx2]-[MMLV RT(codon opt.)]-[bpNLS(SV40)]-[NLS(c-Myc)].

In this case, the "bpNLS(SV40)" refers to a bipartite SV40 NLS (see [Chen, Peter J., et al. "Enhanced prime editing systems by manipulating cellular determinants of editing outcomes." Cell 184.22 (2021): 5635-5652.; and Wu, Jianrong, Anita H. Corbett, and Keith M. Berland. "The intracellular mobility of nuclear import receptors and NLS cargoes." Biophysical journal 96.9 (2009): 3840-3849.]). Additionally, the "MMLV RT" refers to a Moloney murine leukemia virus RT pentamutant (MMKV RT_D200N, T306K, W313F, T330P, and L603W), "codon opt." refers to human codon optimized, and "NLS(c-Myc)" refers to a c-Myc NLS (see [Chen, Peter J., et al. "Enhanced prime editing systems by manipulating cellular determinants of editing outcomes." Cell 184.22 (2021): 5635-5652.; and Dang, Chi V., and WMk Lee. "Identification of the human c-myc protein nuclear translocation signal." Molecular and cellular biology 8.10 (1988): 4048-4054.]) .

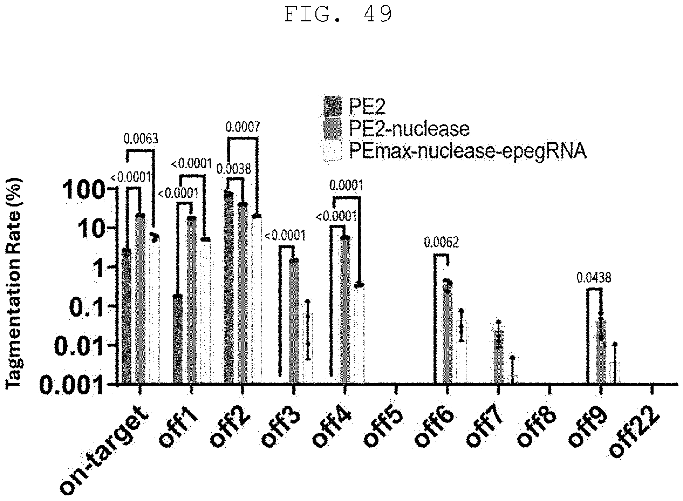

The optimization experiments showed that transient transfection was sufficient for PE2-nuclease (see [Adikusuma, Fatwa, et al. "Optimized nickase-and nuclease-based prime editing in human and mouse cells." Nucleic acids research 49.18 (2021): 10785-10795.]) and PEmax-nuclease (see [Chen, Peter J., et al. "Enhanced prime editing systems by manipulating cellular determinants of editing outcomes." Cell 184.22 (2021): 5635-5652.; and Nelson, James W., et al. "Engineered pegRNAs improve prime editing efficiency." Nature biotechnology 40.3 (2022): 402-410.]) used with engineered pegRNAs (epegRNAs). Furthermore, it was confirmed that a higher tagmentation rate was observed compared to that confirmed in TAPE-seq performed using PE2. The results thereof are disclosed in FIG. 49. Specifically, FIG. 49 discloses the results for each tagmentation rate in TAPE-seq using PE2, PE2-nuclease, and PEmax-nuclease with epegRNAs. The tagmentation rates were confirmed for *HEK4* on-target sites and various off-target sites (two-sided unpaired student t-test; n = 3, for individual transfections).

It was confirmed that the on-target tagmentation rates for PE2-nuclease and PEmax-nuclease using epegRNAs (that is, a prime editing system using epegRNAs and PEmax-nuclease) were significantly higher than that for PE2 (see FIG. 49). There were only 1110 on-target TAPE-seq reads for PE2-nuclease and 906 on-target TAPE-seq reads for PEmax-nuclease with epegRNAs. This compares to 62565 reads in the PE2 sample (Week 2) (see Analysis Data 2 and Tables 2 to 10). PE2-nuclease and PEmax-nuclease using epegRNAs identified 30 and 27 candidates, respectively, compared to the PE2 sample (Week 2) identifying 8 candidates.

The inventors of the present application performed TAPE-seq on 10 different pegRNAs using PE2-nuclease and PEmax-nuclease used with epegRNAs, respectively. Then, the results thereof were compared with the results of TAPE-seq using PE2 using Venn diagrams. The comparison results are disclosed in FIGS. 50 to 54. Specifically, FIGS. 50 to 54 disclose the TAPE-seq results using the prime editing system using PEmax nuclease and epegRNAs, the TAPE-seq results using PE2, and the TAPE-seq results using PE2-nuclease, and the validated off-target sites using the Venn diagrams. FIG. 50 discloses the results for *HEK4* (+2 G to T) pegRNA (or epegRNA) and *HEK4* (+3 TAA ins) pegRNA. Regarding FIG. 50, PE2 TAPE-seq shows a combination of the PE2 TAPE-seq results of HEK4 (+2 G to T) pegRNA and the PE2 TAPE-seq results of HEK4 (+3 TAA ins) pegRNA. FIG. 51 discloses the results for *HBB* (+4 A to T) pegRNA and *DNMT1* (+6 G to C) pegRNA. FIG. 52 discloses the results for *VEGFA* (+5 G to T) pegRNA and *EMX1*(+5 G to T) pegRNA. FIG. 53 discloses the results for *FANCF* (+6 G to C) pegRNA and *HEK3* (+1 CTT ins) pegRNA. FIG. 54 discloses the results for *RNF2* (+6 G to A) pegRNA and *RUNX1* (+6 G to C) pegRNA.

Furthermore, the inventors of the present application compared the TAPE-seq results of PEmax-nuclease using epegRNAs with the prediction results of GUIDE-seq and nDigenome-seq using Venn diagrams. The results are disclosed in FIGS. 55 to 59. Specifically, FIGS. 55 to 59 disclose the comparison results for the off-target prediction results of nDigenome-seq, GUIDE-seq, and TAPE-seq (using PEmax-nuclease and epegRNAs) with the off-target validation results. FIG. 55 discloses the results for *HEK4* (+2 G to T) pegRNA and *HEK4* (+3 TAA ins) pegRNA. FIG. 56 discloses the results for *HBB* (+4 A to T) pegRNA and *DNMT1* (+6 G to C) pegRNA. FIG. 57 discloses the results for *VEGFA* (+5 G to T) pegRNA and *EMX1* (+5 G to T) pegRNA. FIG. 58 discloses the results for *FANCF* (+6 G to C) pegRNA and *HEK3* (+1 CTT ins) pegRNA. FIG. 59 discloses the results for *RNF2* (+6 G to A) pegRNA and *RUNX1* (+6 G to C) pegRNA. For convenience, epegRNAs are improved version of pegRNAs and thus called pegRNAs.

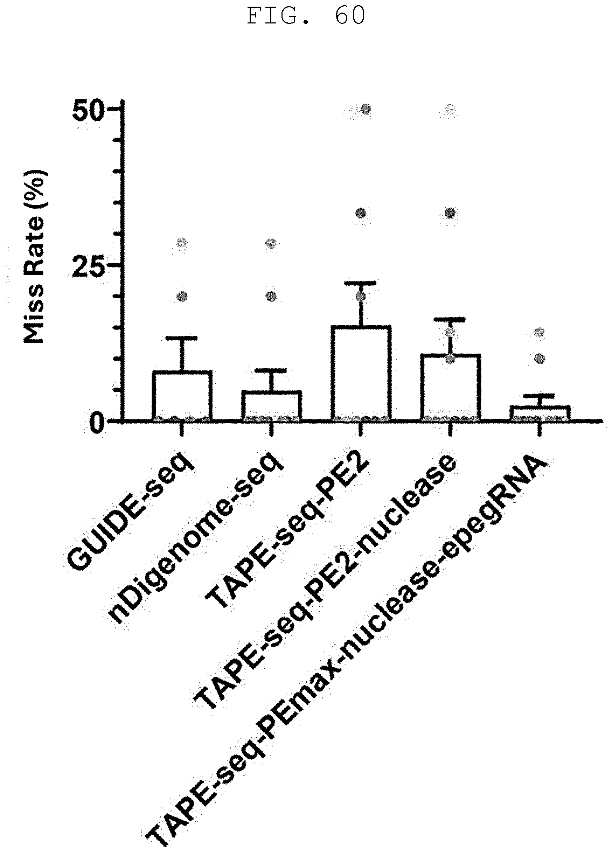

The inventors of the present application compared miss rates of TAPE-seq performed using PE2, PE2-nuclease, and PEmax-nuclease used with epegRNAs. The miss rates were compared for 10 different pegRNAs (pegRNAs used in FIGS. 50 to 59). Furthermore, the results were compared with those of GUIDE-seq and nDigenome-seq (see Analysis Data 6 and Tables 27 to 35) (see FIG. 60). In this case, the miss rate was defined as the number of validated off-target sites missed by the prediction method divided by the total number of validated off-target sites. The results thereof are disclosed in FIG. 60. Specifically, the miss rates of each of the prediction methods, GUIDE-seq, nDigenome-seq, TAPE-seq (PE2), TAPE-seq (PE2-nuclease), and TAPE-seq (using PEmax-nuclease with epegRNAs), were compared (bars represent means; error bars represent standard deviations). In FIG. 60, the results for each pegRNA were indicated by dots (n = 6 in GUIDE-seq, for independent experiments; n = 10 in the rest, for independent experiments).

TAPE-seq using PEmax-nuclease used with epegRNAs showed the lowest miss rate. Compared to the results in FIG. 39, it should be noted that there was an increase in the results for validated off-target sites missed for PE2. This is because newly validated off-target sites were confirmed in TAPE-seq using PE2-nuclease and PEmax-nuclease used with epegRNAs.

### ROC curve analysis of TAPE-seq

A receiver operating characteristic curve (ROC curve) is a plot showing the diagnostic ability of a binary classifier. The inventors of the present application constructed ROC curves for TAPE-seq analysis using PE2, PE2-nuclease, and PE2-nuclease with epegRNAs. These ROC curves were compared with those for GUIDE-seq and nDigenome-seq. The inventors of this application reasoned that the diagnostic ability of each method could be quantitatively compared through a comparison of ROC curves. The diagnostic ability of the TAPE-seq metric (copy number) was compared with those of GUIDE-seq (copy number) and nDigenome-seq (DNA cleavage score). The analysis results of the ROC curve for each off-target prediction method are disclosed in FIGS. 61 to 66. Specifically, the ROC curve results of the GUIDE-seq, nDigenome-seq, TAPE-seq (PE2), TAPE-seq (PE2-nuclease), and TAPE-seq (PEmax-nuclease and epegRNAs) prediction methods are disclosed in FIGS. 61 to 66. FIG. 61 shows the results for *HEK4* (+2 G to T) pegRNA and *HEK4* (+3 TAA ins) pegRNA. FIG. 62 shows the results for *HBB* (+4 A to T) pegRNA and *DNMT1* (+6 G to C) pegRNA. FIG. 63 shows the results for *HEK3* (+1 CTT ins) pegRNA. FIG. 64 shows the results for *EMX1* (+5 G to T) pegRNA and *FANCF* (+6 G to C) pegRNA. FIG. 65 shows the results for *RNF2* (+6 G to A) pegRNA and *RUNX1* (+6 G to C) pegRNA. FIG. 66 shows the results for *VEGFA* (+5 G to T) pegRNA. For convenience, the epegRNA was also expressed as the pegRNA.

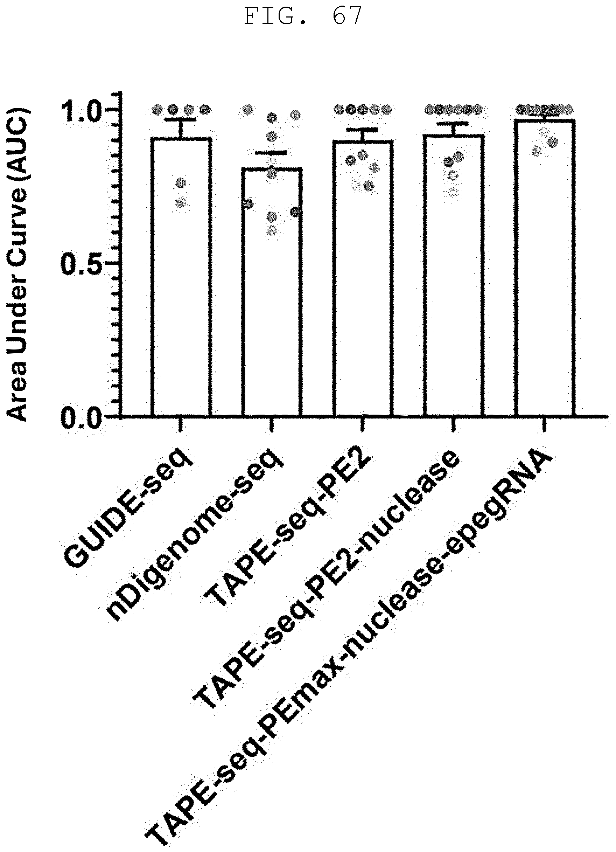

On the basis of the ROC curve analysis results, the area under the ROC curves (AUC) was calculated for each off-target prediction method. The results are disclosed in FIG. 67 (bars represent means; error bars represent standard deviations). In FIG. 67, the results for each pegRNA were represented by dots (n = 6 in GUIDE-seq, for independent experiments; n = 10 in the rest, for independent experiments).

When comparing the AUC for each off-target prediction method, TAPE-seq results using PEmax-nuclease used with epegRNAs showed the highest value. These results suggest that the TAPE-seq metric shows superior diagnostic ability in predicting off-target regions compared to GUIDE-seq and nDigenome-seq.

### Editing patterns at validated off-target sites

Editing patterns of all validated off-target sites were analyzed through a comparison of targeted deep sequencing results (see FIGS. 68 to 87). As pegRNAs, *HEK4* (+2 G to T) pegRNA, *HEK4* (+3 TAA ins) pegRNA, *HBB* (+4 A to T) pegRNA, *DNMT1* (+6 G to C) pegRNA, and *VEGFA* (+5 G to T) pegRNA were used. As prime editing systems, PE2, PE2-nuclease, and PEmax-nuclease used with the epegRNA were used. Furthermore, the analysis was performed in HEK293T, HeLa, and K562 cells.

An editing pattern induced by *HEK4* (+3 TAA ins) pegRNA at the HEK4-off3 site predicted by TAPE-seq is disclosed in FIG. 68 (bars represent means; error bars represent standard deviations; n = 3, for individual transfections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. In other words, the lowercase letters in the WT sequence indicate mismatches when compared to on-target sequences corresponding to a spacer and a PAM of pegRNA. The major edited rate corresponds to the frequency of edited sequences.

Editing patterns induced by *HEK4* (+2 G to T) pegRNA at the *HEK*-off7, *HEK*-off10, and *HEK-*off22 sites predicted by TAPE-seq are disclosed in FIGS. 69 to 71 (bars represent means; error bars represent standard deviations; n = 3, for individual transfections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

Results for editing patterns at validated off-target sites related to *HEK4* (+2 G to T) pegRNA are disclosed in FIGS. 72 to 75 (bars represent means; error bars represent standard deviations; n = 3, for individual transfections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

FIG. 76 discloses the results for an editing pattern at a validated off-target site related to *HBB* (+4 A to T) pegRNA (bars represent means; error bars represent standard deviations; n = 3, for individual transfections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

FIGS. 77 to 80 disclose the results for editing patterns at validated off-target sites related to *HEK4*(+3 TAA ins) pegRNA (bars represent means; error bars represent standard deviations; n = 3, for individual transfections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

FIGS. 81 to 82 disclose the results in HeLa cells. Specifically, the results for *HEK4* (+3 TAA ins) pegRNA and *HEK4* (+2 G to T) pegRNA are disclosed (bars represent mean; error bars represent standard deviations; n = 3, independent traits for infections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

FIGS. 83 to 84 show the results in K562 cells. Specifically, the results for *HEK4* (+3 TAA ins) pegRNA and *HEK4* (+2 G to T) pegRNA are disclosed (bars represent mean; error bars represent standard deviations; n = 3, independent traits for infections; two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

FIGS. 85 to 87 disclose the results for editing patterns at validated off-target sites only identified by TAPE-seq performed using PEmax-nuclease. Specifically, the results for *HEK4* (+2 G to T) pegRNA, *DNMT1* (+6 G to C) pegRNA, *HBB* (+4 A to T) pegRNA, and *VEGFA* (+5 to T) pegRNA are disclosed (bars represent means; error bars represent standard deviations; n = 3, two-sided unpaired student t-test). NC refers to the negative control. Lowercase letters indicate mismatches when compared to the pegRNA. For convenience of sequence comparison, a sequence of pegRNA, expressed as pegRNA in the drawing, is expressed on the basis of the on-target sequence. The major edited rate corresponds to the frequency of edited sequences.

The sequences disclosed in FIGS. 68 to 87 were assigned sequence numbers as follows. In each sequence where a bulge is indicated by a "-", a version of the sequence without the "-" is listed in the sequence listing.

In FIG. 68, the sequence labeled WT was assigned SEQ ID NO: 153, the sequence labeled edited was assigned SEQ ID NO: 154, and the sequence labeled pegRNA was assigned SEQ ID NO: 155.

In FIG. 69, the sequence labeled WT was assigned SEQ ID NO: 156, the sequence labeled edited was assigned SEQ ID NO: 157, and the sequence labeled pegRNA was assigned SEQ ID NO: 158.

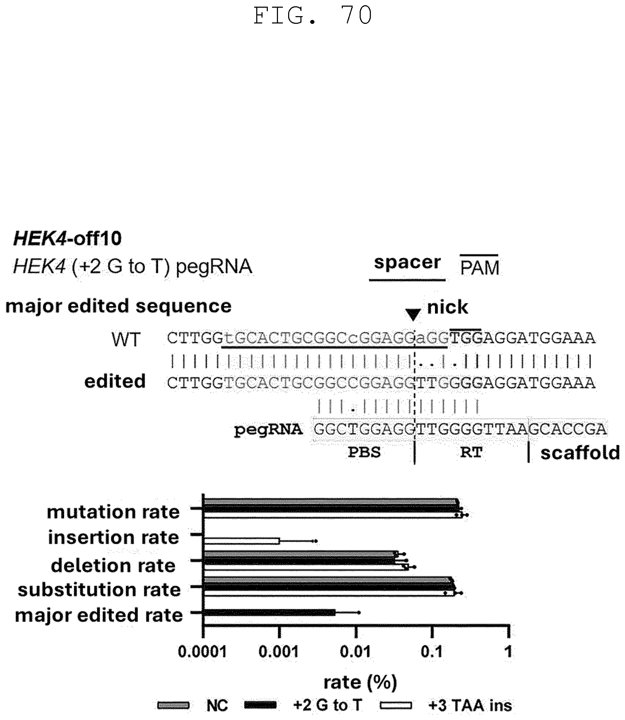

In FIG. 70, the sequence labeled WT was assigned SEQ ID NO: 159, the sequence labeled edited was assigned SEQ ID NO: 160, and the sequence labeled pegRNA was assigned SEQ ID NO: 161.

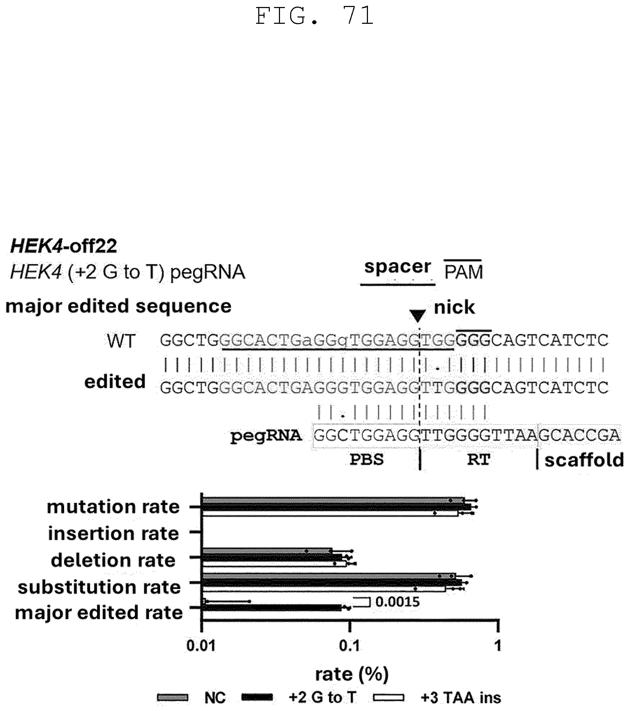

In FIG. 71, the sequence labeled WT was assigned SEQ ID NO: 162, the sequence labeled edited was assigned SEQ ID NO: 163, and the sequence labeled pegRNA was assigned SEQ ID NO: 164.

Regarding *HEK4*-off1 in FIG. 72, the sequence labeled WT was assigned SEQ ID NO: 165, the sequence labeled edited was assigned SEQ ID NO: 166, and the sequence labeled pegRNA was assigned SEQ ID NO: 167.

Regarding *HEK4*-off2 in FIG. 72, the sequence labeled WT was assigned SEQ ID NO: 168, the sequence labeled edited was assigned SEQ ID NO: 169, and the sequence labeled pegRNA was assigned SEQ ID NO: 170.

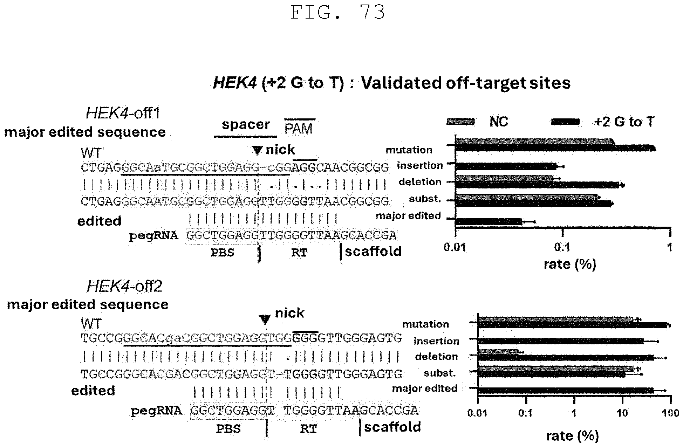

Regarding *HEK4-*off4 in FIG. 73, the sequence labeled WT was assigned SEQ ID NO: 171, the sequence labeled edited was assigned SEQ ID NO: 172, and the sequence labeled pegRNA was assigned SEQ ID NO: 173.

Regarding *HEK4*-off6 in FIG. 73, the sequence labeled WT was assigned SEQ ID NO: 174, the sequence labeled edited was assigned SEQ ID NO: 175, and the sequence labeled pegRNA was assigned SEQ ID NO: 176.

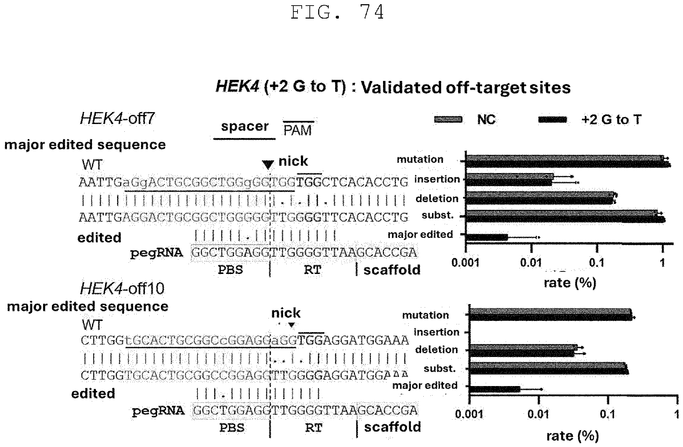

Regarding *HEK4*-off7 in FIG. 74, the sequence labeled WT was assigned SEQ ID NO: 177, the sequence labeled edited was assigned SEQ ID NO: 178, and the sequence labeled pegRNA was assigned SEQ ID NO: 179.

Regarding *HEK4*-off10 in FIG. 74, the sequence labeled WT was assigned SEQ ID NO: 180, the sequence labeled edited was assigned SEQ ID NO: 181, and the sequence labeled pegRNA was assigned SEQ ID NO: 182.

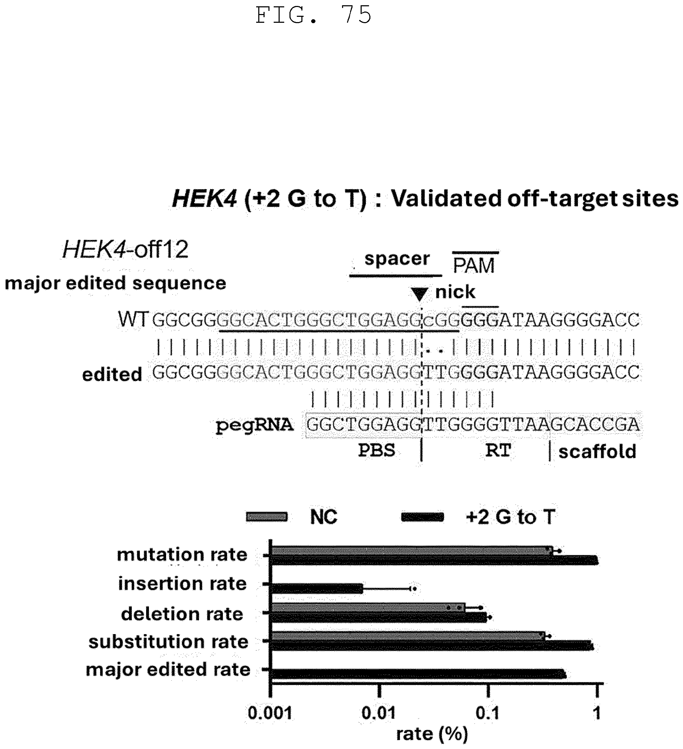

Regarding *HEK4*-off12 in FIG. 75, the sequence labeled WT was assigned SEQ ID NO: 183, the sequence labeled edited was assigned SEQ ID NO: 184, and the sequence labeled pegRNA was assigned SEQ ID NO: 185.

Regarding *HBB*-off1 in FIG. 76, the sequence labeled WT was assigned SEQ ID NO: 186, the sequence labeled edited was assigned SEQ ID NO: 187, and the sequence labeled pegRNA was assigned SEQ ID NO: 188.

Regarding *HEK4*-off1 in FIG. 77, the sequence labeled WT was assigned SEQ ID NO: 189, the sequence labeled edited was assigned SEQ ID NO: 190, and the sequence labeled pegRNA was assigned SEQ ID NO: 191.

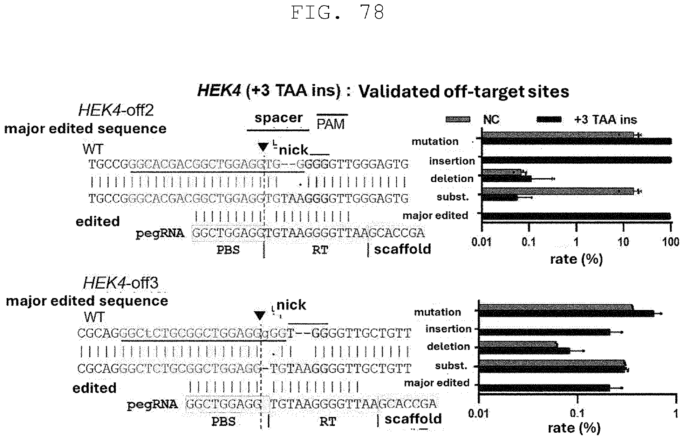

Regarding *HEK4*-off2 in FIG. 78, the sequence labeled WT was assigned SEQ ID NO: 192, the sequence labeled edited was assigned SEQ ID NO: 193, and the sequence labeled pegRNA was assigned SEQ ID NO: 194.

Regarding *HEK4*-off3 in FIG. 78, the sequence labeled WT was assigned SEQ ID NO: 195, the sequence labeled edited was assigned SEQ ID NO: 196, and the sequence labeled pegRNA was assigned SEQ ID NO: 197.

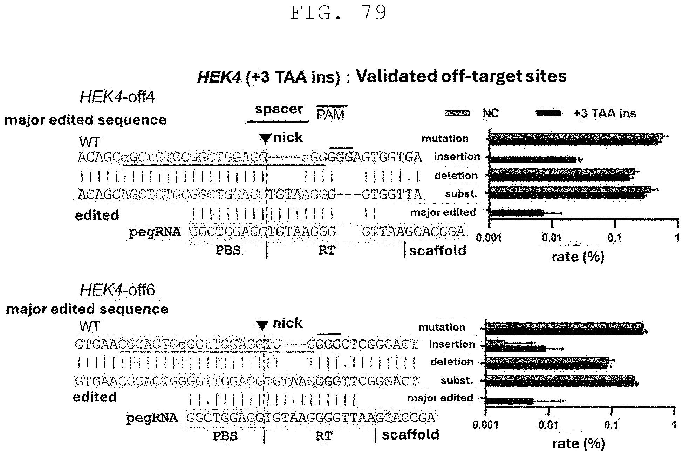

Regarding *HEK4*-off4 in FIG. 79, the sequence labeled WT was assigned SEQ ID NO: 198, the sequence labeled edited was assigned SEQ ID NO: 199, and the sequence labeled pegRNA was assigned SEQ ID NO: 200.

Regarding *HEK4*-off6 in FIG. 79, the sequence labeled WT was assigned SEQ ID NO: 201, the sequence labeled edited was assigned SEQ ID NO: 202, and the sequence labeled pegRNA was assigned SEQ ID NO: 203.

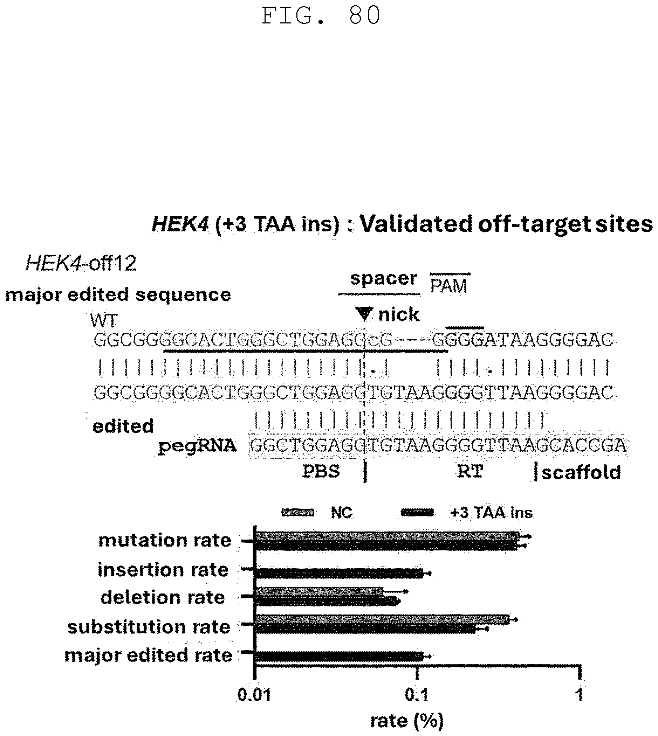

Regarding *HEK4*-off12 in FIG. 80, the sequence labeled WT was assigned SEQ ID NO: 204, the sequence labeled edited was assigned SEQ ID NO: 205, and the sequence labeled pegRNA was assigned SEQ ID NO: 206.

Regarding *HEK4*-off2 (+3 TAA ins) in FIG. 81, the sequence labeled WT was assigned SEQ ID NO: 207, the sequence labeled edited was assigned SEQ ID NO: 208, and the sequence labeled pegRNA was assigned SEQ ID NO: 209.

Regarding *HEK4*-off3 (+3 TAA ins) in FIG. 81, the sequence labeled WT was assigned SEQ ID NO: 210, the sequence labeled edited was assigned SEQ ID NO: 211, and the sequence labeled pegRNA was assigned SEQ ID NO: 212.

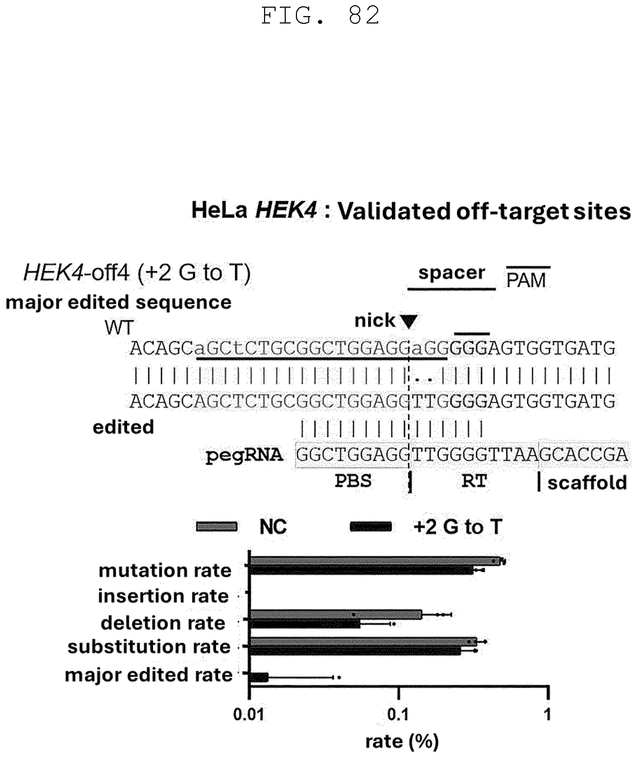

Regarding *HEK4*-off4 (+2 G to T) in FIG. 82, the sequence labeled WT was assigned SEQ ID NO: 213, the sequence labeled edited was assigned SEQ ID NO: 214, and the sequence labeled pegRNA was assigned SEQ ID NO: 215.

Regarding *HEK4*-off1 (+2 G to T) in FIG. 83, the sequence labeled WT was assigned SEQ ID NO: 216, the sequence labeled edited was assigned SEQ ID NO: 217, and the sequence labeled pegRNA was assigned SEQ ID NO: 218.

Regarding *HEK4*-off3 (+2 G to T) in FIG. 83, the sequence labeled WT was assigned SEQ ID NO: 219, the sequence labeled edited was assigned SEQ ID NO: 220, and the sequence labeled pegRNA was assigned SEQ ID NO: 221.

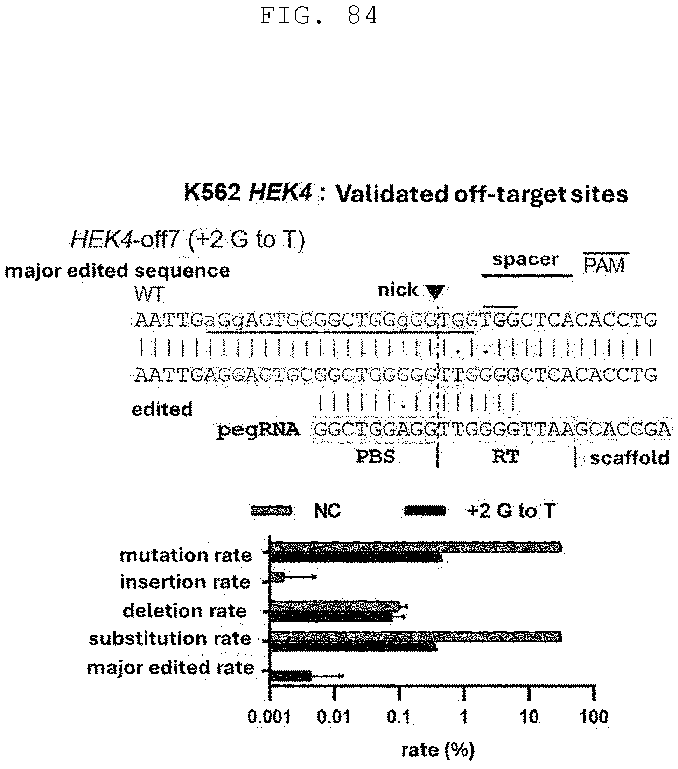

Regarding *HEK4*-off7 (+2 G to T) in FIG. 84, the sequence labeled WT was assigned SEQ ID NO: 222, the sequence labeled edited was assigned SEQ ID NO: 223, and the sequence labeled pegRNA was assigned SEQ ID NO: 224.

Regarding *HEK4*-off22 (+2 G to T) in FIG. 85, the sequence labeled WT was assigned SEQ ID NO: 225, the sequence labeled edited was assigned SEQ ID NO: 226, and the sequence labeled pegRNA was assigned SEQ ID NO: 227.

Regarding *HEK4*-off28 (+2 G to T) in FIG. 85, the sequence labeled WT was assigned SEQ ID NO: 228, the sequence labeled edited was assigned SEQ ID NO: 229, and the sequence labeled pegRNA was assigned SEQ ID NO: 230.

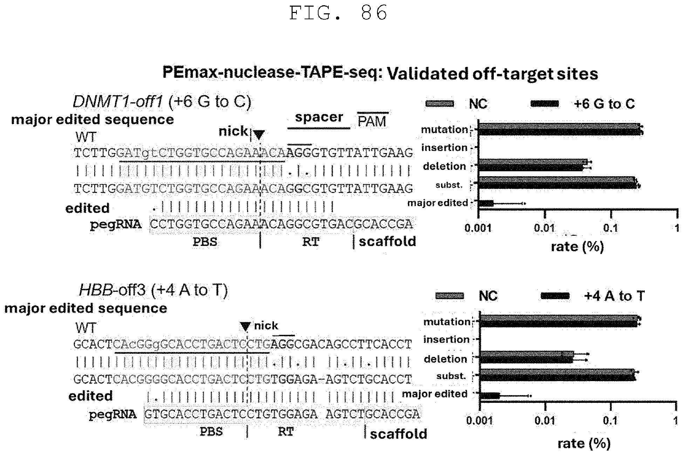

Regarding *DNMT1-*off1 (+6 G to C) in FIG. 86, the sequence labeled WT was assigned SEQ ID NO: 231, the sequence labeled edited was assigned SEQ ID NO: 232, and the sequence labeled pegRNA was assigned SEQ ID NO: 233.

Regarding *HBB*-off3 (+4 A to T) in FIG. 86, the sequence labeled WT was assigned SEQ ID NO: 234, the sequence labeled edited was assigned SEQ ID NO: 235, and the sequence labeled pegRNA was assigned SEQ ID NO: 236.

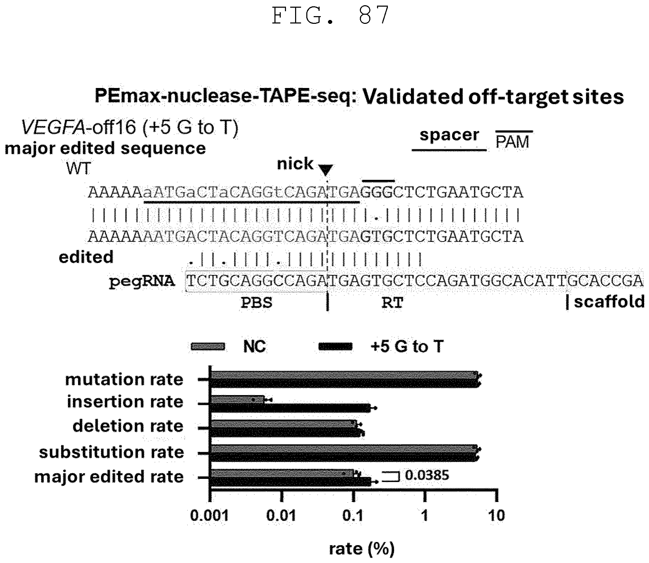

Regarding *VEGFA*-off16 (+5 G to T) in FIG. 87, the sequence labeled WT was assigned SEQ ID NO: 237, the sequence labeled edited was assigned SEQ ID NO: 238, and the sequence labeled pegRNA was assigned SEQ ID NO: 239.

Referring to FIGS. 68 to 71, only *HEK4* (+3 TAA ins) pegRNA induced editing at the *HEK4*-off3 site predicted by TAPE-seq, whereas only *HEK4* (+2 G to T) pegRNA caused off-target effects at *HEK4*-off7, *HEK4*-off10, and *HEK4*-off22 predicted by TAPE-seq. These results suggest that off-target effects may also be dependent on the sequence of the RT template. This tendency may partially demonstrate the higher area under the ROC curve in TAPE-seq compared to GUIDE-seq or nDigenome-seq. This is because these two methods are performed using a single guide RNA (sgRNA) free of the sequence of the RT template.

### Mismatch analysis by region

The inventors of the present application tabulated the number of mismatches in the PBS, RT template, and spacer region of the pegRNA and listed the results with the validation results (see Analysis Data 7 and Tables 36 to 44) .

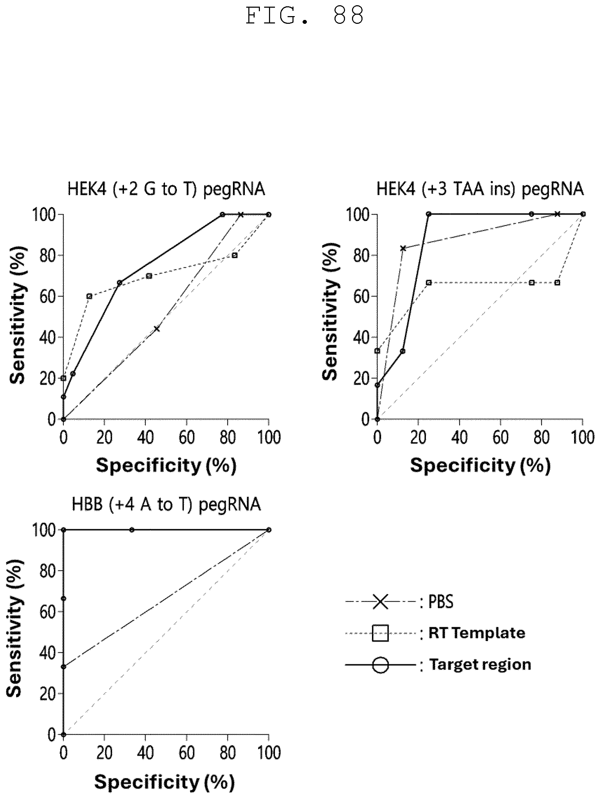

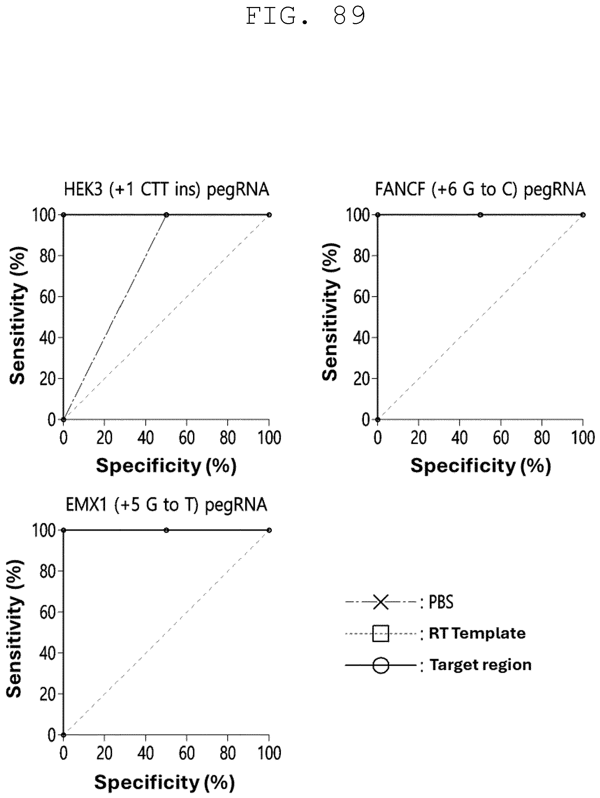

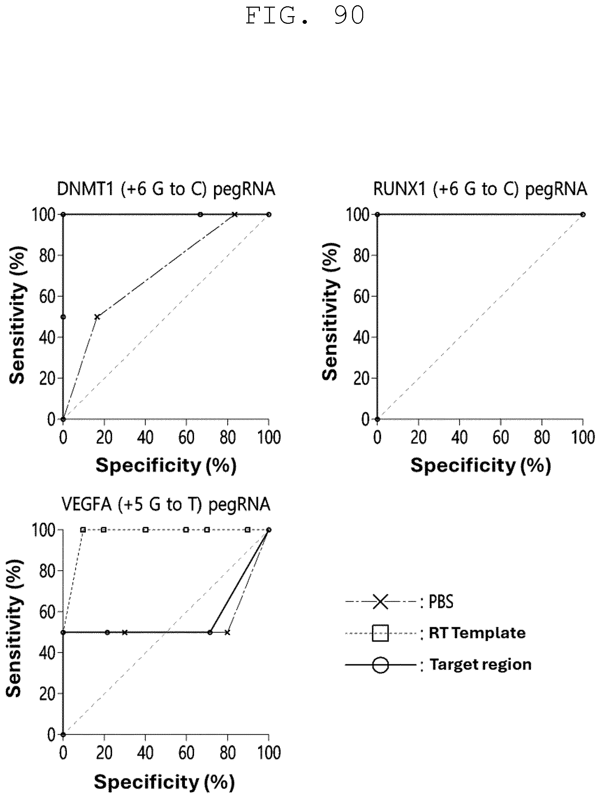

ROC curves were constructed using the number of mismatches, instead of the copy number, as the metric for predicting validation results in binary classification. The results thereof are disclosed in FIGS. 88 to 90 (in which case RNF2 was excluded as there was only one sample). Specifically, the analysis results for the ROC curves for mismatches in the PBS region, the RT template region, and a target region are disclosed in FIGS. 88 to 90. FIG. 88 shows the results for *HEK4* (+2 G to T) pegRNA, *HEK4* (+3 TAA ins) pegRNA, and *HBB* (+4 A to T) pegRNA. FIG. 89 shows the results for *HEK3* (+1 CTT ins) pegRNA, *FANCF* (+6 G to C) pegRNA, and *EMX1* (+5 G to T) pegRNA. FIG. 90 shows the results for *DNMT1* (+6 G to C) pegRNA, *RUNX1* (+6 G to C) pegRNA, and *VEGFA* (+5 G to T) pegRNA.

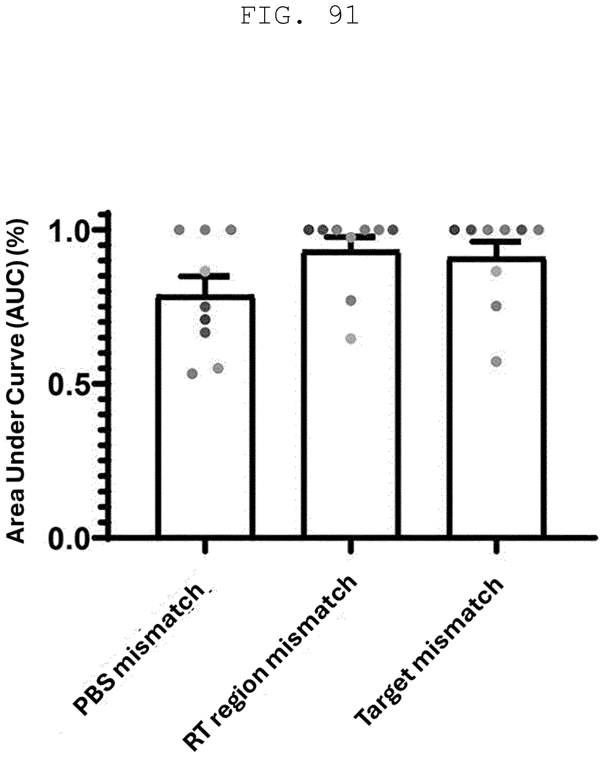

FIG. 91 discloses the results for the AUC by mismatches in each region, calculated from FIGS. 88 to 90. The results thereof are disclosed for nine different pegRNAs. Each result for the pegRNA is indicated by a dot (n = 10, in which case RNF2 was excluded as there was only one sample).

In most cases, the AUC for mismatches in the RT template region was higher than that in the PBS.

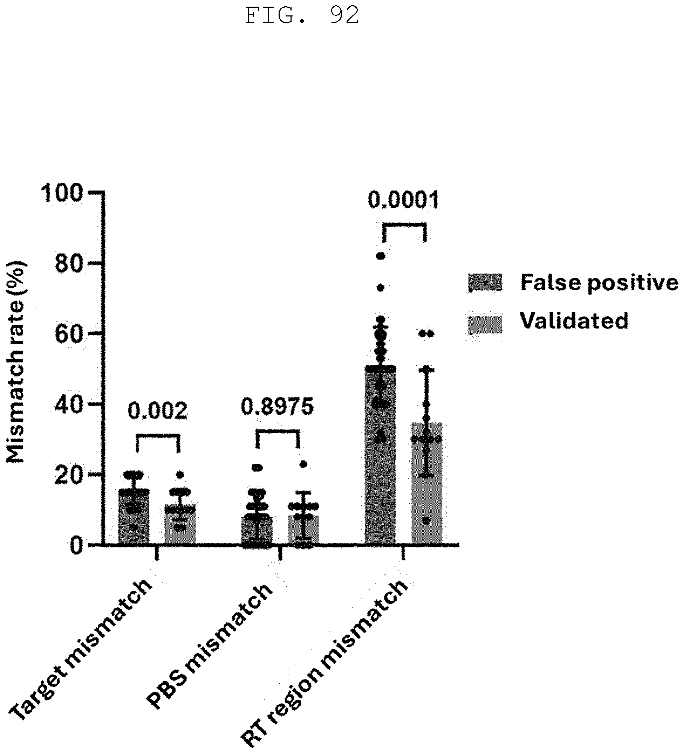

FIG. 92 shows the mismatch rates for sites predicted by TAPE-seq, false positive sites, and validated sites. Regarding the false positive sites, target mismatches and RT region mismatches were confirmed for independent target loci of n = 54. PBS mismatches were confirmed for target loci of n = 47. Seven loci with DNA/RNA expansions for PBS mismatches were excluded from the analysis. Regarding the validated sites, target mismatches and RT region mismatches were confirmed for independent target loci of n = 13. PBS mismatches were confirmed for target loci of n = 12. One locus with DNA/RNA expansions for PBS mismatches was excluded from the analysis (two-sided unpaired student t-test; bars represent means; error bars represent standard deviations).

When comparing the mismatch rates for the false and validated targets in the PBS, target, and RT template regions, the rate for false was significantly higher in the target and RT template regions, but not in the PBS region, than the rate for validated. Generally, the RT template mismatch appears to be as influential as target mismatches in predicting the effectiveness of potential off-target sites. Unlike TAPE-seq, GUIDE-seq and nDigenome-seq do not include RT-related mechanisms in the protocol, so the ability to accommodate the molecular mechanisms of RT is limited in the off-target prediction process. While methods using only existing Cas9, such as GUIDE-seq or nDigenome-seq, are free of RT sequences in reverse transcriptases or pegRNAs, TAPE-seq is based on the molecular mechanism of prime editing (using pegRNAs, involving reverse transcriptases, and the like) and thus is presumed to show a superior diagnostic ability by effectively removing false positive off-target sites.

The inventors of the present application have developed TAPE-seq, an off-target prediction method suitable for application to prime editing systems. This TAPE-seq system, an off-target prediction method that well includes the characteristics of prime editing, provides a new platform. As early developers of TAPE-seq, the inventors of the present application have confirmed the results for TAPE-seq using various versions of PE. In particular, the application of the prime editing system using PEmax-nuclease and epegRNAs showed remarkably excellent results. Nevertheless, it will be apparent to those skilled in the art that the experimental examples disclosed in the present application do not preclude the use of other versions of PE from the present specification and the claims based thereon.

Furthermore, one of the inventive features of TAPE-seq is in the use of pegRNAs (or epegRNAs) including tag templates. Thus, it will be apparent to those skilled in the art that not only the versions of prime editing proteins developed before the present application, but also versions of prime editing proteins developed after the present application are applicable to the TAPE-seq method. Additionally, it will be apparent to those skilled in the art that inventions that inherit the inventive features of TAPE-seq, for example, even in the case of using other modified pegRNAs, are included herein and the claims based thereon.

TAPE-seq identified fewer off-target loci for PE2 compared to DSB-inducing Cas9 targeting the same sites. In recent years, various techniques have been developed and are being developed to increase the efficiency of PE2. Some of these technologies are applied to the TAPE-seq protocol to increase the tagmentation rate, which also increases the sensitivity of TAPE-seq for identifying new off-target loci missed by previous methods. As described above, it is expected that improved versions of the prime editing system developed by and/or to be developed after the filing date of the present application filing date of the present disclosure are applicable to improve performance, such as the sensitivity, of TAPE-seq. Increasing the sensitivity of TAPE-seq is expected to enable more off-target loci, which have been previously missed, to be identified. Furthermore, optimizing tagmentation conditions is expected to enable the sensitivity of TAPE-seq to increase.

The advantages of TAPE-seq include being an unbiased, cell-based method enabling cell type-specific prime editing events to be detectable with a high validation rate, low miss rate, and high area under the ROC curve. This method directly measures PE genome editing activity by embracing the RT mechanism, unlike other methods such as GUIDE-seq and nDigenome-seq, which provide only indirect aspects of the nickase or DSB activity of Cas9. Furthermore, a major limitation of GUIDE-seq is that transfection of some doublestranded oligodeoxynucleotide (dsODN) tags is inevitable, which may be toxic in some intolerant cells or impossible in animal models. In contrast, the tag sequence of TAPE-seq is embedded in the pegRNA itself, so toxicity caused by dsODN is irrelevant to TAPE-seq. Furthermore, in vivo delivery of TAPE-seq vectors may also be possible.

The following considerations may be addressed for the design of tagmentation pegRNAs for TAPE-seq. Because pegRNA is single-stranded, the tag sequence may form secondary structures with adjacent RT or PBS sequences. Such events are detectable by low target tagmentation rates, in which case the tag sequence is required to be corrected before performing the final TAPE-seq analysis. A reverse complementary sequence to the 34-bp tag sequence may be used, or other tag sequences that do not form secondary structures may be designed. In such a process, tools that help design pegRNAs may be used. For example, prediction tools such as Vienna2.0 (see [Lorenz, Ronny, et al. "ViennaRNA Package 2.0." Algorithms for molecular biology 6.1 (2011): 1-14.]) may help design epegRNAs (see [Nelson, James W., et al. "Engineered pegRNAs improve prime editing efficiency." Nature biotechnology 40.3 (2022): 402-410.]).

More PE-based therapeutics are expected to be developed in the future (see [Liu, Pengpeng, et al. "Improved prime editors enable pathogenic allele correction and cancer modelling in adult mice." Nature communications 12.1 (2021): 1-13.; Jang, Hyewon, et al. "Application of prime editing to the correction of mutations and phenotypes in adult mice with liver and eye diseases." Nature biomedical engineering 6.2 (2022): 181-194.; Kim, Yohan, et al. "Adenine base editing and prime editing of chemically derived hepatic progenitors rescue genetic liver disease." cell stem cell 28.9 (2021): 1614-1624.; Schene, Imre F., et al. "Prime editing for functional repair in patient-derived disease models." Nature communications 11.1 (2020): 1-8.; and Petri, Karl, et al. "CRISPR prime editing with ribonucleoprotein complexes in zebrafish and primary human cells." Nature biotechnology 40.2 (2022): 189-193.]. Accordingly, TAPE-seq is expected to become one of the powerful cell-based methods for studying the safety of PE-based drugs.

### Table for analysis data

Hereinbelow, results obtained by the experimental method disclosed in the present application are to be disclosed using tables. Each table provided below was cited appropriately in the section where the results related to the table were discussed.

### Analysis Data 1. Analysis of PE2 TAPE-seq on-target tagmentation sequences

Hereinbelow, the analysis results of PE2 TAPE-seq on-target tagmentation sequences are to be disclosed in Tables 1-1 and 1-2.

Tables 1-1 and 1-2 show associated results. The first column of Table 1-2 enables information in Table 1-2 to be associated with the corresponding information in Table 1-1. In Table 1-2, percent represents the percentage of the indicated sequence out of the total NGS reads. Regarding the entry "sequence", sequences in "Bold" refer to sequences corresponding to spacer regions, sequences in italics (italicized) refer to sequences corresponding to PAMs, and sequences underlined refer to mismatches between the edited sequence and the sequence before being edited.

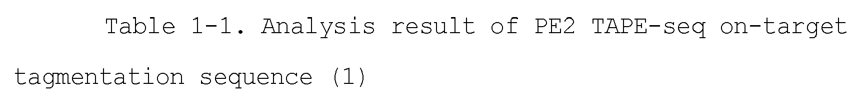

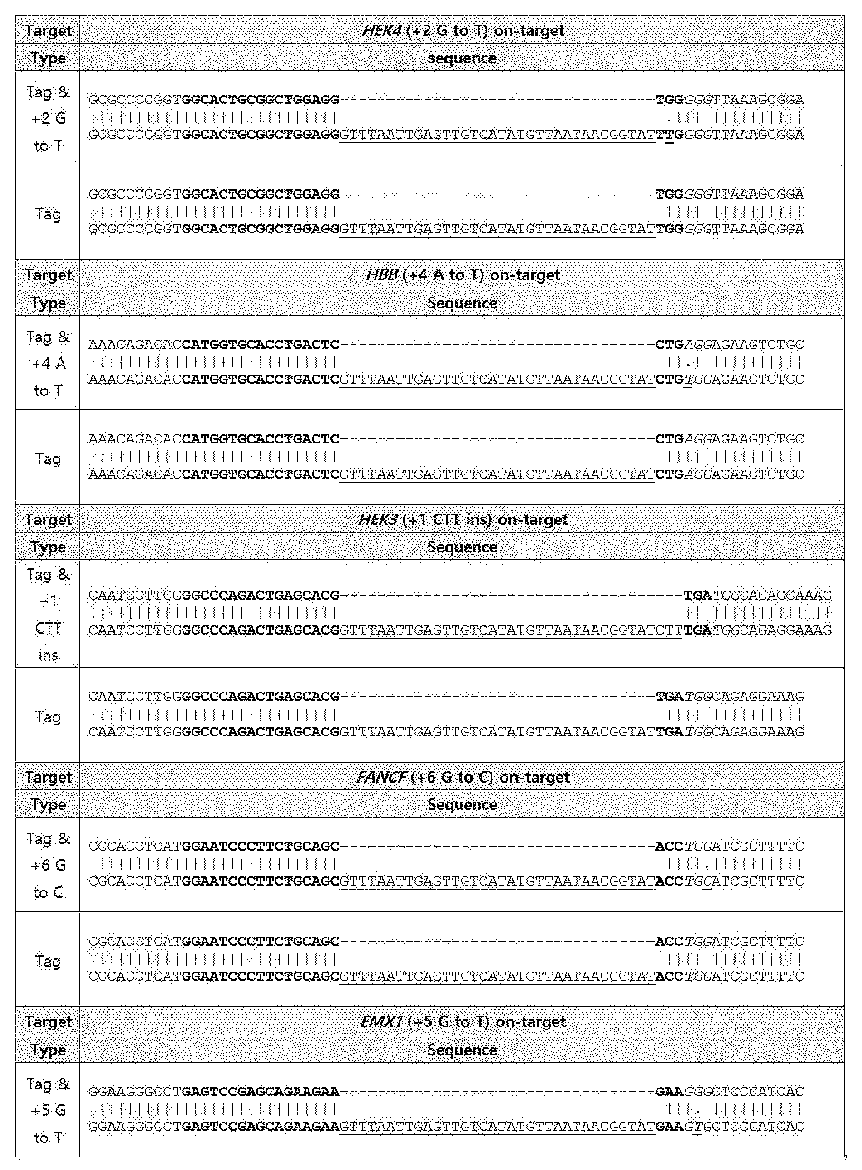

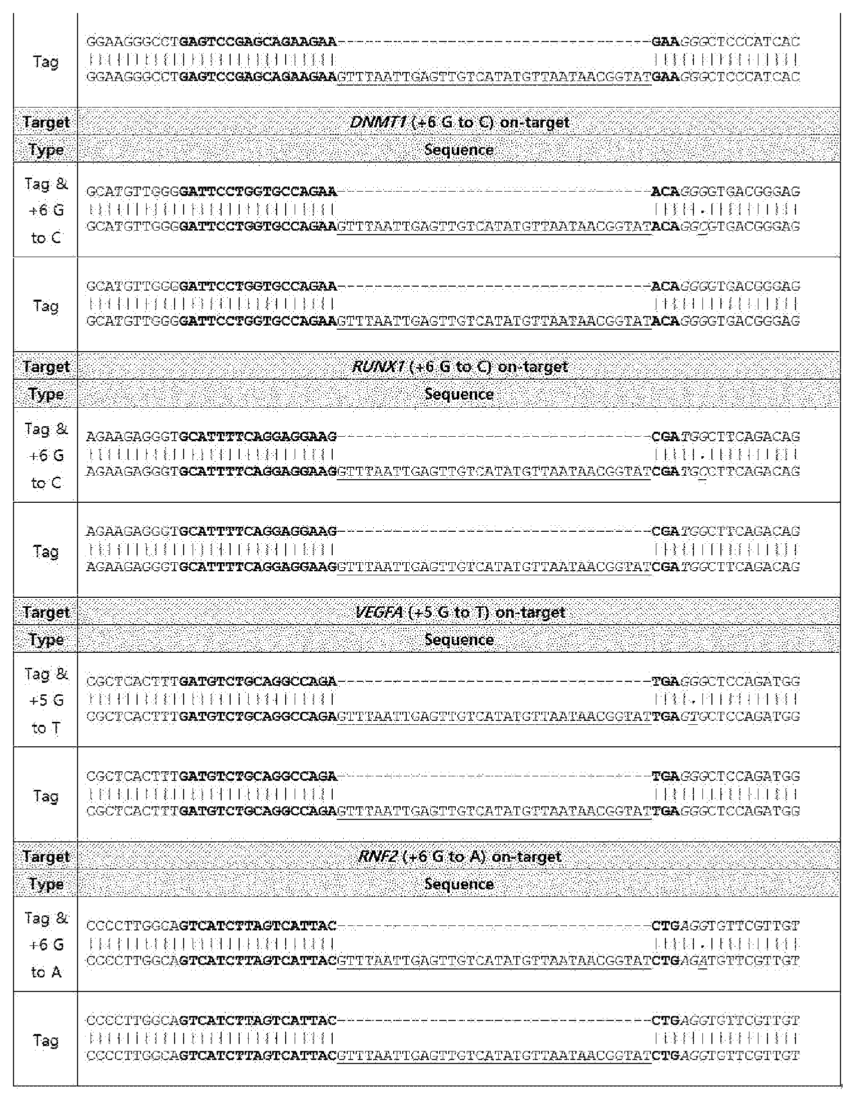

The sequences disclosed in Table 1-1 were assigned SEQ ID NOs: 240 to 275. In Table 1-1, the sequences were assigned SEQ ID NOs: 240 to 275 in such a disclosed order, and the "-" indicating a bulge was omitted from each sequence disclosed in the sequence listing.

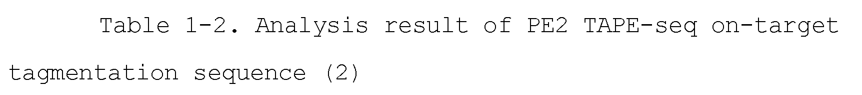

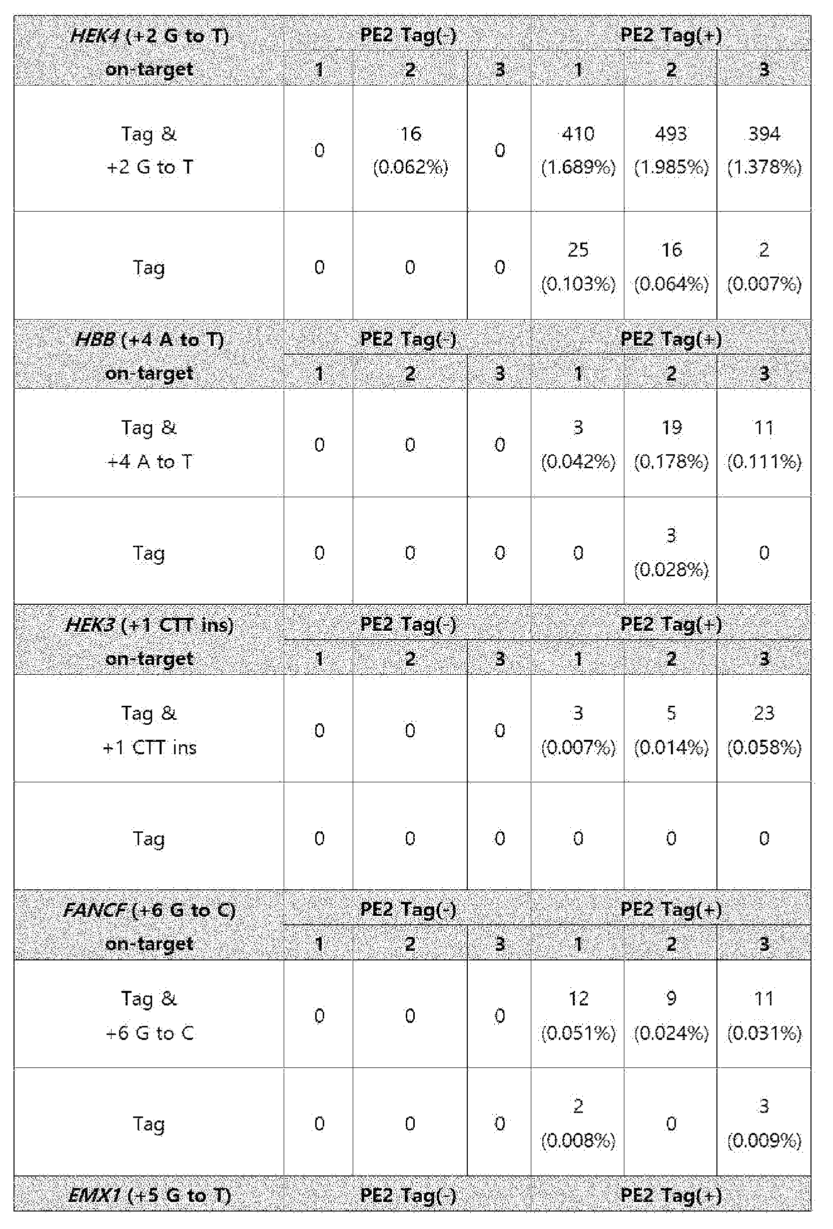

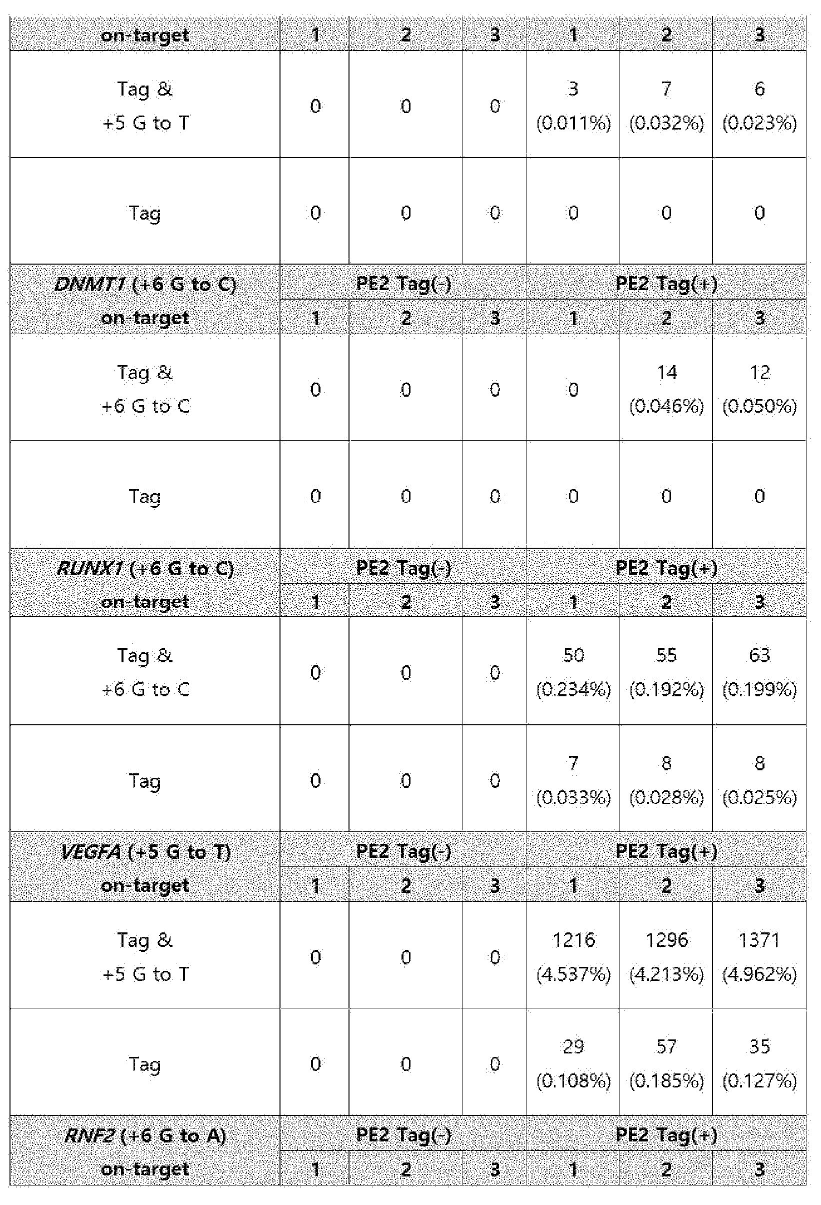

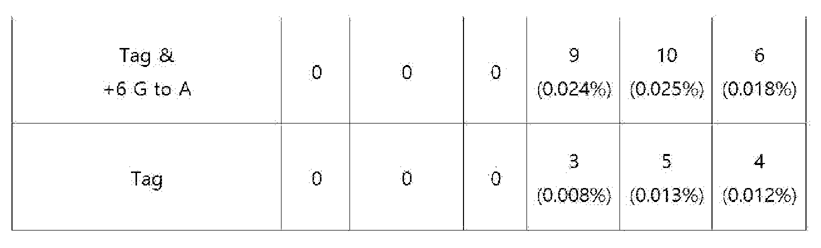

### Analysis Data 2. Comparison of on-target and off-target sites confirmed by TAPE-seq with off-target sites for sgRNA targeting same region confirmed by nDigenome-seq and GUIDE-seq

Hereinbelow, the analysis results for on-target and off-target sites identified by TAPE-seq are to be disclosed. Furthermore, the comparison results with off-target regions for the sgRNA targeting the same region, confirmed by nDigenome-seq and GUIDE-seq, are to be disclosed. In each table, the entry "Type" indicates whether the bulge is an RNA bulge or a DNA bulge. The associated results are expressed as Table n-x. For example, Tables 2-1, 2-2, 2-3, 2-4, and 2-5 are associated results. In a single associated group of tables, each piece of information in each table is associated with the information in another related table through the information in the first column. Regarding the entry "Target", lowercase letters and "-" indicate mismatches with the corresponding regions of the on-target and pegRNA.

Each sequence disclosed in the following tables was assigned a sequence number as follows (see sequence listing). The "-" indicating a bulge was omitted from each sequence disclosed in the sequence listing.

The target sequence of HEK4 on-target was assigned SEQ ID NO: 276. The respective target sequences of the off-targets predicted, HEK4-off1 to HEK4-off39, were assigned SEQ ID NOs: 277 to 315.

The target sequence of HBB on-target was assigned SEQ ID NO: 316. The respective target sequences of the off-targets predicted, HBB-off1 to HBB-off5, were assigned SEQ ID NOs: 317 to 321.

The target sequence of EMX1 on-target was assigned SEQ ID NO: 322. The respective target sequences of the off-targets predicted, EMX1-off1 and EMX1-off2, were assigned SEQ ID NOs: 323 and 324.

The target sequence of FANCF on-target was assigned SEQ ID NO: 325. The respective target sequences of the off-targets predicted, FANCF-off1 and FANCF-off2, were assigned SEQ ID NOs: 326 and 327.

The target sequence of HEK3 on-target was assigned SEQ ID NO: 328. The respective target sequences of the off-targets predicted, HEK3-off1 and HEK3-off2, were assigned SEQ ID NOs: 329 and 330.

The target sequence of RNF2 on-target was assigned SEQ ID NO: 331.

The target sequence of DNMT1 on-target was assigned SEQ ID NO: 332. The respective target sequences of the off-targets predicted, DNMT1-off1 to DNMT1-off7, were assigned SEQ ID NOs: 333 to 339.

The target sequence of RUNX1 on-target was assigned SEQ ID NO: 340. The target sequence of the off-target predicted, RUNX1-off1, was assigned SEQ ID NO: 341.

The target sequence of VEGFA on-target was assigned SEQ ID NO: 342. The respective target sequences of the off-targets predicted, VEGFA-off1 to VEGFA-off16, were assigned SEQ ID NOs: 343 to 358.

The sequence corresponding to the PBS of HEK4 on-target was assigned SEQ ID NO: 359. The sequences corresponding to the PBSs of HEK4-off1 to HEK4-off39 were assigned SEQ ID NOs: 360 to 398, respectively. The sequence corresponding to the RT of HEK4 on-target was assigned SEQ ID NO: 399. The sequences corresponding to the RTs of HEK4-off1 to HEK4-off39 were assigned SEQ ID NOs: 400 to 438, respectively (see Table 36).

The sequence corresponding to the PBS of HBB on-target was assigned SEQ ID NO: 439. The sequences corresponding to the PBSs of HBB-off1 to HBB-off5 were assigned SEQ ID NOs: 440 to 444, respectively. The sequence corresponding to the RT of HBB on-target was assigned SEQ ID NO: 445. The sequences corresponding to the RTs of HBB-off1 to HBB-off5 were assigned SEQ ID NOs: 446 to 450, respectively (see Table 37).

The sequence corresponding to the PBS of EMX1 on-target was assigned SEQ ID NO: 451. The sequences corresponding to the PBSs of EMX1-off1 and EMX1-off2 were assigned SEQ ID NOs: 452 and 453, respectively. The sequence corresponding to the RT of EMX1 on-target was assigned SEQ ID NO: 454. The sequences corresponding to the RTs of EMX1-off1 and EMX1-off2 were assigned SEQ ID NOs: 455 and 456, respectively (see Table 38).

The sequence corresponding to the PBS of FANCF on-target was assigned SEQ ID NO: 457. The sequences corresponding to the PBSs of FANCF-off1 and FANCF-off2 were assigned SEQ ID NOs: 458 and 459, respectively. The sequence corresponding to the RT of FANCF on-target was assigned SEQ ID NO: 460. The sequences corresponding to the RTs of FANCF-off1 and FANCF-off2 were assigned SEQ ID NOs: 461 and 462, respectively (see Table 39).

The sequence corresponding to the PBS of HEK3 on-target was assigned SEQ ID NO: 463. The sequences corresponding to the PBSs of HEK3-off1 and HEK3-off2 were assigned SEQ ID NOs: 464 and 465, respectively. The sequence corresponding to the RT of HEK3 on-target was assigned SEQ ID NO: 466. The sequences corresponding to the RTs of HEK3-off1 and HEK3-off2 were assigned SEQ ID NOs: 467 and 468, respectively (see Table 40).

The sequence corresponding to the PBS of RNF2 on-target was assigned SEQ ID NO: 469. The sequence corresponding to the RT of RNF2 on-target was assigned SEQ ID NO: 470 (see Table 41).

The sequence corresponding to the PBS of DNMT1 on-target was assigned SEQ ID NO: 471. The sequences corresponding to the PBSs of DNMT1-off1 to DNMT1-off7 were assigned SEQ ID NOs: 472 to 478, respectively. The sequence corresponding to the RT of DNMT1 on-target was assigned SEQ ID NO: 479. The sequences corresponding to the RTs of DNMT1-off1 to DNMT1-off7 were assigned SEQ ID NOs: 480 to 486, respectively (see Table 42).

The sequence corresponding to the PBS of RUNX1 on-target was assigned SEQ ID NO: 487. The sequence corresponding to the PBS of RUNX1-off1 was assigned SEQ ID NO: 488. The sequence corresponding to the RT of RUNX1 on-target was assigned SEQ ID NO: 489. The sequence corresponding to the RT of RUNX1-off1 was assigned SEQ ID NO: 490 (see Table 43).

The sequence corresponding to the PBS of VEGFA on-target was assigned SEQ ID NO: 491. The sequences corresponding to the PBSs of VEGFA-off1 to VEGFA-off16 were assigned SEQ ID NOs: 492 to 507, respectively. The sequence corresponding to the RT of VEGFA on-target was assigned SEQ ID NO: 508. The sequences corresponding to the RTs of VEGFA-off1 to VEGFA-off16 were assigned SEQ ID NOs: 509 to 524, respectively (see Table 44).

**Table 2-1. HEK4 TAPE-seq average read depth (1)**

| **HEK4 TAPE-seq average read depth** | | | | | | | |
|---|---|---|---|---|---|---|---|
| **HEK4** | **chr** | **position** | **Target** | **PAM** | **Mis match** | **type** | **bulge size** |
| on-target | chr20 | 31349753 | GGCACTGCGGCTGGAGGTGG | GGG | 0 | X | 0 |
| *HEK4-*off1 | chr4 | 56815181 | GGCAaTGCGGCTGGAGGcGG | AGG | 2 | X | 0 |
| *HEK4-*off2 | chr10 | 126694857 | GGCACgaCGGCTGGAGGTGG | GGG | 2 | X | 0 |
| *HEk4-*off3 | chr19 | 33382076 | GGCtCTGCGGCTGGAGGgGG | TGG | 2 | X | 0 |
| *HEK4-*off4 | chrX | 104846026 | aGCtCTGC-GCGCTGGAGGaGG | GGG | 3 | RNA | 1 |
| *HEK4-*off5 | chr13 | 107743553 | GGaACTGaGGCT-CTGAGGgtG | GGG | 4 | RNA | 1 |
| *HEK4-*off6 | chr13 | 27629405 | GGGAGTGgGGtTGGAGGTGG | GGG | 2 | X | 0 |
| *HEK4-*off7 | chr7 | 54561420 | aGgACTGCGGCTGGgGGTGG | TGG | 3 | X | 0 |
| *HEK4-*off8 | chr19 | 129506.9 | GaCACTGaGGCaGGAGGTGG | GGG | 3 | X | 0 |
| *HEK4-*off9 | chr19 | 46887169 | GaggCTGCGGCTGGgGGTGG | AGG | 4 | X | 0 |
| *HEK4-*off10 | chr20 | 60010545 | tGCACTGCGGCcGGAGGaGG | TGG | 3 | X | 0 |
| *HEK4-*off11 | chr3 | 49055357 | GGgACTGCGGCTGGAGGTGGG | AAG | 1 | DNA | 1 |
| *HEK4-*off12 | chr19 | 2474639 | GGCACTG-TGGGCTGGAGGcGG | GGG | 1 | RNA | 1 |
| *HEK4-*off13 | chr20 | 60895666 | GGCACaGCaGCTGGAGGTGc | TGG | 3 | X | 0 |
| *HEK4-*off14 | chr10 | 126708851 | aGCACT-CTGGgTGGgGGTtG | GGG | 4 | RNA | 2 |
| *HEK4-*off15 | chr3 | 31866516 | GGCACTGaGGgTtGgGGGATGG | AGG | 4 | DNA | 2 |
| *HEK4-*off16 | chr10 | 77103102 | GGCAtcaCGGCTGGAGGTGG | AGG | 3 | X | 0 |
| *HEK4-*off17 | chr3 | 51725447 | GGCtCTGtGGCTGGAGGaGG | TGG | 3 | x | 0 |
| *HEK4-*off18 | chr15 | 41044237 | GGCgCTGCGGCgGGAGGTGG | AGG | 2 | X | 0 |
| *HEK4-*off19 | chr13 | 88900987 | caCACTGCaGCTGGAGGTGG | TGG | 3 | X | 0 |
| *HEK4-*off20 | chr17 | 72251117 | GGtACTctGGCTGGAGGTGG | TGG | 3 | X | 0 |
| *HEK4-*off21 | chr17 | 75429275 | GaCACcaCGGCTGGAGaTGG | TGG | 4 | X | 0 |
| *HEK4-*off22 | chr20 | 45342993 | GGCACTGaGGgTGGAGGTGG | GGG | 2 | X | 0 |
| *HEK4-*off23 | chr2 | 241640049 | GGggCTGCGGCcGGAGGTGG | TGG | 3 | X | 0 |
| *HEK4-*off24 | chr1 | 38557167 | aGCACaaaGGCTGGAGGTGG | AGG | 4 | x | 0 |
| *HEK4-*off25 | chr1 | 204463906 | GGCgCTGCGGCTGGAGCCGG | CGG | 3 | X | 0 |
| *HEK4-*off26 | chr6 | 160517876 | GGCACTGCtGCTGGgGGTGG | TGG | 2 | X | 0 |
| *HEK4-*off27 | chr8 | 119227128 | GGCACaatGGCTGGAGGTGa | AGG | 4 | X | 0 |
| *HEK4-*off28 | chr10 | 13692619 | GGCACTGgGGCTGGgGGaGG | GGG | 3 | X | 0 |
| *HEK4-*off29 | chr16 | 28266963 | GGCtCTtCGGCTGGAGGTaG | CGG | 3 | X | 0 |
| *HEK4-*off30 | chr2 | 25348462 | GGaACTGtGGCTGGAGGTGG | CAG | 2 | X | 0 |
| *HEK4-*off31 | chr6 | 36761675 | ccCACTGgGGCTGGAGGTGG | GGG | 3 | X | 0 |
| *HEK4-*off32 | chr19 | 38616181 | GGCACTGaGaCTGGgGGTGG | GGG | 3 | X | 0 |
| *HEK4-*off33 | chr20 | 1151849 | GGCACTGtGGCTGcAGGTGG | AGG | 2 | X | 0 |
| *HEK4-*off34 | chr18 | 37194553 | GGCACTGCGGgTGGAGGcGG | GGG | 2 | X | 0 |
| *HEK4-*off35 | chr16 | 50300329 | aGCACTGtGGCTGGgGGaGG | GGG | 4 | X | 0 |
| *HEK4-*off36 | chr16 | 78413344 | GGtACaGtGGCTGGAGGTGG | AAG | 3 | X | 0- |
| *HEK4-*off37 | chr2 | 3691610 | aGgACTGCG--CGTGGAGaTGG | TGG | 3 | RNA | 2 |
| *HEK4-*off38 | chr1 | 243064859 | GcCACTGgTTGGCTGGgcGTGG | TGG | 4 | DNA | 2 |
| *HEK4-*off39 | chr5 | 172049290 | tGCACaGaGGCTGGAGGCTGa | AAG | 4 | DNA | 1 |

**Table 2-2. HEK4 TAPE-seq average read depth (2)**

| **HEK4 TAPE-seg average read depth** | | | | | | |
|---|---|---|---|---|---|---|
| **HEK293T** | | | | | | |
| **HEK4** | **PE2 (+2 G to T / Mi-seq)** | | | | **PE2(+2 G to T)** | **PE2 (+3 TAA ins)** |
| | **2 weeks (Mi-seq (1))** | **4 weeks** | **6 weeks** | **7 weeks** | | |
| on-target | 62565 | 2369 | 1060 | 1594 | 7274 | 5856 |
| HEK4-off1 | 11262 | 156 | 58 | 91 | 7383 | 1032 |
| HEM-off2 | 1953 | 32 | 19 | 35 | 5625 | 283 |
| HEK4-off3 | 46 | 0 | 0 | 0 | 270 | 10 |
| HEK4-off4 | 31 | 26 | 5 | 4 | 0 | 48 |
| HEK4-off5 | 10 | 0 | 0 | 0 | 0 | 0 |
| HEK4-off6 | 6 | 0 | 5 | 0 | 0 | 0 |
| HEK4-off7 | 3 | 0 | 0 | 0 | 0 | 4 |
| HEK4-off8 | 0 | 0 | 0 | 0 | 136 | 0 |
| HEM-off9 | 0 | 0 | 0 | 0 | 107 | 0 |
| HEK4-off10 | 0 | 0 | 0 | 0 | 0 | 5 |
| HEK4-off11 | 0 | 0 | 0 | 0 | 0 | 2 |
| HEK4-off12 | 0 | 0 | 0 | 0 | 0 | 2 |
| HEK4-off13 | 0 | 0 | 0 | 0 | 0 | 2 |
| HEK4-off14 | 0 | 0 | 17 | 0 | 0 | 0 |
| HEK4-off15 | 0 | 0 | 0 | 0 | 0 | 0 |
| *HEK4-*off16 | 0 | 0 | 0 | 0 | 0 | 0 |
| *HEK4-*off17 | 0 | 0 | 0 | 0 | 0 | 0 |
| *HEK4-*off18 | 0 | 0 | 0 | 0 | 0 | 0 |
| *HEK4-*off19 | 0 | 0 | 0 | 0 | 0 | 0. |
| *HEK4-*off20 | 0 | 0 | 0 | 0 | 0 | 0 |
| *HEK4-*off21 | 0 | 0 | 0 | 0 | 0 | 0 |
| *HEK4-*off22 | 0 | 0 | 0 | 0 | 0 | 0 |
| *HEK4-*off23 | 0 | 0 | 0 | 0 | 0 | 0 |
| *HEK4-*off24 | 0 | 0 | 0 | 0 | 0 | 0 |
| *HEK4-*off25 | 0 | 0 | 0 | 0 | 0 | 0 |
| *HEK4-*off26 | 0 | 0 | 0 | 0 | 0 | 0 |
| *HEK4-*off27 | 0 | 0 | 0 | 0 | 0 | 0 |
| *HEK4-*off28 | 0 | 0 | 0 | 0 | 0 | 0 |
| *HEK4-*off29 | 0 | 0 | 0 | 0 | 0 | 0 |
| *HEK4-*off30 | 0 | 0 | 0 | 0 | 0 | 0 |
| *HEK4-*off31 | 0 | 0 | 0 | 0 | 0 | 0 |
| *HEK4-*off32 | 0 | 0 | 0 | 0 | 0 | 0 |
| *HEK4-*off33 | 0 | 0 | 0 | 0 | 0 | 0 |
| *HEK4-*off34 | 0 | 0 | 0 | 0 | 0 | 0 |
| *HEK4-*off35 | 0 | 0 | 0 | 0 | 0 | 0 |
| *HEK4-*off36 | 0 | 0 | 0 | 0 | 0 | 0 |
| *HEK4-*off37 | 0 | 0 | 0 | 0 | 0 | 0 |
| *HEK4-*off38 | 0 | 0 | 0 | 0 | 0 | 0 |
| *HEK4-*off39 | 0 | 0 | 0 | 0 | 0 | 0 |
| *Number of Candidate* | 8 | 4 | 6 | 4 | 6 | 10 |

**Table 2-3. HEK4 TAPE-seq average read depth (3)**

| **HEK4 TAPE-seq average read depth** | | | | | | | | |
|---|---|---|---|---|---|---|---|---|
| **HEK293T** | | | | | | | | |
| **HEK4** | **PE2(+2 G to T-Hi seq)** | | | **PE4(+2 G to T / Hi-seq)** | | | **PE2-nuclease (+2 G to T / Mi-seq)** | **PEmax-nuclease + epegRHA (+2 G to T / Hi-seq)** |
| | (3) | (4) | (5) | (1) | (2) | (3) | | |
| on-target | 986 | 573 | 68.9 | 816 | 971 | 947 | 1110 | 906 |
| HEK4-off1 | 1.38 | 143 | 118 | 55 | 73 | 55 | 194 | 529 |
| HEK4-off2 | 83 | 61 | 96 | 20 | 35 | 15 | 297 | 492 |
| HEK4-off3 | 0 | 0 | 0 | 0 | 6 | 2 | 23 | 34 |
| HEK4-off4 | 0 | 0 | 1 | 0 | 0 | 2 | 351 | 258 |
| HEK4-off5 | 0 | 0 | 0 | 0 | 0 | 0 | 0 | 0 |
| HEK4-off6 | 0 | 0 | 0 | 0. | 0 | 0 | 1 | 23 |
| HEK4-off7 | 0 | 0 | 0 | 0 | 4 | 0 | 22 | 13 |
| HEK4-off8 | 0 | 0 | 0 | 0 | 0 | 0 | 3 | 0 |
| HEK4-off9 | 0 | 0 | 0 | 0 | 0 | 0 | 2 | 0- |
| HEK4-off10 | 0 | 0 | 0 | 0 | 0 | 0 | 16 | 52 |
| HEK4-off11 | 0 | 0 | 0 | 0 | 0 | 0 | 6 | 3 |
| HEK4-off12 | 0 | 0 | 0 | 0 | 0 | 0 | 0 | 0 |
| HEK4-off13 | 0 | 0 | 0 | 0 | 0 | 0 | 6 | 10 |
| HEK4-off14 | 0 | 0 | 0 | 0 | 0 | 0 | 0 | 0 |
| HEK4-off15 | 0 | 0 | 0 | 0 | 0 | 0 | 0 | 0 |
| *HEK4-*off16 | 0 | 0 | 0 | 0 | 0 | 0 | 26 | 20 |
| *HEK4-*off17 | 0 | 0 | 0 | 0 | 0 | 0 | 11 | 3 |
| *HEK4-*off18 | 0 | 0 | 0 | 0 | 0 | 0 | 6 | 9 |
| *HEK4-*off19 | 0 | 0 | 0 | 0 | 0 | 0 | 5 | 4 |
| *HEK4-*off21 | 0 | 0 | 0 | 0 | 0 | 0 | 5 | 4 |
| *HEK4-*off21 | 0 | 0 | 0 | 0 | 0 | 0 | 5 | 4 |
| *HEK4-*off22 | 0 | 0 | 0 | 0 | 0 | 0 | 4 | 3 |
| *HEK4-*off23 | 0 | 0 | 0 | 0 | 0 | 0 | 3 | 0 |
| *HEK4-*off24 | 0 | 0 | 0 | 0 | 0 | 0 | 2 | 0 |
| *HEK4-*off25 | 0 | 0 | 0 | 0 | 0 | 0 | 2 | 2 |
| *HEK4-*off26 | 0 | 0 | 0 | 0 | 0 | 0 | 2 | 0 |
| *HEK4-*off27 | 0 | 0 | 0 | 0 | 0 | 0 | 2 | 3 |
| *HEK4-*off28 | 0 | 0 | 0 | 0 | 0 | 0 | 2 | 2 |
| *HEK4-*off29 | 0 | 0 | 0 | 0 | 0 | 0 | 2 | 0 |
| *HEK4-*off30 | 0 | 0 | 0 | 0 | 0 | 0 | 1 | 0 |
| *HEK4-*off31 | 0 | 0 | 0 | 0 | o | 0 | 1 | 0 |
| *HEK4-*off32 | 0 | 0 | 0 | 0 | 0 | 0 | 1 | 2 |
| *HEK4-*off33 | 0 | 0 | 0 | 0 | 0 | 0 | 1 | 0 |
| *HEK4-*off34 | 0 | 0 | 0 | 0 | 0 | 0 | 0 | 4 |
| *HEK4-*off35 | 0 | 0 | 0 | 0 | 0 | 0 | 0 | 2 |
| *HEK4-*off36 | 0 | 0 | 0 | 0 | 0 | 0 | 0 | 2 |
| *HEK4-*off37 | 0 | 0 | 0 | 0 | 0 | 0 | 0 | 6 |
| *HEK4-*off38 | 0 | 0 | 0 | 0 | 0 | 0 | 0 | 2 |
| *HEK4-*off39 | 0 | 0 | 0 | 0 | 0 | 0 | 0 | 1 |
| *Number of Candid ate* | 3 | 3 | 4 | 3 | 5 | 5 | 30 | 27 |

**Table 2-4. HEK4 TAPE-seq average read depth (4)**

| **HEK4 TAPE-seq average read depth** | | | | | | |
|---|---|---|---|---|---|---|
| **K562** | | | | | | |
| **HEK4** | **PE2(+2 G to T/Hi-seq)** | | | **PE4(+2 G to T / Hi-seq)** | | |
| | (1) | (2) | (3) | (1) | (2) | (3) |
| on-target | 1044 | 1290 | 1394 | 1222 | 1137 | 1276 |
| HEK4-off1 | 120 | 251 | 284 | 247 | 174 | 242 |
| HEK4-off2 | 10 | 2 | 32 | 9 | 21 | 8 |
| HEK4-off3 | 10 | 12 | 4 | 2 | 15 | 0 |
| HEK4-off4 | 0 | 8 | 0 | 16 | 0 | 0 |
| HEK4-off5 | 0 | 0 | 0 | 0 | 0 | 0 |
| HEK4-off6 | 0 | 0 | 0 | 0 | 0 | 4 |
| HEK4-off7 | 0 | 0 | 0 | 0 | 0 | 0 |
| HEK4-off8 | 0 | 0 | 0 | 0 | 0 | 0 |
| HEK4-off9 | 0 | 0 | 0 | 0 | 0 | 0 |
| HEK4-off10 | 0 | 0 | 0 | 0 | 0 | 0 |
| HEK4-off11 | 0 | 0 | 0 | 0 | 0 | 0 |
| HEK4-off12 | 0 | 0 | 0 | 0 | 4 | 7 |
| HEK4-off13 | 0 | 0 | 0 | 0 | 5 | 0 |
| HEK4-offl4 | 0 | 0 | 0 | 0 | 0 | 0 |
| HEK4-off15 | 0 | 0 | 0 | 6 | 0 | 0 |
| *HEK4-*off16 | 0 | 0 | 0 | 0 | 0 | 0 |
| *HEK4-*off17 | 0 | 0 | 0 | 0 | 0 | 0 |
| *REK4-*off18 | 0 | 0 | 0 | 0 | 0 | 0 |
| *HEK4-*off19 | 0 | 0 | 0 | 0 | 0 | 0 |
| *HEK4-*off20 | 0 | 0 | 0 | 0 | 0 | 0 |
| *HEK4-*off21 | 0 | 0 | 0 | 0 | 0 | 0 |
| *HEK4-*off22 | 0 | 0 | 0 | 0 | 0 | 0 |
| *HEK4-*off23 | 0 | 0 | 0 | 0 | 0 | 0 |
| *HEK4-*off24 | 0 | 0 | 0 | 0 | 0 | 0 |
| *HEK4-*off25 | 0 | 0 | 0 | 0 | 0 | 0 |
| *HEK4-*off26 | 0 | 0 | 0 | 0 | 0 | 0 |
| *HEK4-*off27 | 0 | 0 | 0 | 0 | 0 | 0 |
| *HEK4-*off2 8 | 0 | 0 | 0 | 0 | 0 | 0 |
| *HEK4-*off29 | 0 | 0 | 0 | 0 | 0 | 0 |
| *HEK4-*off30 | 0 | 0 | 0 | 0 | 0 | 0 |
| *HEK4-*off31 | 0 | 0 | 0 | 0 | 0 | 0 |
| *HEK4-*off32 | 0 | 0 | 0 | 0 | 0 | 0 |
| *HEK4-*off33 | 0 | 0 | 0 | 0 | 0 | 0 |
| *HEK4-*off34 | 0 | 0 | 0 | 0 | 0 | 0 |
| *HEK4-*off35 | 0 | 0 | 0 | 0 | 0 | 0 |
| HEK4-off36 | 0 | 0 | 0 | 0 | 0 | 0 |
| *HEK4-*off37 | 0 | 0 | 0 | 0 | 0 | 0 |
| *HEK4-*off38 | 0 | 0 | 0 | 0 | 0 | 0 |
| *HEK4-*off39 | 0 | 0 | 0 | 0 | 0 | 0 |
| *Number of Candidate* | 4 | 5 | 4 | 6 | 6 | 5 |

**Table 2-5. HEK4 TAPE-seq average read depth (5)**

| **HEK4 TAPE-seq average read depth** | | | | | | | | |
|---|---|---|---|---|---|---|---|---|
| **HeLa** | | | | | | | **nDigenome-seq** | **GUIDE-seq Rank** |
| **HEK4** | **PE2(+2 G to T/Hi-seq)** | | | **PE4(+2 G to T & Hi-seq)** | | | | |
| | (1) | (2) | (3) | (1) | (2) | (3) | | |
| on-target | 1767 | 2847 | 865 | 1143 | 1288 | 1005 | HEK4-001 | 5 |
| HEK4-off1 | 17 | 65 | 143 | 30 | 43 | 159 | HEK4-013 | 25 |
| HEK4-off2 | 7 | 18 | 39 | 14 | 15 | 18 | HEK4-014 | 3 |
| HEK4-off3 | 0 | 6 | 4 | 0 | 0 | 6 | HEK4-009 | 2 |
| HEK4-off 4 | 0 | 4 | 0 | 0 | 0 | 0 | N/F | N/F |
| HEK4-off5 | 0 | 0 | 0 | 0 | 0 | 0 | N/F | N/F |
| HEK4-off6 | 0 | 0 | 0 | 0 | 0 | 0 | HEK4-016 | 11 |
| HEK4-off7 | 0 | 0 | 0 | 0 | 0 | 0 | HEK4-022 | 12 |
| HEK4-off8 | 0 | 0 | 0 | 0 | 0 | 0 | HEK4-034 | 36 |
| HEK4-off9 | 0 | 0 | 0 | 0 | 0 | 0 | HEK4-190 | 26 |
| HEK4-off10 | 0 | 0 | 0 | 0 | 0 | 0 | HEK4-100 | 1 |
| HEK4-off11 | 0 | 0 | 0 | 0 | 0 | 0 | N/F | 33 |
| HEK4-off12 | 0 | 0 | 0 | 0 | 0 | 0 | N/F | N/F |
| HEK4-off13 | 0 | 0 | 0 | 0 | 2 | 0 | HEK4-104 | 18 |
| HEK4-off14 | 0 | 0 | 0 | 0 | 0 | 0 | N/F | N/F |
| HEK4-off15 | 0 | 0 | 0 | 0 | 0 | 0 | N/F | N/F |
| *HEK4-*off16 | 0 | 0 | 0 | 0 | 0 | 0 | HEK4-028 | 4 |
| *HEK4-*off17 | 0 | 0 | 0 | 0 | 0 | 0 | HEK4-043 | 21 |
| *HEK4-*off18 | 0 | 0 | 0 | 0 | 0 | 0 | HEK4-011 | 6 |
| *HEK4-*off19 | 0 | 0 | 0 | 0 | 0 | 0 | HEK4-019 | 41 |
| *HEK4-*off20 | 0 | 0 | 0 | 0 | 0 | 0 | N/F | 49 |
| *HEK4-*off21 | 0 | 0 | 0 | 0 | 0 | 0 | HEK4-213 | 14 |
| *HEK4-*off22 | 0 | 0 | 0 | 0 | 0 | 0 | HEK4-012 | 13 |
| *HEK4-*off23 | 0 | 0 | 0 | 0 | 0 | 0 | HEK4-027 | 75 |
| *HEK4-*off24 | 0 | 0 | 0 | 0 | 0 | 0 | N/F | N/F |
| *HEK4-*off25 | 0 | 0 | 0 | 0 | 0 | 0 | HEK4-102 | 66 |
| *HEK4-*off26 | 0 | 0 | 0 | 0 | 0 | 0 | HEK4-015 | 9 |
| *HEK4-*off27 | 0 | 0 | 0 | 0 | 0 | 0 | HEK4-229 | 37 |
| *HEK4-*off28 | 0 | 0 | 0 | 0 | 0 | 0 | HEK4-110 | 10 |
| *HFK4-*off29 | 0 | 0 | 0 | 0 | 0 | 0 | HEK4-032 | 57 |
| *HEK4-*off30 | 0 | 0 | 0 | 0 | 0 | 0 | HEK4-004 | 50 |
| *HEK4-*off31 | 0 | 0 | 0 | 0 | 0 | 0 | HEK4-017 | 47 |
| *HEK4-*off32 | 0 | 0 | 0 | 0 | 0 | 0 | HEK4-109 | 7 |
| *HEK4-*off33 | 0 | 0 | 0 | 0 | 0 | 0 | HEK4-008 | 15 |
| *HEK4-*off34 | 0 | 0 | 0 | 0 | 0 | 0 | HEK4-010 | 23 |
| *HEK4-*off35 | 0 | 0 | 0 | 0 | 0 | 0 | HEK4-194 | 16 |
| *HEK4-*off36 | 0 | 0 | 0 | 0 | 0 | 0 | HEK4-067 | N/F |
| *HEX4-*off37 | 0 | 0 | 0 | 0 | 0 | 0 | N/F | N/F |
| *HEK4-*off38 | 0 | 0 | 0 | 0 | 0 | 0 | N/F | N/F |
| *HEK4-*off39 | 0 | 0 | 0 | 0 | 0 | 0 | N/F | N/F |
| *Number of Candidate* | 3 | 5 | 4 | 3 | 4 | 4 | 29 | 30 |

**Table 3-1. HBB TAPE-seq average read depth (1)**

| **HBB TAPE-seq average read depth** | | | | | | | |
|---|---|---|---|---|---|---|---|
| **HBB** | **chr** | **position** | **Target** | **PAM** | **Mis match** | **type** | **bulge size** |
| on-target | chr11 | 5248231 | CATGGTGCACCTGACTCCTG | AGG | 0 | X | 0 |
| *HBB-*off1 | chr11 | 5255643 | CATGGTGCAtCTGACTCCTG | AGG | 1 | X | 0 |
| *HBB-*off2 | chr6 | 159152766 | CATGGTG-- TGgCgGgCgCCTG | TAG | 4 | RNA | 2 |
| *HBB-*off3 | chr14 | 57943690 | CAcGGgGCACCTGACTCCTG | AGG | 2 | X | 0 |
| *HBB-*off4 | chr6 | 52672647 | aATGGaGCACCTGAGTCCca | AGG | 4 | X | 0 |
| *HBB-*off5 | chr19 | 39884737 | gATGGTGCAtCTGACTCCaG | AGG | 3 | X | 0 |

**Table 3-2. HBB TAPE-seq average read depth (2)**

| **HBB TAPE-seq average read depth** | | | | |
|---|---|---|---|---|
| **HBB** | **HEK2B3T** | | | **nDigenome-seq** |
| | **(+4 A to T / Hi-seq)** | | | |
| | **PE2** | **PE2-nuclease** | **PEmax-nuclease + epegRNA** | |
| on-target | 3209 | 775 | 2489 | HBB-001 |
| *HBB*-off1 | 28 | 66 | 439 | HBB-002 |
| *HBB*-off2 | 0 | 2 | 0 | N/F |
| *HBB-*off3 | 0 | 0 | 7 | HBB-003 |
| *HBB*-off4 | 0 | 0 | 3 | HBB-046 |
| *HBB*-off5 | 0 | 0 | 2 | HBB-009- |
| *Number of Candidate* | 2 | 3 | 5 | 5 |

**Table 4-1. EMX1 TAPE-seq average read depth (1)**

| **EMX1 TAPE-seq avereage read depth** | | | | | | | |
|---|---|---|---|---|---|---|---|
| **EMX1** | **chr** | **position** | **Target** | **PAM** | **Mis match** | **type** | **bulge size** |
| on-target | chr2 | 73160981 | GAGTCCGAGCAGAAGAAGAA | GGG | 0 | X | 0 |
| *EMX1-*off1 | chr5 | 46359062 | GAGTtaGAGCAGAAGAAGAA | AGG | 2 | X | 0 |
| *EMX1-*off2 | chr2 | 219845055 | GAGgCCGAGCAGAAGAAagA | CGG | 3 | X | 0 |

**Table 4-2. EMX1 TAPE-seq average read depth (2)**

| **EMX1 TAPE-seq average read depth** | | | | | |
|---|---|---|---|---|---|
| **EMX1** | **HEK293T** | | | **nDigenome-seq** | **GUIDE-seq Rank** |
| | **/ Hi-seq) (+5G to T / Hi-seq)** | | | | |
| | **PE2** | **PE2-nuclease** | **PEmax-nuclease + epegRNA** | | |
| on-target | 1243 | 1466 | 1819 | EMX1-01 | 1. |
| *EMX1*-off1 | 0 | 6 | 51 | EMX1-06 | 2 |
| *EMX1*-off2 | 0 | 0 | 7 | EMX1-17 | 4 |
| *Number of Candidate* | 1 | 2 | 3 | 3 | 3 |

**Table 5-1. FANCF TAPE-seq average read depth (1)**

| **FANCF TAPE-seq average read depth** | | | | | | | |
|---|---|---|---|---|---|---|---|
| **FANCF** | **chr** | **position** | **Target** | **PAM** | **Mis match** | **type** | **bulge size** |
| on-target | chr11 | 22647333 | GGAATCCCTTCTGCAGCACC | TGG | 0 | X | 0 |
| *FANCF-*off1 | chr18 | 8707523 | GGAAcCCCgTCTGCAGCACC | AGG | 2 | X | 0 |
| *FANCF-*off2 | chr2 | 54853309 | GGAATatCTTCTGCAGCcCC | AGG | 3 | X | 0 |

**Table 5-2. FANCF TAPE-seq average read depth (2)**

| **FANCF TAPE-seq average read depth** | | | | | |
|---|---|---|---|---|---|
| **FANCF** | **HEK293T** | | | **nDiganomeseq** | **GUIDE-seq Rank** |
| | **(+6 G to C / Hi-seq )** | | | | |
| | **PE2** | **PE2-nuclease** | **PEmax-nuclease + epegRNA** | | |
| on-target | 680 | 1128 | 3623 | FANCF-01 | 1 |
| *FANCF-*off1 | 0 | 2 | 78 | FANCF-05 | 2 |
| *FANCF-*off2 | 0 | 0 | 6 | FANCF-11 | N/F |
| *Number of* Candidate | 1 | 2 | 3 | 3 | 2 |

**Table 6-1. HEK3 TAPE-seq average read depth (1)**

| **HEK3 TAPE-sea average read depth** | | | | | | | |
|---|---|---|---|---|---|---|---|
| **HEK3** | **chr** | **position** | **Target** | **PAM** | **Mis match** | **type** | **bulge size** |
| on-target | chr9 | 110184619 | GGCCCAGACTGAGCACGTGA | TGG | 0 | X | 0 |
| *HEK3-*off1 | chr7 | 66968037 | GaCaCAGACcGgGGACGTGA | GGG | 4 | X | 0 |
| *HEK3-*off2 | chr15 | 79749913 | caCCCAGACTGAGCACGTGc | TGG | 3 | X | 0 |

**Table 6-2. HEK3 TAPE-seq average read depth (2)**

| **HEK3 TAPE-seq average read depth** | | | | | |
|---|---|---|---|---|---|
| **HEK3** | **HEK293T** | | | **nDigenome-seq** | **Rank** |
| | **(+1 CTT ins / Hi-seq)** | | | | |
| | | **PE2-nuclease** | **PEmax-nuclease + epegRNA** | | |
| on-target | 3420 | 1233 | 829 | HEK3-01 | 1 |
| *HEK3*-off1 | 0 | 2 | 38 | HEK3-11 | 3 |
| *HEK3*-off2 | 0 | 2 | 68 | HEK3-06 | 2 |
| Number of Candidate | 1 | 3 | 3 | 3 | 3 |

**Table 7-1. RNF2 TAPE-seq average read depth (1)**

| **RNF2 TAPE-seq average read depth** | | | | | | | |
|---|---|---|---|---|---|---|---|
| **RBF2** | **chr** | **position** | **Target** | **PAM** | **Mis match** | **type** | **bulge size** |
| on-target | chr1 | 185056768 | GTCATCTTAGTCATTACGTG | AGG | 0 | X | 0 |

**Table 7-2. RNF2 TAPE-seq average read depth (2)**

| **RNF2 TAPE-seq average read depth** | | | | | |
|---|---|---|---|---|---|
| **RNF2** | **HEK293T** | | | **nDigenome-seq** | **GUlDE-saq Rank** |
| | **(+6 G to A / Hi-seq)** | | | | |
| | **PE2** | **PE2-nuclease** | **PEmax-nuclease + epegRNA** | | |
| on-target | 1561 | 1473 | 3271 | RNF2-01 | 1 |
| *Number of Candidate* | 1 | 1 | 1 | 1 | 1 |

**Table 8-1. DNMT1 TAPE-seq average read depth (1)**

| **DNMT1 TAPE-seq average read depth** | | | | | | | |
|---|---|---|---|---|---|---|---|
| **DNMT1** | **chr** | **position** | **Target** | **PAM** | **Mis match** | **type** | **bulge size** |
| on-target | chr19 | 10244300 | GATTCCTGGTGCCAGAAACA | GGG | 0 | X | 0 |
| *DNMT1-*off1 | chr2 | 121450805 | GATgtCTGGTGCCAGAAACA | AGG | 2 | X | 0 |
| *DNMT1-*off2 | chr10 | 74379333 | GATTCtTGaTGCCAGAAACt | GGG | 3 | X | 0 |
| *DNMT1-*off3 | chr7 | 89940115 | tATTCCTGGaGCCAGAAgCA | GGG | 3 | X | 0 |
| *DNMT1-*off4 | chr17 | 15641875 | ccTcCGTGGTGtCAGAAACA | AGG | 4 | X | 0 |
| *DNMT1-*off5 | chr1 | 97025552 | GgaaCCTGGTGCCAGAAACA | GAG | 3 | X | 0 |
| *DNMT1-*off6 | chr6 | 30333668 | ccgTCCTaGTGCCAGAAACA | TGG | 4 | X | 0 |
| *DNMT1-*off7 | chr22 | 34301627 | GAaaCtTGGTGGCAGAAACA | GGG | 3 | x | 0 |

**Table 8-2. DNMT1 TAPE-seq average read depth (2)**

| **DNMT1 TAPE-seq average read depth** | | | | |
|---|---|---|---|---|
| **DNMT1** | **HEK293T** | | | **nDigenome seq** |
| | **(+6 G td C / H-seq)** | | | |
| | **PE2** | **PE2-nuclease** | **PEmax-nuclease + epegRNA** | |
| on-target | 1247 | 757 | 956 | DNMT1-01 |
| *DNMT1-*off1 | 0 | 8 | 467 | DNMT1-06 |
| *DNMT1-*off2 | 0 | 0 | 17 | DNMT1-12 |
| *DNMT1-*off3 | 0 | 0 | 15 | DNMT1-13 |
| *DNMT1-*off4 | 0 | 0 | 14 | DNMT1-47 |
| *DNMT1-*off5 | 0 | 0 | 7 | DNMT1-31 |
| *DNMT1-*off6 | 0 | 0- | 3 | DNMT1-53 |
| *DNMT1-*off7 | 0 | 0 | 3 | DNMT1-9 |
| *Number of Candidate* | 1 | 2 | 8 | 8 |

**Table 9-1. RUNX1 TAPE-seq average read depth (1)**

| **RUNX1 TAPE-seq average read depth** | | | | | | | |
|---|---|---|---|---|---|---|---|
| **RUNX1** | **chr** | **position** | **Target** | **PAM** | **Mis match** | **type** | **bulge size** |
| on-target | chr21 | 36421194 | GCATTTTCAGGAGGAAGCGA | TGG | 0 | X | 0 |
| *RUNX1-*off1 | chr14 | 97265074 | GCATTTTCAGaAGGAAGCaA | GGG | 2 | X | 0 |

**Table 9-2. RUNX1 TAPE-seq average read depth (2)**

| **RUNX1 TAPE-seq averahe read depth** | | | | |
|---|---|---|---|---|
| **RUNX1** | **HEK294T** | | | **nDigenome-seq** |
| | **(+6 G to C / Ht-seq)** | | | |
| | **PE2** | **PE2-nuclease** | **PEmax-nualease + epegRNA** | |
| on-target | 1142 | 1321 | 2843 | RUNX1-01 |
| *RUNX1-*off1 | 0 | 2 | 110 | RUNX1-04 |
| *Number of Candidate* | 1 | 2 | 2 | 2 |

**Table 10-1. VEGFA TAPE-seq average read depth (1)**

| **VEGFA TAPE-seq average read depth** | | | | | | | |
|---|---|---|---|---|---|---|---|
| **VEGFA** | **chr** | **position** | **Target** | **PAM** | **Mis match** | **type** | **bulge size** |
| on-target | chr6 | 43737145 | GATGTCTGCAGGCCAGATGA | GGG | 0 | X | 0 |
| *VEGFA-off1* | chr22 | 18303003 | acTCTCTGCAGGtCAGATcA | GGG | 4 | x | 0 |
| *VEGFA-off2* | chr12 | 1740975 | tAaGTCTGaAGGCCAGATGA | GGG | 3 | X | 0 |
| *VEGFA-off3* | chr6 | 1941601 | aATGTtTaCAGGCCAGATGAGG | AGG | 3 | DNA | 2 |
| *VEGFA-off4* | chr11 | 70158865 | aATGTCcGCAGcCCAGATGA | TGG | 3 | X | 0 |
| *VEGFA-off5* | chr22 | 49096508 | ccTGTCTGCAGGCCgGATGA | GAG | 3 | x | 0 |
| *VEGFA-off6* | chr16 | 89124143 | GATGTCTGCAGGaCAGATGc | AGG | 2 | X | 0 |
| *VEGFA-off7* | chr11 | 1121009430 | GATGTCTGCAGGCCAGA-Gacg | AGG | 2 | RNA | 1 |
| *VEGFA-off8* | chr10 | 33838152 | GATGTCTGgAGGtCAGATGg | AGG | 3 | X | 0 |
| *VEGFA-off9* | chr2 | 99463359 | GgTGTgTGCAGGCCAGATcA | TGG | 3 | X | 0 |
| *VEGFA-off10* | chr20 | 57576982 | GgTGTCTGCAGGGCCAGATGc | TGG | 2 | DNA | 1 |
| *VEGFA-off11* | chr22 | 48197827 | tgTGTCTGCAGGGCAGAgGg | AGG | 4 | X | 0 |
| *VEGFA-off12* | chr6 | 72236965 | GATGTaTGgAGGCCAGAaGA | GGG | 3 | X | 0 |
| *VEGFA-off13* | chr8 | 59391471 | GtcaTCTGCAGGCCAGATGg | GGG. | 4 | X | 0 |
| *VEGFA-off14* | chr20 | 60162096 | aATaTCTGCAGGtCAGAgGA | GGG | 4 | X | 0 |
| *VEGFA-off15* | chr8 | 145025699 | cccGTCTGCAGGCCAGATGGA | TGG | 3 | DNA | 1 |
| *VEGFA-off16* | chr2 | 50977382 | aATGaCTaCAGGtCAGATGA | GGG | 4 | X | 0 |

**Table 10-2. VEGFA TAPE-seq average read depth (2)**

| **VEG TAPE-seq average read depth** | | | | |
|---|---|---|---|---|
| **VEGFA** | **HEK293T** | | | **nDigenome-seq** |
| | **(+5 G to T / Hi-seq)** | | | |
| | | **PE2-nucleas** | **PEmax-nuclease + epegRNA** | |
| on-target | 7372 | 1507 | 2143 | VEGFA-001 |
| *VEGFA-off1* | 0 | 11 | 51 | VEGFA-115 |
| *VEGFA-off2* | 0 | 9 | 119 | VEGFA-003 |
| *FEGFA-off3* | 0 | 3, | 64 | VEGFA-038 |
| *VEGFA-off4* | 0 | 2 | 11 | VEGFA-014 |
| *VEGEA-off5* | 0 | 2 | 41 | VEGFA-027 |
| *VEGFA-off6* | 0 | 0 | 5 | VEGFA-007 |
| *VEGFA-off7* | 0 | 0 | 281 | VEGFA-003 |
| *VEGFA-off8* | 0 | 0 | 2 | VEGFA-010 |
| *VEGFA-off9* | 0 | 0 | 12 | VEGFA-016 |
| *VEGFA-off10* | 0 | 0 | 2 | N/F |
| *VEGFA-off11* | 0 | 0 | 4 | VEGFA-212 |
| *VEGFA-off12* | 0 | 0 | 8 | VEGFA-020 |
| *VEGFA-off13* | 0 | 0 | 6 | VEGFA-215 |
| *VEGFA-off14* | 0 | 0 | 11 | VEGFA-132 |
| *VEGFA-off15* | 0 | 0 | 5 | VEGFA-250 |
| *VEGFA-off16* | 0 | 0 | 8 | VEGFA-213 |
| *Number of Candidate* | 1 | 6 | 17 | 16 |

### Analysis Data 3. Number of reads generated for sequencing platform and each TAPE-seq analysis

Hereinbelow, the results for the number of reads generated for sequencing platforms and each TAPE-seq analysis are to be disclosed.

**Table 11. Number of reads generated for sequencing platform and each TAPE-seq analysis**

| **Target** | **Cell line** | **Ptatform** | **Raw reads** | **piggyBac sequence reads** | **piggyBac Rate** |
|---|---|---|---|---|---|
| *HEK4* (+2 G to T) (1) | HEK293T | mi-seq | 5,329,399 | 1,010,653 | 18.96% |
| *HEK4* (+2 G to T) 4 weeks | HEK293T | mi-seq | 5,313,548 | 729,706 | 13.73% |
| *HEK4* (+2 G to T) 6 weeks | HEK293T | mi-seq | 2,324,242 | 288,857 | 12.43% |
| *HEK4* (+2 G to T) 7 weeks | HEK293T | mi-seq | 4,021,702 | 497,658 | 12.37% |
| *HEK4* (+2 G to T) (2) | HEK293T | hi-seq | 53,771,178 | 2,214,486 | 4.12% |
| *HEK4* (+2 G to T) (3) | HEK293T | hi-seq | 58,008,078 | 536,807 | 0.93% |
| *HEK4* (+2 G to T) (4) | HEK293T | hi-seq | 61,412,886 | 505,551 | 0.82% |
| *HEK4* (+2 G to T) (5) | HEK293T | hi-seq | 59,390,856 | 707,287 | 1.19% |
| PE4_*HEK4* (+2 G to T) (1) | HEK293T | hi-seq | 50,511,026 | 589,869 | 1.17% |
| PE4*_HEK4* (+2 G to T) (2) | HEK293T | hi-seq | 66,349,430 | 1,418,255 | 2.14% |
| PE4_*HEK4* (+ 2 G to T) (3) | HEK293T | hi-seq | 46,533,876 | 1,121,059 | 2,41% |
| *HEK4* (+2 G to T) (1) | HeLa | hi-seq | 40,706,666 | 14,673 | 0.04% |
| *HEK4 (+2* G to T) (2) | HeLa | hi-seq | 45,042,270 | 149,463 | 0.33%. |
| *HEK4* (+2 G to T) (3) | HeLa | hi-seq | 54,578,504 | 913,167 | 1.67% |
| PE4_*HEK4* (+2 G to T). (1) | HeLa | hi-seq | 36,546,792 | 21,829 | 0.06% |
| PE4_*HEK4* (+2 G to T) (2) | HeLa | hi-seq | 55,193,802 | 1,307,526 | 2.37% |
| PE4_*HEK4* (+2 G to T) (3) | HeLa | hi-seq | 61,754,700 | 2,143,523 | 3.47% |
| *HEK4* (+2 G to T; (1) | K562 | hi-seq | 64,416,366 | 1,376,402 | 2.14% |
| *HEK4* (+2 G to T) (2) | K562 | hi-seq | 65,778,700 | 2,589,380 | 3.94% |
| *HEK4* (+2 G to T) (3) | K562 | hi-seq | 66,970,136 | 3,129,656 | 4.67% |
| PE4_*HEK4* (+2 G to T) (1) | K562 | hi-seq | 53,039,266 | 1,822,624 | 3.44% |
| PE4_*HEK4* (+2 G to T) (2) | K562 | hi-seq | 60,229,112 | 2,039,800 | 3.39% |
| PE4_*HEK4* (+2 G to T) (3) | K562 | hi-seq | 57,263,104 | 1,417,759 | 2.48%. |
| *HEK4* (+3 TAA ins) | HEK293T | mi-seq | 6,293,644 | 1,545,880 | 24.56% |
| *HBB* (+4 A to T) | HEK293T | hi-seq | 42,436,740 | 2,100,391 | 4.95% |
| *EMX1* (+5 G to T) | HEK293T | hi-seq | 42,030,260 | 1,253,176 | 2.98% |
| *FANCF* (*+*6 G to C) | HEK293T | hi-seq | 47,067,908 | 1,125,657 | 2.39% |
| *HEK3* (*+*1 CTT ins) | HEK2.93T | hi-seq | 35,666,448 | 1,203,328 | 3.37% |
| *RNF2* (+6 G to A) | HEK293T | hi-seq | 42,8301,500 | 1,831,028 | 4.29% |
| *DNMT1* (+6 G to C) | HEK293T | hi-seq | 43,145,930 | 1,363,866 | 3.16% |
| *RUNX1* (+6 G to C) | HEK293T | hi-seq | 40,567,726 | 1,339,202 | 3.30% |
| *VEGFA* (+5 G to T) | HEK293T | hi-seq | 37,111,342 | 1,128,518 | 3.04% |
| PE-nuclease_*HEK4* (+2G to T) | HEK293T | mi-seq | 4,638,550 | - | - |
| PE-nuclease_*HBB* (+4 A to T) | HEK293T | hi-seq | 81,364,360 | - | |
| PE-nuciease_*EMX1* (+5 G to T) | HEK293T | hi-seq | 72, 747,902 | - | - |
| PE-nuclease_*FANCF* (+6 G to C) | HEK293T | hi-seq | 72,668,086 | - | - |
| FE-nuclease_*HEK3* (+1 CTT ins) | HEK293T | hi-seq | 74,782,498 | - | - |
| PE-nuclease_*RNF2* (+6 G to A) | HEK293T | hi-seq | 78,125,416 | - | - |
| PE-nudease_*DNMT1* (+6 G to C) | HEK293T | hi-seq | 76,970,264 | - | - |
| PE-nuclease_*RUNX1* (+6 G to C) | HEK293T | hi-seq | 78,195,336 | - | - |
| PE-nuclease_*VEGFA* (+5 G to T) | HEK293T | hi-seq | 64,318,452 | - | - |
| PEmax-nuclease & epegRNA_*HEK4* (+2G to T) | HEK293T | hi-seq | 61,527,186 | - | - |
| PEmax-nuclease & epegRNA_*HBB* (+4 A to T) | HEK294T | hi-seq | 69,702,474 | - | - |
| PEmax-nuclease & epegRNA_*EMX1* (+5 G to T) | HEK295T | hi-seq | 77,858,022 | - | - |
| PEmax-nuclease & epegRNA_*FANCE* (+6 G to C) | HEK296T | hi-seq | 66,097,728 | - | - |
| PEmax-nuclease & epegRNA_HEK3 (+1 CTT ins) | HEK297T | hi-seq | 62,316,104 | - | - |
| PEmax-nuclease & epegRNA_*RNF2* (+6 G to A) | HEK298T | hi-seq | 66,553,332 | - | - |
| PEmax-nuclease & epegRNA_*DNMT1* (+6 G to C) | HEK299T | hi-seq | 83,252,164 | - | |
| PEmax-nuclease & epegRNA_*RUNX1* (+6 G to C) | HEK300T | hi-seq | 75,564,042 | - | - |
| PEmax-nuclease & epegRNA_*VEGFA* (+5 G to T) | HEK301T | hi-seq | 67,342,504 | - | - |

### Analysis Data 4. Targeted deep sequencing analysis of candidate off-target regions confirmed by TAPE-seq

Hereinbelow, the targeted deep sequencing analysis results for on-target and off-target regions predicted by TAPE-seq (editing rate, tagmentation rate, and the like) are to be disclosed. The associated results are expressed as Table n-x. For example, Tables 12-1 and 12-2 are associated results. In a single associated group of tables (such as Table 12), information in each table is associated with the information in another related table through the information in the first column. Regarding the entry "Target", lowercase letters and "-" indicate mismatches with the on-target and pegRNA.

**Table 12-1. Result for each pegRNA on-target region (1)**

| **Cell Type: HEK293T** | | | |
|---|---|---|---|
| | **chr** | **position** | **Target** |
| *HEK4* (+2 G to T) | chr20 | 31349753 | GGCACTGCGGCTGGAGGTGG |
| *HEK4* (+3 TAA ins) | | | |
| *HBB* (+4 A to T) | chr11 | 5248231 | CATGGTGCACCTGACTCCTG |
| *HEK3* (+1 CTT ins) | chr9 | 110184619 | GGCCCAGACTGAGCACGTGA |
| *FANCF* (+6 G to C) | chr11 | 22647333 | GGAATCCCTTCTGCAGCACC |
| *EMX1* (+5 G to T) | chr2 | 73160981 | GAGTCCGAGCAGAAGAAGAA |
| *DNMT1* (+6 G to C) | chr19 | 10244300 | GATTCCTGGTGCCAGAAACA |
| *RUNX1* (+6 G to C) | chr21 | 36421194 | GCATTTTCAGGAGGAAGCGA |
| *VEGFA* (+5 G to T) | chr6 | 43737145 | GATGTCTGCAGGCCAGATGA |
| *RNF2* (+6 G to A) | chr1 | 185056768 | GTCATCTTAGTCATTACCTG |

**Table 12-2. Result for each pegRNA on-target region (2)**

| Cell Type: HEK293T | | | | | | |
|---|---|---|---|---|---|---|
| | chr | position | Prime editing rate | | Tagmentation rate | |
| | | | PE2(+) | PE2(-) | PE2(+) | PE2(-) |
| *HEK4* (+2 G to T) | chr20 | 31349753 | 47.148% | 0.423% | 2.406% | 0.021% |
| *HEK4* (+3 TAA ins) | | | 50.887% | 0.000% | - | - |
| *HBB* (+4 A to T) | chr11 | 5248231 | 52.712% | 0.014% | 0.119% | 0.000% |
| *HEK3* (+1 CTT ins) | chr9 | 110184619 | 72.663% | 0.011% | 0.068% | 0.000% |
| *FANCF* (+6 G to C) | chr1 1 | 22647333 | 35.267% | 0.008% | 0.041% | 0.000% |
| *EMX1* (+5 G to T) | chr2 | 73160981 | 47.535% | 0.047% | 0.024% | 0.000% |
| *DNMT1* (+6 G to C) | chr19 | 10244300 | 48.071% | 0.013% | 0.036% | 0.000% |
| *RUNX1*(+6 G to C) | chr21 | 36421194 | 35.284% | 0.005% | 0.237% | 0.000% |
| *VEGFA* (+5 G to T) | chr6 | 43737145 | 88.045% | 0.034% | 4.967% | 0.000% |
| *RNF2* (+6 G to A) | chr1 | 185056768 | 64.406% | 0.007% | 0.040% | 0.000% |

**Table 13-1. Result for each pegRNA off-target region predicted by PE2 TAPE-seq (HEK293T, HeLa, and K562) (1)**

| **HEK293T, *HEK4* (+2 G to T)** | | | |
|---|---|---|---|
| | chr | position | Target |
| *HEK4*-off1 | chr4 | 56815181 | GGCAaTGCGGCTGGAGGcGG |
| *HEK4*-off2 | chr10 | 126694857 | GGCACgaCGGCTGGAGGTGG |
| *HEK4*-off3 | chr19 | 33382076 | GGCtCTGCGGCTGGAGGgGG |
| *HEK4*-off4 | chrX | 104846026 | aGCtCTGC-GCGCTGGAGGaGG |
| *HEK4*-off5 | chr13 | 107743553 | GGaACTGaGGCT-CTGAGGgtG |
| *HEK4*-off6 | chr13 | 27629405 | GGCACTGgGGtTGGAGGTGG |
| *HEK4*-off7 | chr7 | 54561420 | aGgACTGCGGCTGGgGGTGG |
| *HEK4*-off8 | chr19 | 1295069 | GaCACTGaGGCaGGAGGTGG |
| *HEK4*-off9 | chr19 | 46887169 | GaggCTGCGGCTGGgGGTGG |
| *HEK4*-off10 | chr20 | 60010545 | tGCACTGCGGCcGGAGGaGG |
| *HEK4*-off11 | chr3 | 49055357 | GGgACTGCGGCTGGAGGTGGG |
| *HEK4*-off12 | chr19 | 2474639 | GGCACTG-TGGGCTGGAGGcGG |
| *HEK4*-off13 | chr20 | 60895666 | GGCACaGCaGCTGGAGGTGc |
| *HEK4*-off14 | chr10 | 126708851 | aGCACT-CTGGgTGGgGGTtG |
| *HEK4*-off15 | chr3 | 31866516 | GGCACTGaGGgTtGgGGGATGG |
| *HEK4*-off16 | chr10 | 77103102 | GGCAtcaCGGCTGGAGGTGG |
| *HEK4*-off17 | chr3 | 51725447 | GGCtCTGtGGCTGGAGGaGG |
| *HEK4*-off18 | chr15 | 41044237 | GGCgCTGCGGCgGGAGGTGG |
| *HEK4*-off19 | chr13 | 88900987 | caCACTGCaGCTGGAGGTGG |
| *HEK4*-off20 | chr17 | 72251117 | GGtACTctGGCTGGAGGTGG |
| *HEK4*-off21 | chr17 | 75429275 | GaCACcaCGGCTGGAGaTGG |
| *HEK4*-off22 | chr20 | 45342993 | GGCACTGaGGgTGGAGGTGG |
| *HEK4*-off23 | chr2 | 241640849 | GGggCTGCGGCcGGAGGTGG |
| *HEK4*-off24 | chr1 | 38557167 | aGCACaaaGGCTGGAGGTGG |
| *HEK4*-off25 | chr1 | 204463906 | GGCgCTGCGGCTGGAGccGG |
| *HEK4*-off26 | chr6 | 160517876 | GGCACTGCtGCTGGgGGTGG |
| *HEK4*-off27 | chr8 | 119227128 | GGCACaatGGCTGGAGGTGa |
| *HEK4*-off28 | chr10 | 13692619 | GGCACTGgGGCTGGgGGaGG |
| *HEK4*-off29 | chr16 | 28266963 | GGCtCTtCGGCTGGAGGTaG |
| *HEK4*-off30 | chr2 | 25348462 | GGaACTGtGGCTGGAGGTGG |
| *HEK4*-off31 | chr6 | 36761675 | ccCACTGgGGCTGGAGGTGG |
| *HEK4*-CM33 | chr20 | 1151849 | GGCACTGtGGCTGcAGGTGG |
| | | | |

| **HeLa, *HEK4* (+2 G to T)** | | | |
|---|---|---|---|
| | chr | position | Target |
| *HEK4*-off1 | chr4 | 56815181 | GGCAaTGCGGCTGGAGGcGG |
| *HEK4*-off2 | chr10 | 126694857 | GGCACgaCGGCTGGAGGTGG |
| *HEK4*-off3 | chr19 | 33382076 | GGCtCTGCGGCTGGAGGgGG |
| *HEK4*-off4 | chrX | 104846026 | aGCtCTGC-GCGCTGGAGGaGG |
| *HEK4*-off5 | chr13 | 107743553 | GGaACTGaGGCT-CTGAGGgtG |
| *HEK4*-off6 | chr13 | 27629405 | GGCACTGgGGtTGGAGGTGG |
| *HEK4*-off7 | chr7 | 54561420 | aGgACTGCGGCTGGgGGTGG |
| *HEK4*-off8 | chr19 | 1295069 | GaCACTGaGGCaGGAGGTGG |
| *HEK4*-off9 | chr19 | 46887169 | GaggCTGCGGCTGGgGGTGG |
| *HEK4*-off10 | chr20 | 60010545 | tGCACTGCGGCcGGAGGaGG |
| *HEK4*-off11 | chr3 | 49055357 | GGgACTGCGGCTGGAGGTGGG |
| *HEK4*-off12 | chr19 | 2474639 | GGCACTG-TGGGCTGGAGGcGG |
| *HEK4*-off13 | chr20 | .60895666 | GGCACaGCaGCTGGAGGTGc |
| *HEK4*-off14 | chr10 | 126708851 | aGCACT--CTGGgTGGgGGTtG |
| *HEK4*-off15 | chr3 | 31866516 | GGCACTGaGGgTtGgGGGATGG |
| *HEK4*-off22 | chr20 | 45342993 | GGCACTGaGGgTGGAGGTGG |
| | | | |

| **K562, *HEK4* (+2 G to T)** | | | |
|---|---|---|---|
| | chr | position | Target |
| *HEK4*-off1 | chr4 | 56815181 | GGCAaTGCGGCTGGAGGcGG |
| *HEK4*-off2 | chr10 | 126694857 | GGCACgaCGGCTGGAGGTGG |
| *HEK4*-off3 | chr19 | 33382076 | GGCtCTGCGGCTGGAGGgGG |
| *HEK4*-off4 | chrX | 104846026 | aGCtCTGC-GCGCTGGAGGaGG |
| *HEK4*-off5 | chr13 | 107743553 | GGaACTGaGGCT-CTGAGGgtG |
| *HEK4*-off6 | chr13 | 27629405 | GGCACTGgGGtTGGAGGTGG |
| *HEK4*-off7 | chr7 | 54561420 | aGgACTGCGGCTGGgGGTGG |
| *HEK4*-off8 | chr19 | 1295069 | GaCACTGaGGCaGGAGGTGG |
| *HEK4*-off9 | chr19 | 46887169 | GaggCTGCGGCTGGgGGTGG |
| *HEK4*-off10 | chr20 | 60010545 | tGCACTGCGGCcGGAGGaGG |
| *HEK4*-off11 | chr3 | 49055357 | GGgACTGCGGCTGGAGGTGGG |
| *HEK4*-off12 | chr19 | 2474639 | GGCACTG-TGGGCTGGAGGcGG |
| *HEK4*-off13 | chr20 | 60895666 | GGCACaGCaGCTGGAGGTGc |
| *HEK4*-off14 | chr10 | 126708851 | aGCACT--CTGGgTGGgGGTtG |
| *HEK4*-off15 | chr3 | 31866516 | GGCACTGaGGgTtGgGGGATGG |
| *HEK4*-off22 | chr20 | 45342993 | GGCACTGaGGgTGGAGGTGG |
| | | | |

| **HEK293T, *HEK4* (+3 TAA ins)** | | | |
|---|---|---|---|
| | chr | position | Target |
| *HEK4*-off1 | chr4 | 56815181 | GGCAaTGCGGCTGGAGGcGG |
| *HEK4*-off2 | chr10 | 126694857 | GGCACgaCGGCTGGAGGTGG |
| *HEK4*-off3 | chr19 | 33382076 | GGCtCTGCGGCTGGAGGgGG |
| *HEK4*-off4 | chrX | 104846026 | aGCtCTGC-GCGCTGGAGGaGG |
| *HEK4*-off5 | chr13 | 107743553 | GGaACTGaGGCT-CTGAGGgtG |
| *HEK4*-off6 | chr13 | 27629405 | GGCACTGgGGtTGGAGGTGG |
| *HEK4*-off7 | chr7 | 54561420 | aGgACTGCGGCTGGgGGTGG |
| *HEK4*-off8 | chr19 | 1295069 | GaCACTGaGGCaGGAGGTGG |
| *HEK4*-off9 | chr19 | 46887169 | GaggCTGCGGCTGGgGGTGG |
| *HEK4*-off10 | chr20 | 60010545 | tGCACTGCGGCcGGAGGaGG |
| *HEK4*-off11 | chr3 | 49055357 | GGgACTGCGGCTGGAGGTGGG |
| *HEK4*-off12 | chr19 | 2474639 | GGCACTG-TGGGCTGGAGGcGG |
| *HEK4*-off13 | chr20 | 60895666 | GGCACaGCaGCTGGAGGTGc |
| *HEK4*-off14 | chr10 | 126708851 | aGCACT-CTGGgTGGgGGTtG |
| *HEK4*-off15 | chr3 | 31866516 | GGCACTGaGGgTtGgGGGATGG |
| *HEK4*-off22 | chr20 | 45342993 | GGCACTGaGGgTGGAGGTGG |
| | | | |

| **HeLa, *HEK4* (+ 3 TAA ins)** | | | |
|---|---|---|---|
| | chr | position | Target |
| *HEK4*-off1 | chr4 | 56815181 | GGCAaTGCGGCTGGAGGcGG |
| *HEK4*-off2 | chr10 | 126694857 | GGCACgaCGGCTGGAGGTGG |
| *HEK4*-off3 | chr19 | 33382076 | GGCtCTGCGGCTGGAGGgGG |
| *HEK4*-off4 | chrX | 104846026 | aGCtCTGC-GCGCTGGAGGaGG |
| *HEK4*-off5 | chr13 | 107743553 | GGaACTGaGGCT-CTGAGGgtG |
| *HEK4*-off6 | chr13 | 27629405 | GGCACTGgGGtTGGAGGTGG |
| *HEK4*-off7 | chr7 | 54561420 | aGgACTGCGGCTGGgGGTGG |
| *HEK4*-off8 | chr19 | 1295069 | GaCACTGaGGCaGGAGGTGG |
| *HEK4*-off9 | chr19 | 46887169 | GaggCTGCGGCTGGgGGTGG |
| *HEK4-*off10 | chr20 | 60010545 | tGCACTGCGGCcGGAGGaGG |
| *HEK4*-off11 | chr3 | 49055357 | GGgACTGCGGCTGGAGGTGGG |
| *HEK4-*off12 | chr19 | 2474639 | GGCACTG-TGGGCTGGAGGcGG |
| *HEK4*-off13 | chr20 | 60895666 | GGCACaGCaGCTGGAGGTGc |
| *HEK4*-off14 | chr10 | 126708851 | aGCACT--CTGGgTGGgGGTtG |
| | chr3 | 31866516 | GGCACTGaGGgTtGgGGGATGG |
| *HEK4*-off22 | chr20 | 45342993 | GGCACTGaGGgTGGAGGTGG |
| | | | |

| **K562, *HEK4* (+3 TAA ins)** | | | |
|---|---|---|---|
| | chr | position | Target |
| *HEK4*-off1 | chr4 | 56815181 | GGCAaTGCGGCTGGAGGcGG |
| *HEK4*-off2 | chr10 | 126694857 | GGCACgaCGGCTGGAGGTGG |
| *HEK4*-off3 | chr19 | 33382076 | GGCtCTGCGGCTGGAGGgGG |
| *HEK4*-off4 | chrX | 104846026 | aGCtCTGC-GCGCTGGAGGaGG |
| *HEK4*-off5 | chr13 | 107743553 | GGaACTGaGGCT-CTGAGGgtG |
| *HEK4*-off6 | chr13 | 27629405 | GGCACTGgGGtTGGAGGTGG |
| *HEK4*-off7 | chr7 | 54561420 | aGgACTGCGGCTGGgGGTGG |
| *HEK4*-off8 | chr19 | 1295069 | GaCACTGaGGCaGGAGGTGG |
| *HEK4*-off9 | chr19 | 46887169 | GaggCTGCGGCTGGgGGTGG |
| *HEK4*-off10 | chr20 | 60010545 | tGCACTGCGGCcGGAGGaGG |
| *HEK4*-off11 | chr3 | 49055357 | GGgACTGCGGCTGGAGGTGGG |
| *HEK4*-off12 | chr19 | 2474639 | GGCACTG-TGGGCTGGAGGcGG |
| *HEK4*-off13 | chr20 | 60895666 | GGCACaGCaGCTGGAGGTGc |
| *HEK4*-off14 | chr10 | 126708851 | aGCACT-CTGGgTGGgGGTtG |
| *HEK4*-off15 | chr3 | 31866516 | GGCACTGaGGgTtGgGGGATGG |
| *HEK4*-off22 | chr20 | 45342993 | GGCACTGaGGgTGGAGGTGG |
| | | | |

| **HEK293T, *HBB* (+4 A to T)** | | | |
|---|---|---|---|
| | chr | position | Target |
| *HBB*-off1 | chr11 | 5255643 | CATGGTGCAtCTGACTCCTG |
| *HBB*-off2 | chr6 | 159152766 | CATGGTG-TGgCgGgCgCCTG |
| *HBB*-off3 | chr14 | 57943690 | CAcGGgGCACCTGACTCCTG |
| *HBB*-off4 | chr6 | 52672647 | aATGGaGCACCTGACTCCca |
| *HBB*-off5 | chr19 | 39884737 | gATGGTGCAtCTGACTCCaG |
| | | | |

| **HE4293T, *EMX1* (+5 G to T)** | | | |
|---|---|---|---|
| | chr | position | Target |
| *EMX*-off1 | chr5 | 45359062 | GAGTtaGAGCAGAAGAAGAA |
| *EMX*-off2 | chr2 | 219845055 | GAGgCCGAGCAGAAGAAagA |
| | | | |

| **HEK29ET, *FANCF* (+6 G to C)** | | | |
|---|---|---|---|
| | chr | position | Target |
| *FANC*-off1 | chr18 | 8707523 | GGAAcCCCgTCTGCAGCACC |
| *FACF*-off2 | chr2 | 54853309 | GGAATatCTTCTGCAGCcCC |
| | | | |

| **HEK293T, *HEK3* (+1 CTT ins)** | | | |
|---|---|---|---|
| | chr | position | Target |
| *HEK3*-off1 | chr7 | 66968037 | GaCaCAGACcGgGCACGTGA |
| *HEK3*-off2 | chr15 | 79749913 | caCCCAGACTGAGCACGTGc |
| | | | |

| **HEK293T, *DNMT1* (+6 G to C)** | | | |
|---|---|---|---|
| | chr | position | Target |
| *DNMT1*-off1 | chr2 | 121450805 | GATgtCTGGTGCCAGAAACA |
| *DNMT1*-off2 | chr10 | 74379333 | GATTCtTGaTGCCAGAAACt |
| *DNMT1*-off3 | chr7 | 89940115 | tATTCCTGGaGCCAGAAgCA |
| *DNMT1*-off4 | chr17 | 15641875 | ccTcCCTGGTGtCAGAAACA |
| *DNMT1*-off5 | chr1 | 97025552 | GgaaCCTGGTGCCAGAAACA |
| *DNMT1*-off6 | chr6 | 30333668 | ccgTCCTaGTGGCAGAAACA |
| *DNMT1*-off7 | chr22 | 34301627 | GAaaCtTGGTGCCAGAAACA |
| | | | |

| **HEK293T, *RUNX1* (+6 G to C)** | | | |
|---|---|---|---|
| | chr | position | Target |
| *RUN1*-off1 | chr14 | 97265074 | GCATTTTCAGaAGGAAGCaA |
| | | | |

| **HEK293T, *VEGFA* (+5 G to T)** | | | |
|---|---|---|---|
| | chr | position | Target |
| *VEGFA*-off1 | chr22 | 18303003 | acTGTCTGCAGGtCAGATcA |
| *VEGFA*-off2 | chr12 | 1740975 | tAaGTCTGaAGGCCAGATGA |
| *VEGFA*-off3 | chr6 | 1941601 | aATGTtTaCAGGCCAGATGAGG |
| *VEGFA*-off4 | chr11 | 70158865 | aATGTCcGCAGcCCAGATGA |
| *VEGFA*-off5 | chr22 | 49096508 | ccTGTCTGCAGGCCgGATGA |
| *VEGFA*-off6 | chr16 | 89124143 | GATGTCTGCAGGaCAGATGc |
| *VEGFA*-off7 | chr11 | 121009430 | GATGTCTGCAGGCCAGA-Gacg |
| *VEGFA*-off8 | chr10 | 33838152 | GATGTCTGgAGGtCAGATGg |
| *VEGFA*-off8 | chr2 | 99463359 | GgTGTgTGCAGGCCAGATcA |
| *VEGFA*-off10 | chr20 | 57576982 | GgTGTCTGCAGGGCCAGATGc |
| *VEGFA*-off11 | chr22 | 48197827 | tgTGTCTGCAGGCCAGAgGg |
| *VEGFA*-off12 | chr6 | 72236965 | GATGTaTGgAGGCCAGAaGA |
| *VEGFA*-off13 | chr8 | 59391471 | GtcaTCTGCAGGCCAGATGg |
| *VEGFA*-off14 | chr20 | 60162096 | aATaTCTGCAGGtCAGAgGA |
| *VEGFA*-off15 | chr8 | 145025899 | cccGTCTGCAGGCCAGATGGA |
| *VEGFA*-off16 | chr2 | 50977382 | aATGaCTaCAGGtCAGATGA |

**Table 13-2. Result for each pegRNA off-target region predicted by PE2 TAPE-seq (HEK293T, HeLa, and K562) (2)**

| **HEK293T, *HEK4* (±2 G to T)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(+) |
| *HEK4*-off1 | 0.042% | 0.000% | 0.280% | 0.210% | 0.331% | 0.080% |
| *HEK4*-off2 | 43.011% | 0.000% | 11.314% | 16.337% | 45.094% | 0.069% |
| *HEK4*-off3 | 0.000% | 0.000% | 0.279% | 0.301% | 0.092% | 0.061% |
| *HEK4*-off4 | 0.095% | 0.000% | 0.406% | 0.326% | 0.165% | 0.208% |
| *HEK4*-off5 | 0.000% | 0.000% | 0.338% | 0.453% | 0.091% | 0.075% |
| *HEK4*-off6 | 0.231% | 0.016% | 0.417% | 0.199% | 0.095% | 0.090% |
| *HEK4*-off7 | 0.004% | 0.000% | 0.955% | 0.767% | 0.161% | 0.173% |
| *HEK4*-off8 | 0.000% | 0.000% | 6.591% | 5.527% | 0.111% | 0.097% |
| *HEK4*-off9 | 0.000% | 0.000% | 0.275% | 0.374% | 0.127% | 0.139% |
| *HEK4*-off10 | 0.005% | 0.000% | 0.191% | 0.178% | 0.033% | 0.036% |
| *HEK4*-off1 1 | 0.000% | 0.000% | 0.243% | 0.260% | 0.054% | 0.038% |
| *HEK4*-off12 | 0.489% | 0.000% | 0.871% | 0.330% | 0.096% | 0,061% |
| *HEK4*-off13 | 0.000% | 0.000% | 0.269% | 0.269% | 0.058% | 0.057% |
| *HEK4*-off14 | 0.000% | 0.000% | 0.258% | 0.302% | 0.118% | 0.136% |
| *HEK4*-off15 | 0.000% | 0.000% | 0.558% | 0.326% | 0.097% | 0.080% |
| *HEK4*-off16 | 0.000% | 0.000% | 0.128% | 0.146% | 0.033% | 0.054% |
| *HEK4*-off17 | 0.000% | 0.000% | 0.141% | 0.079% | 0.033% | 0.039% |
| *HEK4*-off18 | 0.000% | 0.000% | 0.146% | 0.141% | 0.063% | 0.053% |
| *HEK4*-off19 | 0.000% | 0.000% | 0.645% | 1.059% | 43.418% | 15.860% |
| *HEK4*-off20 | 0.000% | 0.000% | 0.009% | 0.000% | 0.000% | 0.000% |
| *HEK4*-off21 | 0.000% | 0.000% | 0.182% | 0.123% | 0.066% | 0.060% |
| *HEK4*-off22 | 0.087% | 0.011% | 0.565% | 0.515% | 0.088% | 0,076% |
| *HEK4*-off23 | 0.000% | 0.000% | 0.149% | 0.190% | 0.061% | 0.032% |
| *HEK4*-off24 | 0.000% | 0.000% | 0.181% | 0.183% | 0.056% | 0.053% |
| *HEK4*-off25 | 0.000% | 0.000% | 4.862% | 4.554% | 0.003% | 0.000% |
| *HEK4*-off26 | 0.000% | 0.000% | 0.186% | 0.190% | 0.088% | 0.069% |
| *HEK4*-off27 | 0.000% | 0.000% | 0.172% | 0.131% | 0.048% | 0.047% |
| *HEK4*-off28 | 0.004% | 0.000% | 0.233% | 0.238% | 0.109% | 0.155% |
| *HEK4*-off29 | 0.000% | 0.000% | 0.537% | 0.473% | 0.038% | 0.023% |
| *HEK4*-off30 | 0.000% | 0.000% | 0.133% | 0.156% | 0.012% | 0.015% |
| *HEK4*-off31 | 0.000% | 0.000% | 0.144% | 0.139% | 0.090% | 0.079% |
| *HEK4*-off33 | 0.000% | 0.000% | 0.146% | 0.108% | 0.020% | 0.032% |
| | | | | | | |

| **HeLa, *HEK4* (+ 2 G to T)** | | | | | | |
|---|---|---|---|---|---|---|
| | Major editing rate | | Substitution rate | | Deletion rate | |
| | PEG(+) | PE2(-) | PE2(+) | PE2(-) | PE2(+) | PE2(-) |
| *HEK4*-off1 | 0.000% | 0.000% | 0.154% | 0.187% | 0.137% | 0.044% |
| *HEK4*-off2 | 0.000% | 0.000% | 74.743% | 56.124% | 0.097% | 0.289% |
| *HEK4*-off3 | 0.000% | 0.000% | 0.356% | 0.431% | 0.055% | 0.066% |
| *HEK4*-off4 | 0.013% | 0.000% | 0.260% | 0.335% | 0.055% | 0.144% |
| *HEK4*-off5 | 0.000% | 0.000% | 0.197% | 0.303% | 0.097% | 0.072% |
| *HEK4*-off6 | 0.000% | 0.000% | 0.186% | 0.386% | 0.056% | 0.042% |
| *HEK4*-off7 | 0.000% | 0.000% | 0.222% | 30.251% | 0.114% | 0.059% |
| *HEK4*-off8 | 0.000% | 0.000% | 7.541% | 6.6.31% | 0.100% | 0.063% |
| *HEK4*-off9 | 0.000% | 0.000% | 8.707% | 0.409% | 0.262% | 0.117% |
| *HEK4*-off10 | 0.000% | 0.000% | 0.187% | 0.128% | 0.043% | 0.027% |
| *HEK4*-off11 | 0.000% | 0.000% | 0.108% | 0.206% | 0.026% | 0.035% |
| *HEK4*-off12 | 0.000% | 0.000% | 0.470% | 0.208% | 0.086% | 0.102% |
| *HEK4*-off13 | 0.000% | 0.000% | 0.404% | 0.240% | 0.145% | 0.048% |
| *HEK4*-off14 | 0.000% | 0.000% | 0.164% | 0.140% | 0.080% | 0.201% |
| *HEK4*-off15 | 0.000% | 0.000% | 0.606% | 0.584% | 0.052% | 0.073% |
| *HEK4*-off22 | 0.000% | 0.000% | 0.343% | 0.874% | 0.078% | 0.075% |
| | | | | | | |

| **K562, *HEK4* (+2 G to T)** | | | | | | |
|---|---|---|---|---|---|---|
| | Major editing rate | | Substitution rate | | Deletion rate | |
| | PE2(+) | PE2(-) | PE2(+) | PE2(-) | PE2(+) | PE2(-) |
| *HEK4*-off1 | 0.006% | 0.000% | 0;135% | 0.195% | 0.116% | 0.053% |
| *HEK4*-off2 | 0.000% | 0.000% | 1.687% | 42.668% | 67.944% | 2.283% |
| *HEK4*-off3 | 0.000% | 0.000% | 0.396% | 0.409% | 0.077% | 0.083% |
| *HEK4*-off4 | 0.000% | 0.000% | 0.250% | 0.309% | 0.076% | 0.115% |
| *HEK4*-off5 | 0.000% | 0.000% | 0.200% | 0.312% | 0.062% | 0.064% |
| *HEK4-*off6 | 0.000% | 0.000% | 0.176% | 0.421% | 0.057% | 0.051% |
| *HEK4*-off7 | 0.004% | 0.000% | 0.350% | 30.672% | 0.077% | 0.098% |
| *HEK4*-off8 | 0.000% | 0.000% | 8.953% | 6.059% | 0.084% | 0.072% |
| *HEK4*-off9 | 0.000% | 0.000% | 1.646% | 1.035% | 0.161% | 0.374% |
| *HEK4*-off10 | 0.000% | 0.000% | 0.141% | 0.184% | 0.013% | 0.020% |
| *HEK4*-off11 | 0.000% | 0.000% | 0.226% | 0.238% | 0.036% | 0.031% |
| *HEK4*-off12 | 0.000% | 0.000% | 0.403% | 0.268% | 0.077% | 0.141% |
| *HEK4*-off13 | 0.000% | 0.000% | 0.357% | 0.243% | 0.109% | 0.063% |
| *HEK4*-off14 | *-* | *-* | *-* | - | *-* | - |
| *HEK4*-off15 | 0.000% | 0.000% | 0.574% | 0.599% | 0.093% | 0.124% |
| *HEK4*-off22 | 0.000% | 0.000% | 0.479% | 1.086% | 0.080% | 0.088% |
| | | | | | | |

| **HEK293T, *HEK4* (+3 TAA ins)** | | | | | | |
|---|---|---|---|---|---|---|
| | Major edit rate | | Substitution rate | | Deletion rate | |
| | PE2(+) | PE2(-) | PR2(+) | PE2(-) | PE2(+) | PE2(-) |
| *HEK4*-off1 | 0.009% | 0.000% | 0.286% | 0.230% | 0.672% | 0.080% |
| *HEK4*-off2 | 94.734% | 0.000% | 0.056% | 16.337% | 0.112% | 0.063% |
| *HEK4*-off3 | 0.213% | 0.000% | 0.300% | 0.301% | 0.084% | 0.061% |
| *HEK4*-off4 | 0.011% | 0.000% | 0.298% | 0.377% | 0.168% | 0.208% |
| *HEK4*-off5 | 0.000% | 0,000% | 0,220% | 0.453% | 0.074% | 0.075% |
| *HEK4*-off6 | 0.006% | 0.000% | 0.224% | 0.221% | 0.084% | 0.090% |
| *HEK4*-off7 | 0.000% | 0.000% | 0.687% | 0.783% | 0.159% | 0.165% |
| *HEK4*-off8 | 0.000% | 0.000% | 4.545% | 5.553% | 0.087% | 0.097% |
| *HEK4*-off9 | 0.000% | 0.000% | 0.172% | 0.410% | 0.124% | 0.139% |
| *HEK4*-off10 | 0.000% | 0.000% | 0,196% | 0.197% | 0.049% | 0.036% |
| *HEK4*-off11 | 0.000% | 0.000% | 0.303% | 0.284% | 0.049% | 0.038% |
| *HEK4*-off12 | 0.108% | 0.000% | 0.228% | 0.366% | 0.074% | 0.061% |
| *HEK4*-off13 | 0.000% | 0.000% | 0,267% | 0.291% | 0.059% | 0.057% |
| *HEK4-*off14 | 0.000% | 0.000% | 0.181% | 0.301% | 0.133% | 0.118% |
| *HEK4*-off15 | 0.000% | 0.000% | 0.526% | 0.326% | 0.092% | 0.080% |
| *HEK4*-off22 | 0.000% | 0.000% | 0.442% | 0.515% | 0.095% | 0.076% |

| **HeLa, *HEK4* (+3 TAA ins)** | | | | | | |
|---|---|---|---|---|---|---|
| | Majo editing rate | | Substitution rate | | Deletion rate | |
| | PE2(+) | PE2(-) | PE2(+) | PE2(-) | PE2(+) | PE2(-) |
| *HEK4*-off1 | 0.000% | 0.000% | 0.259% | 0.201% | 0.321% | 0.044% |
| *HEK4*-off2 | 99.364% | 0.319% | 0.154% | 56.606% | 0.000% | 0.289% |
| *HEK4*-off3 | 0.006% | 0.000% | 0.427% | 0.458% | 0.064% | 0.066% |
| *HEK4*-off4 | 0.000% | 0.000% | 0.253% | 0.380% | 0.061% | 0.144% |
| *HEK4*-off5 | 0.000% | 0.000% | 0.131% | 1.129% | 0.043% | 0.072% |
| *HEK4*-off6 | 0.000% | 0.000% | 0.126% | 0.447% | 0.067% | 0.042% |
| *HEK4*-off7 | 0.000% | 0.000% | 0.429% | 30.869% | 0.105% | 0.059% |
| *HEK4*-off8 | 0.000% | 0.000% | 7.093% | 6.750% | 0.111% | 0.063% |
| *HEK4*-off9 | 0.000% | 0.000% | 3.626% | 0.444% | 0.258% | 0.117% |
| *HEK4*-off10 | 0.000% | 0.000% | 0.225% | 0.139% | 0.043% | 0.027% |
| *HEK4*-off11 | 0.000% | 0.000% | 0.227% | 0.211% | 0.035% | 0.035% |
| *HEK4*-off12 | 0.000% | 0.000% | 0.414% | 0.208% | 0.095% | 0.102% |
| *HEK4*-off13 | 0.000% | 0.000% | 0.395% | 0.281% | 0.108% | 0.048% |
| *HEK4*-off14 | 0.000% | 0.000% | 0.342% | 0.140% | 0.134% | 0.201% |
| *HEK4*-off15 | 0.000% | 0.000% | 0.496% | 0.584% | 0.079% | 0.073% |
| *HEK4*-off22 | 0.000% | 0.000% | 0.384% | 40.587% | 0.097% | 0.286% |
| | | | | | | |

| **K562, *HEK4* (+3 TAA ins)** | | | | | | |
|---|---|---|---|---|---|---|
| | Major editing rate | | Substitution rate | | Deletion rate | |
| | PE2(+) | | PE2(+) | PE2(-) | PE2(+) | PE2(-) |
| *HEK4*-off1 | 0.000% | 0.000% | 0.235% | 0.216% | 0.373% | 0.053% |
| *HEK4*-off2 | 0.000% | 0.000% | 11.048% | 42.668% | 82.296% | 2.283% |
| *HEK4*-off3 | 0.003% | 0.000% | 0.470% | 0.437% | 0.080% | 0.083% |
| *HEK4*-off4 | 0.000% | 0.000% | 0.244% | 0.343% | 0.052% | 0.115% |
| *HEK4*-off5 | 0.000% | 0.000% | 0.286% | 1.032% | 0.073% | 0.064% |
| *HEK4*-off6 | 0.000% | 0.000% | 0.275% | 0.475% | 0.083% | 0.051% |
| *HEK4*-off7 | 0.000% | 0.000% | 0.513% | 30.877% | 0.120% | 0.098% |
| *HEK4*-off8 | 0.000% | 0.000% | 9.122% | 6.144% | 0.090% | 0.072% |
| *HEK4*-off9 | 0.000% | 0.000% | 5.266% | 1.088% | 0.192% | 0.374% |
| *HEK4*-off10 | 0.000% | 0.000% | 0.176% | 0.191% | 0.024% | 0.020% |
| *HEK4*-off11 | 0.000% | 0.000% | 0.273% | 0.250% | 0.013% | 0.031% |
| *HEK4*-off12 | 0.000% | 0.000% | 0.467% | 0.276% | 0.105% | 0.141% |
| *HEK4*-off13 | 0.000% | 0.000% | 0.386% | 0.274% | 0.142% | 0.063% |
| *HEK4*-off14 | - | - | - | - | - | - |
| *HEK4*-off15 | 0.000% | 0.000% | 0.367% | 0.619% | 0.046% | 0.124% |
| *HEK4*-off22 | 0.000% | 0.000% | 0.518% | 1.124% | 0.125% | 0.088% |
| | | | | | | |

| **HEK293T, *HBB* (+4 A to T)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(-) |
| *HBB*-off1 | 3.259% | 0.000% | 5.124% | 0.243% . | 0.050% | 0.039% |
| *HBB*-off2 | 0.000% | 0.000% | 0.636% | 0.770% | 0.085% | 0.069% |
| *HBB*-off3 | 0.002% | 0.000% | 0.228% | 0.231% | 0.026% | 0.028% |
| *HBB*-off4 | 0.000% | 0.000% | 1.158% | 0.789% | 0.178% | 0.146% |
| *HBB*-off5 | 0.000% | 0.000% | 0.236% | 0.210% | 0.022% | 0.025% |
| | | | | | | |

| **HEK293T, *EMX1* (+5 G to T)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(-) |
| *EMX1*-off1 | 0.000% | 0.000% | 0.381% | 0.383% | 0.139% | 0.113% |
| *EMX1*-off2 | 0.000% | 0,000% | 0.544% | 0.536% | 0.075% | 0.100% |
| | | | | | | |

| **HEK293T, *FANCF* (+6 G to C)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(-) |
| *FANCF*-off1 | 0.000% | 0.000% | 0.303% | 0.296% | 0.054% | 0.055% |
| *FANCF*-off2 | 0.000% | 0.000% | 1.297% | 1.170% | 0.008% | 0.046% |
| | | | | | | |

| **HEK293T, *HEK3* (+1 CTT ins)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(-) |
| *HEK3*-off1 | 0.000% | 0.000% | 0.579% | 0.626% | 0.034% | 0.030% |
| *HEK3*-off2 | 0.000% | 0.000% | 0.184% | 0.182% | 0.029% | 0.040% |
| | | | | | | |

| **HEK293T, *DNMT1* (+6 G to C)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(-) |
| *DNMT1*-off1 | 0.002% | 0.000% | 0.240% | 0.229% | 0.037% | 0.044% |
| *DNMT1-off2* | 0.000% | 0.000% | 0.441% | 0.428% | 0.016% | 0.040% |
| *DNMT1*-off3 | 0.000% | 0.000% | 0.253% | 0.279% | 0.033% | 0.043% |
| *DNMT1*-off4 | 0.000% | 0.000% | 5.374% | 6.442% | 0.080% | 0.073% |
| *DNMT1*-off5 | 0.000% | 0.000% | 0.311% | 0.320% | 0.052% | 0.060% |
| *DNMT1*-off6 | 0.000% | 0.000% | 0.189% | 0.206% | 0.026% | 0.034% |
| *DNMT1*-off7 | 0.000% | 0.000% | 0.549% | 0.505% | 0.025% | 0.056% |
| | | | | | | |

| **HEK293T, *RUNX1* (+6 G to C)** | | | | | | |
|---|---|---|---|---|---|---|
| | Major edit rate | | Substitution rate | | Deletion rate | |
| | PE2(+) | PE2(-) | PE2(+) | PE2(-) | PE2(+) | PE2(-) |
| *RUNX1*-off1 | 0.000% | 0.000% | 0.771% | 0.724% | 0.057% | 0.063% |
| | | | | | | |

| **HEK293T, *VEGFA* (+5 G to T)** | | | | | | |
|---|---|---|---|---|---|---|
| | Major edit rate | | | | Deletion rate | |
| | PE2(+) | PE2(-) | | PE2(-) | PE2(+) | PE2(-) |
| *VEGFA*-off1 | 0.000% | 0.000% | 0.296% | 0.313% | 0.037% | 0.041% |
| *VEGFA*₋off2 | 0.000% | 0.000% | 0.297% | 0.238% | 0.035% | 0.044% |
| *VEGFA*-off3 | 0.000% | 0.000% | 0.819% | 0,849% | 0.056% | 0.046% |
| *VEGFA*-off4 | 0.000% | 0.000% | 0.285% | 0.272% | 0.078% | 0.053% |
| *VEGFA*-off5 | 0.000% | 0.000% | 0.261% | 0.249% | 0.023% | 0.020% |
| *VEGFA*-off6 | - | - | - | - | - | - |
| *VEGFA*-off7 | 0.000% | 0.000% | 0.140% | 0.150% | 0.003% | 0.005% |
| *VEGFA*-off8 | 0.000% | 0.000% | 7.345% | 7.449% | 0.017% | 0.026% |
| *VEGFA*-off9 | 0.000% | 0.000% | 0.382% | 0.350% | 0.028% | 0.026%, |
| *VEGFA*-off10 | 0.000% | 0.000% | 4.218% | 4.558% | 0.032% | 0.013% |
| *VEGFA*-off11 | 0.000% | 0.000% | 0.501% | 0.603% | 0.042% | 0.047% |
| *VEGFA*-off12 | 0.000% | 0.000%, | 0.276% | 0.294% | 0.058% | 0.055% |
| *VEGFA-*off13 | 0.000% | 0.000% | 8.692% | 8.395% | 0.032% | 0.036% |
| *VEGFA*-off14 | 0.000% | 0.000% | 0.257% | 0.235% | 0.032% | 0.037% |
| *VEGFA*-off15 | 0.000% | 0.000% | 0.160% | 0.150% | 0.014% | 0.018% |
| *VEGFA*-off16 | 0.173% | 0.100% | 5.067% | 5.289% | 0.124% | 0.111% |

**Table 13-3. Result for each pegRNA off-target region predicted by PE2 TAPE-seq (HEK293T, HeLa, and K562) (3)**

| **HEK293T, *HEK4* (+2 G to T)** | | | | | |
|---|---|---|---|---|---|
| | Insertion rate | | Mutation rate | | Validation |
| | PE2(+) | PE2(-) | PE2(+) | PE2(-) | |
| *HEK4-*off1 | 0.037% | 0.000% | 0.698% | 0.289% | Validated |
| *HEK4-*off2 | 27.392% | 0.000% | 83.801% | 16.405% | Validated |
| *HEK4-*off3 | 0.000% | 0.000% | 0.371% | 0.362% | False |
| *HEK4-*off4 | 0.028% | 0.000% | 0.600% | 0.533% | Validated |
| *HEK4-*off5 | 0.002% | 0.000% | 0.430% | 0.528% | False |
| *HEK4-*off6 | 0.006% | 0.002% | 0.518% | 0.291% | Validated |
| *HEK4-*off7 | 0.018% | 0.018% | 1.134% | 0.958% | Validated |
| *HEK4-*off8 | 0.000% | 0.000% | 6.702% | 5.624% | False |
| *HEK4-*off9 | 0.000% | 0.000% | 0.402% | 0.513% | False |
| *HEK4-*off10 | 0.000% | 0.000% | 0.224% | 0.214% | Validated |
| *HEK4-*off11 | 0.000% | 0.000% | 0.297% | 0.298% | False |
| *HEK4-*off12 | 0.007% | 0.000% | 0.974% | 0.391% | Validated |
| *HEK4-*off13 | 0.000% | 0.000% | 0.327% | 0.327% | False |
| *HEK4-*off14 | 0.000% | 0.001 % | 0.376% | 0.439% | False |
| *HEK4-*off15 | 0.000% | 0.000% | 0.655% | 0.407% | False |
| *HEK4-*off16 | 0.000% | 0:000% | 0.161% | 0.200% | False |
| *HEK4-*off17 | 0.000% | 0.000% | 0.173% | 0.119% | False |
| *HEK4-*off18 | 0.000% | 0.000% | 0.209% | 0.194% | False |
| *HEK4-*off19 | 5.509% | 7.651% | 49.571% | 24.569% | False |
| *HEK*4-off20 | 0.000% | 0.000% | 0.009% | 0.000% | False |
| *HEK4*-off21 | 0.000% | 0.000% | 0.247% | 0.183% | False |
| *HEK4*-off22 | 0.000% | 0.000% | 0.653% | 0.591% | Validated |
| *HEK4*-off23 | 0.000% | 0.000% | 0.210% | 0.222% | False |
| *HEK4*-off24 | 0.000% | 0.000% | 0.237% | 0.236% | False |
| *HEK4*-off25 | 0.019% | 0.023% | 4.884% | 4.578% | False |
| *HEK4*-off26 | 0.005% | 0.000% | 0.279% | 0.259% | False |
| *HEK4*-off27 | 0.000% | 0.000% | 0.220% | 0.178% | False |
| *HEK4*-off28 | 0.000% | 0.000% | 0.342% | 0.393% | Validated |
| *HEK4*-off29 | 0.000% | 0.000% | 0.574% | 0.496% | False |
| *HEK4*-off30 | 0.000% | 0.000% | 0.145% | 0.171% | False |
| *HEK4*-off31 | 0.000% | 0.003% | 0.233% | 0.221% | False |
| *HEK4*-off33 | 0.000% | 0.000% | 0.166% | 0.140% | False |
| | | | | | |

| **HeLa, *HEK4* (+2 G to T)** | | | | | |
|---|---|---|---|---|---|
| | Insertion rate | | Mutation rate | | Validation |
| | PE2(+) | PE2(-) | PE2(+) | PE2(-) | |
| *HEK4*-off1 | 0.000% | 0.000% | 0.291 % | 0.231% | False |
| *HEK4*-off2 | 0.013% | 0.319% | 74.852% | 56.731% | False |
| *HEK4*-off3 | 0.000% | 0.000% | 0.411% | 0.497% | False |
| *HEK4*-off4 | 0.000% | 0.000% | 0.315% | 0.479% | Validated |
| *HEK4*-off5 | 0.004% | 0.004% | 0.298% | 0.380% | False |
| *HEK4*-off6 | 0.000% | 0.000% | 0.242% | 0.428% | False |
| *HEK4*-off7 | 0.000% | 0.004% | 0.335% | 30.314% | False |
| *HEK4*-off8 | 0.000% | 0.000% | 7.641% | 6.694% | False |
| *HEK4*-off9 | 0.000% | 0.001% | 8.969% | 0.527% | False |
| *HEK4*-off10 | 0.002% | 0.000% | 0.232% | 0.155% | False |
| *HEK4*-off11 | 0.000% | 0.000% | 0.134% | 0.241 % | False |
| *HEK4*-off12 | 0.000% | 0.004% | 0.556% | 0.314% | False |
| *HEK4*-off13 | 0.000% | 0.004% | 0.548% | 0.293% | False |
| *HEK4*-off14 | 0.000% | 0.000% | 0.245% | 0.341% | False |
| *HEK4*-off15 | 0.000% | 0.000% | 0.658% | 0.657% | False |
| *HEK4*-off22 | 0.000% | 0.002% | 0.422% | 0.951% | False |
| | | | | | |

| **K562, *HEK4* (+2 G to T)** | | | | | |
|---|---|---|---|---|---|
| | Insertion rate | | Mutation rate | | Validation |
| | PE2(+) | PE2(-) | PE2(+) | PE2(-) | |
| *HEK4*-off1 | 0.011% | 00.000% | 0.262% | 0.248% | Validated |
| *HEK4*-off2 | 5.169% | 44.245% | 74.800% | 89.196% | False |
| *HEK4*-off3 | 0.000% | 0.000% | 0.473% | 0.492% | False |
| *HEK4*-off4 | 0.000% | 0.000% | 0.326% | 0.424% | False |
| *HEK4*-off5 | 0.006% | 0.003% | 0.268% | 0.379% | False |
| *HEK4*-off6 | 0.000% | 0.000% | 0.233% | 0.471% | False |
| *HEK4*-off7 | 0.000% | 0.002% | 0.427% | 30.771% | Validated |
| *HEK4*-off8 | 0.000% | 0.003% | 9.037% | 6.134% | False |
| *HEK4*-off9 | 0.000% | 0.010% | 1.807% | 1.419% | False |
| *HEK4*-off10 | 0.000% | 0.000% | 0.154% | 0.204% | False |
| *HEK4*-off11 | 0.000% | 0.000% | 0.262% | 0.269% | False |
| *HEK4*-off12 | 0.009% | 0.010% | 0.490% | 0.419% | False |
| *HEK4*-off13 | 0.000% | 0.001% | 0.465% | 0.306% | False |
| *HEK4*-off14 | - | - | - | - | PCR Fail |
| *HEK4*-off15 | 0.000% | 0.000% | 0.668% | 0.723% | False |
| *HEK4*-off22 | 0.000% | 0.000% | 0.560% | 1.174% | False |
| | | | | | |

| **HEK293T, *HEK4* (+3 TAA ins)** | | | | | |
|---|---|---|---|---|---|
| | Insertion rate | | Mutation rate | | Validation |
| | PE2(+) | PE2(-) | PE2(+) | PE2(-) | |
| *HEK4*-off1 | 0.043% | 0.000% | 1.001 % | 0.309% | Validated |
| *HEK4*-off2 | 99.659% | 0.000% | 99.827% | 16.405% | Validated |
| *HEK4*-off3 | 0.214% | 0.000% | 0 598% | 0.362% | Validated |
| *HEK4*-off4 | 0.024% | 0.000% | 0.489% | 0.585% | Validated |
| *HEK4*-off5 | 0.000% | 0.000% | 0.294% | 0.528% | False |
| *HEK4*-off6 | 0.009% | 0.002% | 0.317% | 0.313% | Validated |
| *HEK4*-off7 | 0.019% | 0.010% | 0.865% | 0.958% | False |
| *HEK4*-off8 | 0.000% | 0.000% | 4.632% | 5.650% | False |
| *HEK4*-off9 | 0.000% | 0.000% | 0.296% | 0.549% | False |
| *HEK4*-off10 | 0.001% | 0.000% | 0.246% | 0.233% | False |
| *HEK4*-off11 | 0.000% | 0.000% | 0.352% | 0.322% | False |
| *HEK4*-off12 | 0.108% | 0.000% | 0.411% | 0.426% | Validated |
| *HEK4*-off13 | 0.002% | 0.000% | 0.329% | 0.348% | False |
| *HEK4*-off14 | 0.000% | 0.000% | 0.314% | 0.419% | False |
| *HEK4*-off15 | 0.000% | 0.000% | 0.618% | 0.407% | False |
| *HEK4*-off22 | 0.000% | 0.000% | 0.537% | 0.591% | False |

| **HeLa, *HEK4* (+3 TAA ins)** | | | | | |
|---|---|---|---|---|---|
| | Insertion rate | | Mutation rate | | Validation |
| | PE2(+) | PE2(-) | PE2(+) | PE2(-) | |
| *HEK4*-off1 | 0.010% | 0.000% | 0.590% | 0.245% | False |
| *HEK4*-off2 | 99.563% | 0.319% | 99.717% | 57.214% | Validated |
| *HEK4*-off3 | 0.006% | 0.000% | 0.497% | 0.523% | Validated |
| *HEK4*-off4 | 0.000% | 0.000% | 0.314% | 0.524% | False |
| *HEK4*-off5 | 0.009% | 0.004% | 0.183% | 1.206% | False |
| *HEK4*-off6 | 0.000% | 0.000% | 0.193% | 0.489% | False |
| *HEK4*-off7 | 0.008% | 0.004% | 0.542% | 30.932% | False |
| *HEK4*-off8 | 0.000% | 0.000% | 7.204% | 6,813% | False |
| *HEK4*-off9 | 0.000% | 0.001% | 3.884% | 0.562% | False |
| *HEK4*-off10 | 0.000% | 0.000% | 0.269% | 0.166% | False |
| *HEK4*-off11 | 0.000% | 0.000% | 0.262% | 0.246% | False |
| *HEK4*-off12 | 0.000% | 0.004% | 0.509% | 0.314% | False |
| *HEK4*-off13 | 0.002% | 0.004% | 0.005% | 0.334% | False |
| *HEK4*-off14 | 0.000% | 0.000% | 0.475% | 0.341% | False |
| *HEK4*-off15 | 0.000% | 0.000% | 0.575% | 0.657% | False |
| *HEK4*-off22 | 0.000% | 0.237% | 0.480% | 41.110% | False |
| | | | | | |

| **K562, *HEK4* (+3 TAA ins)** | | | | | |
|---|---|---|---|---|---|
| | Insertion rate | | Mutation rate | | Validation (K562) |
| | PE2(+) | PE2(-) | PE2(+) | PE2(-) | |
| *HEK4*-off1 | 0.008% | 0.000% | 0.616% | 0.269% | False |
| *HEK4*-off2 | 3.391% | 44.245% | 96.736% | 89.196% | False |
| *HEK4*-off3 | 0.003% | 0.000% | 0.553% | 0.520% | Validated |
| *HEK4*-off4 | 0.000% | 0.000% | 0.296% | 0.458% | False |
| *HEK4*-off5 | 0.005% | 0.003% | 0.364% | 1.098% | False |
| *HEK4*-off6 | 0.000% | 0.000% | 0.358% | 0.526% | False |
| *HEK4*-off7 | 0.004% | 0.002% | 0.637% | 30.977% | False |
| *HEK4*-off8 | 0.000% | 0.003% | 9.212% | 6.218% | False |
| *HEK4*-off9 | 0.000% | 0.010% | 5.457% | 1.472% | False |
| *HEK4*-off10 | 0.000% | 0.000% | 0.200% | 0.212% | False |
| *HEK4*-off11 | 0.000% | 0.000% | 0.285% | 0.281% | False |
| *HEK4*-off12 | 0.008% | 0.010% | 0.579% | 0.428% | False |
| *HEK4*-off13 | 0.000% | 0.001% | 0.528% | 0.338% | False |
| *HEK4*-off14 | - | - | - | - | PCR Fail |
| *HEK4*-off15 | 0.000% | 0.000% | 0.414% | 0.743% | Faise |
| *HEK4*-off22 | 0.003% | 0.000% | 0.646% | 1.211% | False |
| | | | | | |

| **HEK293T, *HBB* (+4 A to T)** | | | | | |
|---|---|---|---|---|---|
| | Insertion rate | | Mutation rate | | Validation |
| | PE2(+) | PE2(-) | PE2(+) | PE2(-) | |
| *HBB*-off1 | 0.005% | 0.000% | 5.179% | 0.282% | Validated |
| *HBB*-off2 | 0.570% | 0.733% | 1.291% | 1.572% | False |
| *HBB*-off3 | 0.000% | 0.000% | 0.254% | 0.258% | Validated |
| *HBB*-off4 | 0.013% | 0.004% | 1.349% | 0.939% | False |
| *HBB*-off5 | 0.000% | 0.000% | 0.257% | 0.234% | False |
| | | | | | |

| **HEK293T, *EMX1* (+5 G to T)** | | | | | |
|---|---|---|---|---|---|
| | Insertion rate | | Mutation rate | | Validation |
| | PE2(+) | PE2(-) | PE2(+) | PE2(-) | |
| *EMX1*-off1 | 0.002% | 0.000% | 0.522% | 0.496% | False |
| *EMX1*-off2 | 0.011% | 0.003% | 0.630% | 0.639% | False |
| | | | | | |

| **HEK293T, *FANCF* (+6 G to C)** | | | | | |
|---|---|---|---|---|---|
| | Insertion rate | | Mutation rate | | Validation |
| | PE2(+) | PE2(-) | PE2(+) | PE2(-) | |
| *FANCF*-off1 | 0.000% | 0.000% | 0.357% | 0.351% | False |
| *FANCF*-off2 | 0.000% | 0.000% | 1.304% | 1.216% | False |
| | | | | | |

| **HEK293T, *HEK3* (+1 CTT ins)** | | | | | |
|---|---|---|---|---|---|
| | Insertion rate | | Mutation rate | | Validation |
| | PE2(+) | PE2(-) | PE2(+) | PE2(-) | |
| *HEK3*-off1 | 0.000% | 0.000% | 0.614% | 0.655% | False |
| *HEK3*-off2 | 0.000% | 0.000% | 0.214% | 0.222% | False |
| | | | | | |

| **HEK293T, *DNMT1* (+6 G to C)** | | | | | |
|---|---|---|---|---|---|
| | Insertion rate | | Mutation rate | | Validation |
| | PE2(+) | PE2(-) | PE2(+) | PE2(-) | |
| *DNMT1*-off1 | 0.000% | 0.000% | 0.277% | 0.273% | Validated |
| *DNMT1*-off2 | 0.000% | 0.000% | 0.457% | 0.468% | False |
| *DNMT1*-off3 | 0.000% | 0.000% | 0.286% | 0.322% | False |
| *DNMT1*-off4 | 13.116% | 16.476% | 18,570% | 22.991% | False |
| *DNMT1*-off5 | 0.000% | 0.000% | 0.362% | 0.379% | False |
| *DNMT1*-off6 | 0.000% | 0.000% | 0.216% | 0.240% | False |
| *DNMT1*-off7 | 0.005% | 0.002% | 0.579% | 0.563% | False |
| | | | | | |

| **HEK293T, *RUNX1* (+6 G to C)** | | | | | |
|---|---|---|---|---|---|
| | Insertion rate | | Mutation rate | | Validation |
| | PE(+) | PE2(-) | PE2(+) | PE2(-) | |
| *RUNX1*-off1 | 0.000% | 0.000% | 0.829% | 0.787% | False |
| | | | | | |

| **HEK293T, *VEGFA* (+5 G to T)** | | | | | |
|---|---|---|---|---|---|
| | Insertion rate | | Mutation rate | | Validation |
| | PE2(+) | PE2(-) | PE2(+) | PE2(-) | |
| *VEGFA-*off1 | 0.000% | 0.002% | 0.333% | 0.357% | False |
| *VEGFA*-off2 | 0.000% | 0.000% | 0.332% | 0.282% | False |
| *VEGFA*-off3 | 0.000% | 0.000% | 0.875% | 0.895% | False |
| *VEGFA*-off4 | 0.000% | 0.000% | 0.364% | 0.325% | False |
| *VEGFA*-off5 | 0.037% | 0.000% | 0.320% | 0.269% | False |
| *VEGFA*-off6 | - | - | - | - | PCR Fail |
| *VEGFA*-off7 | 0.065% | 0.051% | 0.208% | 0.206% | False |
| *VEGFA*-off8 | 0.000% | 0.000% | 7.362% | 7.475% | False |
| *VEGFA*-off9 | 0.002% | 0.000% | 0.412% | 0.375% | False |
| *VEGFA*-off10 | 0.000% | 0.001% | 4.250% | 4.572% | False |
| *VEGFA*-off11 | 0.000% | 0.000% | 0.543% | 0.650% | False |
| *VEGFA*-off12 | 0.000% | 0.000% | 0.333% | 0.348% | False |
| *VEGFA*-off13 | 0.000% | 0.001% | 8.724% | 8.432% | False |
| *VEGFA*-off14 | 0.000% | 0.000% | 0.289% | 0.272% | False |
| *VEGFA*-off15 | 0.020% | 0.016% | 0.193% | 0.185% | False |
| *VEGFA*-off16 | 0.172% | 0.006% | 5.363% | 5.406% | Validated |

**Table 14-1. Tagmentation rates of PE2 TAPE-seq, PE-nuclease TAPE-seq, and PEmax nuclease TAPE-seq (1)**

| | chr | position | Target |
|---|---|---|---|
| *HEK4*-on-target (+2G to T) | chr20 | 31349753 | GGCACTGCGGCTGGAGGTGG |
| nDlgenome-*HEK4*-012 (+2 G to T) | chr20 | 45342993 | GGCACTGaGGgTGGAGGTGG |
| *HEK4*-off1 (+2 G to T) | chr4 | 56815181 | GGCAaTGCGGCTGGAGGcGG |
| *HEK4*-off2 (+2 G to T) | chr10 | 126694857 | GGCACgaCGGCTGGAGGTGG |
| *HEK4*-off3 (+2 G to T) | chr19 | 33382076 | GGCtCTGCGGCTGGAGGgGG |
| *HEK4*-off4 (+2 G to T) | chrX | 104846026 | aGCtCTGC-GCGCTGGAGGaGG |
| *HEK4*-off5 (+2 G to T) | chr13 | 107743553 | GGaACTGaGGCT-CTGAGGgtG |
| *HEK4*-off6 (+2 G to T) | chr13 | 27629405 | GGCACTGgGGtTGGAGGTGG |
| *HEK4*-off7 (+2 G to T) | chr7 | 54561420 | aGgACTGCGGCTGGgGGTGG |
| *HEK4*-off8 (+2 G to T) | chr19 | 1295069 | GaCACTGaGGCaGGAGGTGG |
| *HEK4*-off9 (+2 G to T) | chr19 | 46887169 | GaggCTGCGGCTGGgGGTGG |
| *HBB*-on-target (+4 A to T) | chr11 | 5248231 | CATGGTGCACCTGACTCCTG |
| *HBB*-off1 (+4 A to T) | chr11 | 5255643 | CATGGTGCAtCTGACTCCTG |

**Table 14-2. Tagmentation rates of PE2 TAPE-seq, PE-nuclease TAPE-seq, and PEmax nuclease TAPE-seq (2)**

| **Tagmentation rate** | | | | |
|---|---|---|---|---|
| | PE2(-) | PE2(+) | PE2- nuclease(+) | PEmax-nuclease & epegRNA(+) |
| *HEK4*-on-target (+2G to T) | 0.021 % | 2.406% | 21.187% | 5.860% |
| nDlgenome-*HEK4-*012 (+2 G to T) | 0.000% | 0.000% | 0.000% | 0.000% |
| *HEK4*-off1 (+2 G to T) | 0.000% | 0.181% | 17.833% | 5.107% |
| *HEK4*-off2 (+2 G to T) | 0.000% | 73.888% | 39.564% | 20.629% |
| *HEK4*-off3 (+2 G to T) | 0.000% | 0.000% | 1.468% | 0.067% |
| *HEK4*-off4 (+2 G to T) | 0.000% | 0.000% | 5.592% | 0.362% |
| *HEK4*-off5 (+2 G to T) | 0.000% | 0.000% | 0.000% | 0.000% |
| *HEK4*-off6 (+2 G to T) | 0.000% | 0.000% | 0.361% | 0.043% |
| *HEK4*-off7 (+2 G to T) | 0.000% | 0.000% | 0.024% | 0.002% |
| *HEK4*-off8 (+2 G to T) | 0.000% | 0.000% | 0.000% | 0.000% |
| *HEK4*-off9 (+2 G to T) | 0.000% | 0.000% | 0.043% | 0.004% |
| *HBB*-on-target (+4 A to T) | 0.000% | 0.119% | - | - |
| *HBB*-off1 (+4 A to T) | 0.000% | 0.000% | - | - |

**Table 15. Tagmentation rates of PE2 TAPE-seq and PE4 TAPE-seq**

| HEK4 (+2 G to T) | chr | position | Target | Cell type | Tagmentation rate | |
|---|---|---|---|---|---|---|
| | | | | | PE2(+) | PE4(+) |
| On-target | chr20 | 31349753 | GGCACTGCGGCTGGAGGTGG | HEK293T | 0.518 % | 0.687 % |
| | | | | HeLa | 0.943 % | 0.455 % |
| | | | | K562 | 1 .543 % | 1.991 % |
| Off1 | chr4 | 56815181 | GGCAaTGCGGCTGGAGGcGG | HEK293T | 0.017 % | 0.019 % |
| | | | | HeLa | 0.007 % | 0.003 % |
| | | | | K562 | 0.068 % | 0.133 % |

**Table 16. Tagmentation rates by tag length**

| HEK4 (+2 G to T) | chr | position | Target | Tag length | Tagmentation rate | | |
|---|---|---|---|---|---|---|---|
| | | | | | HEK293T | HeLa | K562 |
| On-target | chr20 | 31349753 | GGCACTGCGGC TGGAGGTGG | 34bp | 0.414% | 0.326% | 2.932% |
| | | | | 29bp | 0.513% | 0.572% | 3.777% |
| | | | | 24bp | 2.022% | 1.106% | 7.192% |
| | | | | 19bp | 3.213% | 1.908% | 7.289% |
| Off1 | chr4 | 56815181 | GGCAaTGCGGC TGGAGGcGG | 34bp | 0.025% | 0.033% | 0.160% |
| | | | | 29bp | 0.010% | 0.006% | 0.022% |
| | | | | 24bp | 0.020% | 0.000% | 0.045% |
| | | | | 19bp | 0.046% | 0.000% | 0.057% |

### Analysis Data 5. Calculation of validation rates for predictions generated by TAPE-seq

Hereinbelow, the calculation results of validation rates for predictions generated by TAPE-seq (PE2, PE2-nuclease, and PEmax-nuclease) and other off-target prediction methods are to be disclosed.

**Table 17. HEK4 (+2 G to T) pegRNA**

| Target | ***HEK4*** (**+2 G to T**) | nDigenome -seq | GUIDE -seq | TAPE-seq PE2 (N=3) | TAPE-seq PE2-nuclease (N=1) | TAPE-seq PEmax-nucelase (N=1) |
|---|---|---|---|---|---|---|
| Number of off-target candidates | | 408 | 134 | 14 | 30 | 27 |
| Validation | Validated | 8 | 8 | 8 | 9 | 9 |
| | FALSE | 48 | 34 | 6 | 21 | 13 |
| | Not Analyzed | 352 | 92 | 0 | 0 | 5 |
| | Number of Missed validated off-target | 2 | 2 | 2 | 1 | 1 |
| | Validation rate | 14.29% | 19.05% | 57.14% | 30.00% | 40.91% |

**Table 18. HEK4 (+3 TAA ins) pegRNA**

| Target | **HEK4 (+ 3 TAA ins)** | nDigenome -seq | GUIDE-seq | TAPE-seq PE2(N=3) | TAPE-seq PE2-nuclease (N=1) | TAPE-seq PEmax-nucelase (N=1) |
|---|---|---|---|---|---|---|
| Number of off-target candidates | | 408 | 134 | 14 | 30 | 27 |
| Validation | Validated | 5 | 5 | 7 | 6 | 6 |
| | FALSE | 6 | 7 | 7 | 7 | 5 |
| | Not Analyzed | 397 | 122 | 0 | 17 | 16 |
| | Number of Missed validated off-target | 2 | 2 | 0 | 1 | 1 |
| | Validation rate | 45.45% | 41.67% | 50.00% | 46.15% | 54.55% |

**Table 19. HBB (+4 A to T) pegRNA**

| Target | ***HBB* (+4 A to T)** | nDigenome-seq | TAPE-seq PE2 (N-1) | TAPE-seq PE2-nuclease (N=1) | TAPE-seq PEmax-nucelase (N=1) |
|---|---|---|---|---|---|
| Number of off-target candidates | | 445 | 2 | 3 | 5 |
| Validation | Validated | 3 | 2 | 2 | 3 |
| | FALSE | 37 | 0 | 1 | 2 |
| | Not Analyzed | 405 | 0 | 0 | 0 |
| | Number of Missed validated off-target | 0 | 1 | 1 | 0 |
| | Validation rate | 7.50% | 100% | 67% | 60.00% |

**Table 20. EMX1 (+5 G to T) pegRNA**

| Target | ***EMX1* (+5 G to T)** | nDigenome-seq | GUIDE-seq | TAPE-seq PE2 (N=1) | TAPE-seq PE2-nuclease (N=1) | TAPE-seq PEmax-nuclease (N=1) |
|---|---|---|---|---|---|---|
| Number of off-target candidates | | 90 | 16 | 1 | 2 | 3 |
| Validation | Validated | 1 | 1 | 1 | 1 | 1 |
| | FALSE | 19 | 9 | 0 | 1 | 2 |
| | Not Analyzed | 70 | 6 | 0 | 0 | 0 |
| | Number of Missed validated off-target | 0 | 0 | 0 | 0 | 0 |
| | Validation rate | 5.00% | 10.00% | 100% | 50% | 33.33% |

**Table 21. FANCF (+6 G to C) pegRNA**

| **Target** | ***FANCF* (+6 G to C)** | nDigenome-seq | GUIDE-seq | TAPE-seq PE2 (N=1) | TAPE-seq PE2-nuclease (N=1) | PEmax-nucelase (N=1) |
|---|---|---|---|---|---|---|
| Number of off-target candidates | | 58 | 9 | 1 | 2 | 3 |
| Validation | Validated | 1 | 1 | 1 | 1 | 1 |
| | FALSE | 57 | 7 | 0 | 1 | 2 |
| | Not Analyzed | 0 | 1 | 0 | 0 | 0 |
| | Number of Missed validated off-target | 0 | 0 | 0 | 0 | 0 |
| | Validation rate | 1.72% | 12.50% | 100% | 50% | 333.33% |

**Table 22. HEK3 (+1 CTT ins) pegRNA**

| Target | ***HEK3* (+1 CTT ins)** | nDigenome-seq | GUIDE-seq | TAPE-seq PE2 (N=1) | TAPE-seq PE2-nuclease (N=1) | TAPE-seq PEmax-nuclease (N=1) |
|---|---|---|---|---|---|---|
| Number of off-target candidates | | 39 | 6 | 1 | 3 | 3 |
| Validation | Validated | 1 | 1 | 1 | 1 | 1 |
| | FALSE | 38 | 5 | 0 | 2 | 2 |
| | Not Analyzed | 0 | 0 | 0 | 0 | 0 |
| | Number of Missed validated off-target | 0 | 0 | 0 | 0 | 0 |
| | Validation rate | 2.56% | 16.67% | 100% | 33% | 33.33% |

**Table 23. RNF2 (+6 G to A) pegRNA**

| Target | ***RNF2* (+6 G to A)** | nDigenome-seq | GUIDE-seq | TAPE-seq PE2 (N=1) | TAPE-seq PE2-nuclease (N=1) | TAPE-seq PEmax-nuclease (N=1) |
|---|---|---|---|---|---|---|
| Number of off-target candidates | | 73 | 1 | 1 | 1 | 1 |
| Validation | Validated | 1 | 1 | 1 | 1 | 1 |
| | FALSE | 17 | 0 | 0 | 0 | 0 |
| | Not Analyzed | 55 | 0 | 0 | 0 | 0 |
| | Number of Missed validated off-target | 0 | 0 | 0 | 0 | 0 |
| | Validation rate | 5.56% | 100% | 100% | 100% | 1 00% |

**Table 24. DNMT1 (+6 G to C) pegRNA**

| Target | ***DNMT1* (+6 G to C)** | nDigenome-seq | TAPE-seq PE2 (N=1) | TAPE-seq nuclease (N=1) | TAPE-seq PEmax-nucelase (N=1) |
|---|---|---|---|---|---|
| Number of off-target candidates | | 217 | 1 | 2 | 8 |
| Validation | Validated | 2 | 1 | 2 | 2 |
| | FALSE | 21 | 0 | 0 | 6 |
| | Not Analyzed | 194 | 0 | 0 | 0 |
| | Number of Missed validated off-target | 0 | 1 | 0 | 0 |
| | Validation rate | 8.70% | 100% | 100% | 25.00% |

**Table 25. RUNX1 (+6 G to C) pegRNA**

| Target | ***RUNX1* (+6 G to C)** | nDigenome-seq | TAPE-seq PE2 (N=1) | TAPE-seq PE2-nuclease (N=1) | TAPE-seq PEmax-nucelase (N=1) |
|---|---|---|---|---|---|
| Number of off-target candidates | | 28 | 1 | 2 | 2 |
| Validation | Validated | 1 | 1 | 1 | 1 |
| | FALSE | 20 | 0 | 1 | 1 |
| | Not Analyzed | 7 | 0 | 0 | 0 |
| | Number of Missed validated off-target | 0 | 0 | 0 | 0 |
| | Validation rate | 4.76% | 100% | 50% | 50.00% |

**Table 26. VEGFA (+5 G to T) pegRNA**

| Gene | ***VEGFA* (+5 G to T)** | nDigenome-seq | TAPE-seq PE2 (N=1) | TAPE-seq PE2-nuclease (N=1) | TAPE-seq PEmax-nucelase (N=1) |
|---|---|---|---|---|---|
| Number of off-target candidates | | 536 | 1 | 6 | 17 |
| Validation | Validated | 2 | 1 | 1 | 2 |
| | FALSE | 26 | 0 | 5 | 14 |
| | Not Analyzed | 508 | 0 | 0 | 1 |
| | Number of Missed validated off-target | 0 | 1 | 1 | 0 |
| | Validation rate | 7.14% | 100% | 17% | 12.50% |

### Analysis Data 6. Miss rates of validated off-targets

Hereinbelow, the results for the miss rate of each off-target prediction method are to be disclosed. A validated off-target predicted by an off-target prediction method is indicated by a +. The miss rate for the remaining targets was confirmed to be 0. The entry "Type" indicates information on whether the bulge is RNA, DNA, or nonexistent.

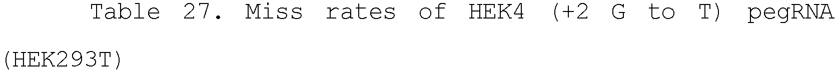

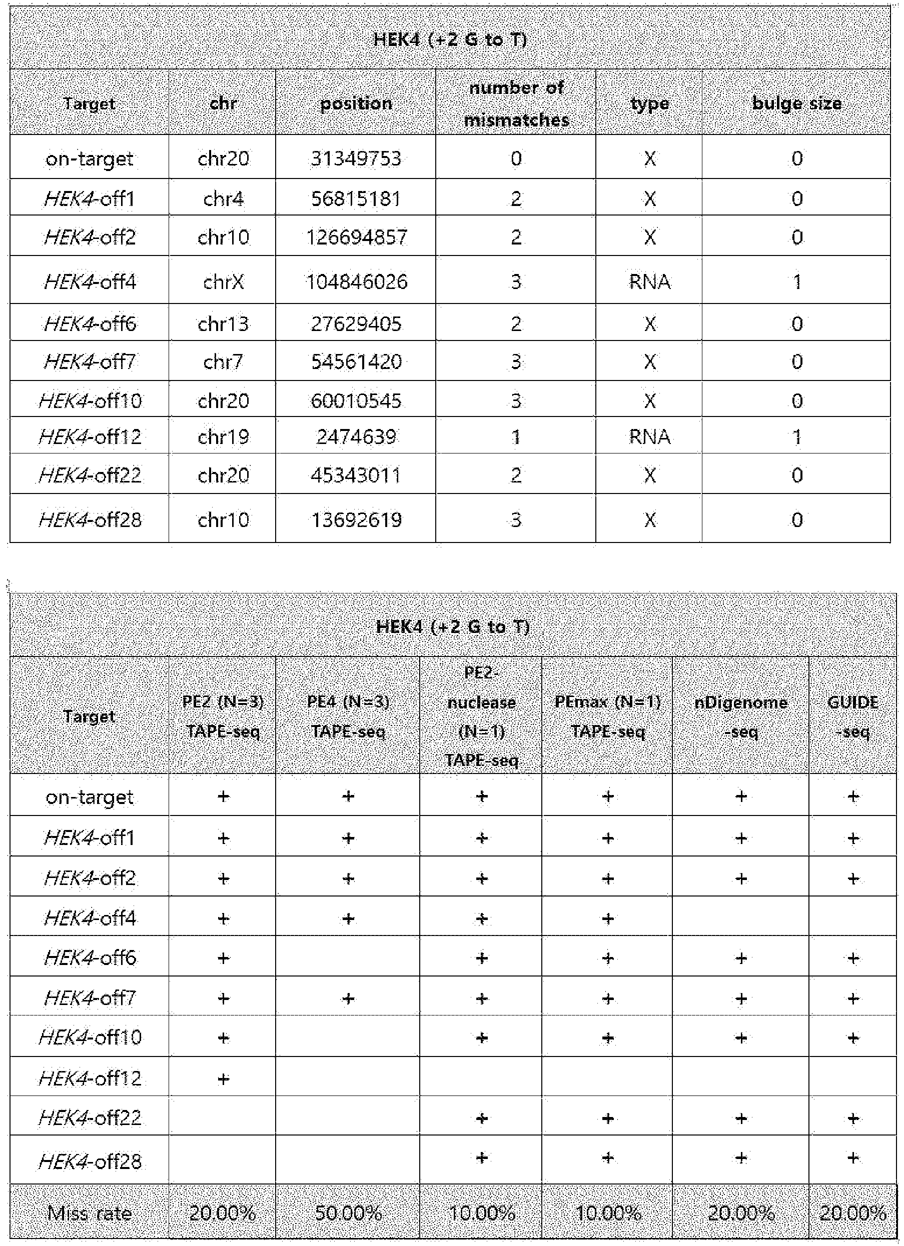

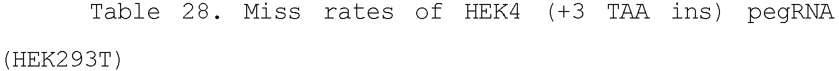

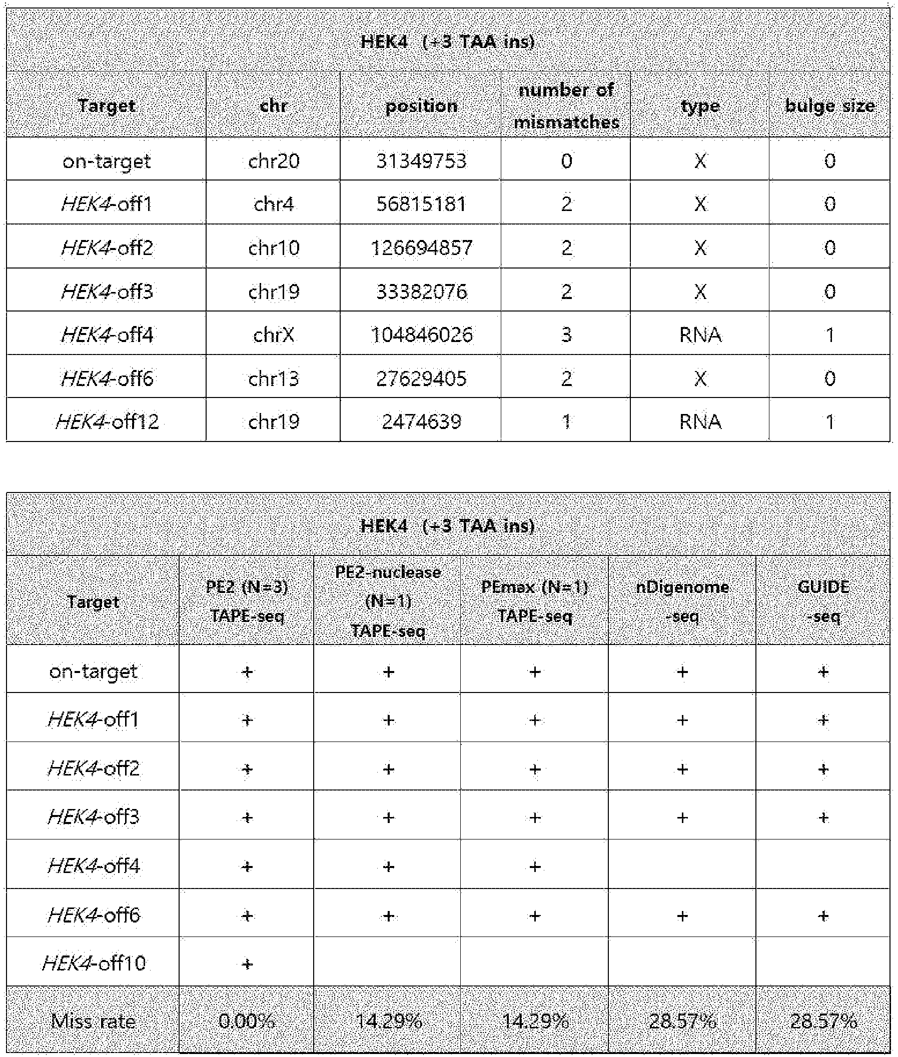

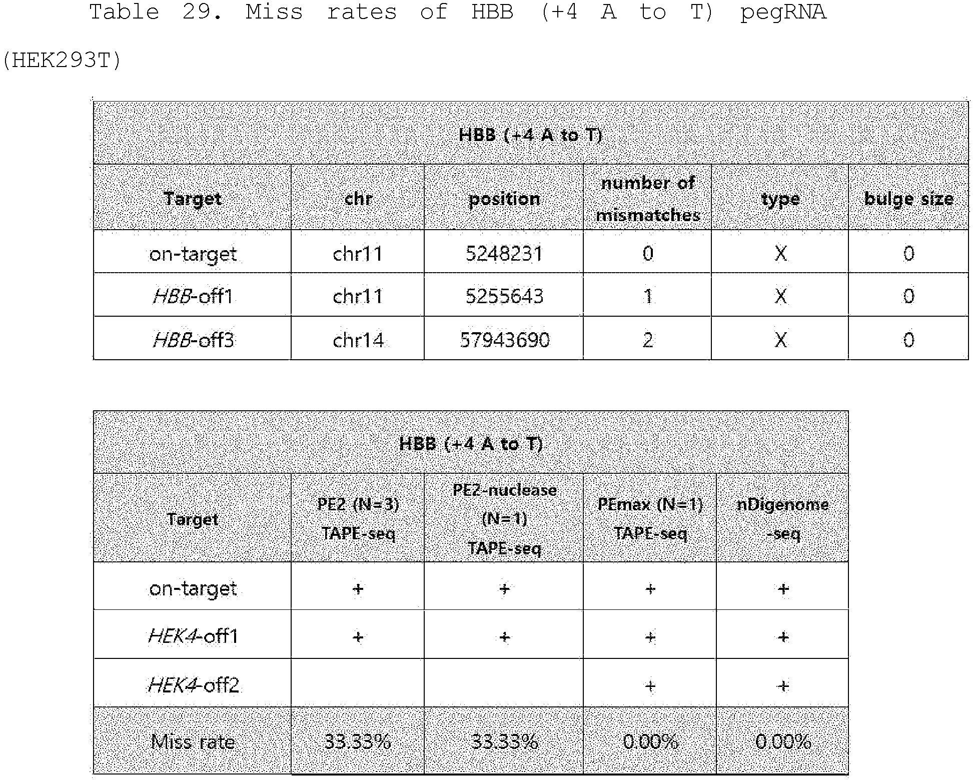

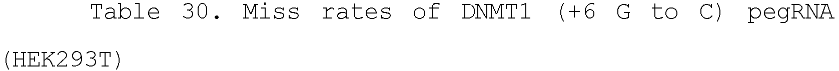

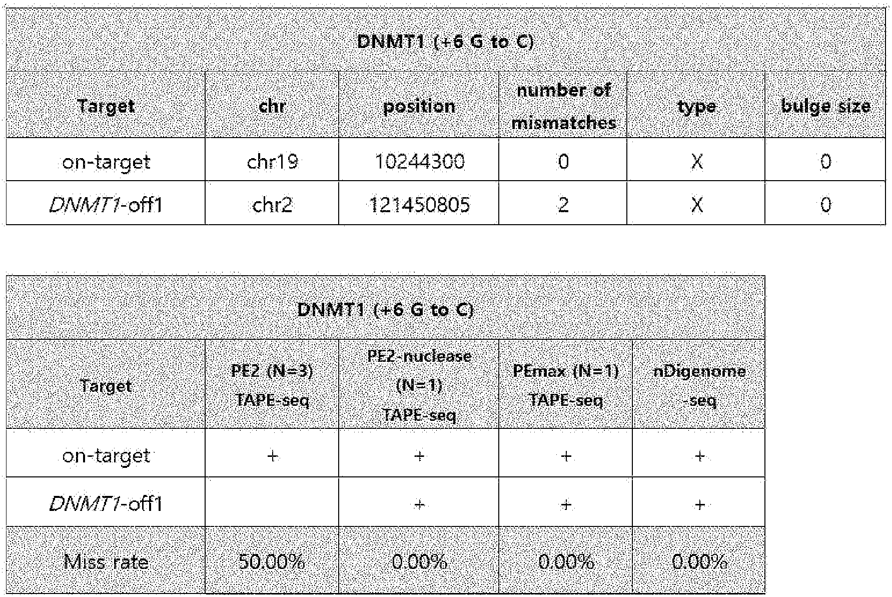

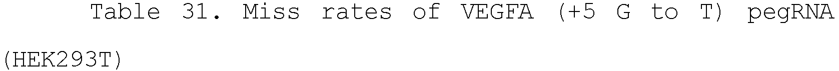

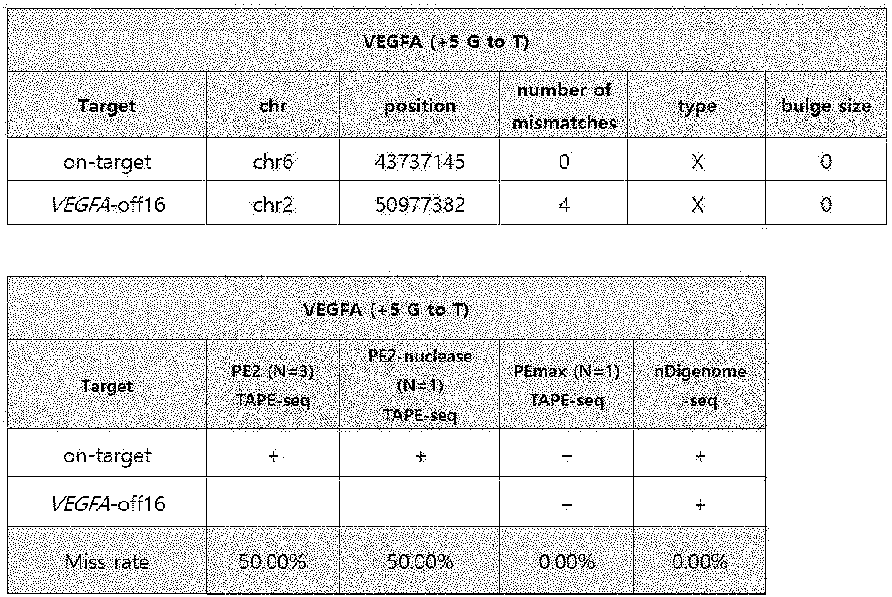

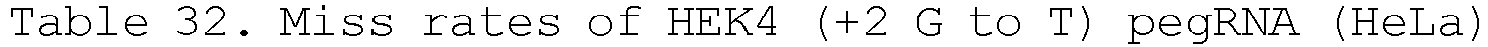

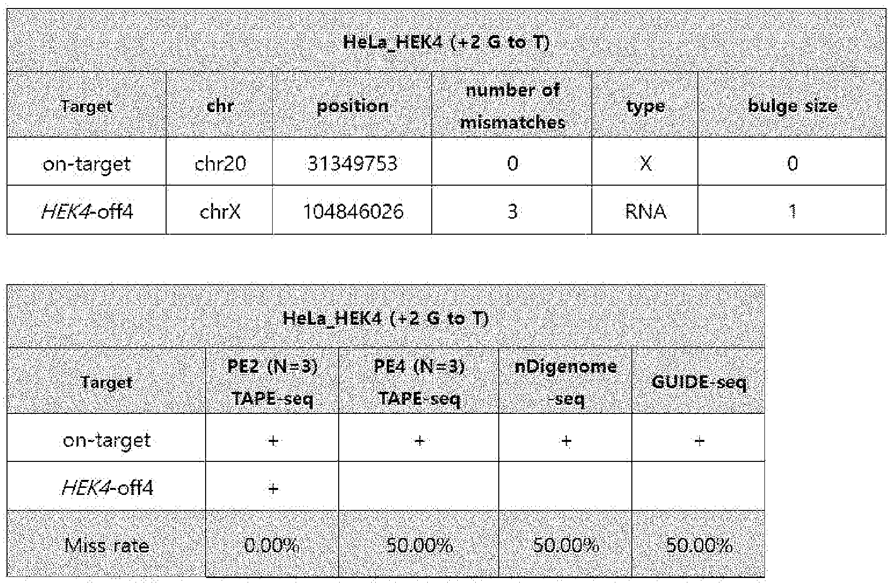

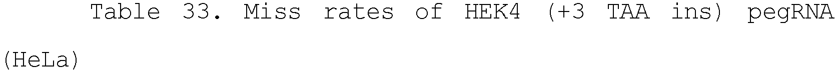

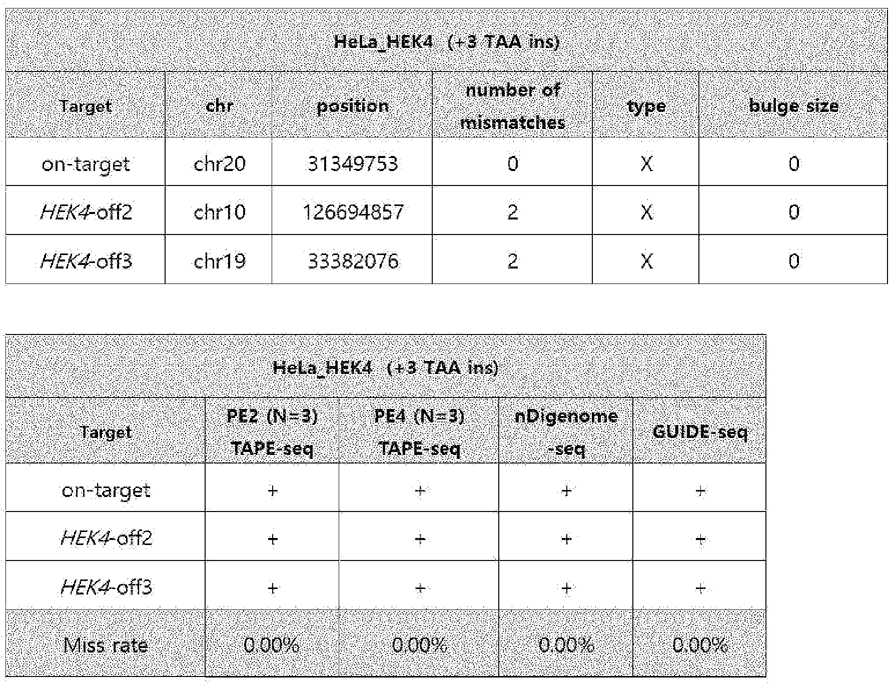

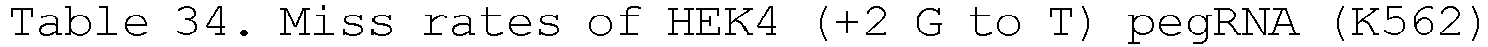

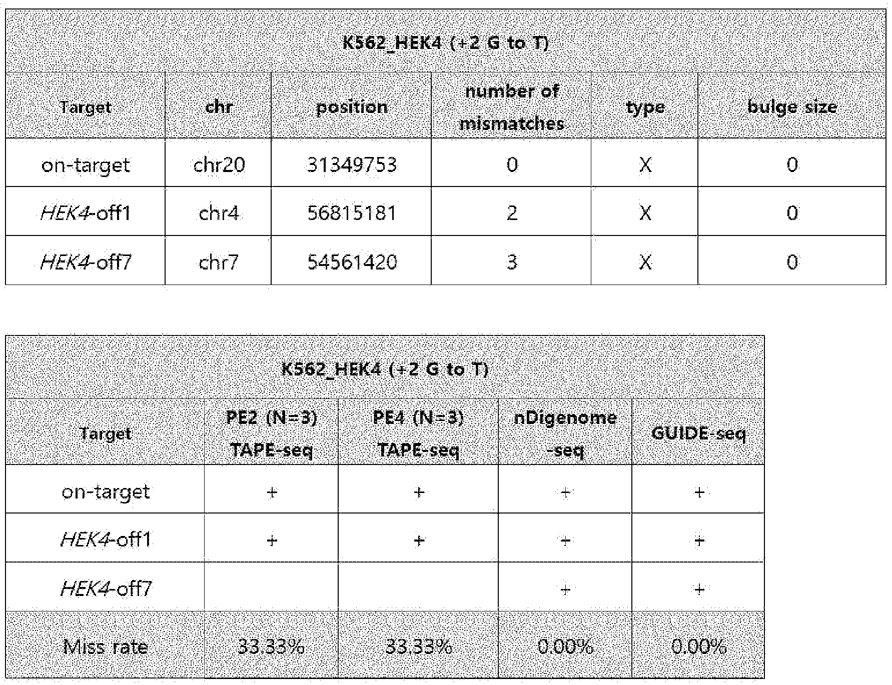

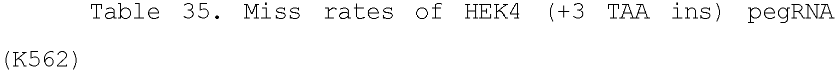

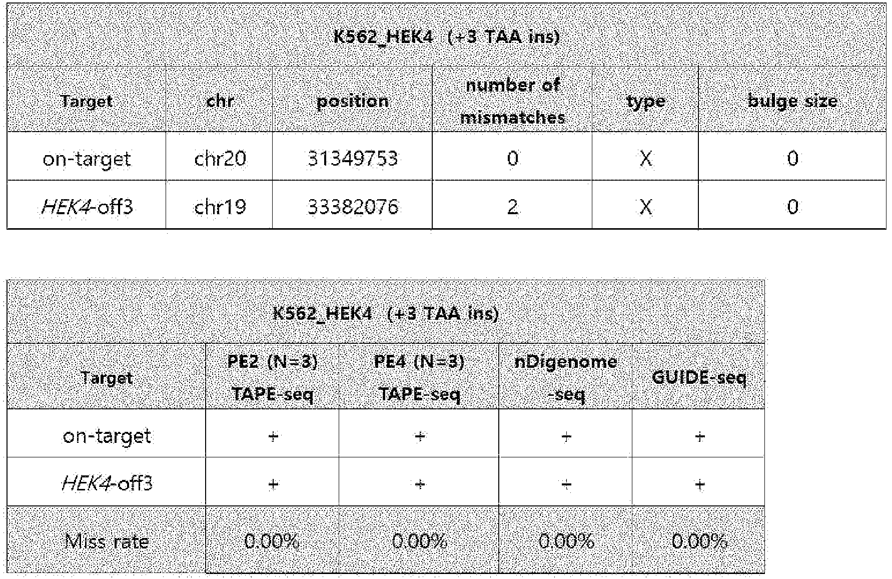

### Analysis Data 7. Mismatch analysis by regions

Hereinbelow, the analysis results for mismatches in each region of the pegRNA are to be disclosed. In the entries "Target", "PBS", and "RT region", mismatches between the on-target sequence and the corresponding region of the pegRNA are indicated by "lowercase letters" and "-". The entry "Type" indicates information on whether the bulge is RNA, DNA, or nonexistent.

**Table 36-1. Analysis result for HEK4 on-target and off-target (1)**

| **HEK4** | **chr** | **position** | **Target** | **PAM** | **Mis match** | **type** | **bulge size** |
|---|---|---|---|---|---|---|---|
| on-target | chr20 | 31349753 | GGCACTGCGGCTGGAGGTGG | GGG | 0 | X | 0 |
| HEK4-off1 | chr4 | 56815181 | GGCAaTGCGGCTGGAGGcGG | AGG | 2 | X | 0 |
| HEK4-off2 | chr10 | 126694857 | GGCACgaCGGC TGGAGGTGG | GGG | 2 | X | 0 |
| HEK4-off3 | chr19 | 33382076 | GGCtCTGCGGCTGGAGGgGG | TGG | 2 | X | 0 |
| HEK4-off4 | chrX | 104846026 | aGCtCTGC-GCGCTGGAGGaGG | GGG | 3 | RNA | 1 |
| HEK4-off5 | chr13 | 107743553 | GGaAGTGaGGCT-CTGAGGgtG | GGG | 4 | RNA | 1 |
| HEK4-off6 | chr13 | 27629405 | GGCACTGgGGtTGGAGGTGG | GGG | 2 | X | 0 |
| HEK4-off7 | chr7 | 54561420 | aGgACTGCGGCTGGgGGTGG | TGG | 3 | X | 0 |
| HEK4-off8 | chr19 | 1295069 | GaCACTGaGGCaGGAGGTGG | GGG | 3 | X | 0 |
| HEK4-off9 | chr19 | 46887169 | GaggCTGCGGCTGGgGGTGG | AGG | 4 | X | 0 |
| HEK4 - off10 | chr20 | 60010545 | tGCACTGCGGCcGGAGGaGG | TGG | 3 | X | 0 |
| HEK4-off11 | chr3 | 49055357 | GGgACTGCGGCTGGAGGTGGG | AAG | 1 | DNA | 1 |
| HEK4-off12 | chr19 | 2474639 | GGCACTG-TGGGCTGGAGGcGG | GGG | 1 | RNA | 1 |
| HEK4-off13 | chr20 | 60895666 | GGCACaGCaGCTGGAGGTGc | TGG | 3 | X | 0 |
| HEK4-off14 | chr10 | 126708851 | aGCACT-CTGGgTGGgGGTtG | GGG | 4 | RNA | 2 |
| HEK4-off15 | chr3 | 31866516 | GGCAGTGaGGgTtGgGGGATGG | AGG | 4 | DNA | 2 |
| *HEK4-*off16 | chr10 | 77103102 | GGCAt caCGGCTGGAGGTGG | AGG | 3 | X | 0 |
| *HEK4-*off17 | chr3 | 51725447 | GGCtCTGtGGCTGGAGGaGG | TGG | 3 | X | 0 |
| *HEK4-*off18 | chr15 | 41044237 | GGCgCTGCGGCgGGAGGTGG | AGG | 2 | X | 0 |
| *HEK4-*off19 | chr13 | 88900987 | caCACTGCaGCTGGAGGTGG | TGG | 3 | X | 0 |
| *HEK4-*off20 | chr17 | 72251117 | GGtACTctGGCTGGAGGTGG | TGG | 3 | X | 0 |
| *HEK4-*off21 | chr17 | 75429275 | GaCACcaCGGCTGGAGaTGG | TGG | 4 | X | 0 |
| *HEK4-*off22 | chr20 | 45342993 | GGCACTGaGGgTGGAGGTGG | GGG | 2 | X | 0 |
| *HEK4-*off23 | chr2 | 241640849 | GGggCTGCGGCcGGAGGTGG | TGG | 3 | X | 0 |
| *HEK4-*off24 | chr1 | 38.557167 | aGCACaaaGGCTGGAGGTGG | AGG | 4 | X | 0 |
| *HEK4-*off25 | chr1 | 204463906 | GGCgCTGCGGCTGGAGccGG | CGG | 3 | X | 0 |
| *HEK4-*off26 | chr6 | 160517876 | GGCACTGCtGCTGGgGGTGG | TGG | 2 | X | 0 |
| *HEK4-*off27 | chr8 | 119227128 | GGCACaatGGCTGGAGGTGa | AGG | 4 | X | 0 |
| *HEK4-*off28 | chr10 | 1369269 | GGCACTGgGGCTGGgGGaGG | GGG | 3 | X | 0 |
| *HEK4-*off29 | chr16 | 28266963 | CGCtCTtCGGCTGGAGGTaG | CGG | 3 | X | 0 |
| *HEK4-*off30 | chr2 | 25348462 | GGaACTGtGGCTGGAGGTGG | CAG | 2 | X | 0 |
| *HEK4-*off31 | chr6 | 36761675 | ccCACTGgGGCTGGAGGTGG | GGG | 3 | X | 0 |
| *HEK4-*off32 | chr19 | 38616181 | GGCACTGaGaCTGGgGGTGG | GGG | 3 | X | 0 |
| *HEK4-*off33 | chr20 | 1151849 | GGCACTGtGGCTGcAGGTGG | AGG | 2 | X | 0 |
| *HEK4-*off34 | chr18 | 37194553 | GGCACTGCGGgTGGAGGcGG | GGG | 2 | X | 0 |
| *HEK4-*off35 | chr16 | 50300329 | aGCACTGtGGCTGGgGGaGG | GGG | 4 | X | 0 |
| *HEK4-*off36 | chr16 | 78413344 | GGtACaGtGGCTGGAGGTGG | AAG | 3 | X | 0 |
| *HEK4-*off37 | chr2 | 3691610 | aGgACTGCG--CGTGGAGaTGG | TGG | 3 | RNA | 2 |
| *HEK4-*off38 | chr1 | 243064859 | GcCACTGgTTGGCTGGgcGTGG | TGG | 4 | DNA | 2 |
| *HEK4-*off39 | chr5 | 172049290 | tGCACaGaGGCTGGAGGCTGa | AAG | 4 | DNA | 1 |

**Table 36-2. Analysis result for HEK4 on-target and off-target (1)**

| **HEK4** | **PBS (9bp) p** | **number of PBS mismatch** | **PBS mismatch Rate** | **RT region (10bp)** | **number of RT template mismatches** | **RT region mismatch** |
|---|---|---|---|---|---|---|
| on-target | GGCTGGAGG | 0 | 0% | TGGGGGTTAA | 0 | 0% |
| HEK4-off1 | GGCTGGAGG | 0 | 0% | cGGaGGcaac | 6 | 60% |
| HEK4-off2 | GGCTGGAGG | 0 | 0% | TGGGGGgTtg | 3 | 30% |
| HEK4-off3 | GGCTGGAGG | 0 | 0% | gGGtGGggtt | 6 | 60% |
| HEK4-off4 | GcGCTGGAGG | DNA bulge 1bp | - | aGGgGGagtg | 6 | 60% |
| HEK4-off5 | GGCT-CTGAGG | RNA bulge 1bp DNA bulge 2bp | - | gtGGGGTgcA | 4 | 40% |
| HEK4-off6 | GGtTGGAGG | 1 | 11% | TGGGGGcTcg | 3 | 30% |
| HEK4-off7 | GGCTGGgGG | 1 | 11% | TGGtGGcTcA | 3 | 30% |
| HEK4-off8 | GGCaGGAGG | 1 | 11% | TGGGGGgcAg | 3 | 30% |
| HEK4-off9 | GGCTGGgGG | 1 | 11% | TGGaGGTggg | 4 | 40% |
| HEK4-off10 | GGCcGGAGG | 1 | 11% | aGGtGGaggA | 5 | 50% |
| HEK4-off11 | GGCTGGAGG | 0 | 0% | GGGaaGaagt | 6 | 60% |
| HEK4-off12 | GGCTGGAGG | 0 | 0% | cGGGGGaTAA | 2 | 200 |
| HEK4-off13 | aGCTGGAGG | 1 | 11% | TGcTGGagcA | 4 | 40% |
| HEK4-off14 | GGgTGGgGG | 2 | 22% | TtGGGGggAt | 4 | 40% |
| HEK4-off15 | GGgTtGgGGGA | 3 DNA bulge 2bp | - | TGGaGGagtg | 5 | 50% |
| *HEK4-*off16 | GGCTGGAGG | 0 | 0% | TGGaGGgggc | 5 | 50% |
| *HEK4-*off17 | GGCTGGAGG | 0 | 0% | aGGtGGaagA | 5 | 50% |
| HEK4-off18 | GGCgGGAGG | 1 | 11% | TGGaGGagct | 5 | 50% |
| *HEK4-*off19 | aGCTGGAGG | 1 | 11% | TGGtGGggAg | 4 | 40% |
| *HEK4-*off20 | GGCTGGAGG | 0 | 0% | TGGtGGggtg | 5 | 50% |
| *HEK4-*off21 | GGCTGGAGa | 1 | 11% | TGGtGGgatg | 5 | 50% |
| *HEK4-*off22 | GGgTGGAGG | 1 | 11% | TGGGGGcagt | 4 | 40% |
| *HEK4-*off23 | GGCcGGAGG | 1 | 11% | TGGtGGcgct | 5 | 50% |
| *HEK4-*off24 | GGCTGGAGG | 0 | 0% | TGGaGGaggt | 5 | 50% |
| *HEK4-*off25 | GGCTGGAGc | 1 | 11% | cGGcGGccgg | 6 | 60% |
| *HEK4-*off26 | tGCTGGgGG | 2 | 22 % | TGGtGGgagg | 5 | 50% |
| *HEK4-*off27 | GGCTGGAGG | 0 | 0% | TGaaGGcagA | 5 | 50% |
| HEK4-off28 | GGCTGGgGG | 1 | 11% | aGGGGGTctA | 3 | 30% |
| *HEK4-*off29 | GGCTGGAGG | 0 | 0% | TaGcGGgTAg | 4 | 40% |
| *HEK4-*off30 | GGCTGGAGG | 0 | 0% | TGGCaGCagc | 6 | 60% |
| *HEK4-*off31 | GGCTGGAGG | 0 | 0% | TGGGGGTgtg | 3 | 30% |
| *HEK4-*off32 | GaCTGGgGG | 2 | 22% | TGGGGGTggg | 3 | 30% |
| *HEK4-*off33 | GGCTGcAGG | 1 | 11% | TGGaGGTact | 4 | 40% |
| *HEK4-*off34 | GGgTGGAGG | 1 | 11% | cGGGGGgTgc | 4 | 40% |
| *HEK4-*off35 | GGCTGGgGG | 1 | 11% | aGGGGGccct | 5 | 50% |
| *HEK4-*off36 | GGCTGGAGG | 0 | 0% | TGGaaGaagc | 6 | 60% |
| *HEK4-*off37 | cG-CGTGGAGa | RNA bulge 2 bp DNA bulge 1bp mismatch 2 | - | TGGtGGcctc | 5 | 50% |
| *HEK4-*off38 | GGCTGGgcG | 2 | 22% | TGGtGGcTcA | 3 | 30% |
| *HEK4-*off39 | GGCTGGAGGC | DNA bulge 1bp | - | TGaaaGcacc | 7 | 70% |

**Table 36-3. Analysis result for HEK4 on-target and off-target (3)**

| | **HEK 293T** | | **K562** | | **HeLa** | |
|---|---|---|---|---|---|---|
| **HEK4** | **+2 G tc T** | **+3 TAA ins** | **+2 G to T** | **+3 TAA ins** | **+2 G to T** | **+3 TAA ins** |
| on-target | Validated | Validated | Validated | Validated | Validated | Validated |
| HEK4-off1 | Validated | Validated | Validated | False | False | False |
| HEK4-off2 | Validated | Validated | False | False | False | Validated |
| HEK4-off3 | False | Validated | False | Validated | False | Validated |
| HEK4-off4 | Validated | Validated | False | False | Validated | False |
| HEK4-off5 | False | False | False | False | False | False |
| HEK4-off6 | Validated | Validated | False | False | False | False |
| HEK4-off7 | Validated | False | Validated | False | False | False |
| HEK4-off8 | False | False | False | False | False | False |
| HEK4-off9 | False | False | False | False | False | False |
| HEK4-off10 | Validated | False | False | False | False | False |
| HEK4-off11 | False | False | False | False | False | False |
| HEK4-off12 | Validated | Validated | False | False | False | False |
| HEK4-off13 | False | False | False | False | False | False |
| HEK4-off14 | False | False | PCR Fail | | False | False |
| HEK4-off15 | False | False | False | False | False | False |
| *HEK4-*off16 | False | - | - | - | - | - |
| *HEK4-*off17 | False | - | - | - | - | - |
| *HEK4-*off18 | False | - | - | - | - | - |
| *HEK4-*off19 | False | - | - | - | - | - |
| *HEK4-*off20 | False | - | - | - | - | - |
| *HEK4-*off21 | False | - | - | - | - | - |
| *HEK4-*off22 | Validated | False | False | False | False | False |
| *HEK4-*off23 | False | - | - | - | - | - |
| *HEK4-*off24 | False | - | - | - | - | - |
| *HEK4-*off25 | False | - | - | - | - | - |
| *HEK4-*off26 | False | - | - | - | - | - |
| *HEK4-*off27 | False | - | - | - | - | - |
| *HEK4-*off28 | Validated | - | - | - | - | - |
| *HEK4-*off29 | False | - | - | - | - | - |
| *HEK4-*off30 | False | - | - | - | - | - |
| *HEK4-*off31 | False | - | - | - | - | - |
| *HEK4-*off32 | False | - | - | - | - | - |
| *HEK4-*off33 | False | - | - | - | - | - |
| *HEK4-*off34 | - | - | - | - | - | - |
| *HEK4-*off35 | - | - | - | - | - | - |
| *HEK4-*off36 | - | - | - | - | - | - |
| *HEK4-*off37 | - | - | - | - | - | - |
| *HEK4-*off38 | - | - | - | - | - | - |
| *HEK4-*off39 | - | - | - | - | - | - |

**Table 37-1. Analysis result for HBB on-target and off-target (1)**

| **HBB** | **chr** | **position** | **Target** | **PAM** | **Mis match** | **type** | **bulge size** |
|---|---|---|---|---|---|---|---|
| on-target | chr11 | 5248231 | CATGGTGCACCTGACTCCTG | AGG | 0 | X | 0 |
| HBB-off1 | chr11 | 5255643 | CATGGTGCAtCTGACTCCTG | AGG | 1 | X | 0 |
| *HBB-*off2 | chr6 | 159152766 | CATGGTG-- TGgCgGgCgCCTG | TAG | 4 | RNA | 2 |
| *HBB-*off3 | chr14 | 57943690 | CAcGGgGCAGCTGACTCCTG | AGG | 2 | X | 0 |
| *HBB-*off4 | chr6 | 52672647 | aATGGaGCACCTGACTCCca | AGG | 4 | X | 0 |
| *HBB-*off5 | chr19 | 39884737 | gATGGTGCAtCTGACTCGaG | AGG | 3 | X | 0 |

**Table 37-2. Analysis result for HBB on-target and off-target (2)**

| **HBB** | **PBS region(13bp)** | **PBS mismatch** | **PBS mismatch Rate** |
|---|---|---|---|
| on-target | GTGCACCTGACTC | 0 | 0% |
| HBB-off1 | GTGCAtCTGACTC | 1 | 8% |
| *HBB-*off2 | GTG--TGgCgGgCgC | RNA bulge 2bp | - |
| | | mismatch 4 | |
| *HBB*-off3 | GgGCACCTGACTC | 1 | 8% |
| *HBB*-off4 | GaGCACCTGACTC | 1 | 8% |
| *HBB-*off5 | GTGCAtCTGACTC | 1 | 8% |

**Table 37-3. Analysis result for HBB on-target and off-target (3)**

| **HBB** | **RT region (14bp)** | **RT region mismatch** | **PBS mismatch Rate** | **HEK 293T** |
|---|---|---|---|---|
| | | | | **-4 A to T** |
| on-target | CTGAGGAGAAGTCT | 0 | 0% | Validated |
| HBB-off1 | CTGAGGAGAAGaCT | 1 | 7% | Validated |
| *HBB*-off2 | CTGtaGtcctagCT | 8 | 57% | False |
| *HBB-*off3 | CTGAGGcGAcagCt | 5 | 36% | Validated |
| *HBB*-off4 | CcaAGGcatgGTaT | 7 | 50% | False |
| *HBB-*off5 | CaGAGGctgtaTtc | 8 | 57% | False |

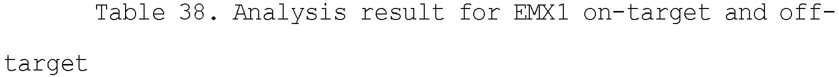

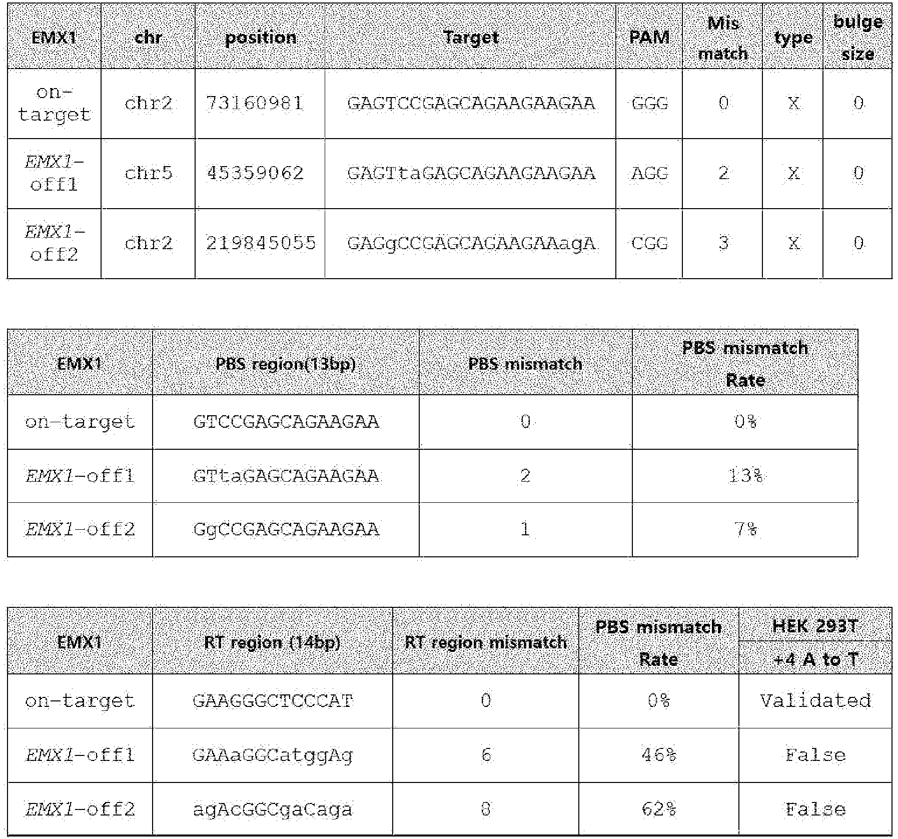

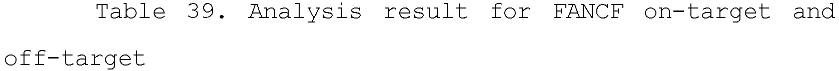

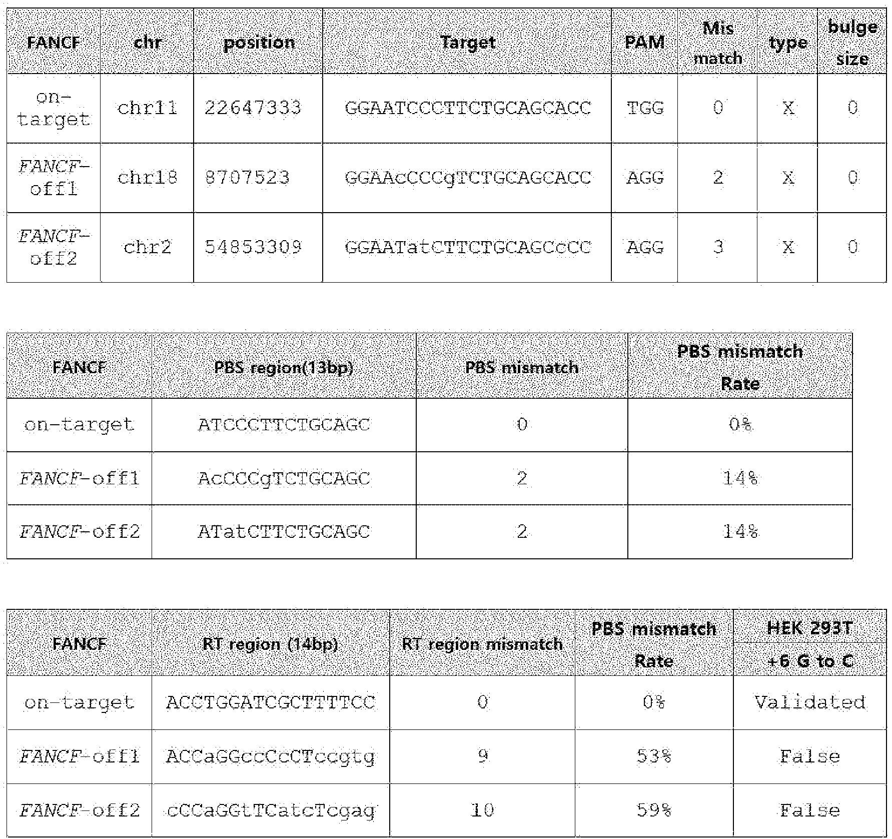

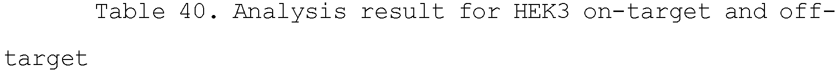

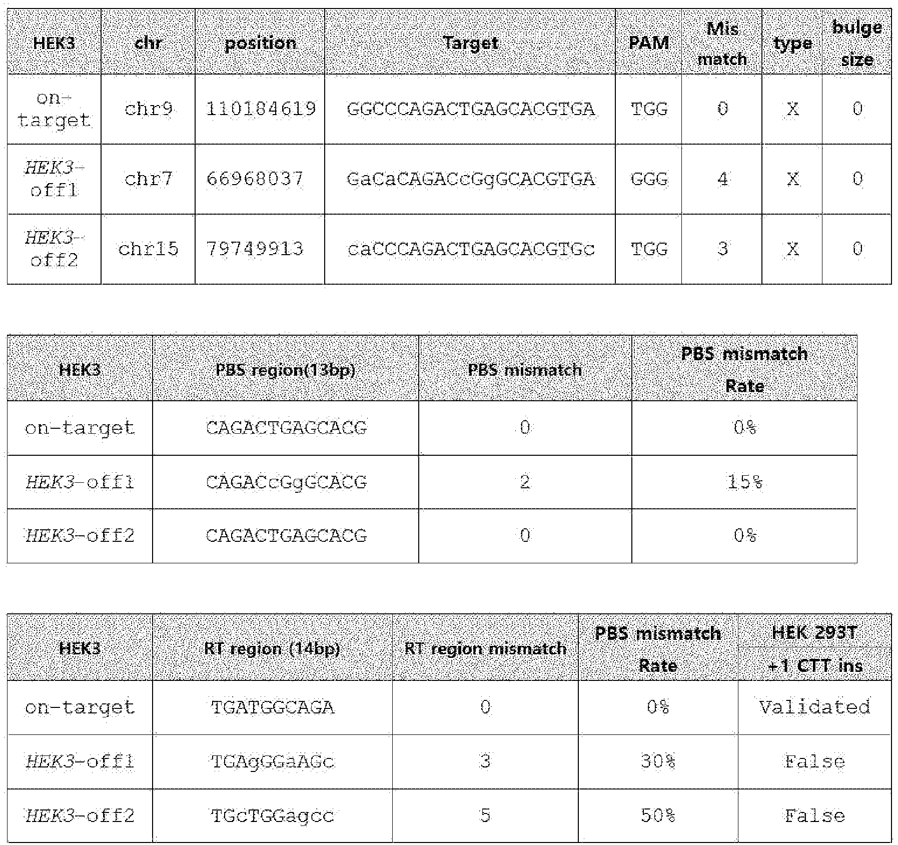

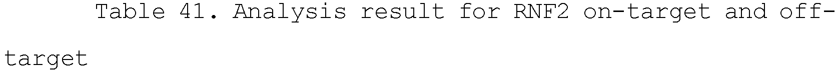

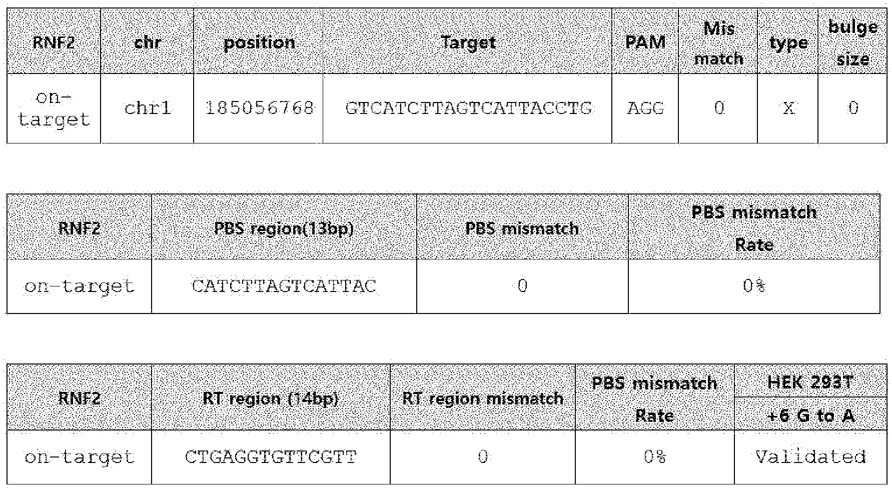

**Table 42-1. Analysis result for DNMT1 on-target and off-target (1)**

| **DNMT1** | **chr** | **position** | **Target** | **PAM** | **Mis match** | **type** | **bulge size** |
|---|---|---|---|---|---|---|---|
| on-target | chr19 | 10244300 | GATTCCTGGTGCCAGAAACA | GGG | 0 | X | 0 |
| *DNMT1-*off1 | chr2 | 121450805 | GATgtCTGGTGCCAGAAACA | AGG | 2 | X | 0 |
| *DNMT1-*off2 | chr10 | 74379333 | GATTCtTGaTGCCAGAAACt | GGG | 3 | X | 0 |
| *DNMT1-*off3 | chr7 | 89940115 | tATTCCTGGaGCCAGAAgCA | GGG | 3 | X | 0 |
| *DNMT1-*off4 | chr17 | 15641875 | ccTcCCTGGTGtCAGAAACA | AGG | 4 | X | 0 |
| *DNMT1-*off5 | chr1 | 97025552 | GgaaCCTGGTGCGAGAAACA | GAG | 3 | X | 0 |
| *DNMT1-*off6 | chr6 | 30333568 | ccgTCCTaGTGCCAGAAACA | TGG | 4 | X | 0 |
| *DNMT1-*off7 | chr22 | 34301627 | GAaaCtTGGTGCGAGAAACA | GGG | 3 | X | 0 |

**Table 42-2. Analysis result for DNMT1 on-target and off-target (2)**

| **DNMT1** | **PBS region(13bp)** | **PBS mismatch** | **PBS mismatch Rate** |
|---|---|---|---|
| on-target | CCTGGTGCCAGAA | 0 | 0% |
| *DNMT1*-off1 | tCTGGTGCCAGAA | 1 | 8% |
| *DNMT1*-off2 | CtTGaTGCCAGAA | 2 | 15% |
| *DNMT1-*off3 | CCTGGaGCCAGAA | 1 | 8% |
| *DNMT1-*off4 | CCTGGTGtCAGAA | 1 | 8% |
| *DNMT1*-off5 | CCTGGTGCCAGAA | 0 | 0% |
| *DNMT1-*off6 | CCTaGTGCCAGAA | 1 | 8% |
| *DNMT1*-off7 | CtTGGTGCCAGAA | 1 | 8% |

**Table 42-3. Analysis result for DNMT1 on-target and off-target (3)**

| **DNMT1** | **RT region (14bp)** | **RT region mismatch** | **PBS mismatch Rate** | **HEK 293T** |
|---|---|---|---|---|
| | | | | **+6 G to C** |
| on-target | ACAGGGGTGAC | 0 | 0% | Validated |
| *DNMT1*-off1 | ACAaGGGTGtt | 3 | 27% | Validated |
| *DNMT1-*off2 | ACtGGGtctta | 6 | 55% | False |
| *DNMT1-*off3 | gCAGGGcaact | 6 | 55% | False |
| *DNMT1*-off4 | ACAaGGtaaAa | 5 | 45% | False |
| *DNMT1-*off5 | ACAGaGacaAa | 5 | 45% | False |
| *DNMT1-*off6 | ACAtGGaaaAt | 5 | 45% | False |
| *DNMT1-*off7 | ACAGGGcattt | 5 | 45% | False |

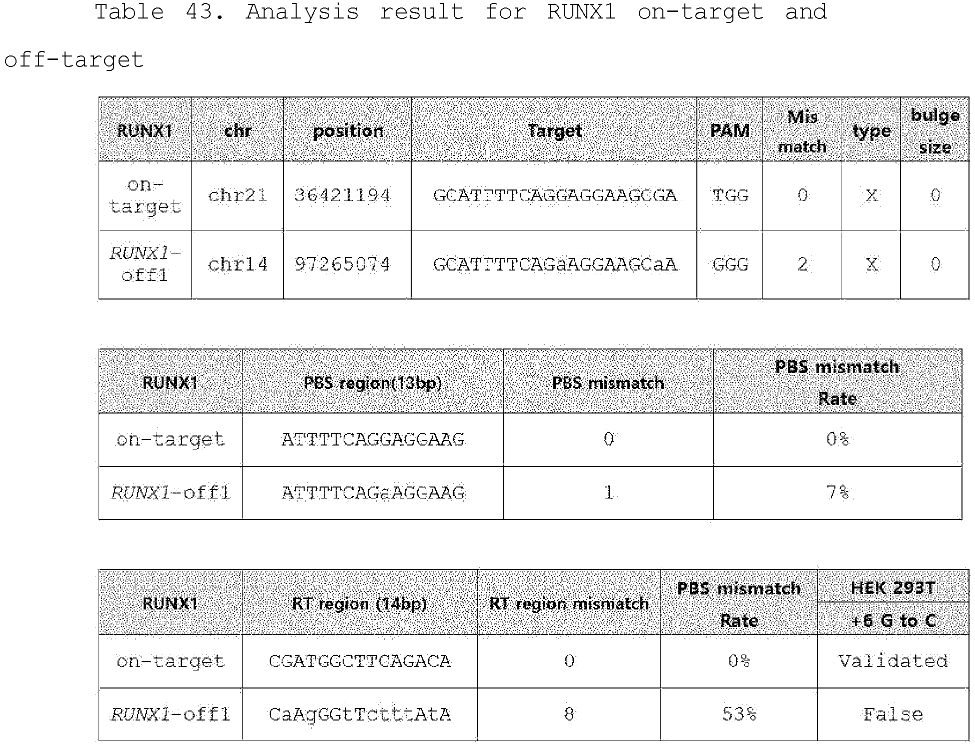

**Table 44-1. Analysis result for VEGFA on-target and off-target (1)**

| **VEGFA** | **chr** | **position** | **Target** | **PAM** | **Mismatch** | **type** | **bulge size** |
|---|---|---|---|---|---|---|---|
| on-target | chr6 | 43737145 | GATGTCTGCAGGCCAGATGA | GGG | 0 | X | 0 |
| *VEGFA-off1* | chr22 | 18303003 | acTGTCTGCAGGtCAGATcA | GGG | 4 | X | 0 |
| *VEGFA-off2* | chr12 | 1740975 | tAaGTCTGaAGGCCAGATGA | GGG | 3 | X | 0 |
| *VEGFA-off3* | chr6 | 1941601 | aATGTtTaCAGGCGAGATAGAG | GAG | 3 | DNA | 2 |
| *VEGFA-off4* | chr11 | 70158865 | aATGTCcGCAGcCCAGATGA | TGG | 3 | X | o |
| *VEGFA-off5* | chr22 | 49096508 | ccTGTCTGCAGGCCgGATGA | GAG | 3 | X | 0 |
| *VEGFA-off6* | chr16 | 89124143 | GATGTCTGCAGGaCAGATGc | AGG | 2 | X | 0 |
| *VEGFA-*off 7 | chr11 | 121009430 | GATGTCTGCAGGCCAGA-Gacg | AGG | 2 | RNA | 1 |
| *VEGFA-off8* | chrlO | 33838152 | GATGTCTGgAGGtCAGATGg | AGG | 3 | X | 0 |
| *VEGFA-off9* | chr2 | 99463359 | GgTGTgTGCAGGCCAGATcA | TGG | 3 | X | 0 |
| *VEGFA-off10* | chr20 | 57576982 | GgTGTCTGCAGGGCCAGATGc | TGG | 2 | DNA | 1 |
| *VEGFA*-*off11* | chr22 | 48197827 | tgTGTCTGCAGGCCAGAgGg | AGG | 4 | X | 0 |
| *VEGFA-off12* | chr6 | 72236965 | GATGTaTGgAGGCCAGAaGA | GGG | 3 | X | 0 |
| *VEGFA-off13* | chr8 | 59391471 | GtcaTCTGCAGGCCAGATGg | GGG | 4 | X | 0 |
| *VEGFA-off14* | chr20 | 60162096 | aATaTCTGCAGGtCAGAgGA | GGG | 4 | X | 0 |
| *VEGFA-off15* | chr8 | 145025899 | cccGTCTGCAGGCCAGATGGA | TGG | 3 | DNA | 1 |
| *VEGFA - off16* | chr2 | 50977332 | aATGaCTaCAGGtCAGATGA | GGG | 4 | X | 0 |

**Table 44-2. Analysis result for VEGFA on-target and off-target (2)**

| **VEGFA** | **PBS region(13bp)** | **PBS mismatch** | **PBS mismatch Rate** |
|---|---|---|---|
| on-target | TCTGCAGGCCAGA | 0 | 0% |
| *VEGFA-off1* | TCTGCAGGtCAGA | 1 | 8% |
| *VEGFA-off2* | TCTGaAGGCCAGA | 1 | 8% |
| *VEGFA-off3* | TtTaCAGGGCAGATA | mismatch 2 | - |
| | | DNA bulge 2bp | |
| *VEGFA-off4* | TCcGCAGcCCAGA | 2 | 15% |
| *VEGFA-off5* | TCTGCAGGCgGA | 1 | 8% |
| *VEGFA-off6* | TCTGCAGGaCAGA | 1 | 8% |
| *VEGFA-off7* | TCTGCAGGCCAGA-G | RNA bulge 1bp | - |
| | | DNA bulge 1bp | |
| *VEGFA-off8* | TCTGgAGGtCAGA | 2 | 15% |
| *VEGFA-off9* | TgTGCAGGCCAGA | 1 | 8% |
| *VEGFA-off10* | TCTGCAGGGCCAGA | DNA bulge 1bp | - |
| *VEGFA-off11* | TCTGCAGGCCAGA | 0 | 0% |
| *VEGFA-off12* | TaTGgAGGCCAGA | 2 | 15% |
| *VEGFA-off13* | TCTGCAGGCCAGA | 0 | 0% |
| *VEGFA-off14* | TCTGCAGGTCAGA | 1 | 8% |
| *VEGFA-off15* | TCTGCAGGCCAGAT | DNA bulge 1bp | - |
| *VEGFA-off16* | aCTaCAGGtCAGA | 3 | 23% |

**Table 44-3. Analysis result for VEGFA on-target and off-target (3)**

| **VEGFA** | **RT region (14bp)** | **RT region mismatch** | **PBS mismatch Rate** | **HEK 293T** |
|---|---|---|---|---|
| | | | | **++5 G to T** |
| on-target | TGAGGGCTCCAGATGGCACATT | 0 | 0% | Validate d |
| *VEGFA-off1* | TcAGGGagaaAGcTGGaAtATa | 9 | 41% | False |
| *VEGFA-off2* | TGAGGGgTtCAcAgcaCcatgg | 11 | 50% | False |
| *VEGFA-off3* | aGAGGaggaagcAgtGagggga | 18 | 82% | False |
| *VEGFA-off4* | TGAtGGCaatgGAaccaAaggc | 13 | 59% | False |
| *VEGFA-off5* | TGAGaGCTtgtctgGcagCtcT | 12 | 55% | False |
| *VEGFA-off6* | TGcaGGgTggAGggaGggtgcc | 14 | 64% | PCR fail |
| *VEGFA-off7* | acgaGGagtgcacaGagctccT | 18 | 82% | False |
| *VEGFA-off8* | TGgaGGgaaCAGtctGggagga | 14 | 64% | False |
| *VEGFA-off9* | TcAtGGCaaatGcaGtCACgaa | 12 | 55% | False |
| *VEGFA-off10* | TGctGGtgaCcGcctGgcCcTg | 13 | 59% | False |
| *VEGFA-off11* | gGgaGGgTgCcLggGGagttga | 7 | 32% | False |
| *VEGFA-off12* | aGAGGGgTagccccactctgaa | 16 | 73% | False |
| *VEGFA-off13* | TGgGGGCTgagGAatctgCtTc | 11 | 50% | False |
| *VEGFA-off14* | gGAGGGaTgtcagcaGaggcTg | 14 | 64% | False |
| *VEGFA-off15* | gGAtGGCTgCAGggtGgAagaa | 11 | 50% | False |
| *VEGFA-off16* | TGAGGGCTCtgaATGCtAtgTg | 7 | 32% | Validate d |

### Vector sequence

### Entire sequence of pRG2-pegRNA and each element included

### - Entire sequence

gacgaagactcaattgtcgattagtgaacggatctcgacggtatcgatcacg
agactagcctcgagcggccgcccccttcacc(1)gagggcctatttcccatgattcctt
catatttgcatatacgatacaaggctgttagagagataattggaattaatttgactgt
aaacacaaagatattagtacaaaatacgtgacgtagaaagtaataatttcttgggtag
tttgcagttttaaaattatgttttaaaatggactatcatatgcttaccgtaacttgaa
agtatttcgatttcttggctttatatatcttgtggaaaggacgaaaca(2)ccggagac
cacatttccccgaaaagtgccacctgacgtctaagaaaccattattatcatgacatta
acctataaaaataggcgtatcacgaggccctttcgtctcgcgcgtttcggtgatgacg
gtgaaaacctctgacacatgcagctcccggagacggtcacagcttgtctgtaagcgga
tgccgggagcagacaagcccgtcagggcgcgtcagcgggtgttggcgggtgtcggggc
tggcttaactatgcggcatcagagcagattgtactgagagtgcaccatatgcggtgtg
aaataccgcacagatgcgtaaggagaaaataccgcatcaggcgccattcgccattcag
gctgcgcaactgttgggaagggcgatcggtgcgggcctcttcgctattacgccagctg
gcgaaagggggatgtgctgcaaggcgattaagttgggtaacgccagggttttcccagt
cacgacgttgtaaaacgacggccagtgccaagcttgcatgcctgcaggtcgactctag
aggatccccgggtaccgagctcgaattcgtaatcatggtcatagctgtttcctgtgtg
aaattgttatccgctcacaattccacacaacatacgagccggaagcataaagtgtaaa
gcctggggtgcctaatagatccggtctcc(3)gttttagagctagaaatagcaagttaa
aataaggctagtccgttatcaacttgaaaaagtggcaccgagtcggtgcggagacg(4)
tgtctgtaagcggatgccgggagcagacaagcccgtcagggcgcgtcagcgggtgttg
gcgggtgtcggggctggcttaactatgcggcatcagagcagattgtactgagagtgca
ccatatgcggtgtgaaataccgcacagatgcgtaaggagaaaataccgcatcaggcgc
cattcgccattcaggctgcgcaactgttgggaagggcgatcggtgcgggcctcttcgc

tattacgccagctggcgaaagggggatgtgctgcaaggcgattaagttgggtaacgcc
agggttttcccagtcacgacgttgtaaaacgacggccagtgccaagcttgcatgcctg
caggtcgactctagaggatccccgggtaccgagctcgaattcgtaatcatggtcatag
ctgtttcctgtgtgaaattgttatccgctcacaattccacacaacatacgagccggcg
tctccctagattcgcgatgtacgggccagatatacgcgttgacattgattattgacta
gttgtcttcctgcattaatgaatcggccaacgcgcggggagaggcggtttgcgtattg
ggcgctcttccgcttcctcgctcactgactcgctgcgctcggtcgttcggctgcggcg
agcggtatcagctcactcaaaggcggtaatacggttatccacagaatcaggggataac
gcaggaaagaac⁽⁵⁾atgtgagcaaaaggccagcaaaaggccaggaaccgtaaaaaggc
cgcgttgctggcgtttttccataggctccgcccccctgacgagcatcacaaaaatcga
cgctcaagtcagaggtggcgaaacccgacaggactataaagataccaggcgtttcccc
ctggaagctccctcgtgcgctctcctgttccgaccctgccgcttaccggatacctgtc
cgcctttctcccttcgggaagcgtggcgctttctcaatgctcacgctgtaggtatctc
agttcggtgtaggtcgttcgctccaagctgggctgtgtgcacgaaccccccgttcagc
ccgaccgctgcgccttatccggtaactatcgtcttgagtccaacccggtaagacacga
cttatcgccactggcagcagccactggtaacaggattagcagagcgaggtatgtaggc
ggtgctacagagttcttgaagtggtggcctaactacggctacactagaaggacagtat
ttggtatctgcgctctgctgaagccagttaccttcggaaaaagagttggtagctcttg
atccggcaaacaaaccaccgctggtagcggtggtttttttgtttgcaagcagcagatt
acgcgcagaaaaaaaggatctcaagaagatcctttgatcttttctacggggtctgacg
ctcagtggaacgaaaactcacgttaagggattttggtcatgagattatcaaaaaggat
cttcacctagatccttttaaattaaaaatgaagttttaaatcaatctaaagtatatat
gagtaaacttggtctgacag⁽⁶⁾ttaccaatgcttaatcagtgaggcacctatctcagc
gatctgtctatttcgttcatccatagttgcctgactccccgtcgtgtagataactacg
atacgggagggcttaccatctggccccagtgctgcaatgataccgcgagatccacgct

caccggctccagatttatcagcaataaaccagccagccggaagggccgagcgcagaag

tggtcctgcaactttatccgcctccatccagtctattaattgttgccgggaagctaga

gtaagtagttcgccagttaatagtttgcgcaacgttgttgccattgctacaggcatcg

tggtgtcacgctcgtcgtttggtatggcttcattcagctccggttcccaacgatcaag

gcgagttacatgatcccccatgttgtgcaaaaaagcggttagctccttcggtcctccg

atcgttgtcagaagtaagttggccgcagtgttatcactcatggttatggcagcactgc

ataattctcttactgtcatgccatccgtaagatgcttttctgtgactggtgagtactc

aaccaagtcattctgagaatagtgtatgcggcgaccgagttgctcttgcccggcgtca

atacgggataataccgcgccacatagcagaactttaaaagtgctcatcattggaaaac

gttcttcggggcgaaaactctcaaggatcttaccgctgttgagatccagttcgatgta

acccactcgtgcacccaactgatcttcagcatcttttactttcaccagcgtttctggg

tgagcaaaaacaggaaggcaaaatgccgcaaaaaagggaataagggcgacacggaaat

gttgaatactcatactcttcctttttcaatattattgaagcatttatcagggttattg

tctcatgagcggatacatatttgaatgtatttagaaaaataaacaaataggggttccg

cgcacatttccccgaaaagtgccacctgacgtc (SEQ ID NO: 146)

- (1) U6 Promoter; (2) Stuffer; (3) sgRNA scaffold; (4) pUC origin; (5) stuffer; (6) Amp resistance

### Entire sequence of pAllinl-PE2 and each element included

### - Entire sequence

       cgatgtacgggccagatatacgcgcgtatatctggcccgtacatcgcgaatc
tag(1)AAAAANNNNNNNNNNNNNNNNNNNNNNNNNNNNNNgcaccgactcggtgccac
tttttcaagttgataacggactagccttattttaacttgctatttctagctctaaaac

NNNNNNNNNNNNNNNNNNNNcggtgtttc(2)gtcctttccacaagatatataaagccaa
gaaatcgaaatactttcaagttacggtaagcatatgatagtccattttaaaacataat
tttaaaactgcaaactacccaagaaattattactttctacgtcacgtattttgtacta
atatctttgtgtttacagtcaaattaattccaattatctctctaacagccttgtatcg
tatatgcaaatatgaaggaatcatgggaaataggccctccgcgtt(3)gacattgatta
ttgactagttattaatagtaatcaattacggggtcattagttcatagcccatatatgg
agttccgcgttacataacttacggtaaatggcccgcctggctgaccgcccaacgaccc
ccgcccattgacgtcaataatgacgtatgttcccatagtaacgccaatagggactttc
cattgacgtcaatgggtggagtatttacggtaaactgcccacttggcagtacatcaag
tgtatcatatgccaagtacgccccctattgacgtcaatgacggtaaatggcccgcctg
gcattatgcccagtacatgaccttatgggactttcctacttggcagtacatctacgta
ttagtcatcgctattaccatggtgatgcggttttggcagtacatcaatgggcgtggat
agcggtttgactcacggggatttccaagtctccaccccattgacgtcaatgggagttt
gttttggcaccaaaatcaacgggactttccaaaatgtcgtaacaactccgccccattg
acgcaaatgggcggtaggcgtgtacggtgggaggtctatataagcagagctggtttag
tgaaccgtcagatccgctagagatccgcggccgctaatacgactcactatagggagag
ccgccaccatgaaacggacagccgacggaagcgagttcgagtca(4)ccaggacatcaa
aagggacacagcgccgaggctagaggcaaccggatggctgaccaagcggcccgaaagg
cagccatcacagagactccagacacctctaccctcctcatagaaaattcatcaccctc
tggcggctcaaaaagaaccgccgacggcagcgaattcgagcccaagaagaagaggaaa
gtctaaccggtcatcatcaccatcaccattgagttttacccctacgacgtgcccgact
acgcctaataactcgagcatgcatctagagggccctattctatagtgtcacctaaatg
ctagagctcgctgatcagcctcgactgtgccttctagttgccagccatctgttgtttg
cccctcccccgtgccttccttgaccctggaaggtgccactcccactgtcctttcctaa
taaaatgaggaaattgcatcgcattgtctgagtaggtgtcattctattctggggggtg

gggtggggcaggacagcaagggggaggattgggaagacaatagcaggcatgctgggga
(5)ggatctgcgatcgctccggtgcccgtcagtgggcagagcgcacatcgcccacagtc
cccgagaagttggggggaggggtcggcaattgaacgggtgcctagagaaggtggcgcg
gggtaaactgggaaagtgatgtcgtgtactggctccgcctttttcccgagggtggggg
agaaccgtatataagtgcagtagtcgccgtgaacgttctttttcgcaacgggtttgcc
gccagaacacagctgaagcttcgaggggctcgcatctctccttcacgcgcccgccgcc
ctacctgaggccgccatccacgccggttgagtcgcgttctgccgcctcccgcctgtgg
tgcctcctgaactgcgtccgccgtctaggtaagtttaaagctcaggtcgagaccgggc
ctttgtccggcgctcccttggagcctacctagactcagccggctctccacgctttgcc
tgaccctgcttgctcaactctacgtctttgtttcgttttctgttctgcgccgttacag
atccaagctgtgaccggcgcctac(6)ATGACCGAGTACAAGCCCACGGTGCGCCTCGC
CACCCGCGACGACGTCCCCAGGGCCGTACGCACCCTCGCCGCCGCGTTCGCCGACTAC
CCCGCCACGCGCCACACCGTCGATCCAGACCGCCACATCGAGCGGGTCACCGAGCTGC
AAGAACTCTTCCTCACGCGCGTCGGGCTCGACATCGGCAAGGTGTGGGTCGCGGACGA
CGGCGCCGCGGTGGCGGTCTGGACCACGCCGGAGAGCGTCGAAGCGGGGGCGGTGTTC
GCCGAGATCGGCCCGCGCATGGCCGAGTTGAGCGGTTCCCGGCTGGCCGCGCAGCAAC
AGATGGAAGGTCTCCTGGCGCCGCACCGGCCCAAGGAGCCCGCGTGGTTCCTGGCCAC
CGTCGGCGTCTCGCCCGACCACCAGGGCAAGGGTCTGGGCAGCGCCGTCGTGCTCCCC
GGAGTGGAGGCGGCCGAGCGCGCCGGGGTGCCCGCCTTCCTGGAGACCTCCGCGCCCC
GCAACCTCCCCTTCTACGAGCGGCTCGGCTTCACCGTCACCGCCGACGTCGAGGTGCC
CGAAGGACCGCGCACCTGGTGCATGACCCGCAAGCCCGGTGCCTGAccatccgtaaga
tgcttttctgtgactggtgagtactcaaccaagtcattctgagaatagtgtatgcggc
gaccgagttgctcttgcccggcgtcaatacgggataataccgcgccacatagcagaac
tttaaaagtgctcatcattggaaaacgttcttcggggcgaaaactctcaaggatctta
ccgctgttgagatccagttcgatgtaacccactcgtgcacccaactgatcttcagcat

cttttactttcaccagcgtttctgggtgagcaaaaacaggaaggcaaaatgccgcaaa
aaagggaataagggcgacacggaaatgttgaatactcatactcttcctttttcaatat
tattgaagcatttatcagggttattgtctcatgagcggatacatatttgaatgtattt
agaaaaataaacaaataggggttccgcgaacttgtttattgcagcttataatggttac
aaataaagcaatagcatcacaaaaatttcacaaataaagcatttttttcactgcattcta
gttgtggtttgtccaaactcatcaatgtatcttatcatgtctgg(7)ccagctagccaa
caagctcgtcatcgctttgcagaagagcagagaggatatgctcatcgtctaaagaact
acccatttattatatattagtcacctcctgttccgaccctgccgcttaccggatacc
tgtccgcctttctcccttcgggaagcgtggcgctttctcatagctcacgctgtaggta
tctcagttcggtgtaggtcgttcgctccaagctgggctgtgtgcacgaaccccccgtt
cagcccgaccgctgcgccttatccggtaactatcgtcttgagtccaacccggtaagac
acgacttatcgccactggcagcagccactggtaacaggattagcagagcgaggtatgt
aggcggtgctacagagttcttgaagtggtggcctaactacggctacactagaaggaca
gtatttggtatctgcgctctgctgaagccagttaccttcggaaaaagagttggtagct
cttgatccggcaaacaaaccaccgctggtagcggtggtttttttgtttgcaagcagca
gattacgcgcagaaaaaaaggatctcaagaagatcctttgatcttttctacggggtct
gacgctcagtggaacgaaaactcacgttaagggattttggtcatgattaaccctagaa
agataatcatattgtgacgtacgttaaagataatcatgcgtaaaattgacgcatgtgt
tttatcggtctgtatatcgaggtttatttattaatttgaatagatattaagttttatt
atatttacacttacatactaataataaattcaacaaacaatttatttatgtttattta
tttattaaaaaaaaacaaaaactcaaaatttcttctataaagtaacaaa(8)actttta
aacattctctcttttacaaaaataaacttattttgtactttaaaaacagtcatgttgt
attataaaataagtaattagcttaacctatacataatagaaacaaattatacttatta
gtcagtcagaaacaaCTTTGGCACATATCAATATTATGCTCTCGTTAATTAA (SEQ
ID NO: 147)

- (1) pegRNA; (2) U6 Promoter; (3) CMV Promoter; (4) PE2; (5) EFlalpha Promoter; (6) Puromycin resistance; (7) ITR; (8) ITR

### Entire sequence of piggy-PE2 and each element included

### - Entire sequence

CGATGTACGGGCCAGATATACGCGTT(1)GACATTGATTATTGACTAGTTATT
AATAGTAATCAATTACGGGGTCATTAGTTCATAGCCCATATATGGAGTTCCGCGTTAC
ATAACTTACGGTAAATGGCCCGCCTGGCTGACCGCCCAACGACCCCCGCCCATTGACG
TCAATAATGACGTATGTTCCCATAGTAACGCCAATAGGGACTTTCCATTGACGTCAAT
GGGTGGAGTATTTACGGTAAACTGCCCACTTGGCAGTACATCAAGTGTATCATATGCC
AAGTACGCCCCCTATTGACGTCAATGACGGTAAATGGCCCGCCTGGCATTATGCCCAG
TACATGACCTTATGGGACTTTCCTACTTGGCAGTACATCTACGTATTAGTCATCGCTA
TTACCATGGTGATGCGGTTTTGGCAGTACATCAATGGGCGTGGATAGCGGTTTGACTC
ACGGGGATTTCCAAGTCTCCACCCCATTGACGTCAATGGGAGTTTGTTTTGGCACCAA
AATCAACGGGACTTTCCAAAATGTCGTAACAACTCCGCCCCATTGACGCAAATGGGCG
GTAGGCGTGTACGGTGGGAGGTCTATATAAGCAGAGCTGGTTTAGTGAACCGTCAGAT
CCGCTAGAGATCCGCGGCCGCTAATACGACTCACTATAGGGAGAGCCGCCACCATGAA
ACGGACAGCCGACGGAAGCGAGTTCGAGTCA(2)CCAGGACATCAAAAGGGACACAGCG
CCGAGGCTAGAGGCAACCGGATGGCTGACCAAGCGGCCCGAAAGGCAGCCATCACAGA
GACTCCAGACACCTCTACCCTCCTCATAGAAAATTCATCACCCTCTGGCGGCTCAAAA
AGAACCGCCGACGGCAGCGAATTCGAGCCCAAGAAGAAGAGGAAAGTCTAACCGGTCA
TCATCACCATCACCATTGAGTTTTACCCCTACGACGTGCCCGACTACGCCTAATAACT
CGAGCATGCATCTAGAGGGCCCTATTCTATAGTGTCACCTAAATGCTAGAGCTCGCTG

ATCAGCCTCGACTGTGCCTTCTAGTTGCCAGCCATCTGTTGTTTGCCCCTCCCCCGTG
CCTTCCTTGACCCTGGAAGGTGCCACTCCCACTGTCCTTTCCTAATAAAATGAGGAAA
TTGCATCGCATTGTCTGAGTAGGTGTCATTCTATTCTGGGGGGTGGGGTGGGGCAGGA
CAGCAAGGGGGAGGATTGGGAAGACAATAGCAGGCATGCTGGGGA(3)GGATCTGCGAT
CGCTCCGGTGCCCGTCAGTGGGCAGAGCGCACATCGCCCACAGTCCCCGAGAAGTTGG
GGGGAGGGGTCGGCAATTGAACGGGTGCCTAGAGAAGGTGGCGCGGGGTAAACTGGGA
AAGTGATGTCGTGTACTGGCTCCGCCTTTTTCCCGAGGGTGGGGGAGAACCGTATATA
AGTGCAGTAGTCGCCGTGAACGTTCTTTTTCGCAACGGGTTTGCCGCCAGAACACAGC
TGAAGCTTCGAGGGGCTCGCATCTCTCCTTCACGCGCCCGCCGCCCTACCTGAGGCCG
CCATCCACGCCGGTTGAGTCGCGTTCTGCCGCCTCCCGCCTGTGGTGCCTCCTGAACT
GCGTCCGCCGTCTAGGTAAGTTTAAAGCTCAGGTCGAGACCGGGCCTTTGTCCGGCGC
TCCCTTGGAGCCTACCTAGACTCAGCCGGCTCTCCACGCTTTGCCTGACCCTGCTTGC
TCAACTCTACGTCTTTGTTTCGTTTTCTGTTCTGCGCCGTTACAGATCCAAGCTGTGA
CCGGCGCCTAC(4)ATGACCGAGTACAAGCCCACGGTGCGCCTCGCCACCCGCGACGAC
GTCCCCAGGGCCGTACGCACCCTCGCCGCCGCGTTCGCCGACTACCCCGCCACGCGCC
ACACCGTCGATCCAGACCGCCACATCGAGCGGGTCACCGAGCTGCAAGAACTCTTCCT
CACGCGCGTCGGGCTCGACATCGGCAAGGTGTGGGTCGCGGACGACGGCGCCGCGGTG
GCGGTCTGGACCACGCCGGAGAGCGTCGAAGCGGGGATAAACCAGCCAGCCGGAAGGG
CCGAGCGCAGAAGTGGTCCTGCAACTTTATCCGCCTCCATCCAGTCTATTAATTGTTG
CCGGGAAGCTAGAGTAAGTAGTTCGCCAGTTAATAGTTTGCGCAACGTTGTTGCCATT
GCTACAGGCATCGTGGTGTCACGCTCGTCGTTTGGTATGGCTTCATTCAGCTCCGGTT
CCCAACGATCAAGGCGAGTTACATGATCCCCCATGTTGTGCAAAAAAGCGGTTAGCTC
CTTCGGTCCTCCGATCGTTGTCAGAAGTAAGTTGGCCGCAGTGTTATCACTCATGGTT
ATGGCAGCACTGCATAATTCTCTTACTGTCATGCCATCCGTAAGATGCTTTTCTGTGA
CTGGTGAGTACTCAACCAAGTCATTCTGAGAATAGTGTATGCGGCGACCGAGTTGCTC

TTGCCCGGCGTCAATACGGGATAATACCGCGCCACATAGCAGAACTTTAAAAGTGCTC
ATCATTGGAAAACGTTCTTCGGGGCGAAAACTCTCAAGGATCTTACCGCTGTTGAGAT
CCAGTTCGATGTAACCCACTCGTGCACCCAACTGATCTTCAGCATCTTTTACTTTCAC
CAGCGTTTCTGGGTGAGCAAAAACAGGAAGGCAAAATGCCGCAAAAAAGGGAATAAGG
GCGACACGGAAATGTTGAATACTCATACTCTTCCTTTTTCAATATTATTGAAGCATTT
ATCAGGGTTATTGTCTCATGAGCGGATACATATTTGAATGTATTTAGAAAAATAAACA
AATAGGGGTTCCGCGAACTTGTTTATTGCAGCTTATAATGGTTACAAATAAAGCAATA
GCATCACAAAAATTTCACAAATAAAGCATTTTTTTCACTGCATTCTAGTTGTGGTTTGTC
CAAACTCATCAATGTATCTTATCATGTCTGG(5)CCAGCTAGCCAACAAGCTCGTCATC
GCTTTGCAGAAGAGCAGAGAGGATATGCTCATCGTCTAAAGAACTACCCATTTTATTA
TATATTAGTCACCTCCTGTTCCGACCCTGCCGCTTACCGGATACCTGTCCGCCTTTCT
CCCTTCGGGAAGCGTGGCGCTTTCTCATAGCTCACGCTGTAGGTATCTCAGTTCGGTG
TAGGTCGTTCGCTCCAAGCTGGGCTGTGTGCACGAACCCCCCGTTCAGCCCGACCGCT
GCGCCTTATCCGGTAACTATCGTCTTGAGTCCAACCCGGTAAGACACGACTTATCGCC
ACTGGCAGCAGCCACTGGTAACAGGATTAGCAGAGCGAGGTATGTAGGCGGTGCTACA
GAGTTCTTGAAGTGGTGGCCTAACTACGGCTACACTAGAAGGACAGTATTTGGTATCT
GCGCTCTGCTGAAGCCAGTTACCTTCGGAAAAAGAGTTGGTAGCTCTTGATCCGGCAA
ACAAACCACCGCTGGTAGCGGTGGTTTTTTTGTTTGCAAGCAGCAGATTACGCGCAGA
AAAAAAGGATCTCAAGAAGATCCTTTGATCTTTTCTACGGGGTCTGACGCTCAGTGGA
ACGAAAACTCACGTTAAGGGATTTTGGTCATGATTAACCCTAGAAAGATAATCATATT
GTGACGTACGTTAAAGATAATCATGCGTAAAATTGACGCATGTGTTTTATCGGTCTGT
ATATCGAGGTTTATTTATTAATTTGAATAGATATTAAGTTTTATTATATTTACACTTA
CATACTAATAATAAATTCAACAAACAATTTATTTATGTTTATTTATTTATTAAAAAAA
AACAAAAACTCAAAATTTCTTCTATAAAGTAACAAA(6)ACTTTTAAACATTCTCTCTT
TTACAAAAATAAACTTATTTTGTACTTTAAAAACAGTCATGTTGTATTATAAAATAAG

TAATTAGCTTAACCTATACATAATAGAAACAAATTATACTTATTAGTCAGTCAGAAAC

AACTTTGGCACATATCAATATTATGCTCTCGTTAATTAA (SEQ ID NO: 148)

- (1) CMV Promoter; (2) PE2; (3) EFlalpha Promoter; (4) Puromycin resistance (5) ITR (6) ITR

### Entire sequence of pRG2-epegRNA and each element included

### - Entire sequence

GACGAAGACTCAATTGTCGATTAGTGAACGGATCTCGACGGTATCGATCACG
AGACTAGCCTCGAGCGGCCGCCCCCTTCACC(1)GAGGGCCTATTTCCCATGATTCCTT
CATATTTGCATATACGATACAAGGCTGTTAGAGAGATAATTGGAATTAATTTGACTGT
AAACACAAAGATATTAGTACAAAATACGTGACGTAGAAAGTAATAATTTCTTGGGTAG
TTTGCAGTTTTAAAATTATGTTTTAAAATGGACTATCATATGCTTACCGTAACTTGAA
AGTATTTCGATTTCTTGGCTTTATATATCTTGTGGAAAGGACGAAACA(2)CCGGAGAC
CACATTTCCCCGAAAAGTGCCACCTGACGTCTAAGAAACCATTATTATCATGACATTA
ACCTATAAAAATAGGCGTATCACGAGGCCCTTTCGTCTCGCGCGTTTCGGTGATGACG
GTGAAAACCTCTGACACATGCAGCTCCCGGAGACGGTCACAGCTTGTCTGTAAGCGGA
TGCCGGGAGCAGACAAGCCCGTCAGGGCGCGTCAGCGGGTGTTGGCGGGTGTCGGGGC
TGGCTTAACTATGCGGCATCAGAGCAGATTGTACTGAGAGTGCACCATATGCGGTGTG
AAATACCGCACAGATGCGTAAGGAGAAAATACCGCATCAGGCGCCATTCGCCATTCAG
GCTGCGCAACTGTTGGGAAGGGCGATCGGTGCGGGCCTCTTCGCTATTACGCCAGCTG
GCGAAAGGGGGATGTGCTGCAAGGCGATTAAGTTGGGTAACGCCAGGGTTTTCCCAGT
CACGACGTTGTAAAACGACGGCCAGTGCCAAGCTTGCATGCCTGCAGGTCGACTCTAG
AGGATCCCCGGGTACCGAGCTCGAATTCGTAATCATGGTCATAGCTGTTTCCTGTGTG
AAATTGTTATCCGCTCACAATTCCACACAACATACGAGCCGGAAGCATAAAGTGTAAA

GCCTGGGGTGCCTAATAGATCCGGTCTCC⁽³⁾GTTTTAGAGCTAGAAATAGCAAGTTAA
AATAAGGCTAGTCCGTTATCAACTTGAAAAAGTGGCACCGAGTCGGTGCGGAGACG⁽⁴⁾
TGTCTGTAAGCGGATGCCGGGAGCAGACAAGCCCGTCAGGGCGCGTCAGCGGGTGTTG
GCGGGTGTCGGGGCTGGCTTAACTATGCGGCATCAGAGCAGATTGTACTGAGAGTGCA
CCATATGCGGTGTGAAATACCGCACAGATGCGTAAGGAGAAAATACCGCATCAGGCGC
CATTCGCCATTCAGGCTGCGCAACTGTTGGGAAGGGCGATCGGTGCGGGCCTCTTCGC
TATTACGCCAGCTGGCGAAAGGGGGATGTGCTGCAAGGCGATTAAGTTGGGTAACGCC
AGGGTTTTCCCAGTCACGACGTTGTAAAACGACGGCCAGTGCCAAGCTTGCATGCCTG
CAGGTCGACTCTAGAGGATCCCCGGGTACCGAGCTCGAATTCGTAATCATGGTCATAG
CTGTTTCCTGTGTGAAATTGTTATCCGCTCACAATTCCACACAACATACGAGCCGGCG
TCTCC⁽⁵⁾CGCGGTTCTATCTAGTTACGCGTTAAACCAACTAGAATTTTTTAGATATAC
GCGTTGACATTGATTATTGACTAGTTGTCTTCCTGCATTAATGAATCGGCCAACGCGC
GGGGAGAGGCGGTTTGCGTATTGGGCGCTCTTCCGCTTCCTCGCTCACTGACTCGCTG
CGCTCGGTCGTTCGGCTGCGGCGAGCGGTATCAGCTCACTCAAAGGCGGTAATACGGT
TATCCACAGAATCAGGGGATAACGCAGGAAAGAAC⁽⁶⁾ATGTGAGCAAAAGGCCAGCAA
AAGGCCAGGAACCGTAAAAAGGCCGCGTTGCTGGCGTTTTTCCATAGGCTCCGCCCCC
CTGACGAGCATCACAAAAATCGACGCTCAAGTCAGAGGTGGCGAAACCCGACAGGACT
ATAAAGATACCAGGCGTTTCCCCCTGGAAGCTCCCTCGTGCGCTCTCCTGTTCCGACC
CTGCCGCTTACCGGATACCTGTCCGCCTTTCTCCCTTCGGGAAGCGTGGCGCTTTCTC
AATGCTCACGCTGTAGGTATCTCAGTTCGGTGTAGGTCGTTCGCTCCAAGCTGGGCTG
TGTGCACGAACCCCCCGTTCAGCCCGACCGCTGCGCCTTATCCGGTAACTATCGTCTT
GAGTCCAACCCGGTAAGACACGACTTATCGCCACTGGCAGCAGCCACTGGTAACAGGA
TTAGCAGAGCGAGGTATGTAGGCGGTGCTACAGAGTTCTTGAAGTGGTGGCCTAACTA
CGGCTACACTAGAAGGACAGTATTTGGTATCTGCGCTCTGCTGAAGCCAGTTACCTTC
GGAAAAAGAGTTGGTAGCTCTTGATCCGGCAAACAAACCACCGCTGGTAGCGGTGGTT

TTTTTGTTTGCAAGCAGCAGATTACGCGCAGAAAAAAAGGATCTCAAGAAGATCCTTT

GATCTTTTCTACGGGGTCTGACGCTCAGTGGAACGAAAACTCACGTTAAGGGATTTTG

GTCATGAGATTATCAAAAAGGATCTTCACCTAGATCCTTTTAAATTAAAAATGAAGTT

TTAAATCAATCTAAAGTATATATGAGTAAACTTGGTCTGACAG(7)TTACCAATGCTTA

ATCAGTGAGGCACCTATCTCAGCGATCTGTCTATTTCGTTCATCCATAGTTGCCTGAC

TCCCCGTCGTGTAGATAACTACGATACGGGAGGGCTTACCATCTGGCCCCAGTGCTGC

AATGATACCGCGAGATCCACGCTCACCGGCTCCAGATTTATCAGCAATAAACCAGCCA

GCCGGAAGGGCCGAGCGCAGAAGTGGTCCTGCAACTTTATCCGCCTCCATCCAGTCTA

TTAATTGTTGCCGGGAAGCTAGAGTAAGTAGTTCGCCAGTTAATAGTTTGCGCAACGT

TGTTGCCATTGCTACAGGCATCGTGGTGTCACGCTCGTCGTTTGGTATGGCTTCATTC

AGCTCCGGTTCCCAACGATCAAGGCGAGTTACATGATCCCCCATGTTGTGCAAAAAAG

CGGTTAGCTCCTTCGGTCCTCCGATCGTTGTCAGAAGTAAGTTGGCCGCAGTGTTATC

ACTCATGGTTATGGCAGCACTGCATAATTCTCTTACTGTCATGCCATCCGTAAGATGC

TTTTCTGTGACTGGTGAGTACTCAACCAAGTCATTCTGAGAATAGTGTATGCGGCGAC

CGAGTTGCTCTTGCCCGGCGTCAATACGGGATAATACCGCGCCACATAGCAGAACTTT

AAAAGTGCTCATCATTGGAAAACGTTCTTCGGGGCGAAAACTCTCAAGGATCTTACCG

CTGTTGAGATCCAGTTCGATGTAACCCACTCGTGCACCCAACTGATCTTCAGCATCTT

TTACTTTCACCAGCGTTTCTGGGTGAGCAAAAACAGGAAGGCAAAATGCCGCAAAAAA

GGGAATAAGGGCGACACGGAAATGTTGAATACTCATACTCTTCCTTTTTCAATATTAT

TGAAGCATTTATCAGGGTTATTGTCTCATGAGCGGATACATATTTGAATGTATTTAGA

AAAATAAACAAATAGGGGTTCCGCGCACATTTCCCCGAAAAGTGCCACCTGACGTC

(SEQ ID NO: 149)

- (1) U6 promoter; (2) Stuffer; (3) sgRNA scaffold; (4) Stuffer; (5) tevopreQ1; (6) pUC origin; (7) Amp resistance

### Entire sequence of pAllinl-PE4 and each element included

### - Entire sequence

CGATGTACGGGCCAGATATACGCGCGTATATCTGGCCCGTACATCGCGAATC
TAG(1)AAAAANNNNNNNNNNNNNNNNNNNNNNNNNNNGCACCGACTCGGTGCCAC
TTTTTCAAGTTGATAACGGACTAGCCTTATTTTAACTTGCTATTTCTAGCTCTAAAAC
NNNNNNNNNNNNNNNNNNNCGGTGTTTC(2)GTCCTTTCCACAAGATATATAAAGCCAA
GAAATCGAAATACTTTCAAGTTACGGTAAGCATATGATAGTCCATTTTAAAACATAAT
TTTAAAACTGCAAACTACCCAAGAAATTATTACTTTCTACGTCACGTATTTTGTACTA
ATATCTTTGTGTTTACAGTCAAATTAATTCCAATTATCTCTCTAACAGCCTTGTATCG
TATATGCAAATATGAAGGAATCATGGGAAATAGGCCCTCCGCGTT(3)GACATTGATTA
TTGACTAGTTATTAATAGTAATCAATTACGGGGTCATTAGTTCATAGCCCATATATGG
AGTTCCGCGTTACATAACTTACGGTAAATGGCCCGCCTGGCTGACCGCCCAACGACCC
CCGCCCATTGACGTCAATAATGACGTATGTTCCCATAGTAACGCCAATAGGGACTTTC
CATTGACGTCAATGGGTGGAGTATTTACGGTAAACTGCCCACTTGGCAGTACATCAAG
TGTATCATATGCCAAGTACGCCCCCTATTGACGTCAATGACGGTAAATGGCCCGCCTG
GCATTATGCCCAGTACATGACCTTATGGGACTTTCCTACTTGGCAGTACATCTACGTA
TTAGTCATCGCTATTACCATGGTGATGCGGTTTTGGCAGTACATCAATGGGCGTGGAT
AGCGGTTTGACTCACGGGGATTTCCAAGTCTCCACCCCATTGACGTCAATGGGAGTTT
GTTTTGGCACCAAAATCAACGGGACTTTCCAAAATGTCGTAACAACTCCGCCCCATTG
ACGCAAATGGGCGGTAGGCGTGTACGGTGGGAGGTCTATATAAGCAGAGCTGGTTTAG
TGAACCGTCAGATCCGCTAGAGATCCGCGGCCGCTAATACGACTCACTATAGGGAGAG
CCGCCACC(4)GGCCATGCTGGCTCTGGATAGCCCAGAAAGCGGCTGGACAGAAGAGGA
CGGACCTAAAGAGGGGCTGGCTGAATACATCGTGGAGTTCCTGAAGAAAAAGGCCGAG

ATGCTGGCCGACTACTTTTCTCTGGAAATCGACGAGGAAGGCAACCTGATCGGCCTGC
CTCTGCTGATCGATAACTACGTGCCTCCCCTGGAAGGCCTGCCCATCTTCATCCTGAG
ACTGGCTACAGAGGTGAACTGGGACGAGGAAAAGGAATGCTTCGAGTCTCTGAGCAAG
GAGTGCGCCATGTTCTATAGCATCAGAAAACAGTACATCTCTGAAGAGAGCACTCTGT
CTGGCCAGCAGAGTGAAGTGCCCGGAAGCATCCCCAACAGCTGGAAGTGGACCGTGGA
ACACATCGTGTACAAGGCCCTGCGGAGCCACATTCTCCCTCCTAAGCACTTCACCGAG
GACGGCAACATCCTGCAGCTGGCCAACCTGCCCGACCTTTATAAGGTTTTCTAACTCG
AGCATGCATCTAGAGGGCCCTATTCTATAGTGTCACCTAAATGCTAGAGCTCGCTGAT
CAGCCTCGACTGTGCCTTCTAGTTGCCAGCCATCTGTTGTTTGCCCCTCCCCCGTGCC
TTCCTTGACCCTGGAAGGTGCCACTCCCACTGTCCTTTCCTAATAAAATGAGGAAATT
GCATCGCATTGTCTGAGTAGGTGTCATTCTATTCTGGGGGGTGGGGTGGGGCAGGACA
GCAAGGGGGAGGATTGGGAAGACAATAGCAGGCATGCTGGGGA(5)GGATCTGCGATCG
CTCCGGTGCCCGTCAGTGGGCAGAGCGCACATCGCCCACAGTCCCCGAGAAGTTGGGG
GGAGGGGTCGGCAATTGAACGGGTGCCTAGAGAAGGTGGCGCGGGGTAAACTGGGAAA
GTGATGTCGTGTACTGGCTCCGCCTTTTTCCCGAGGGTGGGGGAGAACCGTATATAAG
TGCAGTAGTCGCCGTGAACGTTCTTTTTCGCAACGGGTTTGCCGCCAGAACACAGCTG
AAGCTTCGAGGGGCTCGCATCTCTCCTTCACGCGCCCGCCGCCCTACCTGAGGCCGCC
ATCCACGCCGGTTGAGTCGCGTTCTGCCGCCTCCCGCCTGTGGTGCCTCCTGAACTGC
GTCCGCCGTCTAGGTAAGTTTAAAGCTCAGGTCGAGACCGGGCCTTTGTCCGGCGCTC
CCTTGGAGCCTACCTAGACTCAGCCGGCTCTCCACGCTTTGCCTGACCCTGCTTGCTC
AACTCTACGTCTTTGTTTCGTTTTCTGTTCTGCGCCGTTACAGATCCAAGCTGTGACC
GGCGCCTAC(6)ATGACCGAGTACAAGCCCACGGTGCGCCTCGCCACCCGCGACGACGT
CCCCAGGGCCGTACGCACCCTCGCCGCCGCGTTCGCCGACTACCCCGCCACGCGCCAC
ACCGTCGATCCAGACCGCCACATCGAGCGGGTCACCGAGCTGCAAGAACTCTTCCTCA
CGCGCGTCGGGCTCGACATCGGCAAGGTGTGGGTCGCGGACGACGGCGCCGCGGTGGC

GGTCTGGACCACGCCGGAGAGCGTCGAAGCGGGGGCGGTGTTCGCCGAGATCGGCCCG
CGCATGGCCGAGTTGAGCGGTTCCCGGCTGGCCGCGCAGCAACAGATGGAAGGTCTCC
TGGCGCCGCACCGGCCCAAGGAGCCCGCGTGGTTCCTGGCCACCGTCGGCGTCTCGCC
CGACCACCAGGGCAAGGGTCTGGGCAGCGCCGTCGTGCTCCCCGGAGTGGAGGCGGCC
GAGCGCGCCGGGGTGCCCGCCTTCCTGGAGACCTCCGCGCCCCGCAACCTCCCCTTCT
ACGAGCGGCTCGGCTTCACCGTCACCGCCGACGTCGAGGTGCCCGAAGGACCGCGCAC
CTGGTGCATGACCCGCAAGCCCGGTGCCTGACCATCCGTAAGATGCTTTTCTGTGACT
GGTGAGTACTCAACCAAGTCATTCTGAGAATAGTGTATGCGGCGACCGAGTTGCTCTT
GCCCGGCGTCAATACGGGATAATACCGCGCCACATAGCAGAACTTTAAAAGTGCTCAT
CATTGGAAAACGTTCTTCGGGGCGAAAACTCTCAAGGATCTTACCGCTGTTGAGATCC
AGTTCGATGTAACCCACTCGTGCACCCAACTGATCTTCAGCATCTTTTACTTTCACCA
GCGTTTCTGGGTGAGCAAAAACAGGAAGGCAAAATGCCGCAAAAAAGGGAATAAGGGC
GACACGGAAATGTTGAATACTCATACTCTTCCTTTTTCAATATTATTGAAGCATTTAT
CAGGGTTATTGTCTCATGAGCGGATACATATTTGAATGTATTTAGAAAAATAAACAAA
TAGGGGTTCCGCGAACTTGTTTATTGCAGCTTATAATGGTTACAAATAAAGCAATAGC
ATCACAAATTTCACAAATAAAGCATTTTTTTCACTGCATTCTAGTTGTGGTTTGTCCA
AACTCATCAATGTATCTTATCATGTCTGG(7)cagctagccaacaagctcgtcatcgct
ttgcagaagagcagagaggatatgctcatcgtctaaagaactacccattTCTCCTGTT
CCGACCCTGCCGCTTACCGGATACCTGTCCGCCTTTCTCCCTTCGGGAAGCGTGGCGC
TTTCTCATAGCTCACGCTGTAGGTATCTCAGTTCGGTGTAGGTCGTTCGCTCCAAGCT
GGGCTGTGTGCACGAACCCCCCGTTCAGCCCGACCGCTGCGCCTTATCCGGTAACTAT
CGTCTTGAGTCCAACCCGGTAAGACACGACTTATCGCCACTGGCAGCAGCCACTGGTA
ACAGGATTAGCAGAGCGAGGTATGTAGGCGGTGCTACAGAGTTCTTGAAGTGGTGGCC
TAACTACGGCTACACTAGAAGGACAGTATTTGGTATCTGCGCTCTGCTGAAGCCAGTT
ACCTTCGGAAAAAGAGTTGGTAGCTCTTGATCCGGCAAACAAACCACCGCTGGTAGCG

GTGGTTTTTTTGTTTGCAAGCAGCAGATTACGCGCAGAAAAAAAGGATCTCAAGAAGA
TCCTTTGATCTTTTCTACGGGGTCTGACGCTCAGTGGAACGAAAACTCACGTTAAGGG
ATTTTGGTCATGATTAACCCTAGAAAGATAATCATATTGTGACGTACGTTAAAGATAA
TCATGCGTAAAATTGACGCATGTGTTTTATCGGTCTGTATATCGAGGTTTATTTATTA
ATTTGAATAGATATTAAGTTTTATTATATTTACACTTACATACTAATAATAAATTCAA
CAAACAATTTATTTATGTTTATTTATTTATTAAAAAAAAACAAAAACTCAAAATTTCT
TCTATAAAGTAACAAA(8)acttttaaacattctctcttttacaaaaataaacttattt
tgtactttaaaaacagtcatgttgtattataaaataagtaattagcttaacctataca
taatagaaacaaattatacttattagtcagtcagaaacaaCTTTGGCACATATCAATA
TTATGCTCTCGTTAATTAA (SEQ ID NO: 150)

- (1) pegRNA; (2) U6 Promoter; (3) CMV Promoter; (4) PE4; (5) EFlalpha Promoter; (6) Puromycin resistance; (7) ITR; (8) ITR

### Entire sequence of pAllinl-PE2-nuclease and each element included

### - Entire sequence

CGATGTACGGGCCAGATATACGCGCGTATATCTGGCCCGTACATCGCGAATC
TAGA(1)AAAAANNNNNNNNNNNNNNNNNNNNNNNNNNNNNGCACCGACTCGGTGCCAC
TTTTTCAAGTTGATAACGGACTAGCCTTATTTTAACTTGCTATTTCTAGCTCTAAAAC
NNNNNNNNNNNNNNNNNNNCGGTGTTTC(2)GTCCTTTCCACAAGATATATAAAGCCAA
GAAATCGAAATACTTTCAAGTTACGGTAAGCATATGATAGTCCATTTTAAAACATAAT
TTTAAAACTGCAAACTACCCAAGAAATTATTACTTTCTACGTCACGTATTTTGTACTA
ATATCTTTGTGTTTACAGTCAAATTAATTCCAATTATCTCTCTAACAGCCTTGTATCG
TATATGCAAATATGAAGGAATCATGGGAAATAGGCCCTCCGCGTT (3)GACATTGATT

ATTGACTAGTTATTAATAGTAATCAATTACGGGGTCATTAGTTCATAGCCCATATATG
GAGTTCCGCGTTACATAACTTACGGTAAATGGCCCGCCTGGCTGACCGCCCAACGACC
CCCGCCCATTGACGTCAATAATGACGTATGTTCCCATAGTAACGCCAATAGGGACTTT
CCATTGACGTCAATGGGTGGAGTATTTACGGTAAACTGCCCACTTGGCAGTACATCAA
GTGTATCATATGCCAAGTACGCCCCCTATTGACGTCAATGACGGTAAATGGCCCGCCT
GGCATTATGCCCAGTACATGACCTTATGGGACTTTCCTACTTGGCAGTACATCTACGT
ATTAGTCATCGCTATTACCATGGTGATGCGGTTTTGGCAGTACATCAATGGGCGTGGA
TAGCGGTTTGACTCACGGGGATTTCCAAGTCTCCACCCCATTGACGTCAATGGGAGTT
TGTTTTGGCACCAAAATCAACGGGACTTTCCAAAATGTCGTAACAACTCCGCCCCATT
GACGCAAATGGGCGGTAGGCGTGTACGGTGGGAGGTCTATATAAGCAGAGCTCTCTGG
CTAACTAGAGAACCCACTGCTTACTGGCTTATCGAAATTTCCCTATCAGTGATAGAGA
TTGACATCCCTATCAGTGATAGAGATACTGAGCACATCAGCAGGACGCACTGACCAGG
GAGACCCAAGCTTGCCACC(4)GCCCTACTAAAAGCCCTCTTTCTGCCCAAAAGACTTA
GCATAATCCATTGTCCAGGACATCAAAAGGGACACAGCGCCGAGGCTAGAGGCAACCG
GATGGCTGACCAAGCGGCCCGAAAGGCAGCCATCACAGAGACTCCAGACACCTCTACC
CTCCTCATAGAAAATTCATCACCCTCTGGCGGCTCAAAAAGAACCGCCGACGGCAGCG
AATTCGAGCCCAAGAAGAAGAGGAAAGTCTAACCGGTCATCATCACCATCACCATTGA
GTTTTACCCCTACGACGTGCCCGACTACGCCTAATAACTCGAGCATGCATCTAGAGGG
CCCTATTCTATAGTGTCACCTAAATGCTAGAGCTCGCTGATCAGCCTCGACTGTGCCT
TCTAGTTGCCAGCCATCTGTTGTTTGCCCCTCCCCCGTGCCTTCCTTGACCCTGGAAG
GTGCCACTCCCACTGTCCTTTCCTAATAAAATGAGGAAATTGCATCGCATTGTCTGAG
TAGGTGTCATTCTATTCTGGGGGGTGGGGTGGGGCAGGACAGCAAGGGGGAGGATTGG
GAAGACAATAGCAGGCATGCTGGGGA(5)GGATCTGCGATCGCTCCGGTGCCCGTCAGT
GGGCAGAGCGCACATCGCCCACAGTCCCCGAGAAGTTGGGGGGAGGGGTCGGCAATTG
AACGGGTGCCTAGAGAAGGTGGCGCGGGGTAAACTGGGAAAGTGATGTCGTGTACTGG

CTCCGCCTTTTTCCCGAGGGTGGGGGAGAACCGTATATAAGTGCAGTAGTCGCCGTGA
ACGTTCTTTTTCGCAACGGGTTTGCCGCCAGAACACAGCTGAAGCTTCGAGGGGCTCG
CATCTCTCCTTCACGCGCCCGCCGCCCTACCTGAGGCCGCCATCCACGCCGGTTGAGT
CGCGTTCTGCCGCCTCCCGCCTGTGGTGCCTCCTGAACTGCGTCCGCCGTCTAGGTAA
GTTTAAAGCTCAGGTCGAGACCGGGCCTTTGTCCGGCGCTCCCTTGGAGCCTACCTAG
ACTCAGCCGGCTCTCCACGCTTTGCCTGACCCTGCTTGCTCAACTCTACGTCTTTGTT
TCGTTTTCTGTTCTGCGCCGTTACAGATCCAAGCTGTGACCGGCGCCTAC(6)ATGACC
GAGTACAAGCCCACGGTGCGCCTCGCCACCCGCGACGACGTCCCCAGGGCCGTACGCA
CCCTCGCCGCCGCGTTCGCCGACTACCCCGCCACGCGCCACACCGTCGATCCAGACCG
CCACATCGAGCGGGTCACCGAGCTGCAAGAACTCTTCCTCACGCGCGTCGGGCTCGAC
ATCGGCAAGGTGTGGGTCGCGGACGACGGCGCCGCGGTGGCGGTCTGGACCACGCCGG
AGAGCGTCGAAGCGGGGGCGGTGTTCGCCGAGATCGGCCCGCGCATGGCCGAGTTGAG
CGGTTCCCGGCTGGCCGCGCAGCAACAGATGGAAGGTCTCCTGGCGCCGCACCGGCCC
AAGGAGCCCGCGTGGTTCCTGGCCACCGTCGGCGTCTCGCCCGACCACCAGGGCAAGG
GTCTGGGCAGCGCCGTCGTGCTCCCCGGAGTGGAGGCGGCCGAGCGCGCCGGGGTGCC
CGCCTTCCTGGAGACCTCCGCGCCCCGCAACCTCCCCTTCTACGAGCGGCTCGGCTTC
ACCGTCACCGCCGACGTCGAGGTGCCCGAAGGACCGCGCACCTGGTGCATGACCCGCA
AGCCCGGTGCCTGACCATCCGTAAGATGCTTTTCTGTGACTGGTGAGTACTCAACCAA
GTCATTCTGAGAATAGTGTATGCGGCGACCGAGTTGCTCTTGCCCGGCGTCAATACGG
GATAATACCGCGCCACATAGCAGAACTTTAAAAGTGCTCATCATTGGAAAACGTTCTT
CGGGGCGAAAACTCTCAAGGATCTTACCGCTGTTGAGATCCAGTTCGATGTAACCCAC
TCGTGCACCCAACTGATCTTCAGCATCTTTTACTTTCACCAGCGTTTCTGGGTGAGCA
AAAACAGGAAGGCAAAATGCCGCAAAAAAGGGAATAAGGGCGACACGGAAATGTTGAA
TACTCATACTCTTCCTTTTTCAATATTATTGAAGCATTTATCAGGGTTATTGTCTCAT
GAGCGGATACATATTTGAATGTATTTAGAAAAATAAACAAATAGGGGTTCCGCGAACT

TGTTTATTGCAGCTTATAATGGTTACAAATAAAGCAATAGCATCACAAAAATTTCACAAA
TAAAGCATTTTTTTCACTGCATTCTAGTTGTGGTTTGTCCAAACTCATCAATGTATCT
TATCATGTCTGGC(7)cagctagccaacaagctcgtcatcgctttgcagaagagcagag
aggatatgctcatcgtctaaagaactacccattGAAGCTCCCTCGTGCGCTCTCCTGT
TCCGACCCTGCCGCTTACCGGATACCTGTCCGCCTTTCTCCCTTCGGGAAGCGTGGCG
CTTTCTCATAGCTCACGCTGTAGGTATCTCAGTTCGGTGTAGGTCGTTCGCTCCAAGC
TGGGCTGTGTGCACGAACCCCCCGTTCAGCCCGACCGCTGCGCCTTATCCGGTAACTA
TCGTCTTGAGTCCAACCCGGTAAGACACGACTTATCGCCACTGGCAGCAGCCACTGGT
AACAGGATTAGCAGAGCGAGGTATGTAGGCGGTGCTACAGAGTTCTTGAAGTGGTGGC
CTAACTACGGCTACACTAGAAGGACAGTATTTGGTATCTGCGCTCTGCTGAAGCCAGT
TACCTTCGGAAAAAGAGTTGGTAGCTCTTGATCCGGCAAACAAACCACCGCTGGTAGC
GGTGGTTTTTTTGTTTGCAAGCAGCAGATTACGCGCAGAAAAAAAGGATCTCAAGAAG
ATCCTTTGATCTTTTCTACGGGGTCTGACGCTCAGTGGAACGAAAACTCACGTTAAGG
GATTTTGGTCATGATTAACCCTAGAAAGATAATCATATTGTGACGTACGTTAAAGATA
ATCATGCGTAAAATTGACGCATGTGTTTTATCGGTCTGTATATCGAGGTTTATTTATT
AATTTGAATAGATATTAAGTTTTATTATATTTACACTTACATACTAATAATAAATTCA
ACAAACAATTTATTTATGTTTATTTATTTATTAAAAAAAAACAAAAACTCAAAATTTC
TTCTATAAAGTAACAAA(8)acttttaaacattctctcttttacaaaaataaacttatt
ttgtactttaaaaacagtcatgttgtattataaaataagtaattagcttaacctatac
ataatagaaacaaattatacttattagtcagtcagaaacaaCTTTGGCACATATCAAT
ATTATGCTCTCGTTAATTAA (SEQ ID NO: 151)

- (1) pegRNA; (2) U6 Promoter; (3) CMV Promoter; (4) PE2-nuclease; (5) EFlalpha Promoter; (6) Puromycin resistance; (7) ITR; (8) ITR

### Entire sequence of pAllinl-PEmax-nuclease and each element included

### - Entire sequence

CGATGTACGGGCCAGATATACGCGCAATGTCAACGCGTATATCT(1)AAAAAA
TTCTAGTTGGTTTAACGCGTAACTAGATAGAACCGCGNNNNNNNNNNNNNNNNNN
NNNNNNNNNGCACCGACTCGGTGCCACTTTTTCAAGTTGATAACGGACTAGCCTTATT
TTAACTTGCTATTTCTAGCTCTAAAACNNNNNNNNNNNNNNNNNNNCGGTGTTTC(2)G
TCCTTTCCACAAGATATATAAAGCCAAGAAATCGAAATACTTTCAAGTTACGGTAAGC
ATATGATAGTCCATTTTAAAACATAATTTTAAAACTGCAAACTACCCAAGAAATTATT
ACTTTCTACGTCACGTATTTTGTACTAATATCTTTGTGTTTACAGTCAAATTAATTCC
AATTATCTCTCTAACAGCCTTGTATCGTATATGCAAATATGAAGGAATCATGGGAAAT
AGGCCCTCCGCGTT(3)GACATTGATTATTGACTAGTTATTAATAGTAATCAATTACGG
GGTCATTAGTTCATAGCCCATATATGGAGTTCCGCGTTACATAACTTACGGTAAATGG
CCCGCCTGGCTGACCGCCCAACGACCCCCGCCCATTGACGTCAATAATGACGTATGTT
CCCATAGTAACGCCAATAGGGACTTTCCATTGACGTCAATGGGTGGAGTATTTACGGT
AAACTGCCCACTTGGCAGTACATCAAGTGTATCATATGCCAAGTACGCCCCCTATTGA
CGTCAATGACGGTAAATGGCCCGCCTGGCATTATGCCCAGTACATGACCTTATGGGAC
TTTCCTACTTGGCAGTACATCTACGTATTAGTCATCGCTATTACCATGGTGATGCGGT
TTTGGCAGTACATCAATGGGCGTGGATAGCGGTTTGACTCACGGGGATTTCCAAGTCT
CCACCCCATTGACGTCAATGGGAGTTTGTTTTGGCACCAAAATCAACGGGACTTTCCA
AAATGTCGTAACAACTCCGCCCCATTGACGCAAATGGGCGGTAGGCGTGTACGGTGGG
AGGTCTATATAAGCAGAGCTGGTTTAGTGAACCGTCAGATCCGCTAGAGATCCGCGGC
CGCTAATACGACTCACTATAGGGAGAGCCGCCACC(4)GCCCTGTTTCTGCCCAAGCGG
CTGAGCATCATCCACTGTCCTGGACACCAGAAGGGACACTCCGCCGAGGCAAGGGGCA
ATCGGATGGCCGACCAGGCCGCCAGAAAGGCTGCTATTACTGAAACTCCCGACACTTC

CACTCTGCTGATTGAAAACTCCTCCCCTTCTGGCGGCTCAAAAAGAACCGCCGACGGC
AGCGAATTCGAGTCTCCCAAGAAGAAGAGGAAAGTCGGCTCTGGCCCTGCCGCTAAGA
GAGTGAAGCTGGACTAACCGGTCATCATCACCATCACCATTGAGTTTTACCCCTACGA
CGTGCCCGACTACGCCTAATAACTCGAGCATGCATCTAGAGGGCCCTATTCTATAGTG
TCACCTAAATGCTAGAGCTCGCTGATCAGCCTCGACTGTGCCTTCTAGTTGCCAGCCA
TCTGTTGTTTGCCCCTCCCCCGTGCCTTCCTTGACCCTGGAAGGTGCCACTCCCACTG
TCCTTTCCTAATAAAATGAGGAAATTGCATCGCATTGTCTGAGTAGGTGTCATTCTAT
TCTGGGGGGTGGGGTGGGGCAGGACAGCAAGGGGGAGGATTGGGAAGACAATAGCAGG
CATGCTGGGGA(5)GGATCTGCGATCGCTCCGGTGCCCGTCAGTGGGCAGAGCGCACAT
CGCCCACAGTCCCCGAGAAGTTGGGGGGAGGGGTCGGCAATTGAACGGGTGCCTAGAG
AAGGTGGCGCGGGGTAAACTGGGAAAGTGATGTCGTGTACTGGCTCCGCCTTTTTCCC
GAGGGTGGGGGAGAACCGTATATAAGTGCAGTAGTCGCCGTGAACGTTCTTTTTCGCA
ACGGGTTTGCCGCCAGAACACAGCTGAAGCTTCGAGGGGCTCGCATCTCTCCTTCACG
CGCCCGCCGCCCTACCTGAGGCCGCCATCCACGCCGGTTGAGTCGCGTTCTGCCGCCT
CCCGCCTGTGGTGCCTCCTGAACTGCGTCCGCCGTCTAGGTAAGTTTAAAGCTCAGGT
CGAGACCGGGCCTTTGTCCGGCGCTCCCTTGGAGCCTACCTAGACTCAGCCGGCTCTC
CACGCTTTGCCTGACCCTGCTTGCTCAACTCTACGTCTTTGTTTCGTTTTCTGTTCTG
CGCCGTTACAGATCCAAGCTGTGACCGGCGCCTAC(6)ATGACCGAGTACAAGCCCACG
GTGCGCCTCGCCACCCGCGACGACGTCCCCAGGGCCGTACGCACCCTCGCCGCCGCGT
TCGCCGACTACCCCGCCACGCGCCACACCGTCGATCCAGACCGCCACATCGAGCGGGT
CACCGAGCTGCAAGAACTCTTCCTCACGCGCGTCGGGCTCGACATCGGCAAGGTGTGG
GTCGCGGACGACGGCGCCGCGGTGGCGGTCTGGACCACGCCGGAGAGCGTCGAAGCGG
GGGCGGTGTTCGCCGAGATCGGCCCGCGCATGGCCGAGTTGAGCGGTTCCCGGCTGGC
CGCGCAGCAACAGATGGAAGGTCTCCTGGCGCCGCACCGGCCCAAGGAGCCCGCGTGG
TTCCTGGCCACCGTCGGCGTCTCGCCCGACCACCAGGGCAAGGGTCTGGGCAGCGCCG

TCGTGCTCCCCGGAGTGGAGGCGGCCGAGCGCCGGGGTGCCCGCCTTCCTGGAGAC
CTCCGCGCCCCGCAACCTCCCCTTCTACGAGCGGCTCGGCTTCACCGTCACCGCCGAC
GTCGAGGTGCCCGAAGGACCGCGCACCTGGTGCATGACCCGCAAGCCCGGTGCCTGAC
CATCCGTAAGATGCTTTTCTGTGACTGGTGAGTACTCAACCAAGTCATTCTGAGAATA
GTGTATGCGGCGACCGAGTTGCTCTTGCCCGGCGTCAATACGGGATAATACCGCGCCA
CATAGCAGAACTTTAAAAGTGCTCATCATTGGAAAACGTTCTTCGGGGCGAAAACTCT
CAAGGATCTTACCGCTGTTGAGATCCAGTTCGATGTAACCCACTCGTGCACCCAACTG
ATCTTCAGCATCTTTTACTTTCACCAGCGTTTCTGGGTGAGCAAAAACAGGAAGGCAA
AATGCCGCAAAAAAGGGAATAAGGGCGACACGGAAATGTTGAATACTCATACTCTTCC
TTTTTCAATATTATTGAAGCATTTATCAGGGTTATTGTCTCATGAGCGGATACATATT
TGAATGTATTTAGAAAAATAAACAAATAGGGGTTCCGCGAACTTGTTTATTGCAGCTT
ATAATGGTTACAAATAAAGCAATAGCATCACAAATTTCACAAATAAAGCATTTTTTTC
ACTGCATTCTAGTTGTGGTTTGTCCAAACTCATCAATGTATCTTATCATGTCTGGC(7)
cagctagccaacaagctcgtcatcgctttgcagaagagcagagaggatatgctcatcg
tctaaagaactacccattGAAGCTCCCTCGTGCGCTCTCCTGTTCCGACCCTGCCGCT
TACCGGATACCTGTCCGCCTTTCTCCCTTCGGGAAGCGTGGCGCTTTCTCATAGCTCA
CGCTGTAGGTATCTCAGTTCGGTGTAGGTCGTTCGCTCCAAGCTGGGCTGTGTGCACG
AACCCCCCGTTCAGCCCGACCGCTGCGCCTTATCCGGTAACTATCGTCTTGAGTCCAA
CCCGGTAAGACACGACTTATCGCCACTGGCAGCAGCCACTGGTAACAGGATTAGCAGA
GCGAGGTATGTAGGCGGTGCTACAGAGTTCTTGAAGTGGTGGCCTAACTACGGCTACA
CTAGAAGGACAGTATTTGGTATCTGCGCTCTGCTGAAGCCAGTTACCTTCGGAAAAAG
AGTTGGTAGCTCTTGATCCGGCAAACAAACCACCGCTGGTAGCGGTGGTTTTTTTGTT
TGCAAGCAGCAGATTACGCGCAGAAAAAAAGGATCTCAAGAAGATCCTTTGATCTTTT
CTACGGGGTCTGACGCTCAGTGGAACGAAAACTCACGTTAAGGGATTTTGGTCATGAT
TAACCCTAGAAAGATAATCATATTGTGACGTACGTTAAAGATAATCATGCGTAAAATT

GACGCATGTGTTTTATCGGTCTGTATATCGAGGTTTATTTATTAATTTGAATAGATAT
TAAGTTTTATTATATTTACACTTACATACTAATAATAAATTCAACAAACAATTTATTT
ATGTTTATTTATTTATTAAAAAAAAACAAAAACTCAAAATTTCTTCTATAAAGTAACA
AA(8)acttttaaacattctctcttttacaaaaataaacttattttgtactttaaaaac
agtcatgttgtattataaaataagtaattagcttaacctatacataatagaaacaaat
tatacttattagtcagtcagaaacaaCTTTGGCACATATCAATATTATGCTCTCGTTA
ATTAA (SEQ ID NO: 152)

- (1) epegRNA; (2) U6 Promoter; (3) CMV Promoter; (4) PEmax-nuclease; (5) EFlalpha Promoter; (6) Puromycin resistance; (7) ITR; (8) ITR

### Reference

Hereinbelow, some of the references incorporated herein by reference are to be disclosed. The documents referenced herein may or may not have been mentioned in the relevant paragraphs.

1. Tsai, S.Q. et al. GUIDE-seq enables genome-wide profiling of off-target cleavage by CRISPR-Cas nucleases. Nat Biotechnol 33, 187-197 (2015).
2. Liang, S.Q. et al. Genome-wide detection of CRISPR editing in vivo using GUIDE-tag. Nat Commun 13, 437 (2022).
3. Yan, W.X. et al. BLISS is a versatile and quantitative method for genome-wide profiling of DNA double-strand breaks. Nat Commun 8, 15058 (2017).
4. Crosetto, N. et al. Nucleotide-resolution DNA double-strand break mapping by next-generation sequencing. Nat Methods 10, 361-365 (2013).
5. Wienert, B. et al. Unbiased detection of CRISPR off-targets in vivo using DISCOVER-Seq. Science 364, 286-289 (2019).
6. Wang, X. et al. Unbiased detection of off-target cleavage by CRISPR-Cas9 and TALENs using integrase-defective lentiviral vectors. Nat Biotechnol 33, 175-178 (2015) .
7. Chiarle, R. et al. Genome-wide translocation sequencing reveals mechanisms of chromosome breaks and rearrangements in B cells. Cell 147, 107-119 (2011).
8. Kim, H.S. et al. CReVIS-Seq: A highly accurate and multiplexable method for genome-wide mapping of lentiviral integration sites. Mol Ther Methods Clin Dev 20, 792-800 (2021) .
9. Breton, C., Clark, P.M., Wang, L., Greig, J.A. & Wilson, J.M. ITR-Seq, a next-generation sequencing assay, identifies genome-wide DNA editing sites in vivo following adeno-associated viral vector-mediated genome editing. BMC Genomics 21, 239 (2020).
10. Huang, H. et al. Tag-seq: a convenient and scalable method for genome-wide specificity assessment of CRISPR/Cas nucleases. Commun Biol 4, 830 (2021).
11. Dobbs, F.M. et al. Precision digital mapping of endogenous and induced genomic DNA breaks by INDUCE-seq. Nat Commun 13, 3989 (2022).
12. Kim, D. et al. Digenome-seq: genome-wide profiling of CRISPR-Cas9 off-target effects in human cells. Nat Methods 12, 237-243, 231 p following 243 (2015).
13. Kim, D. & Kim, J.S. DIG-seq: a genome-wide CRISPR off-target profiling method using chromatin DNA. Genome Res 28, 1894-1900 (2018).
14. Lazzarotto, C.R. et al. CHANGE-seq reveals genetic and epigenetic effects on CRISPR-Cas9 genome-wide activity. Nat Biotechnol 38, 1317-1327 (2020).
15. Tsai, S.Q. et al. CIRCLE-seq: a highly sensitive in vitro screen for genome-wide CRISPR-Cas9 nuclease off-targets. Nat Methods 14, 607-614 (2017).
16. Cameron, P. et al. Mapping the genomic landscape of CRISPR-Cas9 cleavage. Nat Methods 14, 600-606 (2017).
17. Bae, S., Park, J. & Kim, J.S. Cas-OFFinder: a fast and versatile algorithm that searches for potential off-target sites of Cas9 RNA-guided endonucleases. Bioinformatics 30, 1473-1475 (2014).
18. Concordet, J.P. & Haeussler, M. CRISPOR: intuitive guide selection for CRISPR/Cas9 genome editing experiments and screens. Nucleic Acids Res 46, W242-W245 (2018) .
19. Montague, T.G., Cruz, J.M., Gagnon, J.A., Church, G.M. & Valen, E. CHOPCHOP: a CRISPR/Cas9 and TALEN web tool for genome editing. Nucleic Acids Res 42, W401-407 (2014).
20. Gillmore, J.D. et al. CRISPR-Cas9 In Vivo Gene Editing for Transthyretin Amyloidosis. N Engl J Med 385, 493-502 (2021).
21. Maeder, M.L. et al. Development of a gene-editing approach to restore vision loss in Leber congenital amaurosis type 10. Nat Med 25, 229-233 (2019).
22. Frangoul, H. et al. CRISPR-Cas9 Gene Editing for Sickle Cell Disease and beta-Thalassemia. N Engl J Med 384, 252-260 (2020) .
23. Komor, A.C., Kim, Y.B., Packer, M.S., Zuris, J.A. & Liu, D.R. Programmable editing of a target base in genomic DNA without double-stranded DNA cleavage. Nature 533, 420-424 (2016).
24. Gaudelli, N.M. et al. Programmable base editing of A*T to G*C in genomic DNA without DNA cleavage. Nature 551, 464-471 (2017).
25. Petri, K. et al. Global-scale CRISPR gene editor specificity profiling by ONE-seq identifies population-specific, variant off-target effects. bioRxiv, 2021.2004.2005.438458 (2021).
26. Lei, Z. et al. Detect-seq reveals out-of-protospacer editing and target-strand editing by cytosine base editors. Nat Methods 18, 643-651 (2021).
27. Kim, D. et al. Genome-wide target specificities of CRISPR RNA-guided programmable deaminases. Nat Biotechnol 35, 475-480 (2017).
28. Kim, D., Kim, D.E., Lee, G., Cho, S.I. & Kim, J.S. Genome-wide target specificity of CRISPR RNA-guided adenine base editors. Nat Biotechnol 37, 430-435 (2019).
29. Liang, P. et al. Genome-wide profiling of adenine base editor specificity by EndoV-seq. Nat Commun 10, 67 (2019) .
30. Anzalone, A.V. et al. Search-and-replace genome editing without double-strand breaks or donor DNA. Nature 576, 149-157 (2019).
31. Kim, D.Y., Moon, S.B., Ko, J.H., Kim, Y.S. & Kim, D. Unbiased investigation of specificities of prime editing systems in human cells. Nucleic Acids Res 48, 10576-10589 (2020) .
32. Jin, S. et al. Genome-wide specificity of prime editors in plants. Nat Biotechnol 39, 1292-1299 (2021).
33. Kim, D., Kang, B.C. & Kim, J.S. Identifying genome-wide off-target sites of CRISPR RNA-guided nucleases and deaminases with Digenome-seq. Nat Protoc 16, 1170-1192 (2021) .
34. Li, X. et al. piggyBac transposase tools for genome engineering. Proc Natl Acad Sci U S A 110, E2279-2287 (2013).
35. Malinin, N.L. et al. Defining genome-wide CRISPR-Cas genome-editing nuclease activity with GUIDE-seq. Nat Protoc 16, 5592-5615 (2021).
36. Zheng, Z. et al. Anchored multiplex PCR for targeted next-generation sequencing. Nat Med 20, 1479-1484 (2014) .
37. Iafrate, A.J., Le, L.P. & Zheng, Z., Vol. US 9.487,828 B2 (The General Hospital Corporation, Boston, MA (US), US; 2016).
38. Altschul, S.F., Gish, W., Miller, W., Myers, E.W. & Lipman, D.J. Basic local alignment search tool. J Mol Biol 215, 403-410 (1990).
39. Zhang, Z., Schwartz, S., Wagner, L. & Miller, W. A greedy algorithm for aligning DNA sequences. J Comput Biol 7, 203-214 (2000).
40. Hwang, G.H. et al. PE-Designer and PE-Analyzer: web-based design and analysis tools for CRISPR prime editing. Nucleic Acids Res 49, W499-W504 (2021).
41. Chen, P.J. et al. Enhanced prime editing systems by manipulating cellular determinants of editing outcomes. Cell 184, 5635-5652 e5629 (2021).
42. Adikusuma, F. et al. Optimized nickase- and nuclease-based prime editing in human and mouse cells. Nucleic Acids Res 49, 10785-10795 (2021).
43. Nelson, J.W. et al. Engineered pegRNAs improve prime editing efficiency. Nat Biotechnol (2021).
44. Liu, P. et al. Improved prime editors enable pathogenic allele correction and cancer modelling in adult mice. Nat Commun 12, 2121 (2021).
45. Choi, J. et al. Precise genomic deletions using paired prime editing. Nat Biotechnol (2021).
46. Lin, Q. et al. High-efficiency prime editing with optimized, paired pegRNAs in plants. Nat Biotechnol 39, 923-927 (2021).
47. Song, M. et al. Generation of a more efficient prime editor 2 by addition of the Rad51 DNA-binding domain. Nat Commun 12, 5617 (2021).
48. Lorenz, R. et al. ViennaRNA Package 2.0. Algorithms Mol Biol 6, 26 (2011).
49. Jang, H. et al. Application of prime editing to the correction of mutations and phenotypes in adult mice with liver and eye diseases. Nat Biomed Eng (2021).
50. Kim, Y. et al. Adenine base editing and prime editing of chemically derived hepatic progenitors rescue genetic liver disease. Cell Stem Cell 28, 1614-1624 e1615 (2021) .
51. Schene, I.F. et al. Prime editing for functional repair in patient-derived disease models. Nat Commun 11, 5352 (2020) .
52. Petri, K. et al. CRISPR prime editing with ribonucleoprotein complexes in zebrafish and primary human cells. Nat Biotechnol 40, 189-193 (2021).

## Claims

1. A method for predicting off-target occurring in process of genome editing by prime editing system, comprising:
(a) obtaining a manipulated cell,
wherein the manipulated cell comprises a manipulated genome DNA, wherein the manipulated genome DNA comprises a tag sequence, and the manipulated genome DNA is generated through a following process which a prime editor protein and tpegRNA are involved, comprising:
(i) contacting the genome DNA with a prime editor protein and a tpegRNA (tagmentation pegRNA), wherein the prime editor comprises a Cas protein and a reverse transcriptase, and wherein the tpegRNA comprises a spacer and an extension region comprising a tag template,
(ii) the tag sequence is inserted into the genome DNA by reverse transcription process performed by the reverse transcriptase using the tag template of the tpegRNA as a template of reverse transcription;
(b) analyzing the manipulated genome DNA to obtain information on a tagmentation,
wherein the information on the tagmentation comprises information on a region of genome DNA of which the tag sequence is inserted.

2. The method of claim 1,
wherein the method for predicting off-target further comprises:
obtaining information of the off-target based on the information on the tagmentation, wherein the information of the off-target comprises information on whether an off-target candidate exists, and information on the region of the off-target candidate if the off-target candidate exists.

3. The method of claim 3,
wherein the method for predicting off-target further comprises:
verifying information on a on-target, and comparing the information on the on-target with the the information on the tagmentation.

4. The method of claim 1,
wherein the method for predicting off-target further comprises:
confirming information on whether a off-target candidate exists by confirming an on-target information and comparing the on-target information with the information on the tagmentation.

5. The method of claim 1,
wherein the tag sequence is inserted into a region in the genome DNA which is specified by the spacer of tpegRNA.

6. The method of claim 1,
wherein the region where the tag sequence is inserted is associated with (corresponds to) off-target candidate regions or on-target regions.

7. The method of claim 1,
wherein information on the region where the tag sequence is inserted comprises information on a chromosome where the tag sequence is located, and information on a region in the chromosome where the tag sequence is present.

8. The method of claim 2,
wherein the information on the region of the candidate of the off-target comprises information on a chromosome where the off-target candidate is located, and the region in the chromosome where the off-target candidate is present.

9. The method of claim 1,
wherein the information on the tagmentation further comprises:
insertion ratio of the tag sequence by a region where the tag sequence is inserted.

10. The method of claim 2,
wherein the information on the off-target further comprises:
off-target predicting score of the off-target candidate.

11. The method of claim 2,
wherein the information on the off-target further comprises:
a number of the off-target candidates predicted.

12. The method of claim 1,
wherein the manipulated cell is obtained by a method comprises:
contacting the prime editor protein or a nucleic acid encoding the prime editor protein and the tpegRNA or a nucleic acid encoding the tpegRNA with a cell.

13. The method of claim 1,
wherein the manipulated cell is obtained by a method comprises:
introducing the prime editor protein or a nucleic acid encoding the prime editor protein and the tpegRNA or a nucleic acid encoding the tpegRNA into a cell.

14. The method of claim 1,
wherein the method further comprises:
obtaining a DNA from the manipulated cell, wherein it is performed prior to (b).

15. The method of claim 1,
wherein the tpegRNA comprises:
spacer; gRNA core; and an extension region comprising a primer binding site, a tag template, and a reverse transcription template.

16. The method of claim 15,
wherein the reverse transcription template of the tpegRNA comprises an editing template and homology region.

17. The method of claim 15,
wherein the manipulated genome DNA comprises an editing.

18. The method of claim 1,
wherein the spacer, the gRNA core, and the extension region are located in the order of the spacer, the gRNA core, and the extension region in the 5' to 3' direction.

19. The method of claim 1,
wherein the tag template is located in between primer binding site and the reverse transcription template in the extension region.

20. The method of claim 1,
wherein the tpegRNA further comprises 3' engineering region comprising RNA protection motif.

21. The method of claim 1,
wherein the method further comprises:
verifying a predetermined prime editing system, comprising one or more of follows:
information on a predetermined cell, information on a predetermined pegRNA, information on a predetermined prime editor protein.

22. The method of claim 21,
wherein the predetermined cell is different from the cell used in the method for predicting off-target.

23. The method of claim 21,
wherein a sequence of the spacer of the tpegRNA is the same as a sequence of the spacer of the predetermined pegRNA, wherein a sequence of the primer binding site of the tpegRNA is the same as a sequence of the primer binding site of the predetermined pegRNA.

24. The method of claim 21,
wherein a sequence of the spacer of the tpegRNA is the same as a sequence of the spacer of the predetermined pegRNA, wherein a sequence of the primer binding site of the tpegRNA is the same as a sequence of the primer binding site of the predetermined pegRNA,
wherein a sequence of the reverse transcription template of the tpegRNA is the same as a sequence of the reverse transcription template of the predetermined pegRNA.

25. The method of claim 21,
wherein the prime editor protein used in the method for predicting off-target is the same or different to the predetermined prime editor protein.

26. The method of claim 1,
wherein the length of the tag template is 5nt to 60nt.

27. The method of claim 1,
wherein the length of the tag template is 10nt to 50nt.

28. The method of claim 1,
wherein the prime editor protein is a PE-nuclease comprising a Cas protein which have double-strand break activity.

29. The method of claim 1,
wherein the prime editor protein is a PEmax-nuclease.

30. The method of claim 1,
wherein the Cas protein which included in the prime editor protein is nickase.

31. The method of claim 1,
wherein the prime editor protein is a PE2 prime editor protein.

32. The method of claim 1,
wherein the manipulation of DNA genome is further involves any one or more of a dnMLH1, a gRNA, and an additional Cas protein, and an additional prime editor protein.

33. The method of claim 1,
wherein the (b) comprises:
analyzing the manipulated genome DNA tag-specifically.

34. The method of claim 1,
wherein the (b) comprises:
sequencing the manipulated genome DNA.

35. The method of claim 1,
wherein the (b) comprises:
generating a tag-specific library from the manipulated genome DNA; generating an amplified tag-specific library by amplifying tag-specific library; and sequencing the amplified tag-specific library.

36. A method for predicting off-target occurring in process of genome editing by prime editing system, comprising:
(a) preparing a population of cells comprising one or more manipulated cells,
wherein the manipulated cell comprises a manipulated genome DNA, wherein the manipulated genome DNA comprises a tag sequence, and the manipulated genome DNA is generated through a following process which a prime editor protein and tpegRNA are involved, comprising:
(i) contacting the genome DNA with a prime editor protein and a tpegRNA (tagmentation pegRNA), wherein the prime editor protein comprises a Cas protein and a reverse transcriptase, and , wherein the tpegRNA comprises a spacer and an extension region comprising a tag template,
(ii) the tag sequence is inserted into the genome DNA, wherein the insertion of the tag sequence is achieved through a reverse transcription process performed by the reverse transcriptase using the tag template of the tpegRNA as a template of reverse transcription;
(b) obtaining a tagmentation information by analyzing results obtained through a process comprising a sequencing the manipulated genome DNA of one or more manipulated cells,
wherein the tagmentation information comprises information on one or more sites where each tag sequence is inserted; and
(c) obtaining information on the off-target based on the tagmentation information,
wherein the information on the off-target comprises information on whether a off-target candidate exists, and information on the region of one or more off-target candidates.

37. A tagmentation pegRNA (tpegRNA) comprising:
a spacer; a gRNA core; and an extension region compring a tag template.

38. The tpegRNA of claim 37,
wherein the spacer, the gRNA core, and the extension region comprising the tag template are located in the order of the spacer, the gRNA core, and the extension region comprising the tag template in the 5' to 3' direction.

39. The tpegRNA of claim 37,
wherein the extension region comprises the tag template, a primer binding site, and a reverse transcription template.

40. The tpegRNA of claim 39,
wherein the tag template is located in between primer binding site and the reverse transcription template in the extension region.

41. The tpegRNA of claim 39,
wherein the reverse transcription template is located in between the tag template and the primer binding site.

42. The tpegRNA of claim 39,
wherein the primer binding site, the tag template and the reverse transcription template are located in the order of the reverse transcription template, the tag template, and the primer binding site in the 5' to 3' direction in the extension region.

43. The tpegRNA of claim 39,
wherein the reverse transcription template comprises an editing template and a homology region.

44. The tpegRNA of claim 37,
wherein the tag template has a length of 5nt to 60nt.

45. The tpegRNA of claim 37,
wherein the tag template has a length of 10nt to 50nt.

46. The tpegRNA of claim 37,
wherein the tpegRNA further comprises 3' engineering region comprising an RNA protection motif.

47. The tpegRNA of claim 46,
wherein the RNA protection motif has a length of 10nt to 60nt.

48. The tpegRNa of claim 47,
wherein the tpegRNA has a length of 100nt to 350nt.

49. A composition for predicting off-target which are capable of occuring in process of genome editing by prime editing system, comprising:
a tpegRNA of any one of the claims 37 to 48; and
a prime editor comprising a Cas protein and a reverse transcriptase.
